# EUROPEAN PATENT SPECIFICATION

(11) **EP 4 526 298 B1**
(45) Date of publication and mention of the grant of the patent: **28.10.2026**
(21) Application number: 23727300.8
(22) Date of filing: 16.05.2023
(51) Int. Cl.: C07D 401/14, C07D 487/04, A61K 31/4155, A61P 35/00

(54) **PYRAZOLE DERIVATIVES AS STING AGONISTS**
PYRAZOLDERIVATE ALS STING-AGONISTEN
DÉRIVÉS DE PYRAZOLE UTILISÉS EN TANT QU'AGONISTES DE STING

(30) Priority: 18.05.2022 WO PCT/CN2022/093600; 11.10.2022 WO PCT/CN2022/124648; 10.03.2023 WO PCT/CN2023/080862; 11.04.2023 WO PCT/CN2023/087630
(43) Date of publication of application: 26.03.2025
(73) Proprietor: F. Hoffmann-La Roche AG, 4070 Basel (CH)
(72) Inventor: LIU, Haixia, Shanghai 201203 (CN); LIU, Yafei, Shanghai 201203 (CN); WANG, Jianping, Shanghai 201203 (CN); WU, Yao, Shanghai 201203 (CN); ZHANG, Zhiwei, Shanghai 201203 (CN)
(74) Representative: Salud, Carlos E.
(86) International application number: PCT/EP2023/063042
(87) International publication number: WO 2023/222644

(56) References cited:
- WO-A1-2018/118781
- WO-A1-2021/059136
- WO-A1-2022/195462
- WO-A1-2022/248353
- WO-A2-2010/006713

## Description

### FIELD OF THE INVENTION

The present invention relates to organic compounds, in particular to STING agonists, useful for treatment of cancers in a mammal. In particular, the present invention relates to pyrazole derivatives that have STING agonism activity, as well as their manufacture, pharmaceutical compositions containing them and their potential use as medicaments.

### BACKGROUND OF THE INVENTION

Cancer immunotherapy (CIT), represented by immune checkpoint inhibitors (ICI), have rapidly emerged in the past few years. The key to an effective CIT-mediated antitumor responses depend on the priming and activation of T cells by antigen-presenting cells, followed by directed trafficking and infiltration of T cells into the tumor mass so that they recognize and kill cancer cells *(*Chen DS, et al. Immunity. 2013;39*).* As such, immune checkpoint blockade only shows durable responses in highly immunogenic "hot" tumors but has shown little efficacy in non-immunogenic "cold" tumors which are characterized by the lack of T-cell infiltration and impaired T-cell priming. Therefore, attempts have been focused on promoting the transformation of "cold tumors" into "hot tumors", with strategies to activate the innate immune system to re-engage non-immunogenic tumors by boosting anti-cancer adaptive immunity. Antigen presentation cells of the innate immune system, such as dendritic cells or macrophages, serve as a critical link between the innate and adaptive immune systems by phagocytosis to process the foreign antigens and present them on the cell surface to the T cells, thereby activating T cell response.

Stimulator of interferon genes (STING) is a four transmembrane endoplasmic reticulum adaptor that facilitates innate immune signaling. Activation of STING protein by its natural ligand, cyclic guanosine monophosphate adenosine monophosphate (cGAMP) is able to activate NF-kB, IRF3 transcription pathway to induce expression of type I interferon and pro-inflammatory cytokine/chemokines (Ishikawa.et al. Nature 2008*,* Quyang et al. Immunity 2012*,* Chen et al. Cell 2011*)*

STING activation has been demonstrated to enhance the immune response and restrain tumor growth, which is achieved by strengthen cancer antigen presentation, contribute to the priming and activation of T cells, facilitate the trafficking and infiltration of T cells into tumors and promote the recognition and killing of cancer cells by T cells. The first generation of intra-tumor delivered cyclic dinucleotides that have entered clinical development only demonstrated limited single agent activity, mainly due to the poor pharmacokinetic and physicochemical properties, *i.e.,* low bioavailability, instability, and difficulty in bypassing the plasma membrane (Leila et al. J. Clin. Med. 2020, Afsaneh et al. Cancers 2021).

### SUMMARY OF THE INVENTION

The invention is set out in the appended claims. Features that are not in the scope of the appended claims are described for reference only.

Objects of the present invention are novel compounds of formula (I) as defined in the claims, their manufacture, medicaments based on a compound in accordance with the invention and their production as well as the use of compounds of formula (I) as STING agonists for the treatment of a broad spectrum of cancers, with the potential to augment response rates to current immunotherapy, overcome acquired resistance. The compounds of formula (I) show superior STING agonism activity. In addition, the compounds of formula (I) also show good safety and good PK profiles, e.g., good solubility, microsome stability, permeability, and safety margin.

One aspect of the invention pertains to a compound of formula (I), 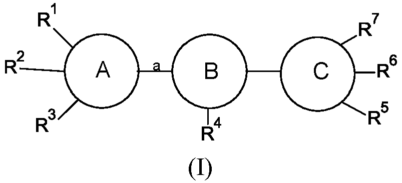 wherein
Ring A is a 5 to 12 membered monocyclic, bicyclic, or tricyclic heteroaryl ring or heterocyclyl ring containing one to four heteroatoms selected from N, O, and S,
   each of R¹, R² and R³ is independently absent or selected from C₁₋₆alkyl, haloC₁₋₆alkyl, hydroxylC₁₋₆alkyl, aminoC₁₋₆alkyl, -C(O)C₁₋₆alkyl, C₁₋₆alkylamino, (C₁₋₆alkyl)₂amino, C₁₋₆alkyl-S-, C₁₋₆alkylSO₂, C₁₋₆alkoxy, haloC₁₋₆alkoxy, C₁₋₆alkoxycarbonyl, carboxyl, hydroxyl, cyano, amino, halogen, acyl, carbamoyl, and a 5 to 8 membered monocyclic heterocyclyl containing one to three heteroatoms selected from N, O, and S, wherein said heterocyclyl can be further substituted by halogen, C₁₋₆alkyl, C₁₋₆alkoxy or amino;
Ring B is a 5 to 8 membered monocyclic or bicyclic heteroaryl ring or heterocyclyl ring containing one to four heteroatoms selected from N, O, and S,
   R⁴ is absent or selected from C₁₋₆alkyl, haloC₁₋₆alkyl, hydroxylC₁₋₆alkyl, aminoC₁₋₆alkyl, - C(O)C₁₋₆alkyl, C₁₋₆alkylamino, (C₁₋₆alkyl)₂amino, C₁₋₆alkyl-S-, C₁₋₆alkylSO₂, C₁₋₆alkoxy, haloC₁₋₆alkoxy, C₁₋₆alkoxycarbonyl, carboxyl, hydroxyl, cyano, amino, halogen, and carbamoyl;
Bond a is a bond connecting ring A and ring B;
Ring C is a 5 to 8 membered monocyclic or bicyclic heteroaryl ring or heterocyclyl ring containing one to four heteroatoms selected from N, O, and S,
   each of R⁵, R⁶ and R⁷ is independently absent or selected from C₁₋₆alkyl, haloC₁₋₆alkyl, hydroxylC₁₋₆alkyl, aminoC₁₋₆alkyl, -C(O)C₁₋₆alkyl, C₁₋₆alkylamino, (C₁₋₆alkyl)₂amino, C₁₋₆alkyl-S-, C₁₋₆alkylSO₂, C₁₋₆alkoxy, C₁₋₆alkoxyC₁₋₆alkyl, haloC₁₋₆alkoxy, C₁₋₆alkoxycarbonyl, haloC₂₋₆alkenyl, C₃₋₇cycloalkyl, C₃₋₇cycloalkylC₁₋₆alkyl, C₂₋₆alkynyl, C₁₋₆alkoxyC₂₋₆alkynyl, carboxyl, hydroxyl, cyano, amino, halogen, carbamoyl, cyanoC₁₋₆alkyl, imidazolylC₁₋₆alkyl, triazolylC₁₋₆alkyl, pyridinylC₁₋₆alkyl, thiazolylC₁₋₆alkyl, and phenylC₁₋₆alkyl, wherein the phenylC₁₋₆alkyl is optionally substituted by halogen, cyano, hydroxylC₁₋₆alkyl, morpholinoC₁₋₆alkyl, C₁₋₆alkoxy, C₁₋₆alkoxyC₁₋₆alkyl, and C₁₋₆alkoxycarbonyl, or
   two of R⁵, R⁶, and R⁷ together with the atoms to which they are bonded form a 5 to 6 membered ring having 0 to 2 heteroatoms selected from N, O, and S;
or a pharmaceutically acceptable salt thereof.

Another aspect of the invention pertains to a process for the preparation of a compound of formula (I), as well as a compound of formula (I) or a pharmaceutically acceptable salt thereof when manufactured according to the process.

Another aspect of the invention pertains to a pharmaceutical composition comprising a compound of formula (I) or a pharmaceutically acceptable salt thereof, and a pharmaceutically acceptable excipient.

The references to the methods of treatment by therapy in the subsequent paragraphs of this description are to be interpreted as references to compounds, pharmaceutical compositions and medicaments of the present invention for use in those methods.

Another aspect of the invention pertains to a compound of formula (I) or a pharmaceutically acceptable salt thereof for use as therapeutically active substance.

Another aspect of the invention pertains to a compound of formula (I) or a pharmaceutically acceptable salt thereof for use in the treatment of cancer.

Another aspect of the invention pertains to the use of a compound of formula (I) or a pharmaceutically acceptable salt thereof for the treatment of cancer.

Another aspect of the invention pertains to the use of a compound of formula (I) or a pharmaceutically acceptable salt thereof for the activation of STING.

Another aspect of the invention pertains to the use of a compound of formula (I) or a pharmaceutically acceptable salt thereof for the preparation of a medicament for the treatment of cancer.

Another aspect of the invention pertains to the use of a compound of formula (I) or a pharmaceutically acceptable salt thereof for the preparation of a medicament for the activation of STING.

Another aspect of the invention pertains to a method for the treatment of cancer, which method comprises administering an effective amount of a compound of formula (I) or a pharmaceutically acceptable salt thereof.

### DETAILED DESCRIPTION OF THE INVENTION

### DEFINITIONS

Unless otherwise defined, all technical and scientific terms used herein have the same meaning as commonly understood by one of ordinary skill in the art to which this invention belongs. Furthermore, the following definitions are set forth to illustrate and define the meaning and scope of the various terms used to describe the invention.

The nomenclature used in this application is based on IUPAC systematic nomenclature, unless indicated otherwise.

The term "compound(s) of this invention" and "compound(s) of the present invention" refers to compounds of formula (I), formula (Ia), formula (Ib), and stereoisomers, solvates or salts thereof (*e*.*g*., pharmaceutically acceptable salts).

The term "substituent" denotes an atom or a group of atoms replacing a hydrogen atom on the parent molecule.

As used herein, the term "C₁₋₆alkyl" alone or in combination signifies a saturated, linear- or branched chain alkyl group containing 1 to 6, particularly 2 to 6 or 1 to 4 carbon atoms, for example, methyl, ethyl, propyl, isopropyl, butyl, isobutyl, *tert*-butyl and the like. Particular "C₁₋₆alkyl" groups are methyl and ethyl.

The term "halogen" or "Halo" denotes fluoro, chloro, bromo, or iodo.

The term "haloC₁₋₆alkyl" denotes an alkyl group wherein at least one of the hydrogen atoms of the alkyl group is replaced by same or different halogen atoms, particularly fluoro atoms. Examples of haloC₁₋₆alkyl include monochloro-, difluoro-or trifluoro-methyl, -ethyl or -propyl, for example difluoromethyl.

The term "heteroaryl" denotes a monovalent aromatic heterocyclic mono- or bicyclic ring system of 5 to 12 ring atoms, comprising 1, 2, 3 or 4 heteroatoms selected from N, O and S, the remaining ring atoms being carbon. Examples of heteroaryl moieties include, but not limited to, pyrrolyl, furanyl, thienyl, imidazolyl, oxazolyl, thiazolyl, triazolyl, oxadiazolyl, thiadiazolyl, tetrazolyl, pyridinyl, pyrazinyl, pyrazolyl, pyridazinyl, pyrimidinyl, triazinyl, isoxazolyl, benzofuranyl, isothiazolyl, benzothienyl, indolyl, isoindolyl, isobenzofuranyl, benzimidazolyl, benzoxazolyl, benzoisoxazolyl, benzothiazolyl, benzoisothiazolyl, benzooxadiazolyl, benzothiadiazolyl, benzotriazolyl, purinyl, quinolinyl, isoquinolinyl, quinazolinyl or quinoxalinyl. Heteroaryl can be further substituted by halogen, C₁₋₆alkyl, haloC₁₋₆alkyl, cyano, C₃₋₇cycloalkyl, (C₁₋₆alkyl)₂amino, or C₁₋₆alkoxy.

The term "heterocyclyl" denotes a monovalent saturated or partly unsaturated mono- or bicyclic ring system of 3 to 9 ring atoms, comprising 1, 2, or 3 ring heteroatoms selected from N, O and S, the remaining ring atoms being carbon. In particular embodiments, heterocyclyl is a monovalent saturated monocyclic ring system of 4 to 7 ring atoms, comprising 1, 2, or 3 ring heteroatoms selected from N, O and S, the remaining ring atoms being carbon. Examples for monocyclic saturated heterocyclyl are aziridinyl, oxiranyl, azetidinyl, oxetanyl, pyrrolidinyl, tetrahydrofuranyl, tetrahydro-thienyl, pyrazolidinyl, imidazolidinyl, oxazolidinyl, isoxazolidinyl, thiazolidinyl, piperidinyl, tetrahydropyranyl, tetrahydrothiopyranyl, piperazinyl, morpholinyl, thiomorpholinyl, 1,1-dioxo-thiomorpholin-4-yl, azepanyl, diazepanyl, homopiperazinyl, oxazepanyl, oxopiperidinyl, oxopiperazinyl or oxopyrrolidinyl. Examples for bicyclic saturated heterocyclyl are azaspiro[3.3]heptanyl, 8-aza-bicyclo[3.2.1]octyl, quinuclidinyl, 8-oxa-3-aza-bicyclo[3.2.1]octyl, 9-aza-bicyclo[3.3.1]nonyl, 3-oxa-9-aza-bicyclo[3.3.1]nonyl, or 3-thia-9-aza-bicyclo[3.3.1]nonyl. Examples for partly unsaturated heterocyclyl are dihydrofuryl, imidazolinyl, dihydro-oxazolyl, tetrahydro-pyridinyl, or dihydropyranyl.

The term "hydroxyC₁₋₆alkyl" denotes a C₁₋₆alkyl group wherein at least one of the hydrogen atoms of the C₁₋₆alkyl group has been replaced by a hydroxy group.

The compounds according to the present invention may exist in the form of their pharmaceutically acceptable salts. The term "pharmaceutically acceptable salt" refers to conventional acid-addition salts or base-addition salts that retain the biological effectiveness and properties of the compounds of formula (I) and are formed from suitable non-toxic organic or inorganic acids or organic or inorganic bases. Acid-addition salts include for example those derived from inorganic acids such as hydrochloric acid, hydrobromic acid, hydroiodic acid, sulfuric acid, sulfamic acid, phosphoric acid and nitric acid, and those derived from organic acids such as *p*-toluenesulfonic acid, trifluoroacetic acid, formic acid, salicylic acid, methanesulfonic acid, oxalic acid, succinic acid, citric acid, malic acid, lactic acid, fumaric acid, and the like. Base-addition salts include those derived from ammonium, potassium, sodium and, quaternary ammonium hydroxides, such as for example, tetramethyl ammonium hydroxide. The chemical modification of a pharmaceutical compound into a salt is a technique well known to pharmaceutical chemists in order to obtain improved physical and chemical stability, hygroscopicity, flowability and solubility of compounds. It is for example described in Bastin R.J., et al., Organic Process Research & Development 2000, 4, 427-435. Particular are the sodium salts of the compounds of formula (I).

The term "therapeutically effective amount" denotes an amount of a compound or molecule of the present invention that, when administered to a subject, (i) treats or prevents the particular disease, condition or disorder, (ii) attenuates, ameliorates or eliminates one or more symptoms of the particular disease, condition, or disorder, or (iii) prevents or delays the onset of one or more symptoms of the particular disease, condition or disorder described herein. The therapeutically effective amount will vary depending on the compound, the disease state being treated, the severity of the disease treated, the age and relative health of the subject, the route and form of administration, the judgement of the attending medical or veterinary practitioner, and other factors.

The term "pharmaceutical composition" denotes a mixture or solution comprising a therapeutically effective amount of an active pharmaceutical ingredient together with pharmaceutically acceptable excipients to be administered to a mammal, *e.g*., a human in need thereof.

### STING Agonists

The present invention relates to (i) a compound of formula (I), 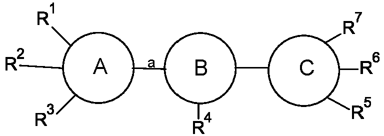 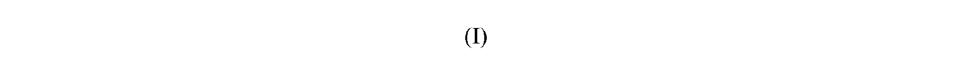 wherein
Ring A is a 5 to 12 membered monocyclic, bicyclic, or tricyclic heteroaryl ring or heterocyclyl ring containing one to four heteroatoms selected from N, O, and S,
   each of R¹, R² and R³ is independently absent or selected from C₁₋₆alkyl, haloC₁₋₆alkyl, hydroxylC₁₋₆alkyl, aminoC₁₋₆alkyl, -C(O)C₁₋₆alkyl, C₁₋₆alkylamino, (C₁₋₆alkyl)₂amino, C₁₋₆alkyl-S-, C₁₋₆alkylSO₂, C₁₋₆alkoxy, haloC₁₋₆alkoxy, C₁₋₆alkoxycarbonyl, carboxyl, hydroxyl, cyano, amino, halogen, acyl, carbamoyl, and a 5 to 8 membered monocyclic heterocyclyl containing one to three heteroatoms selected from N, O, and S, wherein said heterocyclyl can be further substituted by halogen, C₁₋₆alkyl, C₁₋₆alkoxy or amino;
Ring B is a 5 to 8 membered monocyclic or bicyclic heteroaryl ring or heterocyclyl ring containing one to four heteroatoms selected from N, O, and S,
   R⁴ is absent or selected from C₁₋₆alkyl, haloC₁₋₆alkyl, hydroxylC₁₋₆alkyl, aminoC₁₋₆alkyl, - C(O)C₁₋₆alkyl, C₁₋₆alkylamino, (C₁₋₆alkyl)₂amino, C₁₋₆alkyl-S-, C₁₋₆alkylSO₂, C₁₋₆alkoxy, haloC₁₋₆alkoxy, C₁₋₆alkoxycarbonyl, carboxyl, hydroxyl, cyano, amino, halogen, and carbamoyl;
Bond a is a bond connecting ring A and ring B;
Ring C is a 5 to 8 membered monocyclic or bicyclic heteroaryl ring or heterocyclyl ring containing one to four heteroatoms selected from N, O, and S,
   each of R⁵, R⁶ and R⁷ is independently absent or selected from C₁₋₆alkyl, haloC₁₋₆alkyl, hydroxylC₁₋₆alkyl, aminoC₁₋₆alkyl, -C(O)C₁₋₆alkyl, C₁₋₆alkylamino, (C₁₋₆alkyl)₂amino, C₁₋₆alkyl-S-, C₁₋₆alkylSO₂, C₁₋₆alkoxy, C₁₋₆alkoxyC₁₋₆alkyl, haloC₁₋₆alkoxy, C₁₋₆alkoxycarbonyl, haloC₂₋₆alkenyl, C₃₋₇cycloalkyl, C₃₋₇cycloalkylC₁₋₆alkyl, C₂₋₆alkynyl, C₁₋₆alkoxyC₂₋₆alkynyl, carboxyl, hydroxyl, cyano, amino, halogen, carbamoyl, cyanoC₁₋₆alkyl, imidazolylC₁₋₆alkyl, triazolylC₁₋₆alkyl, pyridinylC₁₋₆alkyl, thiazolylC₁₋₆alkyl, and phenylC₁₋₆alkyl, wherein the phenylC₁₋₆alkyl is optionally substituted by halogen, cyano, hydroxylC₁₋₆alkyl, morpholinoC₁₋₆alkyl, C₁₋₆alkoxy, C₁₋₆alkoxyC₁₋₆alkyl, and C₁₋₆alkoxycarbonyl, or
   two of R⁵, R⁶, and R⁷ together with the atoms to which they are bonded form a 5 to 6 membered ring having 0 to 2 heteroatoms selected from N, O, and S;
or a pharmaceutically acceptable salt thereof.

A further embodiment of present invention is (ii) the compound of formula (I), or a pharmaceutically acceptable salt thereof, according to (i), wherein
Ring A is selected from pyridyl, imidazo[1,5-a]pyrazinyl, imidazo[1,5-a]pyridinyl, pyrrolo[1,2-a]pyrazinyl, pyrrolo[2,3-c]pyridinyl, pyrrolo[1,2-c]pyrimidinyl, pyrazolo[3,4-c]pyridinyl, and 1H-pyrazolo[4,3-c]pyridinyl,
   R¹ is carbamoyl,
   R² is selected from C₁₋₆alkyl, haloC₁₋₆alkyl, halogen, acyl, and a 5 to 8 membered monocyclic heterocyclyl containing one to three heteroatoms selected from N, O, and S, preferably R² is C₁₋₆alkyl,
   R³ is absent or halogen;
Ring B is selected from 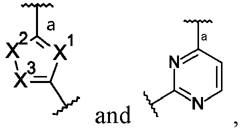
   each of X¹, X² and X³ is independently selected from C, N, and O,
   R⁴ is absent or selected from C₁₋₆alkyl, haloC₁₋₆alkyl, aminoC₁₋₆alkyl, hydroxyC₁₋₆alkyl, and carbamoyl, preferably R⁴ is absent or selected from C₁₋₆alkyl and hydroxyC₁₋₆alkyl;
Ring C is pyrazolyl,
   R⁵ is selected from C₁₋₆alkyl, hydroxylC₁₋₆alkyl, haloC₁₋₆alkyl, cyanoC₁₋₆alkyl, haloC₂₋₆alkenyl, C₃₋₇cycloalkyl, C₃₋₇cycloalkylC₁₋₆alkyl, C₁₋₆alkoxyC₁₋₆alkyl, C₂₋₆alkynyl, C₁₋₆alkoxyC₂₋₆alkynyl, imidazolylC₁₋₆alkyl, triazolylC₁₋₆alkyl, pyridinylC₁₋₆alkyl, thiazolylC₁₋₆alkyl, and phenylC₁₋₆alkyl, wherein the phenylC₁₋₆alkyl is optionally substituted by halogen, cyano, hydroxylC₁₋₆alkyl, morpholinoC₁₋₆alkyl, C₁₋₆alkoxy, C₁₋₆alkoxyC₁₋₆alkyl, and C₁₋₆alkoxycarbonyl, and wherein the phenylC₁₋₆alkyl is preferably optionally substituted by fluoro, cyano, methoxy, hydroxymethyl, morpholinomethyl, methoxymethyl, or methoxycarbonyl, preferably R⁵ is selected from C₁₋₆alkyl, haloC₁₋₆alkyl, haloC₂₋₆alkenyl, C₂₋₆alkynyl, C₃₋₇cycloalkylC₁₋₆alkyl, pyridinylC₁₋₆alkyl, phenylC₁₋₆alkyl, wherein the phenylC₁₋₆alkyl is optionally substituted by halogen, C₁₋₆alkoxy, C₁₋₆alkoxyC₁₋₆alkyl, hydroxylC₁₋₆alkyl, or morpholinoC₁₋₆alkyl, and more preferably R⁵ is selected from C₁₋₆alkyl, haloC₁₋₆alkyl, pyridinylC₁₋₆alkyl, and phenylC₁₋₆alkyl, wherein the phenylC₁₋₆alkyl is optionally substituted by halogen, C₁₋₆alkoxy, C₁₋₆alkoxyC₁₋₆alkyl, or hydroxylC₁₋₆alkyl;
   R⁶ is absent or C₁₋₆alkyl, R⁷ is absent or selected from hydroxyl, halogen, and amino, or R⁶ and R⁷ together with the atoms to which they are bonded form a 5 to 6 membered ring containing zero to two heteroatoms selected from N, O, and S, preferably R⁶ is C₁₋₆alkyl and R⁷ is hydroxyl.

A further embodiment of present invention is (iii) the compound of formula (I), or a pharmaceutically acceptable salt thereof, according to (i) or (ii), wherein
substituted ring A is selected from 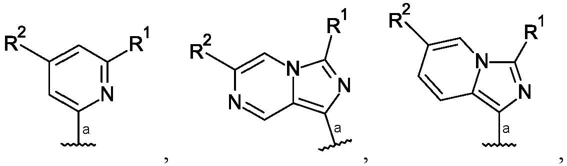 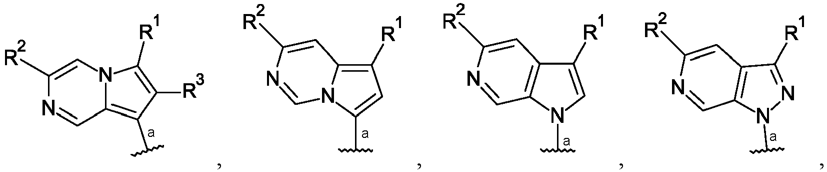 and

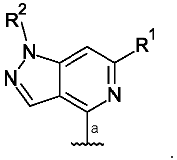

A further embodiment of present invention is (iv) the compound of formula (I), or a pharmaceutically acceptable salt thereof, according to any one of (i) to (iii), wherein
substituted ring A is selected from 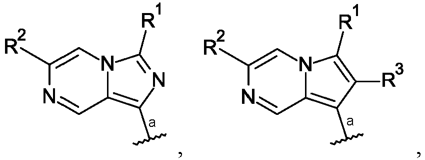 and 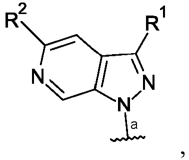
R¹ is carbamoyl,
R² is C₁₋₆alkyl,
R³ is absent or halogen.

A further embodiment of present invention is (v) the compound of formula (I), or a pharmaceutically acceptable salt thereof, according to any one of (i) to (iii), wherein
R¹ is carbamoyl;
R² is selected from methyl, difluoromethyl, acetyl, chloro, and imidazolyl;
R³ is absent or fluoro.

A further embodiment of present invention is (vi) the compound of formula (I), or a pharmaceutically acceptable salt thereof, according to any one of (i) to (v), wherein
R¹ is carbamoyl;
R² is methyl;
R³ is absent or fluoro.

A further embodiment of present invention is (vii) the compound of formula (I), or a pharmaceutically acceptable salt thereof, according to any one of (i) to (vi), wherein ring B is selected from triazolyl, oxazolyl, oxadiazolyl, and pyrimidinyl.

A further embodiment of present invention is (viii) the compound of formula (I), or a pharmaceutically acceptable salt thereof, according to any one of (i) to (vii), wherein
substituted ring B is selected from 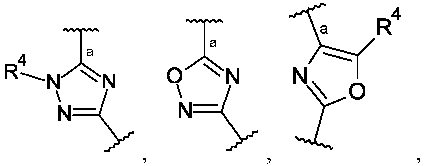 and

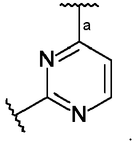

A further embodiment of present invention is (ix) the compound of formula (I), or a pharmaceutically acceptable salt thereof, according to any one of (i) to (viii), wherein
substituted ring B is selected from 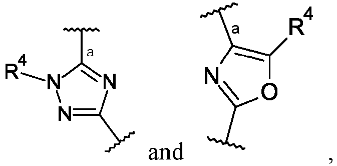
R⁴ is absent or selected from C₁₋₆alkyl and hydroxyC₁₋₆alkyl.

A further embodiment of present invention is (x) the compound of formula (I), or a pharmaceutically acceptable salt thereof, according to any one of (i) to (viii), wherein R⁴ is absent or selected from methyl, difluoromethyl, aminomethyl, hydroxymethyl, and carbamoyl.

A further embodiment of present invention is (xi) the compound of formula (I), or a pharmaceutically acceptable salt thereof, according to any one of (i) to (x), wherein R⁴ is absent or selected from methyl and hydroxymethyl.

A further embodiment of present invention is (xii) the compound of formula (I), or a pharmaceutically acceptable salt thereof, according to any one of (i) to (xi), wherein substitued ring C is

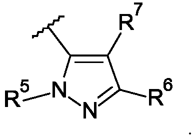

A further embodiment of present invention is (xiii) the compound of formula (I), or a pharmaceutically acceptable salt thereof, according to any one of (i) to (xii), wherein
R⁵ is selected from ethyl, propyl, butyl, cyclopropyl, cyclopropylmethyl, 3,3-difluoropropyl, 3,3-difluoroallyl, 2-cyanoethyl, 3-cyanopropyl, 3-hydroxypropyl, 2-imidazol-1-ylethyl, 2-(triazol-2-yl)ethyl, 2-(triazol-1-yl)ethyl, 2-(1,2,4-triazol-4-yl)ethyl, 2-(1,2,4-triazol-1-yl)ethyl, 3-fluoro-propyl, 2-methoxyethyl, 3-methoxypropyl, 1-pent-3-ynyl, 5-methoxypent-3-ynyl, 2-phenylethyl, 2-(4-methoxyphenyl)ethyl, 2-(3-methoxyphenyl)ethyl, 2-[4-(methoxymethyl)phenyl]ethyl, 2-[3-(methoxymethyl)phenyl]ethyl, 2-(4-fluorophenyl)ethyl, 2-(3-fluorophenyl)ethyl, 2-(3-cyanophenyl)ethyl, 2-(4-cyanophenyl)ethyl, 2-(4-methoxycarbonylphenyl)-ethyl, 2-[4-(hydroxymethyl)phenyl]ethyl, 2-pyridin-4-ylethyl, 2-(3-pyridyl)ethyl, 2-[4-(morpholinomethyl)phenyl]ethyl, and 2-thiazol-2-ylethyl,
R⁶ is absent or methyl, R⁷ is absent, fluoro, amino, or hydroxyl, or R⁶ and R⁷ together with the atoms to which they are bonded form a 5 membered ring.
A further embodiment of present invention is (xiv) the compound of formula (I), or a pharmaceutically acceptable salt thereof, according to any one of (i) to (xiii), wherein
R⁵ is selected from propyl, butyl, cyclopropylmethyl, 3,3-difluoroallyl, 3-fluoro-propyl, 3,3-difluoropropyl, 1-pent-3-ynyl, 2-(3-pyridyl)ethyl, 2-phenylethyl, 2-(4-methoxyphenyl)ethyl, 2-(4-fluorophenyl)ethyl, 2-(3-fluorophenyl)ethyl, 2-[4-(methoxymethyl)phenyl]ethyl, 2-[3-(methoxymethyl)phenyl]ethyl, 2-[4-(hydroxymethyl)phenyl]ethyl, and 2-[4-(morpholinomethyl)phenyl]ethyl,
R⁶ is methyl,
R⁷ is hydroxyl.

A further embodiment of present invention is (xv) the compound of formula (I), or a pharmaceutically acceptable salt thereof, according to any one of (i) or (ii), wherein substituted ring A is selected from 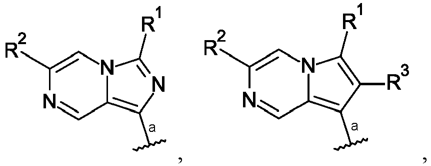 and 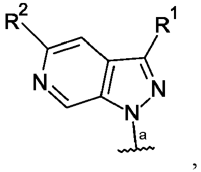
R¹ is carbamoyl,
R² is C₁₋₆alkyl,
R³ is absent or halogen; substituted ring B is selected from 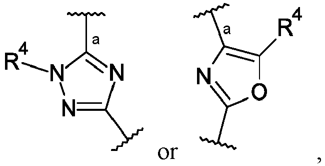
R⁴ is absent or selected from C₁₋₆alkyl and hydroxyC₁₋₆alkyl; substituted ring C is 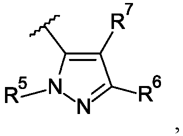
R⁵ is selected from C₁₋₆alkyl, haloC₁₋₆alkyl, haloC₂₋₆alkenyl, C₂₋₆alkynyl, C₃₋₇cycloalkylC₁₋₆alkyl, pyridinylC₁₋₆alkyl, phenylC₁₋₆alkyl, wherein the phenylC₁₋₆alkyl is optionally substituted by halogen, C₁₋₆alkoxy, C₁₋₆alkoxyC₁₋₆alkyl, hydroxylC₁₋₆alkyl, or morpholinoC₁₋₆alkyl,
R⁶ is C₁₋₆alkyl,
R⁷ is hydroxyl.

A further embodiment of present invention is (xvi) the compound of formula (I), or a pharmaceutically acceptable salt thereof, according to (xv), wherein
R¹ is carbamoyl,
R² is methyl,
R³ is absent or fluoro;
R⁴ is absent or selected from methyl and hydroxymethyl;
R⁵ is selected from propyl, butyl, 3-fluoro-propyl, 3,3-difluoropropyl, 3,3-difluoroallyl, 1-pent-3-ynyl, cyclopropylmethyl, 2-(3-pyridyl)ethyl, 2-phenylethyl, 2-(4-methoxyphenyl)ethyl, 2-(4-fluorophenyl)ethyl, 2-(3-fluorophenyl)ethyl, 2-[4-(methoxymethyl)phenyl]ethyl, 2-[3-(methoxymethyl)phenyl]ethyl, 2-[4-(hydroxymethyl)phenyl]ethyl, and 2-[4-(morpholinomethyl)phenyl]ethyl,
R⁶ is methyl,
R⁷ is hydroxyl.

A further embodiment of present invention is (xvii) the compound of formula (I), or a pharmaceutically acceptable salt thereof, according to (xv), wherein
R¹ is carbamoyl,
R² is C₁₋₆alkyl,
R³ is absent or halogen,
R⁴ is absent or hydroxyC₁₋₆alkyl,
R⁵ is selected from C₁₋₆alkyl, haloC₁₋₆alkyl, pyridinylC₁₋₆alkyl, and phenylC₁₋₆alkyl, wherein the phenylC₁₋₆alkyl is optionally substituted by halogen, C₁₋₆alkoxy, C₁₋₆alkoxyC₁₋₆alkyl, or hydroxylC₁₋₆alkyl, preferably R⁵ is selected from C₁₋₆alkyl, haloC₁₋₆alkyl, and phenylC₁₋₆alkyl, wherein the phenylC₁₋₆alkyl is optionally substituted by C₁₋₆alkoxy,
R⁶ is C₁₋₆alkyl,
R⁷ is hydroxyl.

A further embodiment of present invention is (xviii) the compound of formula (I), or a pharmaceutically acceptable salt thereof, according to (xvii), wherein
R¹ is carbamoyl,
R² is methyl,
R³ is absent or fluoro,
R⁴ is absent or hydroxymethyl;
R⁵ is selected from propyl, butyl, 3,3-difluoropropyl, 2-(3-pyridyl)ethyl, 2-phenylethyl, 2-(4-methoxyphenyl)ethyl, 2-(4-fluorophenyl)ethyl, 2-(3-fluorophenyl)ethyl, 2-[4-(methoxymethyl)phenyl]ethyl, and 2-[4-(hydroxymethyl)phenyl]ethyl, preferably R⁵ is selected from propyl, butyl, 3,3-difluoropropyl, 2-phenylethyl, and 2-(4-methoxyphenyl)ethyl, R⁶ is methyl,
R⁷ is hydroxyl.

A further embodiment of present invention is (xix) a compound selected from:
1-[3-(2-ethyl-4-hydroxy-5-methyl-pyrazol-3-yl)-1*H*-1,2,4-triazol-5-yl]-6-methyl-imidazo[1,5-a]pyrazine-3-carboxamide;
1-[5-(2-ethyl-4-hydroxy-5-methyl-pyrazol-3-yl)-2-methyl-1,2,4-triazol-3-yl]-6-methyl-imidazo[1,5-a]pyrazine-3-carboxamide;
8-[3-(2-ethyl-4-hydroxy-5-methyl-pyrazol-3-yl)-1*H*-1,2,4-triazol-5-yl]-3-methyl-pyrrolo[1,2-a]pyrazine-6-carboxamide;
8-[3-(2-ethyl-4-hydroxy-5-methyl-pyrazol-3-yl)-1*H*-1,2,4-triazol-5-yl]-7-fluoro-3-methyl-pyrrolo[1,2-a]pyrazine-6-carboxamide;
4-acetyl-6-[3-(2-ethyl-4-hydroxy-5-methyl-pyrazol-3-yl)-1*H*-1,2,4-triazol-5-yl]pyridine-2-carboxamide;
6-[3-(2-ethyl-4-hydroxy-5-methyl-pyrazol-3-yl)-1*H*-1,2,4-triazol-5-yl]-4-imidazol-1-yl-pyridine-2-carboxamide;
4-[2-(2-ethyl-4-hydroxy-5-methyl-pyrazol-3-yl)pyrimidin-4-yl]-1-methyl-pyrazolo[4,3-c]pyridine-6-carboxamide;
1-[2-(2-ethyl-4-hydroxy-5-methyl-pyrazol-3-yl)pyrimidin-4-yl]-6-methyl-imidazo[1,5-a]pyrazine-3-carboxamide;
1-[2-(2-ethyl-4-hydroxy-5-methyl-pyrazol-3-yl)oxazol-4-yl]-6-methyl-imidazo[1,5-a]pyrazine-3-carboxamide;
4-[5-(difluoromethyl)-2-(2-ethyl-4-hydroxy-5-methyl-pyrazol-3-yl)oxazol-4-yl]-1-methyl-pyrazolo[4,3-c]pyridine-6-carboxamide;
1-[5-(aminomethyl)-2-(2-ethyl-4-hydroxy-5-methyl-pyrazol-3-yl)oxazol-4-yl]-6-methyl-imidazo[1,5-a]pyrazine-3-carboxamide;
4-(6-carbamoyl-1-methyl-pyrazolo[4,3-c]pyridin-4-yl)-2-(2-ethyl-4-hydroxy-5-methyl-pyrazol-3-yl)oxazole-5-carboxamide;
4-[3-(2-ethyl-5,6-dihydro-4*H*-cyclopenta[c]pyrazol-3-yl)-1*H*-1,2,4-triazol-5-yl]-1-methyl-pyrazolo[4,3-c]pyridine-6-carboxamide;
1-[3-[2-(3-hydroxypropyl)-5-methyl-pyrazol-3-yl]-1*H*-1,2,4-triazol-5-yl]-6-methyl-imidazo[1,5-a]pyrazine-3-carboxamide;
1-[3-(2-ethyl-5-methyl-pyrazol-3-yl)-1*H*-1,2,4-triazol-5-yl]-6-methyl-imidazo[1,5-a]pyrazine-3-carboxamide;
1-[3-(2-ethyl-4-fluoro-5-methyl-pyrazol-3-yl)-1*H*-1,2,4-triazol-5-yl]-6-methyl-imidazo[1,5-a]pyrazine-3-carboxamide;
1-[3-(4-amino-2-ethyl-5-methyl-pyrazol-3-yl)-1*H*-1,2,4-triazol-5-yl]-6-methyl-imidazo[1,5-a]pyrazine-3-carboxamide;
6-chloro-1-[3-(2-ethyl-4-hydroxy-5-methyl-pyrazol-3-yl)-1*H*-1,2,4-triazol-5-yl]imidazo[1,5-a]pyrazine-3-carboxamide;
1-[3-(2-ethyl-4-hydroxy-5-methyl-pyrazol-3-yl)-1*H*-1,2,4-triazol-5-yl]-6-methyl-imidazo[1,5-a]pyridine-3-carboxamide;
7-[3-(2-ethyl-4-hydroxy-5-methyl-pyrazol-3-yl)-1*H*-1,2,4-triazol-5-yl]-3-methyl-pyrrolo[1,2-c]pyrimidine-5-carboxamide;
1-[2-(2-ethyl-4-hydroxy-5-methyl-pyrazol-3-yl)oxazol-4-yl]-5-methyl-pyrazolo[3,4-c]pyridine-3 -carboxamide;
1-[3-(2-ethyl-4-hydroxy-5-methyl-pyrazol-3-yl)-1*H*-1,2,4-triazol-5-yl]-5-methyl-pyrazolo[3,4-c]pyridine-3-carboxamide;
4-[3-[4-hydroxy-2-(2-imidazol-1-ylethyl)-5-methyl-pyrazol-3-yl]-1*H*-1,2,4-triazol-5-yl]-1-methyl-pyrazolo[4,3-c]pyridine-6-carboxamide;
4-[3-[2-(3-cyanopropyl)-4-hydroxy-5-methyl-pyrazol-3-yl]-1*H*-1,2,4-triazol-5-yl]-1-methyl-pyrazolo[4,3-c]pyridine-6-carboxamide;
4-[3-[2-(2-cyanoethyl)-4-hydroxy-5-methyl-pyrazol-3-yl]-1*H*-1,2,4-triazol-5-yl]-1-methyl-pyrazolo[4,3-c]pyridine-6-carboxamide;
6-(difluoromethyl)-1-[3-(2-ethyl-4-hydroxy-5-methyl-pyrazol-3-yl)-1*H*-1,2,4-triazol-5-yl]imidazo[1,5-a]pyrazine-3-carboxamide;
7-[3-(2-ethyl-4-hydroxy-5-methyl-pyrazol-3-yl)-1,2,4-oxadiazol-5-yl]-3-methyl-pyrrolo[1,2-c]pyrimidine-5-carboxamide;
8-[2-(2-ethyl-4-hydroxy-5-methyl-pyrazol-3-yl)oxazol-4-yl]-7-fluoro-3-methyl-pyrrolo[1,2-a]pyrazine-6-carboxamide;
1-[2-(2-ethyl-4-hydroxy-5-methyl-pyrazol-3-yl)oxazol-4-yl]-5-methyl-pyrrolo[2,3-c]pyridine-3 -carboxamide;
8-[3-[2-(2-cyanoethyl)-4-hydroxy-5-methyl-pyrazol-3-yl]-1*H*-1,2,4-triazol-5-yl]-7-fluoro-3-methyl-pyrrolo[1,2-a]pyrazine-6-carboxamide;
1-[3-(4-hydroxy-5-methyl-2-propyl-pyrazol-3-yl)-1*H*-1,2,4-triazol-5-yl]-5-methyl-pyrazolo[3,4-c]pyridine-3-carboxamide;
1-[3-[2-(cyclopropylmethyl)-4-hydroxy-5-methyl-pyrazol-3-yl]-1*H*-1,2,4-triazol-5-yl]-5-methyl-pyrazolo[3,4-c]pyridine-3-carboxamide;
1-[3-(2-cyclopropyl-4-hydroxy-5-methyl-pyrazol-3-yl)-1*H*-1,2,4-triazol-5-yl]-5-methyl-pyrazolo[3,4-c]pyridine-3-carboxamide;
4-[3-[2-(3,3-difluoroallyl)-4-hydroxy-5-methyl-pyrazol-3-yl]-1*H*-1,2,4-triazol-5-yl]-1-methyl-pyrazolo[4,3-c]pyridine-6-carboxamide;
4-[3-[4-hydroxy-5-methyl-2-[2-(triazol-2-yl)ethyl]pyrazol-3-yl]-1*H*-1,2,4-triazol-5-yl]-1-methyl-pyrazolo[4,3-c]pyridine-6-carboxamide;
4-[3-[4-hydroxy-5-methyl-2-[2-(triazol-1-yl)ethyl]pyrazol-3-yl]-1*H*-1,2,4-triazol-5-yl]-1-methyl-pyrazolo[4,3-c]pyridine-6-carboxamide;
4-[3-[2-(3,3-difluoropropyl)-4-hydroxy-5-methyl-pyrazol-3-yl]-1*H*-1,2,4-triazol-5-yl]-1-methyl-pyrazolo[4,3-c]pyridine-6-carboxamide;
4-[3-[4-hydroxy-5-methyl-2-[2-(1,2,4-triazol-4-yl)ethyl]pyrazol-3-yl]-1*H*-1,2,4-triazol-5-yl]-1-methyl-pyrazolo[4,3-c]pyridine-6-carboxamide;
4-[3-[4-hydroxy-5-methyl-2-[2-(1,2,4-triazol-1-yl)ethyl]pyrazol-3-yl]-1*H*-1,2,4-triazol-5-yl]-1-methyl-pyrazolo[4,3-c]pyridine-6-carboxamide;
1-[3-[4-hydroxy-5-methyl-2-(2-phenylethyl)pyrazol-3-yl]-1*H*-1,2,4-triazol-5-yl]-5-methyl-pyrazolo[3,4-c]pyridine-3-carboxamide;
1-[3-[2-(3-fluoropropyl)-4-hydroxy-5-methyl-pyrazol-3-yl]-1*H*-1,2,4-triazol-5-yl]-5-methyl-pyrazolo[3,4-c]pyridine-3-carboxamide;
1-[3-[4-hydroxy-2-(2-methoxyethyl)-5-methyl-pyrazol-3-yl]-1*H*-1,2,4-triazol-5-yl]-5-methyl-pyrazolo[3,4-c]pyridine-3-carboxamide ;
1-[3-(2-butyl-4-hydroxy-5-methyl-pyrazol-3-yl)-1*H*-1,2,4-triazol-5-yl]-5-methyl-pyrazolo[3,4-c]pyridine-3-carboxamide;
1-[3-[4-hydroxy-2-(3-methoxypropyl)-5-methyl-pyrazol-3-yl]-1*H*-1,2,4-triazol-5-yl]-5-methyl-pyrazolo[3,4-c]pyridine-3-carboxamide;
4-[3-(4-hydroxy-5-methyl-2-pent-3-ynyl-pyrazol-3-yl)-1*H*-1,2,4-triazol-5-yl]-1-methyl-pyrazolo[4,3-c]pyridine-6-carboxamide;
1-[3-[4-hydroxy-5-methyl-2-[2-(4-pyridyl)ethyl]pyrazol-3-yl]-1*H*-1,2,4-triazol-5-yl]-5-methyl-pyrazolo[3,4-c]pyridine-3-carboxamide;
1-[3-[4-hydroxy-5-methyl-2-[2-(3-pyridyl)ethyl]pyrazol-3-yl]-1*H*-1,2,4-triazol-5-yl]-5-methyl-pyrazolo[3,4-c]pyridine-3-carboxamide;
1-[5-[4-hydroxy-5-methyl-2-(2-phenylethyl)pyrazol-3-yl]-2-methyl-1,2,4-triazol-3-yl]-5-methyl-pyrazolo[3,4-c]pyridine-3-carboxamide;
1-[5-(2-butyl-4-hydroxy-5-methyl-pyrazol-3-yl)-2-methyl-1,2,4-triazol-3-yl]-5-methyl-pyrazolo[3,4-c]pyridine-3-carboxamide;
1-[3-[4-hydroxy-2-[2-(4-methoxyphenyl)ethyl]-5-methyl-pyrazol-3-yl]-1*H*-1,2,4-triazol-5-yl]-5-methyl-pyrazolo[3,4-c]pyridine-3-carboxamide;
1-[3-(4-hydroxy-5-methyl-2-pent-3-ynyl-pyrazol-3-yl)-1*H*-1,2,4-triazol-5-yl]-5-methyl-pyrazolo[3,4-c]pyridine-3-carboxamide;
1-[3-[2-[2-(4-fluorophenyl)ethyl]-4-hydroxy-5-methyl-pyrazol-3-yl]-1*H*-1,2,4-triazol-5-yl]-5-methyl-pyrazolo[3,4-c]pyridine-3-carboxamide;
1-[3-[4-hydroxy-5-methyl-2-(2-phenylethyl)pyrazol-3-yl]-1*H*-1,2,4-triazol-5-yl]-6-methyl-imidazo[1,5-a]pyrazine-3-carboxamide;
1-[3-[4-hydroxy-2-[2-(3-methoxyphenyl)ethyl]-5-methyl-pyrazol-3-yl]-1*H*-1,2,4-triazol-5-yl]-5-methyl-pyrazolo[3,4-c]pyridine-3-carboxamide;
1-(3-(1-(3-fluorophenethyl)-4-hydroxy-3-methyl-1*H*-pyrazol-5-yl)-1*H*-1,2,4-triazol-5-yl)-5-methyl-1*H*-pyrazolo[3,4-c]pyridine-3-carboxamide;
1-[3-[2-[2-(2-fluorophenyl)ethyl]-4-hydroxy-5-methyl-pyrazol-3-yl]-1*H*-1,2,4-triazol-5-yl]-5-methyl-pyrazolo[3,4-c]pyridine-3-carboxamide;
7-fluoro-8-[3-[4-hydroxy-5-methyl-2-(2-phenylethyl)pyrazol-3-yl]-1*H*-1,2,4-triazol-5-yl]-3-methyl-pyrrolo[1,2-a]pyrazine-6-carboxamide;
1-(2-(4-hydroxy-3-methyl-1-propyl-1*H*-pyrazol-5-yl)oxazol-4-yl)-5-methyl-1*H-*pyrazolo[3,4-c]pyridine-3-carboxamide;
1-(2-(1-butyl-4-hydroxy-3-methyl-1*H*-pyrazol-5-yl)oxazol-4-yl)-5-methyl-1*H-*pyrazolo[3,4-c]pyridine-3-carboxamide;
1-[3-[2-[2-(3-cyanophenyl)ethyl]-4-hydroxy-5-methyl-pyrazol-3-yl]-1*H*-1,2,4-triazol-5-yl]-5-methyl-pyrazolo[3,4-c]pyridine-3-carboxamide;
1-[3-[2-[2-(4-cyanophenyl)ethyl]-4-hydroxy-5-methyl-pyrazol-3-yl]-1*H*-1,2,4-triazol-5-yl]-5-methyl-pyrazolo[3,4-c]pyridine-3-carboxamide;
Methyl 4-[2-[5-[5-(3-carbamoyl-5-methyl-pyrazolo[3,4-c]pyridin-1-yl)-1*H*-1,2,4-triazol-3-yl]-4-hydroxy-3-methyl-pyrazol-1-yl]ethyl]benzoate;
1-(3-(1-(3,3-difluoroallyl)-4-hydroxy-3-methyl-1*H*-pyrazol-5-yl)-1*H*-1,2,4-triazol-5-yl)-5-methyl-1*H*-pyrazolo[3,4-c]pyridine-3-carboxamide;
1-[3-[4-hydroxy-2-[2-[4-(methoxymethyl)phenyl]ethyl]-5-methyl-pyrazol-3-yl]-1*H*-1,2,4-triazol-5-yl]-5-methyl-pyrazolo[3,4-c]pyridine-3-carboxamide;
1-[3-[4-hydroxy-2-[2-[3-(methoxymethyl)phenyl]ethyl]-5-methyl-pyrazol-3-yl]-1*H*-1,2,4-triazol-5-yl]-5-methyl-pyrazolo[3,4-c]pyridine-3-carboxamide;
1-(3-(1-(3,3-difluoropropyl)-4-hydroxy-3-methyl-1*H*-pyrazol-5-yl)-1*H*-1,2,4-triazol-5-yl)-5-methyl-1*H*-pyrazolo[3,4-c]pyridine-3-carboxamide;
1-(2-(1-(3,3-difluoropropyl)-4-hydroxy-3-methyl-1*H*-pyrazol-5-yl)oxazol-4-yl)-5-methyl-1*H*-pyrazolo[3,4-c]pyridine-3-carboxamid;
1-[3-[4-hydroxy-2-[2-[4-(hydroxymethyl)phenyl]ethyl]-5-methyl-pyrazol-3-yl]-1*H*-1,2,4-triazol-5-yl]-5-methyl-pyrazolo[3,4-c]pyridine-3-carboxamide;
7-fluoro-8-[3-[4-hydroxy-2-[2-(4-methoxyphenyl)ethyl]-5-methyl-pyrazol-3-yl]-1*H*-1,2,4-triazol-5-yl]-3-methyl-pyrrolo[1,2-a]pyrazine-6-carboxamide;
1-[3-[4-hydroxy-2-[2-(4-methoxyphenyl)ethyl]-5-methyl-pyrazol-3-yl]-1*H*-1,2,4-triazol-5-yl]-6-methyl-imidazo[1,5-a]pyrazine-3-carboxamide;
1-[3-[4-hydroxy-2-[2-[4-(hydroxymethyl)phenyl]ethyl]-5-methyl-pyrazol-3-yl]-1*H*-1,2,4-triazol-5-yl]-6-methyl-imidazo[1,5-a]pyrazine-3-carboxamide;
1-[3-[4-hydroxy-5-methyl-2-[2-(3-pyridyl)ethyl]pyrazol-3-yl]-1*H*-1,2,4-triazol-5-yl]-6-methyl-imidazo[1,5-a]pyrazine-3-carboxamide;
7-fluoro-8-[3-[4-hydroxy-5-methyl-2-[2-(3-pyridyl)ethyl]pyrazol-3-yl]-1*H*-1,2,4-triazol-5-yl]-3-methyl-pyrrolo[1,2-a]pyrazine-6-carboxamide;
1-(2-(4-hydroxy-3-methyl-1-(2-(pyridin-3-yl)ethyl)-1*H*-pyrazol-5-yl)oxazol-4-yl)-5-methyl-1*H*-pyrazolo[3,4-c]pyridine-3-carboxamide;
1-(2-(4-hydroxy-1-(3-methoxyphenethyl)-3-methyl-1*H*-pyrazol-5-yl)oxazol-4-yl)-5-methyl-1*H*-pyrazolo[3,4-c]pyridine-3-carboxamide;
8-[3-(2-butyl-4-hydroxy-5-methyl-pyrazol-3-yl)-1*H*-1,2,4-triazol-5-yl]-7-fluoro-3-methyl-pyrrolo[1,2-a]pyrazine-6-carboxamide;
1-[3-(2-butyl-4-hydroxy-5-methyl-pyrazol-3-yl)-1*H*-1,2,4-triazol-5-yl]-6-methyl-imidazo[1,5-a]pyrazine-3-carboxamide;
1-(2-(4-hydroxy-1-(4-methoxyphenethyl)-3-methyl-1*H*-pyrazol-5-yl)oxazol-4-yl)-5-methyl-1*H*-pyrazolo[3,4-c]pyridine-3-carboxamide;
7-fluoro-8-[3-(4-hydroxy-5-methyl-2-propyl-pyrazol-3-yl)-1*H*-1,2,4-triazol-5-yl]-3-methyl-pyrrolo[1,2-a]pyrazine-6-carboxamide;
1-[3-(4-hydroxy-5-methyl-2-propyl-pyrazol-3-yl)-1*H*-1,2,4-triazol-5-yl]-6-methyl-imidazo[1,5-a]pyrazine-3-carboxamide;
1-[3-[4-hydroxy-2-(5-methoxypent-3-ynyl)-5-methyl-pyrazol-3-yl]-1*H*-1,2,4-triazol-5-yl]-5-methyl-pyrazolo[3,4-c]pyridine-3-carboxamide;
4-(5-(4-hydroxy-3-methyl-1-phenethyl-1*H*-pyrazol-5-yl)-2*H*-1,2,4-triazol-3-yl)-1-methyl-1*H*-pyrazolo[4,3-c]pyridine-6-carboxamide;
1-[5-(hydroxymethyl)-2-(4-hydroxy-5-methyl-2-propyl-pyrazol-3 -yl)oxazol-4-yl]-5-methyl-pyrazolo[3,4-c]pyridine-3-carboxamide;
1-[5-(4-hydroxy-5-methyl-2-propyl-pyrazol-3-yl)-2-methyl-1,2,4-triazol-3-yl]-5-methyl-pyrazolo[3,4-c]pyridine-3-carboxamide;
1-[2-(4-hydroxy-5-methyl-2-propyl-pyrazol-3-yl)oxazol-4-yl]-6-methyl-imidazo[1,5-a]pyrazine-3-carboxamide;
1-[2-(2-butyl-4-hydroxy-5-methyl-pyrazol-3-yl)oxazol-4-yl]-6-methyl-imidazo[1,5-a]pyrazine-3-carboxamide;
1-[3-[4-hydroxy-5-methyl-2-[2-[4-(morpholinomethyl)phenyl]ethyl]pyrazol-3-yl]-1*H*-1,2,4-triazol-5-yl]-6-methyl-imidazo[1,5-a]pyrazine-3-carboxamide;
1-[2-[4-hydroxy-5-methyl-2-[2-[4-(morpholinomethyl)phenyl]ethyl]pyrazol-3-yl]oxazol-4-yl]-5-methyl-pyrazolo[3,4-c]pyridine-3-carboxamide;
1-[2-[4-hydroxy-2-[2-[4-(methoxymethyl)phenyl]ethyl]-5-methyl-pyrazol-3-yl]oxazol-4-yl]-5-methyl-pyrazolo[3,4-c]pyridine-3-carboxamide;
1-[3-[4-hydroxy-5-methyl-2-(2-thiazol-2-ylethyl)pyrazol-3-yl]-1*H*-1,2,4-triazol-5-yl]-6-methyl-imidazo[1,5-a]pyrazine-3-carboxamide;
8-[3-(4-hydroxy-5-methyl-2-propyl-pyrazol-3-yl)-1*H*-1,2,4-triazol-5-yl]-3-methyl-pyrrolo[1,2-a]pyrazine-6-carboxamide;
1-[5-(4-hydroxy-5-methyl-2-propyl-pyrazol-3-yl)-2-methyl-1,2,4-triazol-3-yl]-6-methyl-imidazo[1,5-a]pyrazine-3-carboxamide;
7-fluoro-8-[2-(4-hydroxy-5-methyl-2-propyl-pyrazol-3-yl)oxazol-4-yl]-3-methyl-pyrrolo[1,2-a]pyrazine-6-carboxamide;
8-[2-(4-hydroxy-5-methyl-2-propyl-pyrazol-3-yl)oxazol-4-yl]-3-methyl-pyrrolo[1,2-a]pyrazine-6-carboxamide;
or a pharmaceutically acceptable salt thereof.

A further embodiment of present invention is (xx) a process for the preparation of a compound having the structure of formula (Ia), 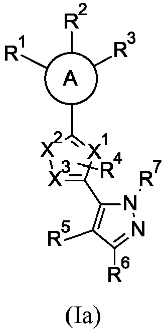 comprising one of the following steps:
(a) reaction of compound of formula (IIa) 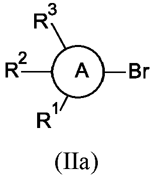 with compound of formula (III) 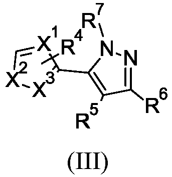 in the presence of a catalyst and a base to provide compound of formula (Ia), wherein the catalyst is preferably selected from Pd(OAc)₂, CuI, and Pd(dppf)Cl₂, and the base is preferably selected from K₂CO₃, Cs₂CO₃, and Li₂CO₃;
(b) reaction of a compound of formula (V) 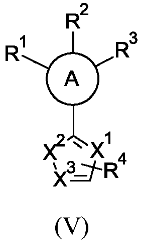 with compound of formula (VI) 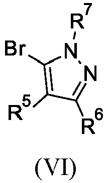 in the presence of a catalyst and a base to provide compound of formula (Ia), wherein the catalyst is preferably Pd(OAc)₂, and the base is preferably selected from K₂CO₃, Na₂CO_{3,} Cs₂CO₃, and KOAc,
(c) reaction of compound of formula (VII), 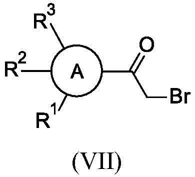 with compound of formula (VIII) 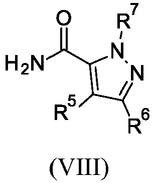 in the presence of a catalyst to proivde compound of formula (Ia), wherein the catalyst is preferably selected from AgOTf and AgBF₄,
(d) reaction of a compound of formula (X) 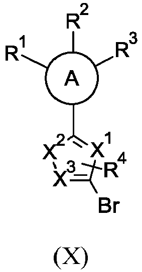 with compound of formula (XI) 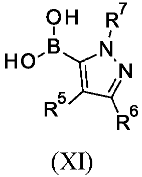 in the presence of a catalyst and a base to provide compound of formula (Ia), wherein the catalyst is preferably Pd(dppf)Cl₂, and the base is preferably K₂CO₃,
   wherein R¹ to R⁷, A, and X¹ to X³ are as defined above.

A further embodiment of present invention is (xxi) a process for the preparation of a compound having the structure of formula (Ib), 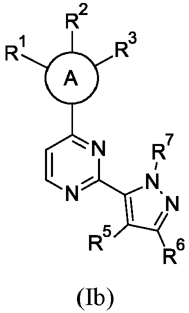 comprising the following step:
reaction of compound of formula (XII) 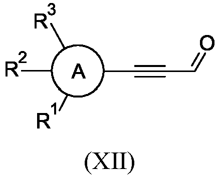
with compound of formula (XIII) 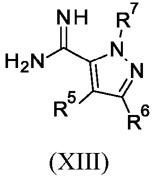
in the presence of a catalyst and a base to provide compound of formula (Ib), wherein the catalyst is preferably PPh₃AuCl₃, and the base is preferably selected from K₂CO₃, Cs₂CO₃, and DBU,
wherein R¹ to R⁷ and ring A are as defined above.

A further embodiment of present invention is (xxii) a compound of formula (I), formula (Ia), formula (Ib), or a pharmaceutically acceptable salt thereof, when manufactured according to the process of (xx) or (xxi).

A further embodiment of present invention is (xxiii) a pharmaceutical composition comprising a compound or a pharmaceutically acceptable salt thereof according to any one of (i) to (xix), and a pharmaceutically acceptable excipient.

### PHARMACEUTICAL COMPOSITIONS AND ADMINISTRATION

The invention also provides pharmaceutical compositions or medicaments containing the compounds of the invention and a therapeutically inert carrier, diluent or excipient, as well as methods of using the compounds of the invention to prepare such compositions and medicaments. In one example, compounds of formula (I) may be formulated by mixing at ambient temperature at the appropriate pH, and at the desired degree of purity, with physiologically acceptable carriers, *i.e.,* carriers that are non-toxic to recipients at the dosages and concentrations employed into a galenical administration form. The pH of the formulation depends mainly on the particular use and the concentration of compound, but preferably ranges anywhere from about 3 to about 8. In one example, a compound of formula (I) is formulated in an acetate buffer, at pH 5. In another embodiment, the compounds of formula (I) are sterile. The compound may be stored, for example, as a solid or amorphous composition, as a lyophilized formulation or as an aqueous solution.

Compositions are formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The "effective amount" of the compound to be administered will be governed by such considerations, and is the minimum amount necessary to activate STING mediated production of Type I IRF responses and proinflammatary cytokines. For example, such amount may be below the amount that is toxic to normal cells, or the mammal as a whole.

In one example, the pharmaceutically effective amount of the compound of the invention administered parenterally per dose will be in the range of about 0.01 to 1000 (*e.g.,* 0.01-100) mg/kg, alternatively about 0.01 to 1000 (*e.g.,* 0.1 to 20) mg/kg of patient body weight per day, with the typical initial range of compound used being 0.3 to 15 mg/kg/day. In another embodiment, oral unit dosage forms, such as tablets and capsules, preferably contain from about 1 to about 1000 (*e.g.,* 25-100) mg of the compound of the invention.

The compounds of the invention may be administered by any suitable means, including oral, topical (including buccal and sublingual), rectal, vaginal, transdermal, parenteral, subcutaneous, intraperitoneal, intrapulmonary, intradermal, intrathecal and epidural and intranasal, and, if desired for local treatment, intralesional administration. Parenteral infusions include intramuscular, intravenous, intraarterial, intraperitoneal, or subcutaneous administration.

The compounds of the present invention may be administered in any convenient administrative form, *e.g.,* tablets, powders, capsules, solutions, dispersions, suspensions, syrups, sprays, suppositories, gels, emulsions, patches, etc. Such compositions may contain components conventional in pharmaceutical preparations, *e.g*., diluents, carriers, pH modifiers, sweeteners, bulking agents, and further active agents.

A typical formulation is prepared by mixing a compound of the present invention and a carrier or excipient. Suitable carriers and excipients are well known to those skilled in the art and are described in detail in, *e.g.,* Ansel, Howard C., et al., Ansel's Pharmaceutical Dosage Forms and Drug Delivery Systems. Philadelphia: Lippincott, Williams & Wilkins, 2004; Gennaro, Alfonso R., et al. Remington: The Science and Practice of Pharmacy. Philadelphia: Lippincott, Williams & Wilkins, 2000; and Rowe, Raymond C. Handbook of Pharmaceutical Excipients. Chicago, Pharmaceutical Press, 2005. The formulations may also include one or more buffers, stabilizing agents, surfactants, wetting agents, lubricating agents, emulsifiers, suspending agents, preservatives, antioxidants, opaquing agents, glidants, processing aids, colorants, sweeteners, perfuming agents, flavoring agents, diluents and other known additives to provide an elegant presentation of the drug (i.e., a compound of the present invention or pharmaceutical composition thereof) or aid in the manufacturing of the pharmaceutical product (i.e., medicament).

An example of a suitable oral dosage form is a tablet containing about 1 to 1000 mg (*e.g.,* 25mg, 50mg, 100mg, 250mg, or 500mg) of the compound of the invention compounded with about 1 to 1000 (*e.g.,* 90-30) mg anhydrous lactose, about 1 to 1000 (*e.g.,* 5-40) mg sodium croscarmellose, about 1 to 1000 (*e.g.,* 5-30mg) mg polyvinylpyrrolidone (PVP) K30, and about 1 to 1000 (*e.g.,* 1-10 mg) mg magnesium stearate. The powdered ingredients are first mixed together and then mixed with a solution of the PVP. The resulting composition can be dried, granulated, mixed with the magnesium stearate and compressed to tablet form using conventional equipment. An example of an aerosol formulation can be prepared by dissolving the compound, for example 1 to 500 mg (*e.g.,* 5-400 mg), of the invention in a suitable buffer solution, *e.g.* a phosphate buffer, adding a tonicifier, *e.g.* a salt such sodium chloride, if desired. The solution may be filtered, *e.g.,* using a 0.2 micron filter, to remove impurities and contaminants.

An embodiment, therefore, includes a pharmaceutical composition comprising a compound of formula (I), or a stereoisomer or pharmaceutically acceptable salt thereof. In a further embodiment includes a pharmaceutical composition comprising a compound of formula (I), or a stereoisomer or pharmaceutically acceptable salt thereof, together with a pharmaceutically acceptable carrier or excipient.

Another embodiment includes a pharmaceutical composition comprising a compound of formula (I) for use in the treatment of cancer.

The following embodiments illustrate typical compositions of the present invention, but serve merely as representative thereof.

### Composition A

A compound of the present invention can be used in a manner known per se as the active ingredient for the production of tablets of the following composition:

### Per tablet

| | |
|---|---|
| Active ingredient | 200 mg |
| Microcrystalline cellulose | 155 mg |
| Corn starch | 25 mg |
| Talc | 25 mg |
| Hydroxypropylmethylcellulose | 20 mg |
| | 425 mg |

### Composition B

A compound of the present invention can be used in a manner known per se as the active ingredient for the production of capsules of the following composition:

### Per capsule

| | |
|---|---|
| Active ingredient | 100.0 mg |
| Corn starch | 20.0 mg |
| Lactose | 95.0 mg |
| Talc | 4.5 mg |
| Magnesium stearate | 0.5 mg |
| | 220.0 mg |

### INDICATIONS AND METHODS OF TREATMENT

The compounds of the invention bind to STING, initiate signaling cascades that induce type I interferon and other pro-inflammatory cytokines and/or co-stimulatory factors. Accordingly, the compounds of the invention are useful for enhancing cancer antigen presentation, contributing to the priming, trafficking and infiltration of T cells, promoting killing of cancers in particular cancer cells. Compounds of the invention are useful for boosting innate immune response in myeloid cells that express STING. Alternatively, compounds of the invention are useful for invigorate T cells in tumors which are T cell present but suppressed, for example by upregulation of co-stimulatory molecules and production of pro-inflammatory cytokines. More broadly, the compounds can be used for the treatment of cancer types which are non-inflamed, *e*.*g*. immune desert or immune excluded by enhancing antigen presentation, T cell priming, recruitment/infiltration, and tumor killing.

Another embodiment includes a method of treating cancer in a mammal in need of such treatment, wherein the method comprises administering to said mammal a therapeutically effective amount of a compound of formula (I), a stereoisomer, tautomer, prodrug or pharmaceutically acceptable salt thereof. Cancer includes but is not limited to sarcomas, adenocarcinomas, blastomas, and carcinomas, of the various organ systems, such as those affecting pancreas, liver, lung, breast, lymphoid, stomach, buliarintestinal (e.g. colon), genitourinary tract (e.g. renal urothelial cells), prostate and pharynx.

A further embodiment of present invention is (xxiv) a compound of the invention for use as therapeutically active substance.

A further embodiment of present invention is (xxiv) a compound of the invention for use in the treatment or cancer.

A further embodiment of present invention is (xxvi) the use of a compound of the invention for the treatment of cancer.

A further embodiment of present invention is (xxvii) the use of a compound of the invention for the activation of STING.

A further embodiment of present invention is (xxviii) the use of a compound of the invention for the preparation of a medicament for the treatment of cancer.

A further embodiment of present invention is (xxix) the use of a compound of the invention for the preparation of a medicament for the activation of STING.

A further embodiment of present invention is (xxx) a method for the treatment of cancer, which method comprises administering an effective amount of a compound of the invention.

A further embodiment of present invention is (xxxi) the use of according to (xxvi) or (xxviii), or the method according to (xxx), wherein the cancer is a cancer in pancreas, liver, lung, breast, lymphoid, stomach, buliarintestinal, genitourinary tract, prostate, or pharynx.

### SYNTHESIS

The compounds of the present invention can be prepared by any conventional means. Suitable processes for synthesizing these compounds as well as their starting materials are provided in the schemes below and in the examples. All substituents, in particular, ring A, X¹ to X³, and R¹ to R⁷ are as defined above unless otherwise indicated. Furthermore, and unless explicitly otherwise stated, all reactions, reaction conditions, abbreviations and symbols have the meanings well known to a person of ordinary skill in organic chemistry.

General synthetic routes for preparing the compound of the invention are shown in **scheme 1.**

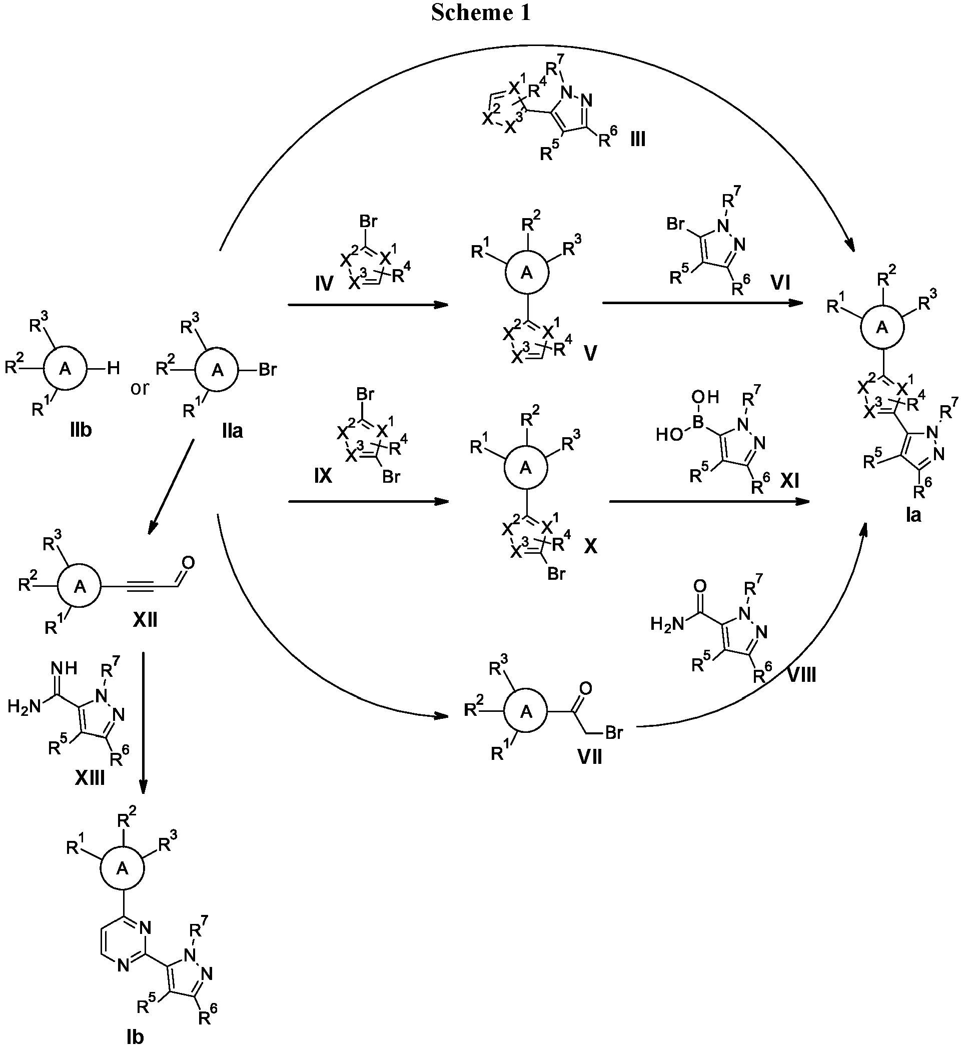

As depicted in **Scheme 1,** a compound having the structure of formula (Ia) can be prepared via a process comprising one of the following steps: 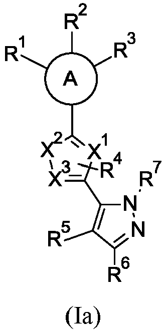
(a) C-H activation between compound of formula (IIa) 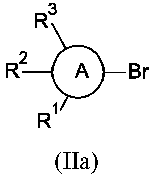 and compound of formula (III) 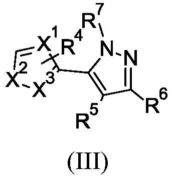 in the presence of a catalyst (such as Pd(OAc)₂, CuI, or Pd(dppf)Cl₂), and a base (such as K₂CO₃, Cs₂CO₃, or Li₂CO₃), provides compound of formula **(Ia);**
(b) bromide compound of formula **(IV)** 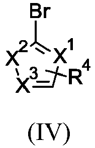 can be converted to boronic ester compound after reacting with B₂pin₂ in the presence of a catalyst (such as Pd(dppf)Cl₂, Pd₂(dba)₃, Pd(OAc)₂, or PdCl₂(PPh₃)₂), which can undergo Suzuki coupling reaction with compound of formula **(IIa)** in the presence of a catalyst (such as Pd(OAc)₂, Pd(dppf)Cl₂, Pd₂(dba)₃, or Pd(PPh₃)₄) to give compound of formula **(V),** 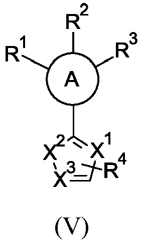 Compound of formula **(V)** can also be prepared from Ullmann reaction between compound of formula **(IIb)** 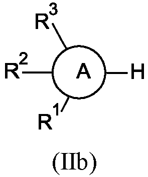 and bromide compound of formula **(IV)** 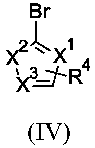 in the presence of a catalyst (such as CuI) and a base (such as K₃PO₄, or Cs₂CO₃), C-H activation between compound of formula **(V)** and compound of formula **(VI)** 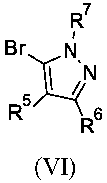 in the presence of a catalyst (such as Pd(OAc)₂) and a base (such as K₂CO₃, Na₂CO₃, Cs₂CO₃, or KOAc) provides compound of formula **(Ia);**
(c) bromide compound of formula **(IIa)** can be converted to *α*-bromocarbonyl compound of formula **(VII)** 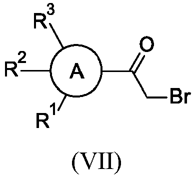 by Stille coupling reaction with tributyl(1-ethoxyvinyl)tin in the presence of a catalyst (such as Pd(PPh₃)₂Cl₂, Pd(OAc)₂, or Pd(MeCN)₂Cl₂), and then bromotion in the presence of brominaion reagent (such as NBS), then the reaction of compound of formula **(VII)** with compound of formula **(VIII)** 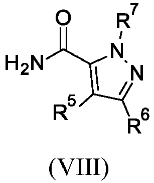 in the presence of a catalyst (such as AgOTf or AgBF₄) can give compound of formula **(Ia);**
(d) di-bromide compound of formula **(IX)** 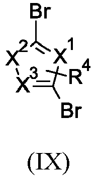 and compound of formula **(IIb)** 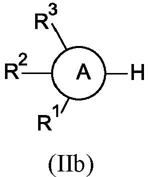 can undergo Ullmann reaction in the presence of catalyst (such as CuI) and a base (such as K₂CO₃, or K₃PO₄,) to provide compound of formula **(X),** 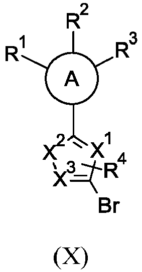 which further reacted with boride compound of formula **(XI)** 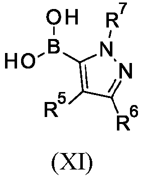 via Suzuki coupling reaction in the presence of catalyst (such as Pd(dppf)Cl₂,) and a base (such as K₂CO₃) to give compound of formula **(Ia).**

Also as depicted in **Scheme 1,** a compound having the structure of formula **(Ib)** can be prepared via a process comprising the following step: 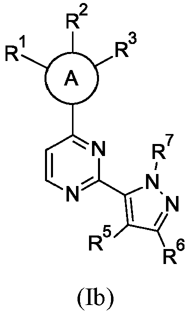 Bromide compound of formula **(IIa)** can be converted to *α*,*β*-unsaturated carbonyl compound of formula **(XII)** 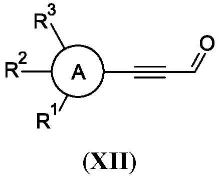 by Sonogashira coupling reaction with tert-butyldimethyl(2-propynyloxy)silane in the presence of a Pd catalyst (such as Pd(PPh₃)₄, Pd(PPh₃)₂Cl₂, Pd(dppf)Cl₂, or Pd(dppp)Cl₂), and a Cu(I) cocatalyst (such as CuI or CuBr), and then deprotection under NH₄F and oxidation in the presence of an oxidant (such as DMP), then the reaction of compound of formula **(XII)** with compound of formula **(XIII)** 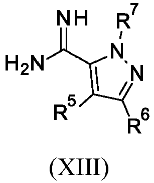 in the presence of a catalyst (such as PPh₃AuCl₃) and a base (such as K₂CO₃, Cs₂CO₃, or DBU) gives compound of formula **(Ib)** via the cyclization of pyrimidine ring.

### EXAMPLES

The invention will be more fully understood by reference to the following examples. They should not, however, be construed as limiting the scope of the invention.

Abbreviations used herein are as follows:
- ACN:: acetonitrile
- Ac₂O:: acetic anhydride
- AcOH:: acetic acid
- AIBN:: azobisisobutyronitrile
- Boc₂O:: di*-tert* butyl dicarbonate
- BnBr:: benzyl bromide
- BzCl:: benzoyl chloride
- B₂pin₂:: bis(pinacolato)diboron
- cataCXium A:: di(1-adamantyl)-n-butylphosphine
- CMBP:: cyanomethylenetributylphosphorane
- DAST:: diethylaminosulfur trifluoride
- DCM:: dichloromethane
- DCE:: dichloroethane
- DIPEA or DIEA:: *N,N*-diisopropylethylamine
- DMAP:: 4-dimethylaminopyridine
- DMBNH₂:: 2,4-dimethoxybenzylamine
- DMF:: *N,N*-dimethylformamide
- DMP:: Dess-Martin Periodinane
- DMSO:: dimethyl sulfoxide
- DPPF:: 1,1'-bis(diphenylphosphino)ferrocene
- EA or EtOAc:: ethyl acetate
- HATU:: 1-[bis(dimethylamino)methylene]-1H-1,2,3-triazolo[4,5-b]pyridinium 3-oxid hexafluorophosphate
- h(s) or hr(s):: hour (s)
- HFIP:: 1,1,1,3,3,3-hexafluoro-2-propanol
- LCMS:: liquid chromatography-mass spectrometry
- LDA:: lithium diisopropylamide
- LiHMDS:: lithium bis(trimethylsilyl)amide
- *m*CPBA*:*: meta-chloroperoxybenzoic acid
- min(s):: minute(s)
- MeOH:: methanol
- MsOH:: methanesulfonic acid
- MTBE:: methyl *tert*-butyl ether
- *n*-BuLi:: *n*-Butyllithium
- NBS:: *N*-bromosuccinimide
- NIS:: *N*-iodosuccinimide
- PCy₃HBF₄:: tri(cyclohexyl)phosphine tetrafluoroborate
- PE:: petroleum ether
- PivOH:: 2,2-dimethylpropanoic acid
- PMBBr:: 4-methoxybenzyl bromide
- PMBNCS:: 4-methoxybenzyl isothiocyanate
- PMBNH₂:: 4-methoxybenzylamine
- prep-HPLC:: preparative high performance liquid chromatography
- prep-TLC:: preparative thin layer chromatography
- PPh₃:: triphenylphosphine
- Pd(dppf)Cl₂:: (1,1'-bis(diphenylphosphino)ferrocene)palladium(II) dichloride
- Pd(OAc)₂:: palladium (II) acetate
- Py:: pyridine
- r.t.:: room temperature
- sat.:: saturated
- Selectfluor: 1-chloromethyl-4-fluoro-1,4-diazoniabicyclo[2.2.2]octane bis(tetrafluoroborate)
- SEMCl:: 2-(trimethylsilyl)ethoxymethyl chloride
- TBAF:: *tetra-n*-butylammonium fluoride
- TBSCl:: *tert*-butyldimethylsilyl chloride
- TEA:: trimethylamine
- TFA:: trifluoroacetic acid
- TFAA:: trifluoroacetic anhydride
- TFE:: 2,2,2-trifluoroethanol
- Tf₂O:: trifluoromethanesulfonic anhydride
- THF:: tetrahydrofuran
- TLC:: thin layer chromatography
- TMSCN:: trimethylsilyl cyanide
- Tol:: toluene
- v/v: volume ratio

### GENERAL EXPERIMENTAL CONDITIONS

Intermediates and final compounds were purified by flash chromatography using one of the following instruments: i) Biotage SP1 system and the Quad 12/25 Cartridge module. ii) ISCO combi-flash chromatography instrument. Silica gel brand and pore size: i) KP-SIL 60 Å, particle size: 40-60 µm; ii) CAS registry NO: Silica Gel: 63231-67-4, particle size: 47-60 micron silica gel; iii) ZCX from Qingdao Haiyang Chemical Co., Ltd, pore: 200-300 or 300-400.

Intermediates and final compounds were purified by preparative HPLC on reversed phase column using XBridge^{™} Prep-C18 (5 µm, OBDTM 30 × 100 mm) column, SunFire^{™} Prep-C18 (5 µm, OBD^{™} 30 × 100 mm) column, Phenomenex Synergi-C18 (10 µm, 25 × 150 mm) or Phenomenex Gemini-C18 (10 µm, 25 × 150 mm). Waters AutoP purification System (Sample Manager 2767, Pump 2525, Detector: Micromass ZQ and UV 2487, solvent system: acetonitrile and 0.1% ammonium hydroxide in water; acetonitrile and 0.1% FA in water or acetonitrile and 0.1% TFA in water). Or Gilson-281 purification System (Pump 322, Detector: UV 156, solvent system: acetonitrile and 0.05% ammonium hydroxide in water; acetonitrile and 0.225% FA in water; acetonitrile and 0.05% HCl in water; acetonitrile and 0.075% TFA in water; or acetonitrile and water).

LC/MS spectra of compounds were obtained using a LC/MS (Waters^{™} Alliance 2795-Micromass ZQ, Shimadzu Alliance 2020-Micromass ZQ or Agilent Alliance 6110-Micromass ZQ), LC/MS conditions were as follows (running time 3 or 1.5 mins):
Acidic condition I: A: 0.1% TFA in H₂O; B: 0.1% TFA in acetonitrile;
Acidic condition II: A: 0.0375% TFA in H₂O; B: 0.01875% TFA in acetonitrile;
Basic condition I: A: 0.1% NH₃·H₂O in H₂O; B: acetonitrile;
Basic condition II: A: 0.025% NH₃·H₂O in H₂O; B: acetonitrile;
Neutral condition: A: H₂O; B: acetonitrile.
Mass spectra (MS): generally only ions which indicate the parent mass are reported, and unless otherwise stated the mass ion quoted is the positive mass ion (MH)⁺.
NMR Spectra were obtained using Bruker Avance 400 MHz, 500 MHz.

The microwave assisted reactions were carried out in a Biotage Initiator Sixty microwave synthesizer. All reactions involving air-sensitive reagents were performed under an argon or nitrogen atmosphere. Reagents were used as received from commercial suppliers without further purification unless otherwise noted.

### PREPARATIVE EXAMPLES

### Intermediate A1

### 1-bromo-N-[(2,4-dimethoxyphenyl)methyl]-6-methyl-imidazo[1,5-a]pyrazine-3-carboxamide

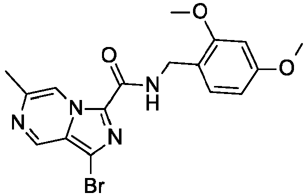

The title compound was prepared according to the following scheme:

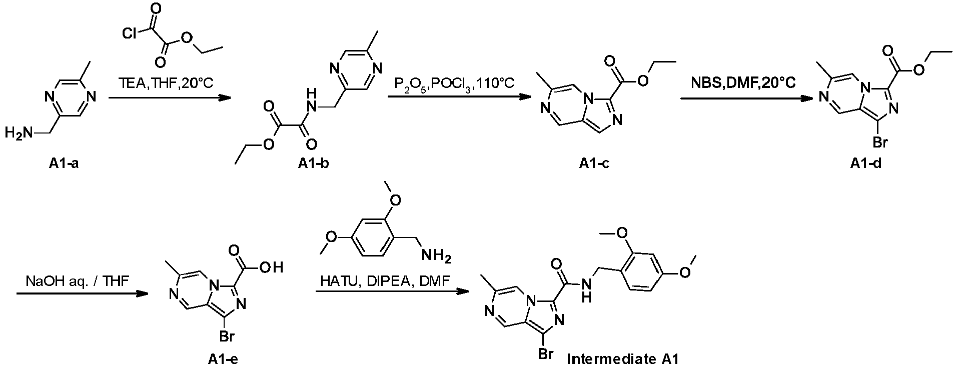

### Step 1: preparation of ethyl 2-[(5-methylpyrazin-2-yl) methylamino]-2-oxo-acetate (compound A1-b)

To a solution 2-(aminomethyl)-5-methylpyrazine **(A1-a,** 7.0 g, 56.84 mmol) in THF (250 mL) was added TEA (12.63 g, 125.04 mmol), the mixture was stirred at r.t. for 1 h, then ethyl 2-chloro-2-oxo-acetate (0.22 g, 1.62 mmol) was added and the mixture was then stirred at r.t. for 2 hrs. The reaction mixture was then poured into water (100 mL) and extracted with DCM (200 mL×4). The combined organic phase was washed with brine, dried over anhydrous Na₂SO₄, and concentrated to give **compound A1-b** (10 g). LCMS (M+H)⁺: 224.

### Step 2: preparation of ethyl 6-methylimidazo[1,5-a]pyrazine-3-carboxylate (compound A1-c)

To a solution of P₂O₅ (31.79 g, 224 mmol) in POCl₃ (100 mL) was added ethyl 2-[(5-methylpyrazin-2-yl) methylamino]-2-oxo-acetate **(A1-b,** 10 g, 44.8 mmol), the mixture was stirred at 110 °C for 12 hrs. The mixture was then concentrated, the residue was quenched with sat. NaHCO₃ solution, extracted with EA (300 mL×3). The combined organic phase was washed with sat. NaHCO₃ solution and brine, dried over anhydrous Na₂SO₄, and concentrated to give **compound A1-c** (8 g), LCMS (M+H)⁺: 206.

### Step 3: preparation of ethyl 1-bromo-6-methyl-imidazo[1,5-a]pyrazine-3-carboxylate (compound Al-d)

To a solution of ethyl 6-methylimidazo[1,5-a]pyrazine-3-carboxylate **(A1-c,** 3.0 g, 14.62 mmol) in DMF (30 mL) was added NBS (2.6 g, 14.62 mmol), the mixture was stirred at 20 °C for 3 hrs. The mixture was then poured into water (80 mL) and extracted with EA (100 mL×2). The combined organic phase was washed with brine, dried over anhydrous Na₂SO₄, filtered and concentrated. The residue was purified by column chromatography to give **compound A1-d** (2.9 g). LCMS (M+H)⁺: 284.

### Step 4: preparation of 1-bromo-6-methyl-imidazo[1,5-a]pyrazine-3-carboxylic acid (compound A1-e)

To a solution of ethyl 1-bromo-6-methyl-imidazo[1,5-a]pyrazine-3-carboxylate (**A1-d**, 2.5 g, 8.8 mmol) in THF (30 mL) was added NaOH (1.41 g, 35.2 mmol) and water (6 mL), the mixture was stirred at 20 °C for 12 hrs. The mixture was then adjusted to pH~3 with HCl (12 N), the mixture was filtered, the filtered cake was dried to give **compound A1-e** (2.3 g). LCMS (M+H)⁺: 256.

### Step 5: preparation of 1-bromo-N-[(2,4-dimethoxyphenyl)methyl]-6-methyl-imidazo[1,5-a]pyrazine-3-carboxamide (Intermediate A1)

To a solution of 1-bromo-6-methyl-imidazo[1,5-a]pyrazine-3-carboxylic acid **(A1-e,** 2.2 g, 8.59 mmol) in DMF (25 mL) was added DIPEA (3.33 g, 25.78 mmol), HATU (3.26 g, 8.59 mmol), and 2,4-dimethoxybenzylamine (1.55 mL, 10.31 mmol), the mixture was stirred at 20 °C for 3 hrs. The mixture was then poured into water (200 mL), and filtered, the filtered cake was dried to give **Intermediate A1** (2.2 g). LCMS (M+H)⁺: 405.

### Intermediate A2

### 4-bromo-N-[(2,4-dimethoxyphenyl)methyl]-1-methyl-pyrazolo[4,3-c]pyridine-6-carboxamide

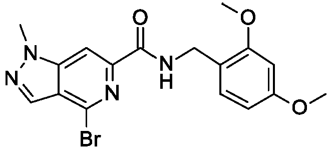

The title compound was prepared according to the following scheme:

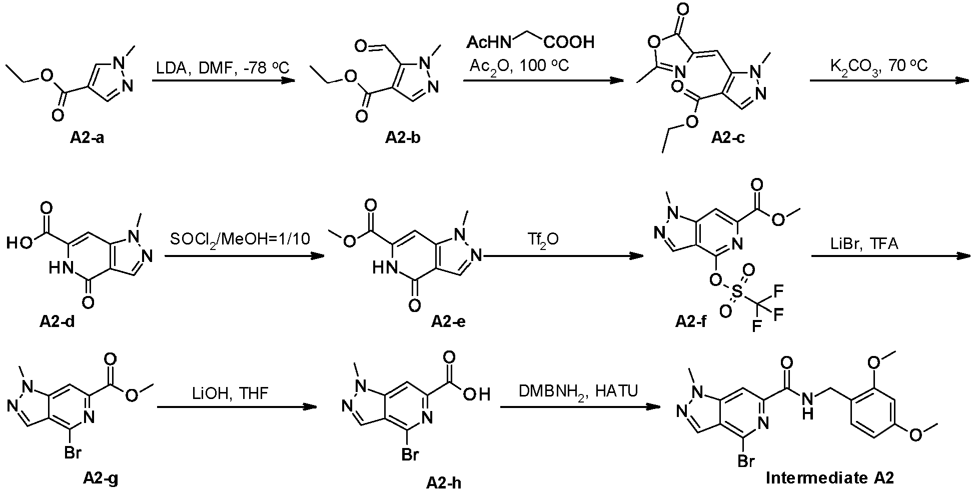

### Step 1: preparation of ethyl 5-formyl-1-methyl-pyrazole-4-carboxylate (compound A2-b)

To a solution of diisopropylamine (43.89 mL, 311.34 mmol) in THF (200 mL) was added n-BuLi (2.5 M in hexane solution, 116.75 mL, 291.89 mmol) dropwise at -78 °C, the mixture was stirred at -78 °C for 1 h, then a solution of ethyl 1-methylpyrazole-4-carboxylate **(A2-a,** 30.0 g, 194.59 mmol) in THF (50 mL) was added dropwise at -78 °C, the mixture was stirred for 1 h. Then the solution of DMF (33.07 mL, 428.1 mmol) in THF (30 mL) was added dropwise, the mixture was stirred at -78 °C for 2 hrs. The mixture was poured into HCl (1 N, 400 mL), extracted with EA (400 mL×3). The combined organic phase was washed with brine, dried over anhydrous Na₂SO₄ and concentrated. The residue was purified by column chromatography to give **compound A2-b** (23.5 g). LCMS (M+H)⁺: 183.

### Step 2: preparation of ethyl 1-methyl-5-[(Z)-(2-methyl-5-oxo-oxazol-4-ylidene) methyl] pyrazole-4-carboxylate (compound A2-c)

To a solution of ethyl 5-formyl-1-methyl-pyrazole-4-carboxylate **(A2-b,** 30.0 g, 164.67 mmol) and 2-acetamidoacetic acid (28.92 g, 247.01 mmol) in acetic anhydride (60 mL) was added KOAc (24.21 g, 247.01 mmol), the mixture was stirred at 100 °C for 2 hrs. The mixture was then quenched by sat. NaHCO₃ solution (500 mL) and extracted with EA (200 mL×3). The combined organic phase was washed with brine, dried over anhydrous Na₂SO₄, and concentrated to give **compound A2-c** (32.0 g). LCMS (M+H)⁺: 264.

### Step 3: preparation of 1-methyl-4-oxo-5H-pyrazolo [4,3-c] pyridine-6-carboxylic acid (compound A2-d)

To a suspension of ethyl 1-methyl-5-[(Z)-(2-methyl-5-oxo-oxazol-4-ylidene) methyl] pyrazole-4-carboxylate **(A2-c,** 32.0 g, 121.56 mmol) in MeOH (200 mL) was added K₂CO₃ (33.6 g, 243.11 mmol), the suspension was stirred at 70 °C for 12 hrs. The mixture was diluted with MTBE (500 mL) and filtered, the filtered cake was washed with MTBE, and then suspended in H₂O (200 mL). Con. HCl was added to adjust to pH~1. The mixture was then filtered, the filtered cake was dried to give **compound A2-d** (20.0 g). LCMS (M+H)⁺: 194.

### Step 4: preparation of methyl 1-methyl-4-oxo-5H-pyrazolo [4,3-c] pyridine-6-carboxylate (compound A2-e)

SOCl₂ (112.74 mL, 1.55 mol) was added to MeOH (400 mL) at 0 °C, then 1-methyl-4-oxo-SH-pyrazolo[4,3-c]pyridine-6-carboxylic acid **(A2-d,** 20.0 g, 103.54 mmol) was added, the mixture was stirred at 60 °C for 16 hrs and then concentrated to give **compound A2-e** (18.9 g). LCMS (M+H)⁺: 208.

### Step 5: preparation of methyl 1-methyl-4-(trifluoromethylsulfonyloxy) pyrazolo [4, 3-c] pyridine-6-carboxylate (compound A2-f)

To a suspension of methyl 1-methyl-4-oxo-5*H*-pyrazolo[4,3-c]pyridine-6-carboxylate **(A2-e,** 18.9 g, 91.22 mmol) in ACN (200 mL) was added Py (22.07 mL, 273.66 mmol) and Tf₂O (46.07 mL, 273.66 mmol) dropwise at 0 °C, the mixture was stirred at 20 °C for 1 h and then used for next step, the **compound A2-f** (30.9 g) was obtained. LCMS (M+H)⁺: 340.

### Step 6: preparation of methyl 4-bromo-1-methyl-pyrazolo [4,3-c] pyridine-6-carboxylate (compound A2-g)

To a solution of methyl 1-methyl-4-(trifluoromethylsulfonyloxy) pyrazolo [4,3-c] pyridine-6-carboxylate **(A2-f,** 30.9 g, 91.08 mmol) in ACN (200 mL) was added LiBr (79.11 g, 910.83 mmol), then TFA (30.44 mL, 409.87 mmol) was added slowly, the mixture was stirred at 20 °C for 16 hrs. The mixture was then quenched with sat. NaHCO₃ solution to pH >7, and the mixture was extracted with EA (500 mL×4). The combined organic phase was washed with brine, dried over anhydrous Na₂SO₄ and concentrated. The residue was purified by Prep-HPLC to give **compound A2-g** (11.5 g). LCMS (M+H)⁺: 270.

### Step 7: preparation of 4-bromo-1-methyl-pyrazolo [4, 3-c] pyridine-6-carboxylic acid (compound A2-h)

To a suspension of methyl 4-bromo-1-methyl-pyrazolo[4,3-c]pyridine-6-carboxylate **(A2-g,** 11.5 g, 42.58 mmol) in THF (50 mL) and water (20 mL) was added LiOH.H₂O (2.66 g, 63.87 mmol), the mixture was stirred at 20 °C for 1 h. The mixture was then adjusted to pH~1 with conc. HCl, the mixture was filtered, and the filtered cake was washed with water to give **compound A2-h** (10.55 g). LCMS (M+H)⁺: 256.

### Step 8: preparation of 4-bromo-N-[(2, 4-dimethoxyphenyl) methyl]-1-methyl-pyrazolo [4, 3-c] pyridine-6-carboxamide (Intermediate A2)

To a suspension of 4-bromo-1-methyl-pyrazolo [4,3-c] pyridine-6-carboxylic acid **(A2-h,** 10.55 g, 41.2 mmol) in DMF (40 mL) was added DIPEA (15.97 g, 123.6 mmol), 2, 4-dimethoxybenzylamine (7.43 mL, 49.44 mmol) and HATU (23.5 g, 61.8 mmol), the mixture was stirred at 20 °C for 1 h. The mixture was then diluted with H₂O (400 mL) and filtered, the filtered cake was washed with H₂O and EA, the solid was concentrated to give **Intermediate A2** (15.5 g). LCMS (M+H)⁺: 405.

### Intermediate B1

### 3-(4-benzyloxy-2-ethyl-5-methyl-pyrazol-3-yl)-4-[(4-methoxyphenyl)methyl]-1,2,4-triazole

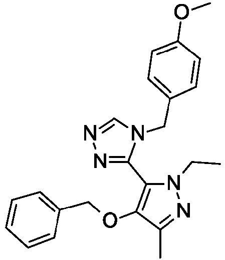

The title compound was prepared according to the following scheme:

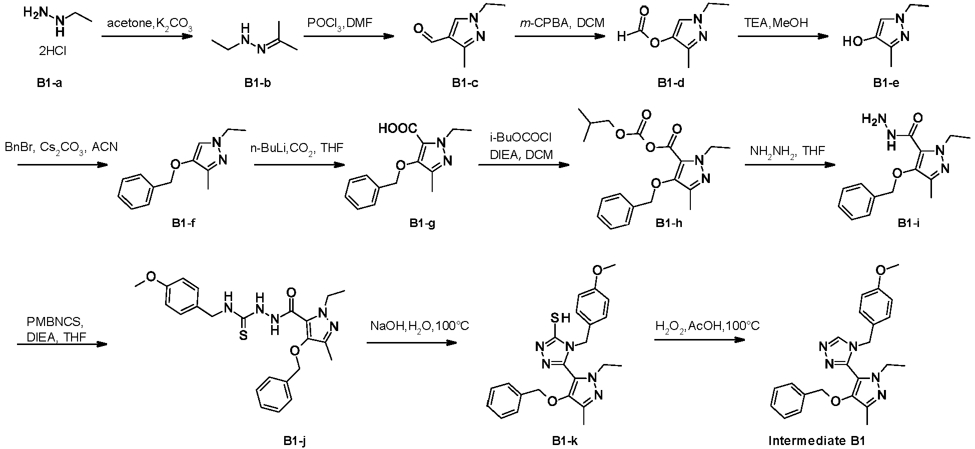

### Step 1: preparation of N-(isopropylideneamino)ethanamine (compound B1-b)

To a solution of ethylhydrazine dihydrochloride **(B1-a,** 40.0 g, 303.03 mmol) in DCM (750 mL) was added acetone (24.54 mL, 333.33 mmol) and K₂CO₃ (105.3 g, 757.58 mmol), the mixture was stirred at 20 °C for 12 hrs. Then the mixture was filtered, the filtered cake was washed with DCM, the filtrate was concentrated to give **compound B1-b** (25.0 g).

### Step 2: preparation of 1-ethyl-3-methyl-pyrazole-4-carbaldehyde (compound B1-c)

POCl₃ (81.68 mL, 873.6 mmol) was added dropwise to DMF (100 mL) at 0 °C, the mixture was stirred for 1 h and then cooled to -20 °C, then the solution of *N*-(isopropylideneamino)-ethanamine **(B1-b,** 35.0 g, 349.44 mmol) in DMF (50 mL) was added dropwise at -20 °C. The mixture was stirred at -20 °C for 3 hrs. Then the mixture was further stirred at 80 ° C for 2 hrs. The mixture was poured into ice slowly, adjusted to pH~9-10 with 30% aq. NaOH, and then extracted with EA (1.5 L×3). The combined organic phase was dried over anhydrous Na₂SO₄ and concentrated. The residue was purified by column chromatography to give **compound B1-c** (26.0 g). LCMS (M+H)⁺: 139.

### Step 3: preparation of (1-ethyl-3-methyl-pyrazol-4-yl) formate (compound B1-d)

To a solution of 1-ethyl-3-methyl-pyrazole-4-carbaldehyde **(B1-c,** 12.0 g, 86.85 mmol) in DCM (120 mL) was added *m*CPBA (35.15 g, 173.7 mmol), the mixture was stirred at 40 °C for 1 h. Then the mixture was poured into sat. Na₂SO₃ solution (800 mL), and extracted with EA (400 mL×3). The combined organic phase was washed with sat. NaHCO₃ solution and brine, then dried over anhydrous Na₂SO₄ and concentrated to give **compound B1-d** (12.0 g). LCMS (M+H)⁺: 155.

### Step 4: preparation of 1-ethyl-3-methyl-pyrazol-4-ol (compound B1-e)

To a solution of (1-ethyl-3-methyl-pyrazol-4-yl) formate **(B1-d,** 10.0 g, 64.86 mmol) in MeOH (100 mL) was added TEA (18.13 mL, 129.73 mmol), the mixture was stirred at r.t. for 0.5 h and then concentrated to give **compound B1-e** (12.0 g). LCMS (M+H)⁺: 127.

### Step 5: preparation of 4-benzyloxy-1-ethyl-3-methyl-pyrazole (compound B1-f)

To a solution of 1-ethyl-3-methyl-pyrazol-4-ol **(B1-e,** 8.0 g, 63.41 mmol) in ACN (100 mL) was added Cs₂CO₃ (41.34 g, 126.82 mmol) and BnBr (11.31 mL, 95.12 mmol), the mixture was stirred at r.t. for 0.5 h. Then the mixture was filtered, the filterate was poured into water (500 mL) and extracted with EA (800 mL×3). The combined organic phase was washed with sat. NaHCO₃ solution and brine, dried over anhydrous Na₂SO₄ and concentrated, the residue was purified by column chromatography to give **compound B1-f** (12.0 g). LCMS (M+H)⁺: 217.

### Step 6: preparation of 4-benzyloxy-2-ethyl-5-methyl-pyrazole-3-carboxylic acid (compound B1-g)

To a solution of 4-benzyloxy-1-ethyl-3-methyl-pyrazole **(B1-f,** 1.5 g, 6.94 mmol) in THF (160 mL) was added n-BuLi (2.5 M in hexane solution, 4.16 mL, 10.4 mmol) at -78 °C and stirred for 1 h. Then CO₂ (3.05 g, 69.35 mmol) was added, the mixture was stirred at r.t. for 1 h and then adjusted to pH~7 with con. HCl, then concentrated to give **compound B1-g** (1.8 g). LCMS (M+H)⁺: 261.

### Step 7: preparation of isobutoxycarbonyl 4-benzyloxy-2-ethyl-5-methyl-pyrazole-3-carboxylate (compound B1-h)

To a solution of 4-benzyloxy-2-ethyl-5-methyl-pyrazole-3-carboxylic acid **(B1-g,** 1.8 g, 6.92 mmol) in DCM (20 mL) was added DIPEA (3.63 mL, 20.75 mmol) and isobutyl chloroformate (1.75 mL, 13.83 mmol), the mixture was stirred at r.t. for 2 hrs and then concentrated to give **compound B1-h** (3.0 g). LCMS (M+H)⁺: 361.

### Step 8: preparation of 4-benzyloxy-2-ethyl-5-methyl-pyrazole-3-carbohydrazide (compound B1-i)

To a solution of hydrazine (0.59 mL, 18.94 mmol) in THF (5 mL) was added the suspension of isobutoxycarbonyl 4-benzyloxy-2-ethyl-5-methyl-pyrazole-3-carboxylate **(B1-h,** 3.0 g, 8.32 mmol) in THF (30 mL) dropwise at 0 °C, the mixture was stirred at r.t. for 0.5 h and then concentrated to give **compound B1-i** (1.6 g). LCMS (M+H)⁺: 275.

### Step 9: preparation of 1-[(4-benzyloxy-2-ethyl-5-methyl-pyrazole-3-carbonyl)amino]-3-[(4-methoxyphenyl)methyl]thiourea (compound B1-j)

To a solution of 4-benzyloxy-2-ethyl-5-methyl-pyrazole-3-carbohydrazide **(B1-i,** 1.6 g, 5.83 mmol) in THF (20 mL) was added DIPEA (1.52 g, 11.67 mmol) and PMBNCS (1.57 g, 8.75 mmol), the mixture was stirred at r.t. for 16 hrs. Then the mixture was diluted with EA (200 mL) and filtered, the filtrate was washed with water, the organic phase was dried with anhydrous Na₂SO₄ and concentrated to give **compound B1-j** (3.0 g). LCMS (M+H)⁺: 454.

### Step 10: preparation of 5-(4-benzyloxy-2-ethyl-5-methyl-pyrazol-3-yl)-4-[(4-methoxyphenyl)methyl]-1,2,4-triazole-3-thiol (compound B1-k)

To a solution of 1-[(4-benzyloxy-2-ethyl-5-methyl-pyrazole-3-carbonyl)amino]-3-[(4-methoxyphenyl)methyl]thiourea **(B1-j,** 3.0 g, 6.61 mmol) in water (12 mL) was added NaOH (6.0 mL, 18.0 mmol, 3 N), the mixture was stirred at 100 °C for 16 hrs. The mixture was then poured into water (500 mL) and extracted with EA (200 mL×3). The combined organic phase was washed with sat. NaHCO₃ solution and brine, dried over anhydrous Na₂SO₄ and concentrated to give **compound B1-k** (3.0 g). LCMS (M+H)⁺: 436.

### Step 11: preparation of 3-(4-benzyloxy-2-ethyl-5-methyl-pyrazol-3-yl)-4-[(4-methoxyphenyl)methyl]-1,2,4-triazole (Intermediate B1)

To a solution of 5-(4-benzyloxy-2-ethyl-5-methyl-pyrazol-3-yl)-4-[(4-methoxyphenyl)-methyl]-1,2,4-triazole-3-thiol **(B1-k,** 1.5 g, 3.44 mmol) in AcOH (60 mL) was added H₂O₂ (15.66 g, 138.12 mmol), the mixture was stirred at r.t. for 1 h. The mixture was then poured into ice water (600 mL) and extracted with EA (300 mL×3). The combined organic phase was washed with brine, dried over anhydrous Na₂SO₄ and concentrated, the residue was purified by column chromatography to give **Intermediate B1** (1.02 g). LCMS (M+H)⁺: 404.

### Intermediate B2

### 3-(4-benzyloxy-2-ethyl-5-methyl-pyrazol-3-yl)-1-methyl-1,2,4-triazole

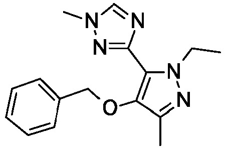

The title compound was prepared according to the following scheme:

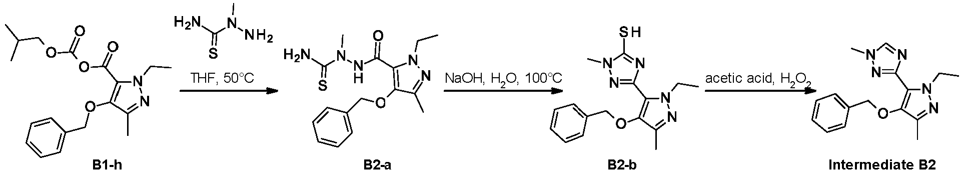

### Step 1: preparation of 1-[(4-benzyloxy-2-ethyl-5-methyl-pyrazole-3-carbonyl)amino]-1-methyl-thiourea (compound B2-a)

To a solution of isobutoxycarbonyl 4-benzyloxy-2-ethyl-5-methyl-pyrazole-3-carboxylate **(B1-h,** 1.0 g, 2.77 mmol) in THF (100 mL) was added 1-amino-1-methyl-thiourea (291.8 mg, 2.77 mmol), the mixture was stirred at 50 °C for 12 hrs. Then the mixture was poured into water (100 mL) and extracted with EA (100 mL×3). The combined organic phase was washed with sat. NaHCO₃ solution and brine, dried over anhydrous Na₂SO₄ and concentrated to give **compound B2-a** (0.85 g). LCMS (M+H)⁺: 348.

### Step 2: preparation of 5-(4-benzyloxy-2-ethyl-5-methyl-pyrazol-3-yl)-2-methyl-1,2,4-triazole-3-thiol (compound B2-b)

To a solution of 1-[(4-benzyloxy-2-ethyl-5-methyl-pyrazole-3-carbonyl)amino]-1-methyl-thiourea **(B2-a,** 0.8 g, 2.3 mmol) in water (12 mL) was added NaOH (2.09 mL, 6.27 mmol, 3 N), the mixture was stirred at 100 °C for 6 hrs. The mixture was then poured into water (200 mL) and extracted with EA (200 mL×3). The combined organic phase was washed with sat. NaHCO₃ solution and brine, dried over anhydrous Na₂SO₄ and concentrated to give **compound B2-b** (0.75 g), LCMS (M+H)⁺: 330.

### Step 3: preparation of 3-(4-benzyloxy-2-ethyl-5-methyl-pyrazol-3-yl)-1-methyl-1,2,4-triazole (Intermediate B2)

To a solution of 5-(4-benzyloxy-2-ethyl-5-methyl-pyrazol-3-yl)-2-methyl-1,2,4-triazole-3-thiol **(B2-b,** 0.7 g, 2.12 mmol) in AcOH (60 mL) was added H₂O₂ (9.64 g, 85.02 mmol), the mixture was stirred at r.t. for 1 h. The mixture was then poured into ice water (200 mL) and extracted with EA (300 mL×3). The combined organic phase was washed with brine, dried over anhydrous Na₂SO₄ and concentrated, the residue was purified by column chromatography to give **Intermediate B2** (300.0 mg). LCMS (M+H)⁺: 298.

### Example 1

### 1-[3-(2-ethyl-4-hydroxy-5-methyl-pyrazol-3-yl)-1H-1,2,4-triazol-5-yl]-6-methyl-imidazo[1,5-a]pyrazine-3-carboxamide

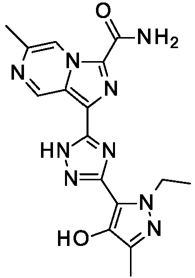

The title compound was prepared according to the following scheme:

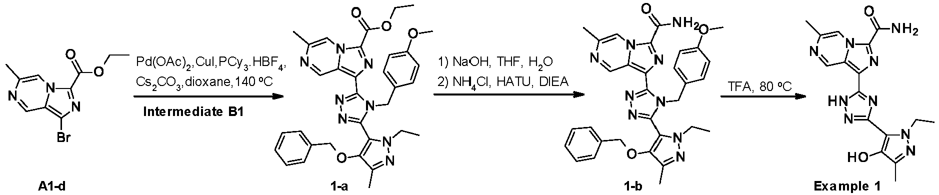

### Step 1: preparation of ethyl 1-[5-(4-benzyloxy-2-ethyl-5-methyl-pyrazol-3-yl)-2-[(4-methoxyphenyl)methyl]-1,2,4-triazol-3-yl]-6-methyl-imidazo[1,5-a]pyrazine-3-carboxylate (compound 1-a)

To a solution of ethyl 1-bromo-6-methyl-imidazo[1,5-a]pyrazine-3-carboxylate **(A1-d,** 338.0 mg, 1.19 mmol) in 1,4-dioxane (1 mL) was added 3-(4-benzyloxy-2-ethyl-5-methyl-pyrazol-3-yl)-4-[(4-methoxyphenyl)methyl]-1,2,4-triazole **(Intermediate B1,** 400.0 mg, 0.99 mmol), Pd(OAc)₂ (66.8 mg, 0.3 mmol), CuI (566.4 mg, 2.97 mmol), PCy₃HBF₄ (145.9 mg, 0.4 mmol) and Cs₂CO₃ (322.2 mg, 0.99 mmol), the mixture was stirred at 140 °C for 12 hrs. Then the mixture was concentrated, the residue was purified by prep-TLC to give **compound 1-a** (190.0 mg), LCMS (M+H)⁺: 607.

### Step 2: preparation of 1-[5-(4-benzyloxy-2-ethyl-5-methyl-pyrazol-3-yl)-2-[(4-methoxyphenyl)methyl]-1,2,4-triazol-3-yl]-6-methyl-imidazo[1,5-a]pyrazine-3-carboxamide (compound 1-b)

To a solution of ethyl 1-[5-(4-benzyloxy-2-ethyl-5-methyl-pyrazol-3-yl)-2-[(4-methoxyphenyl)methyl]-1,2,4-triazol-3-yl]-6-methyl-imidazo[1,5-a]pyrazine-3-carboxylate **(1-a,** 150.0 mg, 0.25 mmol) in THF (10 mL) was added NaOH (49.5 mg, 1.24 mmol) and water (2 mL), the mixture was stirred at r.t. for 12 hrs. Then the mixture was dissolved in EA (30 mL) and washed with brine, the organic layer was concentrated. The residue was dissolved in DMF (2 mL), then DIEA (67.0 mg, 0.52 mmol), NH₄Cl (27.7 mg, 0.52 mmol) and HATU (98.6 mg, 0.26 mmol) were added into, the mixture was stirred at 50 °C for 2 hrs. Then the mixture was concentrated, the residue was purified by reversed-phase column chromatography to give **compound 1-b** (50.0 mg), LCMS (M+H)⁺: 578.

### Step 3: preparation of 1-[3-(2-ethyl-4-hydroxy-5-methyl-pyrazol-3-yl)-1H-1,2,4-triazol-5-yl]-6-methyl-imidazo[1,5-a]pyrazine-3-carboxamide (Example 1)

The solution of 1-[5-(4-benzyloxy-2-ethyl-5-methyl-pyrazol-3-yl)-2-[(4-methoxyphenyl)-methyl]-1,2,4-triazol-3-yl]-6-methyl-imidazo[1,5-a]pyrazine-3-carboxamide **(1-b,** 50.0 mg, 0.09 mmol) in TFA (1.97 g, 17.31 mmol) was stirred at 80 °C for 0.5 h. Then the mixture was purified by reversed-phase column chromatography to give **Example 1** (8.2 mg), LCMS (M+H)⁺: 368. ¹H NMR (400 MHz, DMSO-*d₆*) δ = 9.69 (s, 1H), 9.10 (s, 1H), 8.00 - 7.82 (m, 1H), 4.48 (q,*J* = 6.7 Hz, 2H), 2.58 - 2.55 (m, 3H), 2.15 (s, 3H), 1.34 (t, *J =* 7.0 Hz, 3H).

### Example 2

### 1-[5-(2-ethyl-4-hydroxy-5-methyl-pyrazol-3-yl)-2-methyl-1,2,4-triazol-3-yl]-6-methyl-imidazo[1,5-a]pyrazine-3-carboxamide

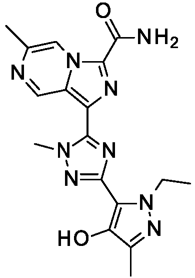

The title compound was prepared according to the following scheme:

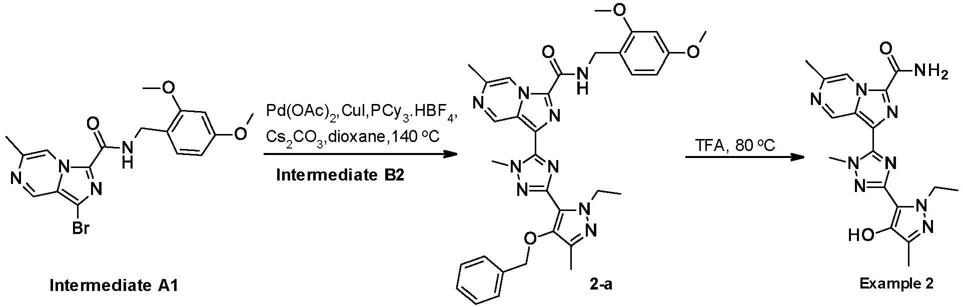

### Step 1: preparation of 1-[5-(4-benzyloxy-2-ethyl-5-methyl-pyrazol-3-yl)-2-methyl-1,2,4-triazol-3-yl]-N-[(2,4-dimethoxyphenyl)methyl]-6-methyl-imidazo[1,5-a]pyrazine-3-carboxamide (compound 2-a)

To a solution of 3-(4-benzyloxy-2-ethyl-5-methyl-pyrazol-3-yl)-1-methyl-1,2,4-triazole **(Intermediate B2,** 200.0 mg, 0.67 mmol) and 1-bromo-*N*-[(2,4-dimethoxyphenyl)methyl]-6-methyl-imidazo[1,5-a]pyrazine-3-carboxamide **(Intermediate A1,** 272.6 mg, 0.67 mmol) in 1,4-dioxane (2 mL) was added Pd(OAc)₂ (45.3 mg, 0.2 mmol), CuI (256.2 mg, 1.35 mmol), PCy₃HBF₄ (99.0 mg, 0.27 mmol) and Cs₂CO₃ (218.6 mg, 0.67 mmol), the mixture was stirred at 140 °C for 18 hrs. The mixture was then concentrated, the residue was purified by prep-TLC to give **compound 2-a** (190.0 mg), LCMS (M+H)⁺: 622.

### Step 2: preparation of 1-[5-(2-ethyl-4-hydroxy-5-methyl-pyrazol-3-yl)-2-methyl-1,2,4-triazol-3-yl]-6-methyl-imidazo[1,5-a]pyrazine-3-carboxamide (Example 2)

The solution of 1-[5-(4-benzyloxy-2-ethyl-5-methyl-pyrazol-3-yl)-2-methyl-1,2,4-triazol-3-yl]-*N*-[(2,4-dimethoxyphenyl)methyl]-6-methyl-imidazo[1,5-a]pyrazine-3-carboxamide **(compound 2-a,** 0.1 g, 0.16 mmol) in TFA (5.0 mL) was stirred at 80 °C for 2 hrs. The mixture was then concentrated, the residue was dissolved in ACN (5 mL) and stirred at r.t. for 0.5 h. The mixture was filtered, the filtered cake was dried to give **Example 2** (34.7 mg). LCMS (M+H)⁺: 382. ¹H NMR (400 MHz, DMSO-*d₆*) δ = 9.73 (s, 1H), 9.15 (s, 1H), 8.38 (s, 1H), 7.97 (s, 1H), 7.93 (br s, 1H), 4.45 (s, 3H), 4.45 - 4.40 (m, 2H), 2.52 (br s, 3H), 2.12 (s, 3H), 1.33 (t, *J =* 7.2 Hz, 3H).

### Example 3

### 8-[3-(2-ethyl-4-hydroxy-5-methyl-pyrazol-3-yl)-1H-1,2,4-triazol-5-yl]-3-methyl-pyrrolo[1,2-a]pyrazine-6-carboxamide

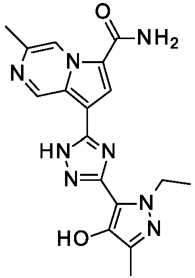

The title compound was prepared according to the following scheme:

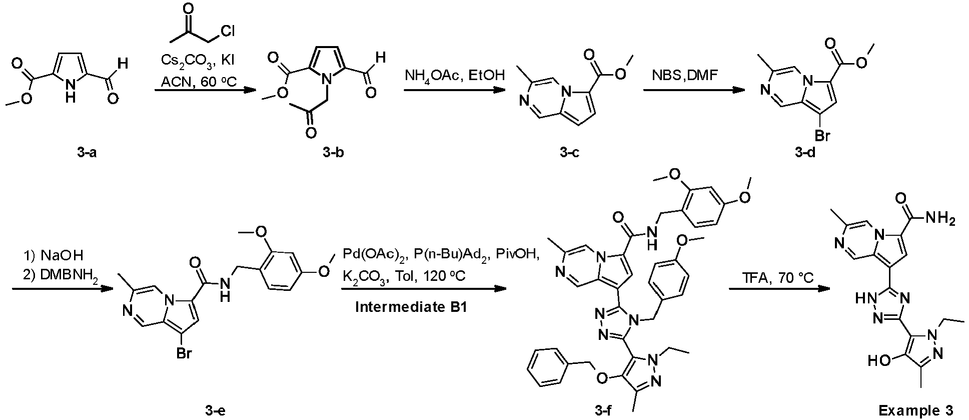

### Step 1: preparation of methyl 1-acetonyl-5-formyl-pyrrole-2-carboxylate (compound 3-b)

To a solution of methyl 5-formyl-1*H*-pyrrole-2-carboxylate **(3-a,** 2.0 g, 13.06 mmol) in ACN (30 mL) was added 1-chloropropan-2-one (1.47 g, 15.93 mmol), Cs₂CO₃ (5.19 g, 15.93 mmol) and KI (4.33 g, 26.12 mmol), the mixture was stirred at 60 °C for 3 hrs. Then the mixture was quenched with sat. NaHCO₃ solution (50 mL), then diluted with water (200 mL) and extracted with EA (250 mL×3). The combined organic phase was dried over anhydrous Na₂SO₄, filtered and concentrated. The residue was purified by column chromatography to give **compound 3-b** (1.5 g).

### Step 2: preparation of methyl 3-methylpyrrolo[1,2-a]pyrazine-6-carboxylate (compound 3-c)

To a solution of methyl 1-acetonyl-5-formyl-pyrrole-2-carboxylate **(3-b,** 2.07 g, 9.89 mmol) in EtOH (40 mL) was added NH₄OAc (2.288 g, 29.68 mmol), the mixture was stirred at 90 °C for 2 hrs. Then the mixture was concentrated and diluted with water (150 mL), extracted with EA (150 mL×3). The combined organic phase was dried over anhydrous Na₂SO₄ and concentrated to give **compound 3-c** (2.1 g), LCMS (M+H)⁺: 191.

### Step 3: preparation of methyl 8-bromo-3-methyl-pyrrolo[1,2-a]pyrazine-6-carboxylate (compound 3-d)

To a solution of methyl 3-methylpyrrolo[1,2-a]pyrazine-6-carboxylate **(3-c,** 2.1 g, 11.04 mmol) in DMF (20 mL) was added NBS (2.36 g, 13.25 mmol), the mixture was stirred at r.t. for 3 hrs. Then the mixture was diluted with water (100 mL) and extracted with EA (120 mL×3). The combined organic phase was dried over anhydrous Na₂SO₄ and concentrated. The residue was purified by column chromatography to give **compound 3-d** (2.02 g). LCMS (M+H)⁺: 269.

### Step 4: preparation of 8-bromo-N-[(2,4-dimethoxyphenyl)methyl]-3-methyl-pyrrolo[1,2-a]pyrazine-6-carboxamide (compound 3-e)

To a solution of methyl 8-bromo-3-methyl-pyrrolo[1,2-a]pyrazine-6-carboxylate **(3-d,** 1.2 g, 4.46 mmol) in THF (10 mL) was added the solution of NaOH (750.0 mg, 18.75 mmol) in water (3 mL), the mixture was stirred at 40 °C for 12 hrs. Then the mixture was diluted with water (30 mL), HCl (1 N) was added to adjust to pH~3, and filtered, the filtered cake was dissolved in DMF (4 mL), DIPEA (0.41 mL, 2.35 mmol), HATU (595.9 mg, 1.57 mmol) and 2,4-dimethoxybenzylamine (0.12 mL, 0.78 mmol) were added, the mixture was stirred at r.t. for 2 hrs. Then the mixture was poured into water (20 mL), then filtered, the filtered cake was collected to give **compound 3-e** (252.0 mg). LCMS (M+H)⁺: 404.

### Step 5: preparation of 8-[5-(4-benzyloxy-2-ethyl-5-methyl-pyrazol-3-yl)-4-[(4-methoxyphenyl)methyl]-1,2,4-triazol-3-yl]-N-[(2,4-dimethoxyphenyl)methyl]-3-methyl-pyrrolo[1,2-a]pyrazine-6-carboxamide (compound 3-f)

The solution of K₂CO₃ (51.2 mg, 0.37 mmol), Pd(OAc)₂ (8.3 mg, 0.04 mmol), PivOH (7.6 mg, 0.07 mmol), cataCXium A (13.3 mg, 0.04 mmol), 8-bromo-*N*-[(2,4-dimethoxyphenyl)-methyl]-3-methyl-pyrrolo[1,2-a]pyrazine-6-carboxamide **(3-e,** 50.0 mg, 0.12 mmol), 3-(4-benzyloxy-2-ethyl-5-methyl-pyrazol-3-yl)-4-[(4-methoxyphenyl)methyl]-1,2,4-triazole **(Intermediate B1,** 49.9 mg, 0.12 mmol) in toluene (1 mL) was stirred at 120 °C for 12 hrs. Then the mixture was purified by prep-TLC to give **compound 3-f** (32.0 mg), LCMS (M+H)⁺: 727.

### Step 6: preparation of 8-[3-(2-ethyl-4-hydroxy-5-methyl-pyrazol-3-yl)-1H-1,2,4-triazol-5-yl]-3-methyl-pyrrolo[1,2-a]pyrazine-6-carboxamide (Example 3)

The solution of 8-[5-(4-benzyloxy-2-ethyl-5-methyl-pyrazol-3-yl)-4-[(4-methoxyphenyl)-methyl]-1,2,4-triazol-3-yl]-*N*-[(2,4-dimethoxyphenyl)methyl]-3-methyl-pyrrolo[1,2-a]pyrazine-6-carboxamide **(3-f,** 32.0 mg, 0.04 mmol) in TFA (2.0 mL) was stirred at 70 °C for 2 hrs. Then the mixture was purified by reversed-phase column chromatography to give **Example 3** (4.6 mg), LCMS (M+H)⁺: 367. ¹H NMR (400 MHz, DMSO-*d₆*) δ = 9.64 (s, 1H), 9.36 (s, 1H), 8.17 (br s, 1H), 4.49 (br d, *J =* 6.8 Hz, 2H), 2.47 (s, 3H), 2.15 (s, 3H), 1.35 (t, *J =* 7.1 Hz, 3H).

### Example 4

### 8-[3-(2-ethyl-4-hydroxy-5-methyl-pyrazol-3-yl)-1H-1,2,4-triazol-5-yl]-7-fluoro-3-methyl-pyrrolo[1,2-a]pyrazine-6-carboxamide

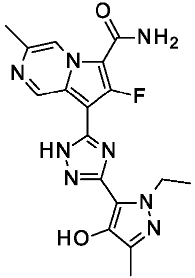

The title compound was prepared according to the following scheme:

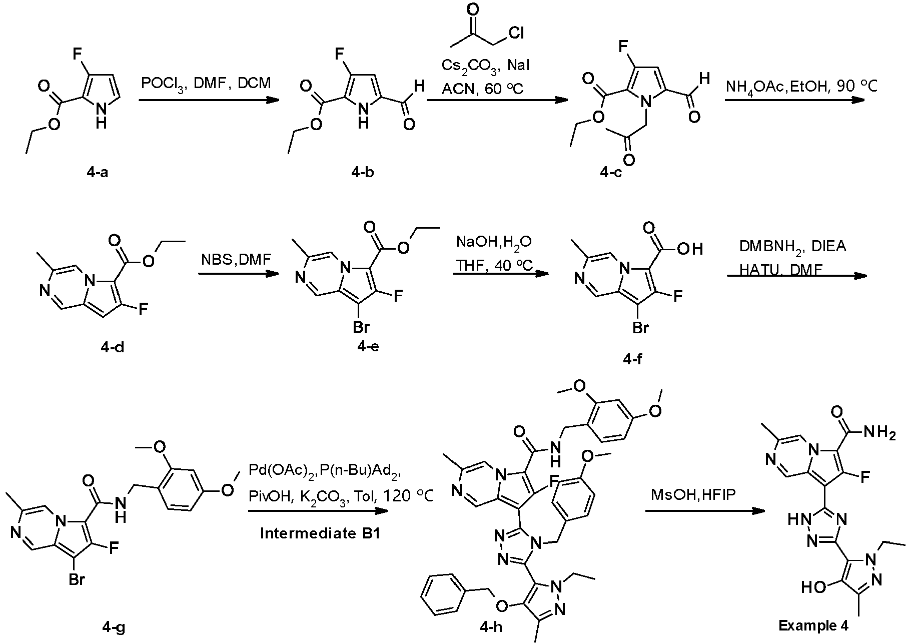

### Step 1: preparation of ethyl 3-fluoro-5-formyl-1H-pyrrole-2-carboxylate (compound 4-b)

POCl₃ (3.57 mL, 38.18 mmol) was added to the solution of DMF (3.7 mL, 47.73 mmol) in DCM (10 mL) at 0 °C, the mixture was stirred at 0 °C for 15 mins, then the solution of ethyl 3-fluoro-1*H*-pyrrole-2-carboxylate **(4-a,** 3.0 g, 19.09 mmol) in DCM (5 mL) was added dropwise. The mixture was poured into ice slowly. Then the mixture was adjusted to pH~9-10 with 30% NaOH aq. and then extracted with EA (50 mL×3). The combined organic phase was dried over anhydrous Na₂SO₄ and concentrated. The residue was purified by column chromatography to give **compound 4-b** (1.8 g). (M+H)⁺: 186.

### Step 2~7: preparation of 8-[5-(4-benzyloxy-2-ethyl-5-methyl-pyrazol-3-yl)-4-[(4-methoxyphenyl)methyl]-1,2,4-triazol-3-yl]-N-[(2,4-dimethoxyphenyl)methyl]-7-fluoro-3-methyl-pyrrolo[1,2-a]pyrazine-6-carboxamide (compound 4-h)

**Compound 4-h** was prepared in analogy to the preparation of **compound 3-f** by using **compound 4-b** instead of methyl 5-formyl-1*H*-pyrrole-2-carboxylate **(3-a),** LCMS (M+H)⁺: 745.

### Step 8: preparation of 8-[3-(2-ethyl-4-hydroxy-5-methyl-pyrazol-3-yl)-1H-1,2,4-triazol-5-yl]-7-fluoro-3-methyl-pyrrolo[1,2-a]pyrazine-6-carboxamide (Example 4)

To a solution of 8-[5-(4-benzyloxy-2-ethyl-5-methyl-pyrazol-3-yl)-4-[(4-methoxyphenyl)-methyl]-1,2,4-triazol-3-yl]-*N*-[(2,4-dimethoxyphenyl)methyl]-7-fluoro-3-methyl-pyrrolo[1,2-a]-pyrazine-6-carboxamide **(4-h,** 62.0 mg, 0.08 mmol) in HFIP (2.0 mL) was added MsOH (79.91 mg, 0.83 mmol), the mixture was stirred at r.t. for 1 h. Then the mixture was purified by reversed-phase column chromatography to give **Example 4** (22.0 mg). LCMS (M+H)⁺: 385. ¹H NMR (400 MHz, DMSO-*d₆*) δ = 9.63 (s, 1H), 9.23 (s, 1H), 7.93 - 7.71 (m, 1H), 7.57 - 7.35 (m, 1H), 4.49 (q, *J =* 6.9 Hz, 2H), 2.49 (br s, 3H), 2.16 (s, 3H), 1.36 (t, *J =* 7.1 Hz, 3H).

### Example 5

### 4-acetyl-6-[3-(2-ethyl-4-hydroxy-5-methyl-pyrazol-3-yl)-1H-1,2,4-triazol-5-yl]pyridine-2-carboxamide

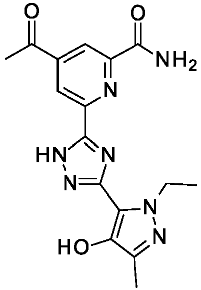

The title compound was prepared according to the following scheme:

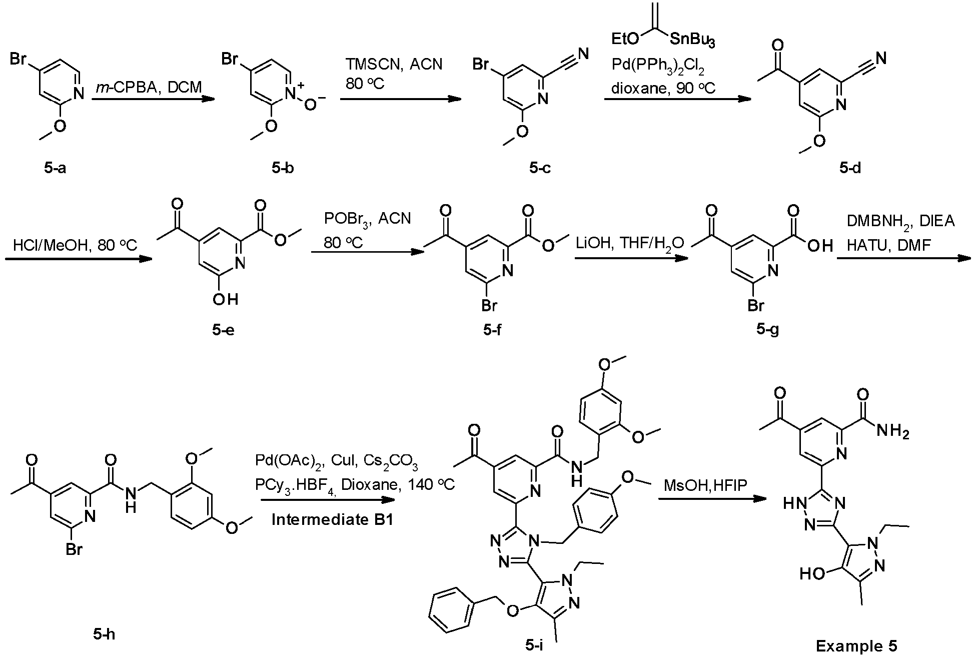

### Step 1: preparation of 4-bromo-2-methoxy-1-oxido-pyridin-1-ium (compound 5-b)

To a solution of 4-bromo-2-methoxypyridine **(5-a,** 25.0 g, 132.96 mmol) in DCM (400 mL) was added *m*-CPBA (32.39 g, 159.56 mmol) in portions, the mixture was stirred at r.t. for 12 hrs. Then the mixture was purified by column chromatography to give **compound 5-b** (16 g). LCMS (M+H)⁺: 204.

### Step 2: preparation of 4-bromo-6-methoxy-pyridine-2-carbonitrile (compound 5-c)

To a solution of 4-bromo-2-methoxy-1-oxido-pyridin-1-ium **(5-b,** 16.0 g, 78.42 mmol) in ACN (150 mL) was added TEA (15.84 g, 156.85 mmol) and TMSCN (23.34 g, 235.27 mmol) in portions, the mixture was stirred at 80 °C for 12 hrs under N₂. Then the mixture was concentrated, the residue was poured into sat. Na₂CO₃ solution (200 mL), extracted with EA (400 mL×2). The combined organic phase was washed with brine, dried over anhydrous Na₂SO₄ and concentrated. The residue was purified by column chromatography to give **compound 5-c** (12 g), LCMS (M+H)⁺: 213.

### Step 3: preparation of 4-acetyl-6-methoxy-pyridine-2-carbonitrile (compound 5-d)

To a solution of 4-bromo-6-methoxy-pyridine-2-carbonitrile **(5-c,** 3 g, 14.08 mmol) in 1,4-dioxane (30 mL) was added tributyl(1-ethoxyvinyl)tin (5.23 g, 14.48 mmol) and Pd(PPh₃)₂Cl₂ (988.5 mg, 1.41 mmol) under N₂. The mixture was stirred at 90 °C for 12 hrs. Then the mixture was quenched with KF solution (45 mL, 1 N). Then HCl solution (60 mL, 1 N) was added to the mixture and stirred at 50 °C for 0.5 h. The mixture was extracted with DCM (100 mL), the organic phase was washed with brine, dried over anhydrous Na₂SO₄ and concentrated. The residue was purified by column chromatography to give **compound 5-d** (1.8 g). LCMS (M+H)⁺: 177.

### Step 4: preparation of methyl 4-acetyl-6-hydroxy-pyridine-2-carboxylate (compound 5-e)

The solution of 4-acetyl-6-methoxy-pyridine-2-carbonitrile **(5-d,** 1 g, 5.68 mmol) in HCl/MeOH (30 mL, 4 N) was stirred at 80 °C for 12 hrs and then concentrated to give **compound 5-e** (1.1 g). LCMS (M+H)⁺: 196.

### Step 5: preparation of methyl 4-acetyl-6-bromo-pyridine-2-carboxylate (compound 5-f)

To a solution of methyl 4-acetyl-6-hydroxy-pyridine-2-carboxylate **(5-e,** 1.1 g, 5.64 mmol) in ACN (30 mL) was added POBr₃ (4.85 g, 16.91 mmol), the mixture was stirred at 80 °C for 2 hrs. Then the mixture was quenched with sat. Na₂CO₃ solution (50 mL), extracted with EA (50 mL×2). The combined organic phase was washed with brine, dried over anhydrous Na₂SO₄, and concentrated to give **compound 5-f** (1.2 g). LCMS (M+H)⁺: 258.

### Step 6: preparation of 4-acetyl-6-bromo-pyridine-2-carboxylic acid (compound 5-g)

The solution of methyl 4-acetyl-6-bromo-pyridine-2-carboxylate **(5-f,** 1.2 g, 4.65 mmol) in THF (10 mL) and water (3 mL) was added LiOH.H₂O (780.5 mg, 18.6 mmol), the mixture was stirred at r.t. for 2 hrs. Then the mixture was diluted with water (50 mL), HCl solution (1 N) was added to adjust to pH~5, the mixture was extracted with EA (50 mL×2). The combined organic phase was washed with brine, dried over anhydrous Na₂SO₄, and concentrated to give **compound 5-g** (900 mg). LCMS (M+H)⁺: 244.

### Step 7: preparation of 4-acetyl-6-bromo-N-[(2,4-dimethoxyphenyl)methyl]pyridine-2-carboxamide (compound 5-h)

To a solution of 4-acetyl-6-bromo-pyridine-2-carboxylic acid **(5-g,** 850.0 mg, 3.48 mmol) in DMF (10 mL) was added DMBNH₂ (0.58 mL, 3.83 mmol), DIEA (1.348 g, 10.45 mmol) and HATU (1.588 g, 4.18 mmol), the mixture was stirred at r.t. for 2 hrs. Then the mixture was poured into water (50 mL) and extracted with EA (50 mL×2). The combined organic phase was washed with brine, dried over anhydrous Na₂SO₄, and concentrated. The residue was purified by column chromatography to give **compound 5-h** (670 mg). LCMS (M+H)⁺: 393.

### Step 8: preparation of 4-acetyl-6-[5-(4-benzyloxy-2-ethyl-5-methyl-pyrazol-3-yl)-4-[(4-methoxyphenyl)methyl]-1,2,4-triazol-3-yl]-N-[(2,4-dimethoxyphenyl)methyl]pyridine-2-carboxamide (compound 5-i)

To a solution of 4-acetyl-6-bromo-N-[(2,4-dimethoxyphenyl)methyl]pyridine-2-carboxamide **(5-h,** 150.0 mg, 0.38 mmol) in 1,4-dioxane (3 mL) was added 3-(4-benzyloxy-2-ethyl-5-methyl-pyrazol-3-yl)-4-[(4-methoxyphenyl)methyl]-1,2,4-triazole **(Intermediate B1,** 153.9 mg, 0.38 mmol), Pd(OAc)₂ (25.7 mg, 0.11 mmol), CuI (145.3 mg, 0.76 mmol), PCy₃HBF₄ (56.2 mg, 0.15 mmol) and Cs₂CO₃ (124.0 mg, 0.38 mmol), the mixture was stirred at 140 °C for 12 hrs. Then the mixture was filtered and concentrated. The residue was purified by prep-TLC to give **compound 5-i** (40 mg). LCMS (M+H)⁺: 716.

### Step 9: preparation of 4-acetyl-6-[3-(2-ethyl-4-hydroxy-5-methyl-pyrazol-3-yl)-1H-1,2,4-triazol-5-yl]pyridine-2-carboxamide (Example 5)

To a solution of 4-acetyl-6-[5-(4-benzyloxy-2-ethyl-5-methyl-pyrazol-3-yl)-4-[(4-methoxyphenyl)methyl]-1,2,4-triazol-3-yl]-N-[(2,4-dimethoxyphenyl)methyl]pyridine-2-carboxamide **(5-i,** 40 mg, 0.06 mmol) in HFIP (1.0 mL) was added MsOH (53.7 mg, 0.56 mmol), the mixture was stirred at r.t. for 1 h. Then the mixture was purified by prep-HPLC to give **Example 5** (11.8 mg), LCMS (M+H)⁺: 356. ¹H NMR (400 MHz, DMSO-*d₆*) δ = 15.4-15.0 (m, 1H), 8.8 - 8.7 (m, 1H), 8.65 (d, *J* = 1.2 Hz, 1H), 8.46 (d, *J* = 1.6 Hz, 1H), 8.01 (br s, 1H), 4.41 (q, *J= 6.8* Hz, 2H), 2.77 (s, 3H), 2.13 (s, 3H), 1.31 (t, *J* = 7.2 Hz, 3H).

### Example 6

### 6-[3-(2-ethyl-4-hydroxy-5-methyl-pyrazol-3-yl)-1H-1,2,4-triazol-5-yl]-4-imidazol-1-yl-pyridine-2-carboxamide

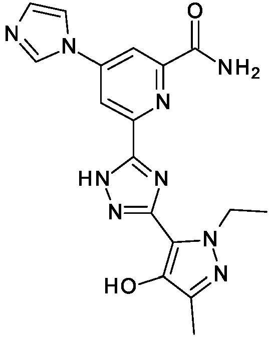

The title compound was prepared according to the following scheme:

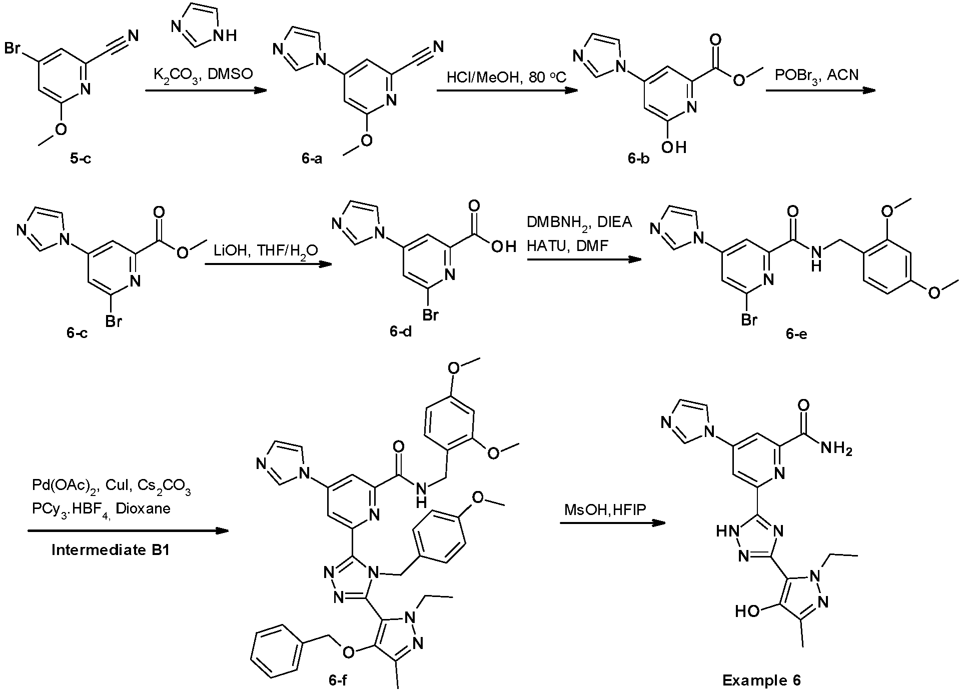

### Step 1: preparation of 4-imidazol-1-yl-6-methoxy-pyridine-2-carbonitrile (compound 6-a)

To a solution of 4-bromo-6-methoxy-pyridine-2-carbonitrile **(5-c,** 5.0 g, 23.47 mmol) in DMSO (100 mL) was added imidazole (2.4 g, 35.21 mmol) and K₂CO₃ (4.87 g, 35.21 mmol), the mixture was stirred at 100 °C for 16 hrs. Then the mixture was poured into H₂O (100 mL), and filtered, the filtered cake was dried to give **compound 6-a** (3.0 g, 14.99 mmol). LCMS (M+H)⁺: 201.

### Step 2~7: preparation of 6-[3-(2-ethyl-4-hydroxy-5-methyl-pyrazol-3-yl)-1H-1,2,4-triazol-5-yl]-4-imidazol-1-yl-pyridine-2-carboxamide (Example 6)

**Example 6** was prepared in analogy to the preparation of **Example 5** by using **compound 6-a** instead of 4-acetyl-6-methoxy-pyridine-2-carbonitrile **(compound 5-d). Example 6** (10.7 mg) was obtained. LCMS (M+H)⁺: 380. ¹H NMR (400 MHz, DMSO-*d₆*+D₂O) δ = 9.70 (br s, 1H), 8.77 (s, 1H), 8.52 (br s, 2H), 7.75 (br s, 1H), 4.43 (q, *J=* 7.1 Hz, 2H), 2.14 (s, 3H), 1.30 (t, *J* = 7.1 Hz, 3H).

### Example 7

### 4-[2-(2-ethyl-4-hydroxy-5-methyl-pyrazol-3-yl)pyrimidin-4-yl]-1-methyl-pyrazolo[4,3-c]pyridine-6-carboxamide

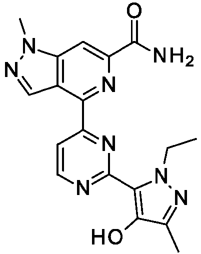

The title compound was prepared according to the following scheme:

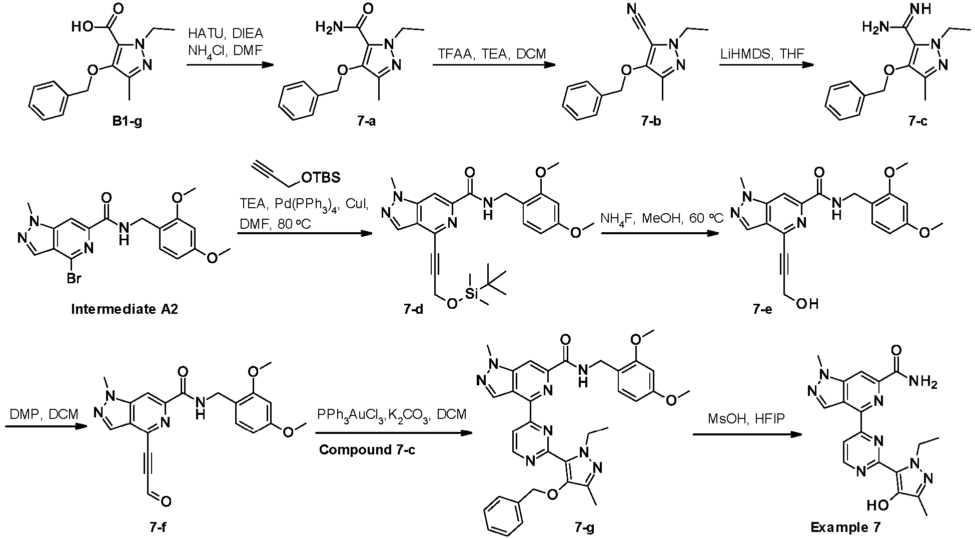

### Step 1: preparation of 4-benzyloxy-2-ethyl-5-methyl-pyrazole-3-carboxamide (compound 7-a)

To a solution of 4-benzyloxy-2-ethyl-5-methyl-pyrazole-3-carboxylic acid **(B1-g,** 5 g, 19.21 mmol) in DMF (50 mL) was added DIEA (7.448 g, 57.63 mmol), NH₄Cl (3.08 g, 57.63 mmol) and HATU (10.96 g, 28.81 mmol), the mixture was stirred at r.t. for 2 hrs. Then the mixture was concentrated. The residue was purified by column chromatography to give **compound 7-a** (2.5 g). LCMS (M+H)⁺: 260.

### Step 2: preparation of 4-benzyloxy-2-ethyl-5-methyl-pyrazole-3-carbonitrile (compound 7-b)

To a solution of 4-benzyloxy-2-ethyl-5-methyl-pyrazole-3-carboxamide **(7-a,** 2.5 g, 9.64 mmol) in DCM (50 mL) was added TEA (2.95 g, 28.92 mmol), the mixture was cooled to -5 °C. Then the solution of TFAA (2.72 mL, 19.28 mmol) in DCM (10 mL) was added into dropwise. Then the mixture was stirred at r.t. for 1 h, then quenched by sat. NaHCO₃ solution (800 mL). The mixture was extracted with EA (450 mL×2). The combined organic phase was washed with brine, dried over anhydrous Na₂SO₄, and concentrated. The residue was purified by column chromatography to give **compound 7-b** (1.5 g). LCMS (M+H)⁺: 242.

### Step 3: preparation of 4-benzyloxy-2-ethyl-5-methyl-pyrazole-3-carboxamidine (compound 7-c)

To a suspension of 4-benzyloxy-2-ethyl-5-methyl-pyrazole-3-carbonitrile **(compound 7-b,** 1.0 g, 4.14 mmol) in THF (10 mL) was added LiHMDS (1 M in THF solution, 12.43 mL, 12.43 mmol), the mixture was stirred at 30 °C for 48 hrs. Then HCl solution (3 N) was added to adjust to pH~3, the mixture was stirred at 0 °C for 1 h. Then the mixture was washed with EA. The aqueous phase was adjusted to pH~14 with NaOH solution (3 N), and extracted with EA (900 mL×3). The combined organic phase was washed with brine, dried over anhydrous Na₂SO₄, and concentrated to give **compound 7-c** (1.2 g). LCMS (M+H)⁺: 259.

### Step 4: preparation of 4-[3-[tert-butyl(dimethyl)silyl]oxyprop-1-ynyl]-N-[(2,4-dimethoxyphenyl)methyl]-1-methyl-pyrazolo[4,3-c]pyridine-6-carboxamide (compound 7-d)

To a solution of 4-bromo-N-[(2,4-dimethoxyphenyl)methyl]-1-methyl-pyrazolo[4,3-c]-pyridine-6-carboxamide **(Intermediate A2,** 200 mg, 0.49 mmol) in DMF (5 mL) was added TEA (149.8 mg, 1.48 mmol), Pd(PPh₃)₄ (57.0 mg, 0.05 mmol), tert-butyldimethyl(2-propynyloxy)silane (252.2 mg, 1.48 mmol) and CuI (18.8 mg, 0.1 mmol), the mixture was stirred at 80 °C for 16 hrs under N₂. Then the mixture was poured into water (300 mL), extracted with EA (100 mL×3). The combined organic phase was washed with sat. NaHCO₃ solution and brine, then dried over anhydrous Na₂SO₄, and concentrated. The residue was purified by column chromatography to give **compound 7-d** (200 mg). LCMS (M+H)⁺: 495.

### Step 5: preparation of N-[(2,4-dimethoxyphenyl)methyl]-4-(3-hydroxyprop-1-ynyl)-1-methyl-pyrazolo[4,3-c]pyridine-6-carboxamide (compound 7-e)

To a solution of 4-[3-[*tert*-butyl(dimethyl)silyl]oxyprop-1-ynyl]-*N*-[(2,4-dimethoxyphenyl)-methyl]-1-methyl-pyrazolo[4,3-c]pyridine-6-carboxamide **(7-d,** 50.0 mg, 0.1 mmol) in MeOH (5 mL) was added NH₄F (0.6 g, 16.17 mmol), the mixture was stirred at 60 °C for 0.5 h under N₂. Then the mixture was filtered, and concentrated to give **compound 7-e** (50 mg). LCMS (M+H)⁺: 381.

### Step 6: preparation of N-[(2,4-dimethoxyphenyl)methyl]-1-methyl-4-(3-oxoprop-1-ynyl)pyrazolo[4,3-c]pyridine-6-carboxamide (compound 7-f)

To a solution of *N*-[(2,4-dimethoxyphenyl)methyl]-4-(3-hydroxyprop-1-ynyl)-1-methyl-pyrazolo[4,3-c]pyridine-6-carboxamide (7-e, 30 mg, 0.08 mmol) in DCM (2 mL) was added DMP (50.2 mg, 0.12 mmol), the mixture was stirred for at r.t for 0.5 h under N₂. Then the mixture was concentrated. The residue was purified by prep-TLC to give **compound 7-f** (28 mg).

### Step 7: preparation of 4-[2-(4-benzyloxy-2-ethyl-5-methyl-pyrazol-3-yl)pyrimidin-4-yl]-N-[(2,4-dimethoxyphenyl)methyl]-1-methyl-pyrazolo[4,3-c]pyridine-6-carboxamide (compound 7-g)

To a solution of 4-benzyloxy-2-ethyl-5-methyl-pyrazole-3-carboxamidine **(7-c,** 23.8 mg, 0.09 mmol) in DCM (20 mL) was added *N*-[(2,4-dimethoxyphenyl)methyl]-1-methyl-4-(3-oxoprop-1-ynyl)pyrazolo[4,3-c]pyridine-6-carboxamide (7-f, 28 mg, 0.08 mmol), PPh₃AuCl₃(39 mg, 0.1 mmol) and K₂CO₃ (21.2 mg, 0.15 mmol), the mixture was stirred for at r.t. for 1 h. Then the mixture was concentrated. The residue was purified by prep-TLC to give **compound 7-g** (45 mg). LCMS (M+H)⁺: 619.

### Step 8: preparation of 4-[2-(2-ethyl-4-hydroxy-5-methyl-pyrazol-3-yl)pyrimidin-4-yl]-1-methyl-pyrazolo[4,3-c]pyridine-6-carboxamide (Example 7)

To a solution of 4-[2-(4-benzyloxy-2-ethyl-5-methyl-pyrazol-3-yl)pyrimidin-4-yl]-*N*-[(2,4-dimethoxyphenyl)methyl]-1-methyl-pyrazolo[4,3-c]pyridine-6-carboxamide **(7-g,** 40 mg, 0.06 mmol) in HFIP (2.0 mL) was added MsOH (62.1 mg, 0.65 mmol), the mixture was stirred at r.t. for 1 h. Then the mixture was diluted with DMF (1mL) and purified by reversed-phase column chromatography to give **Example 7** (11.2 mg). LCMS (M+H)⁺: 379. ¹H NMR (400 MHz, DMSO-*d₆*) δ = 9.34 (br s, 1H), 9.17 - 9.08 (m, 2H), 8.79 (d, *J =* 5.4 Hz, 1H), 8.59 (br s, 1H), 8.54 (s, 1H), 7.87 (br s, 1H), 4.65 (q, *J=* 7.2 Hz, 2H), 4.23 (s, 3H), 2.19 (s, 3H), 1.31 (t, *J=* 7.2 Hz, 3H).

### Example 8

### 1-[2-(2-ethyl-4-hydroxy-5-methyl-pyrazol-3-yl)pyrimidin-4-yl]-6-methyl-imidazo[1,5-a]pyrazine-3-carboxamide

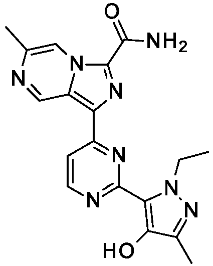

The title compound was prepared according to the following scheme:

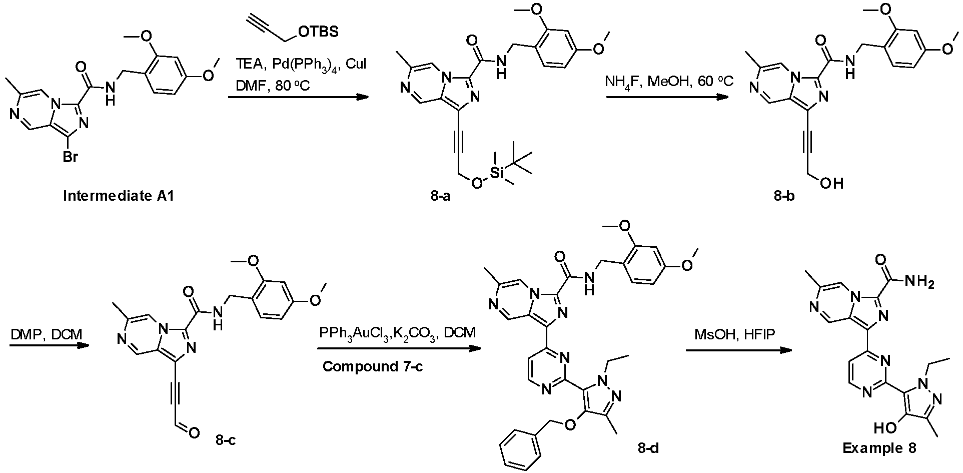

**Example 8** was prepared in analogy to the preparation of **Example 7** by using **Intermediate A1** instead of 4-bromo-N-[(2,4-dimethoxyphenyl)methyl]-1-methyl-pyrazolo[4,3-c]pyridine-6-carboxamide **(Intermediate A2). Example 8** (13.5 mg) was obtained. LCMS (M+H⁺): 379. ¹H NMR (400 MHz, DMSO-*d₆*+D₂O) δ = 9.85 (d, *J=* 1.6 Hz, 1H), 9.10 (s, 1H), 8.89 (d, *J* = 5.4 Hz, 1H), 8.03 (d, *J=* 5.4 Hz, 1H), 4.64 (q, *J =* 7.2 Hz, 2H), 2.48 (br s, 3H), 2.15 (s, 3H), 1.34 (t, *J =* 7.2 Hz, 3H).

### Example 9

### 1-[2-(2-ethyl-4-hydroxy-5-methyl-pyrazol-3-yl)oxazol-4-yl]-6-methyl-imidazo[1,5-a]pyrazine-3-carboxamide

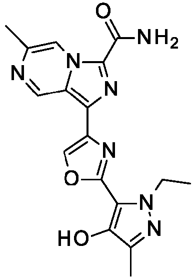

The title compound was prepared according to the following scheme:

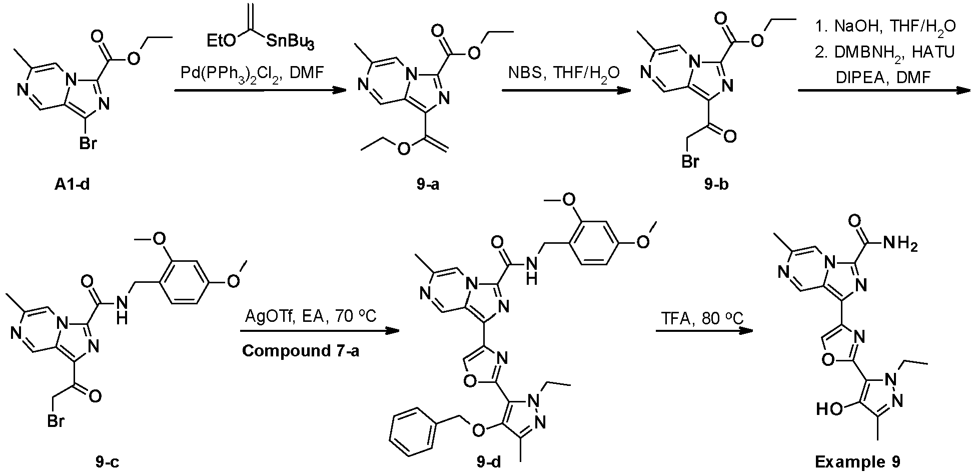

### Step 1: preparation of ethyl 1-(1-ethoxyvinyl)-6-methyl-imidazo[1,5-a]pyrazine-3-carboxylate (compound 9-a)

To a solution of ethyl 1-bromo-6-methyl-imidazo[1,5-a]pyrazine-3-carboxylate (**A1-d**, 600 mg, 2.11 mmol) and tributyl(1-ethoxyvinyl)tin (762.7 mg, 2.11 mmol) in DMF (8 mL) was added Pd(PPh₃)₂Cl₂.CH₂Cl₂ adduct (148.2 mg, 0.21 mmol), the mixture was stirred at 90 °C for 12 hrs under N₂. Then the mixture was quenched with KF solution (15 mL, 1 N), and filtered. The filtrate was extracted with DCM (100 mL), the organic phase was washed with brine, dried over anhydrous Na₂SO₄ and concentrated to give **compound 9a** (400 mg). LCMS (M+H)⁺: 276.

### Step 2: preparation of ethyl 1-(2-bromoacetyl)-6-methylimidazo[1,5-a]pyrazine-3-carboxylate (compound 9-b)

To a solution of ethyl 1-(1-ethoxyvinyl)-6-methylimidazo[1,5-a]pyrazine-3-carboxylate **(9-a,** 400 mg, 1.45 mmol) in THF (8 mL) and water (3 mL) was added NBS (258.6 mg, 1.45 mmol) at 0 °C, the mixture was stirred at r.t. for 1 h. Then the mixture was diluted with DCM (50 mL), poured into water (30 mL), and extracted with DCM (30 mL×2). The combined organic phase was washed with brine, dried over anhydrous Na₂SO₄, and concentrated, the residue was purified by column chromatography to give **compound 9-b** (250 mg). LCMS (M+H)⁺: 326.

### Step 3: preparation of 1-(2-bromoacetyl)-N-[(2,4-dimethoxyphenyl)methyl]-6-methyl-imidazo[1,5-a]pyrazine-3-carboxamide (compound 9-c)

To a solution of ethyl 1-(2-bromoacetyl)-6-methylimidazo[1,5-a]pyrazine-3-carboxylate **(9-b,** 580 mg, 1.78 mmol) in THF (10 mL) and water (3 mL) was added NaOH (355.7 mg, 8.89 mmol), the mixture was stirred at r.t. for 4 hrs. Then the mixture was concentrated, the residue was dissolved in DMF (5 mL), then DMBNH₂ (0.28 mL, 1.87 mmol), DIEA (689.5 mg, 5.33 mmol) and HATU (627.6 mg, 2.67 mmol) were added. The mixture was stirred at r.t. for 12 hrs. Then the mixture was diluted with DCM (100 mL), poured into water (60 mL), and extracted with DCM (100 mL×2). The combined organic phase was washed with brine, dried over anhydrous Na₂SO₄, and concentrated, the residue was purified by column chromatography to give **compound 9-c** (350 mg). LCMS (M+H)⁺: 447.

### Step 4: preparation of 1-[2-(4-benzyloxy-2-ethyl-5-methyl-pyrazol-3-yl)oxazol-4-yl]-N-[(2,4-dimethoxyphenyl)methyl]-6-methyl-imidazo[1,5-a]pyrazine-3-carboxamide (compound 9-d)

To a solution of 1-(2-bromoacetyl)-N-[(2,4-dimethoxyphenyl)methyl]-6-methyl-imidazo[1,5-a]pyrazine-3-carboxamide **(9-c,** 300 mg, 0.67 mmol) in EA (15 mL) was added silver trifluoromethanesulfonate (258.5 mg, 1.01 mmol) and 4-benzyloxy-2-ethyl-5-methylpyrazole-3-carboxamide **(7-a,** 260.9 mg, 1.01 mmol), the mixture was stirred at 70 °C for 12 hrs in the dark under N₂. Then the mixture was concentrated, the residue was purified by reversed-phase column chromatography to give **compound 9-d** (100 mg), LCMS (M+H)⁺: 608.

### Step 5: preparation of 1-[2-(2-ethyl-4-hydroxy-5-methyl-pyrazol-3-yl)oxazol-4-yl]-6-methyl-imidazo[1,5-a]pyrazine-3-carboxamide (Example 9)

The solution of 1-[2-(4-benzyloxy-2-ethyl-5-methyl-pyrazol-3-yl)oxazol-4-yl]-N-[(2,4-dimethoxyphenyl)methyl]-6-methyl-imidazo[1,5-a]pyrazine-3-carboxamide **(9-d,** 98 mg, 0.16 mmol) in TFA (2 mL) was stirred at 80 °C for 4 hrs. The mixture was purified by reversed-phase column chromatography and prep-TLC to give **Example 9** (3.6 mg). LCMS (M+H)⁺: 368. ¹H NMR (400 MHz, DMSO-*d₆*) δ = 9.64 (s, 1H), 9.05 (s, 1H), 8.79 (s, 1H), 8.63 (s, 1H), 8.05 (br d, *J=* 1.5 Hz, 1H), 7.86 (s, 1H), 4.55 - 4.49 (m, 2H), 2.46 (br s, 3H), 2.15 (s, 3H), 1.39 (t, *J=* 7.0 Hz, 3H).

### Example 10

### 4-[5-(difluoromethyl)-2-(2-ethyl-4-hydroxy-5-methyl-pyrazol-3-yl)oxazol-4-yl]-1-methyl-pyrazolo[4,3-c]pyridine-6-carboxamide

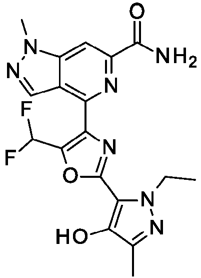

The title compound was prepared according to the following scheme:

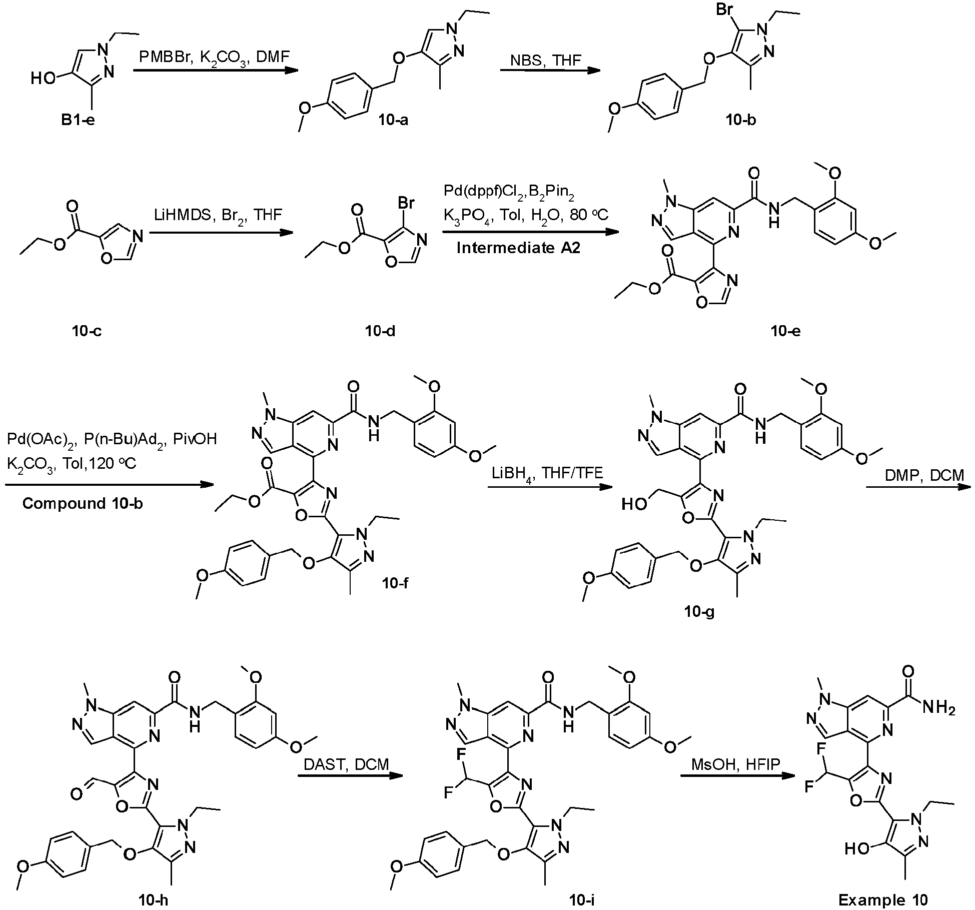

### Step 1: preparation of 1-ethyl-4-[(4-methoxyphenyl)methoxy]-3-methyl-pyrazole (compound 10-a)

To a solution of 1-ethyl-3-methyl-pyrazol-4-ol **(B1-e,** 25.0 g, 198.16 mmol) in DMF (300 mL) was added K₂CO₃ (54.7 g, 396.32 mmol) and PMBBr (59.76 g, 297.24 mmol), the mixture was stirred at r.t. for 16 hrs. Then the mixture was poured into water (300 mL) and extracted with EA (300 mL×3). The combined organic phase was washed with sat. NaHCO₃ solution and brine, dried over anhydrous Na₂SO₄ and concentrated. The residue was purified by flash chromatography to give **compound 10-a** (10 g). LCMS (M+H)⁺: 247.

### Step 2: preparation of 5-bromo-1-ethyl-4-[(4-methoxyphenyl)methoxy]-3-methyl-pyrazole (compound 10-b)

To a solution of 1-ethyl-4-[(4-methoxyphenyl)methoxy]-3-methyl-pyrazole **(10-a,** 1.0 g, 4.06 mmol) in THF (10 mL) was added NBS (794.9 mg, 4.47 mmol), the mixture was stirred at r.t. for 3 hrs. Then the mixture was concentrated, the residue was purified by flash column chromatography to give **compound 10-b** (1.11 g). LCMS (M+H)⁺: 325.

### Step 3: preparation of ethyl 4-bromooxazole-5-carboxylate (Compound 10-d)

To a solution of ethyl oxazole-5-carboxylate **(10-c,** 20 g, 141.72 mmol) in THF (200 mL) and DMF (200 mL) was added LiHMDS (184.24 mL, 184.24 mmol) at -70 °C. After stirring for 2 hrs, Br₂ (29.44 g, 184.24 mmol) was added dropwise. The mixture was stirred at - 70 °C for 2 hrs. Then the mixture was poured into sat. NH₄Cl solution (600 mL), extracted with EA (500 mL×2). The combined organic phase was washed with brine, dried over anhydrous Na₂SO₄, and concentrated. The residue was purified by flash chromatography to give **Compound 10-d** (10 g), LCMS (M+H)⁺: 220.

### Step 4: preparation of ethyl 4-[6-[(2,4-dimethoxyphenyl)methylcarbamoyl]-1-methyl-pyrazolo[4,3-c]pyridin-4-yl]oxazole-5-carboxylate (Compound 10-e)

To a solution of ethyl 4-bromooxazole-5-carboxylate **(10-d,** 2.99 g, 13.57 mmol), 4-bromo-N-[(2,4-dimethoxyphenyl)methyl]-1-methyl-pyrazolo[4,3-c]pyridine-6-carboxamide **(Intermediate A2,** 5.0 g, 12.34 mmol) in toluene (100 mL) and water (25 mL) was added K₃PO₄ (7.86 g, 37.01 mmol), B₂Pin₂ (6.27 g, 24.68 mmol), and Pd(dppf)Cl₂ (1.81 g, 2.47 mmol), the mixture was stirred at 80 °C for 5 hrs under N₂. Then the mixture was concentrated, the residue was purified by flash chromatography to give **Compound 10-e** (860 mg). LCMS (M+H)⁺: 466.

### Step 5: preparation of ethyl 4-[6-[(2,4-dimethoxyphenyl)methylcarbamoyl]-1-methyl-pyrazolo[4,3-c]pyridin-4-yl]-2-[2-ethyl-4-[(4-methoxyphenyl)methoxy]-5-methyl-pyrazol-3-yl]oxazole-5-carboxylate (Compound 10-f)

To a solution of ethyl 4-[6-[(2,4-dimethoxyphenyl)methylcarbamoyl]-1-methyl-pyrazolo-[4,3-c]pyridin-4-yl]oxazole-5-carboxylate **(10-e,** 850 mg, 1.83 mmol), 5-bromo-1-ethyl-4-[(4-methoxyphenyl)methoxy]-3-methyl-pyrazole **(10-b,** 593.9 mg, 1.83 mmol) in toluene (20 mL) was added K₂CO₃ (756.0 mg, 5.48 mmol), cataCXium A (327.3 mg, 0.91 mmol), PivOH (93.1 mg, 0.91 mmol) and Pd(OAc)₂ (123.0 mg, 0.55 mmol), the mixture was stirred at 120 °C for 18 hrs under N₂. Then the mixture was filtered, the filtered cake was washed with DCM/MeOH (V/V=20/1). The residue was purified by column chromatography to give **Compound 10-f (800** mg), LCMS (M+H)⁺: 710.

### Step 6: preparation of N-[(2,4-dimethoxyphenyl)methyl]-4-[2-[2-ethyl-4-[(4-methoxyphenyl)methoxy]-5-methyl-pyrazol-3-yl]-5-(hydroxymethyl)oxazol-4-yl]-1-methyl-pyrazolo[4,3-c]pyridine-6-carboxamide (Compound 10-g)

To a solution of ethyl 4-[6-[(2,4-dimethoxyphenyl)methylcarbamoyl]-1-methyl-pyrazolo[4,3-c]pyridin-4-yl]-2-[2-ethyl-4-[(4-methoxyphenyl)methoxy]-5-methyl-pyrazol-3-yl]-oxazole-5-carboxylate **(10-f,** 800 mg, 1.13 mmol) in THF (20 mL) and TFE (2.0 mL) was added LiBH₄ (2.82 mL, 11.27 mmol) at 0 °C, the mixture was stirred at 15 °C for 4 hrs under N₂. Then the mixture was poured into sat. NH₄Cl solution (20 mL), extracted with DCM (50 mL×2). The combined organic phase was washed with brine, dried over anhydrous Na₂SO₄, and concentrated. The residue was purified by flash chromatography to give **Compound 10-g** (550 mg), LCMS (M+H)⁺: 668.

### Step 7: preparation of N-[(2,4-dimethoxyphenyl)methyl]-4-[2-[2-ethyl-4-[(4-methoxyphenyl)methoxy]-5-methyl-pyrazol-3-yl]-5-formyl-oxazol-4-yl]-1-methyl-pyrazolo[4,3-c]pyridine-6-carboxamide (Compound 10-h)

To a solution of *N*-[(2,4-dimethoxyphenyl)methyl]-4-[2-[2-ethyl-4-[(4-methoxyphenyl)-methoxy]-5-methyl-pyrazol-3-yl]-5-(hydroxymethyl)oxazol-4-yl]-1-methyl-pyrazolo[4,3-c]-pyridine-6-carboxamide **(10-g,** 500 mg, 0.75 mmol) in DCM (10 mL) was added DMP (635.2 mg, 1.5 mmol), the mixture was stirred at r.t. for 1 h under N₂. Then the mixture was filtered, the filtered cake was washed with DCM/MeOH (V/V=20/1), the filtrate was concentrated. The residue was purified by column chromatography to give **Compound 10-h** (250 mg), LCMS (M+H)⁺: 666.

### Step 8: preparation of 4-[5-(difluoromethyl)-2-[2-ethyl-4-[(4-methoxyphenyl)-methoxy]-5-methyl-pyrazol-3-yl]oxazol-4-yl]-N-[(2,4-dimethoxyphenyl)-methyl]-1-methyl-pyrazolo[4,3-c]pyridine-6-carboxamide (Compound 10-i)

To a solution of DAST (3.0 mL, 29.42 mmol) in DCM (3 mL) was added the suspension of *N*-[(2,4-dimethoxyphenyl)methyl]-4-[2-[2-ethyl-4-[(4-methoxyphenyl)methoxy]-5-methyl-pyrazol-3-yl]-5-formyl-oxazol-4-yl]-1-methyl-pyrazolo[4,3-c]pyridine-6-carboxamide **(10-h,** 70 mg, 0.11 mmol ) in DCM (6 mL) dropwise at 0 °C, the mixture was stirred at 0 °C for 8 hrs. Then the mixture was poured into sat. NaHCO₃ solution (20 mL), extracted with DCM (20 mL×2). The combined organic phase was washed with brine, dried over anhydrous Na₂SO₄, and concentrated. The residue was purified by prep-TLC to give **Compound 10-i** (20 mg), LCMS (M+H)⁺: 688.

### Step 9: preparation of 4-[5-(difluoromethyl)-2-(2-ethyl-4-hydroxy-5-methyl-pyrazol-3-yl)oxazol-4-yl]-1-methyl-pyrazolo[4,3-c]pyridine-6-carboxamide (Example 10)

To a solution of 4-[5-(difluoromethyl)-2-[2-ethyl-4-[(4-methoxyphenyl)methoxy]-5-methyl-pyrazol-3-yl]oxazol-4-yl]-*N*-[(2,4-dimethoxyphenyl)-methyl]-1-methyl-pyrazolo[4,3-c]pyridine-6-carboxamide **(10-i,** 20.0 mg, 0.03 mmol) in HFIP (0.5 mL) was added MsOH (27.9 mg, 0.29 mmol), the mixture was stirred at r.t. for 0.5 h. Then the mixture was diluted with MeOH (1 mL), and purified by reversed-phase column chromatography to give **Example 10** (6.5 mg). LCMS (M+H)⁺: 418. ¹H NMR (400 MHz, DMSO-*d₆*) δ = 9.07 (s, 1H), 8.79 (s, 1H), 8.43 (s, 1H), 7.87 - 8.26 (m, 2H), 4.56 (q, *J =* 7.05 Hz, 2H), 4.21 (s, 3H), 2.18 (s, 3H), 1.42 (t, *J* = 7.09 Hz, 3H).

### Example 11

### 1-[5-(aminomethyl)-2-(2-ethyl-4-hydroxy-5-methyl-pyrazol-3-yl)oxazol-4-yl]-6-methyl-imidazo[1,5-a]pyrazine-3-carboxamide

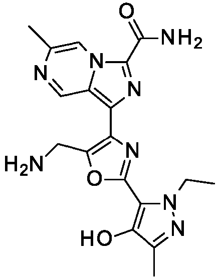

The title compound was prepared according to the following scheme:

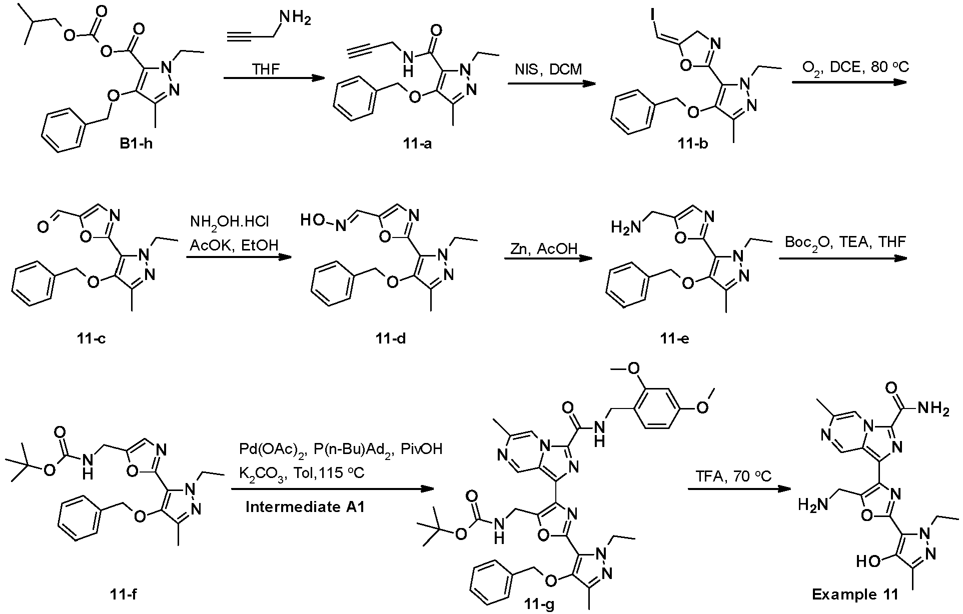

### Step 1: preparation of 4-benzyloxy-2-ethyl-5-methyl-N-prop-2-ynyl-pyrazole-3-carboxamide (Compound 11-a)

To a solution of isobutoxycarbonyl 4-benzyloxy-2-ethyl-5-methyl-pyrazole-3-carboxylate **(B1-h,** 7.0 g, 19.42 mmol) in THF (70 mL) was added propargylamine (1.65 g, 29.92 mmol) dropwise at 0 °C, the mixture was stirred at r.t. for 12 hrs. Then the mixture was concentrated, the residue was purified by column chromatography to give **Compound 11-a** (2.4 g). LCMS (M+H)⁺: 298.

### Step 2: preparation of (5E)-2-(4-benzyloxy-2-ethyl-5-methyl-pyrazol-3-yl)-5-(iodomethylene)-4H-oxazole (Compound 11-b)

To a solution of 4-benzyloxy-2-ethyl-5-methyl-N-prop-2-ynyl-pyrazole-3-carboxamide **(11-a,** 2.4 g, 8.07 mmol) in DCM (35 mL) was added NIS (2.72 g, 12.11 mmol), the mixture was stirred at r.t. for 4 hrs. Then the mixture was concentrated to give **Compound 11-b** (3.0 g), LCMS (M+H)⁺: 424.

### Step 3: preparation of 2-(4-benzyloxy-2-ethyl-5-methyl-pyrazol-3-yl)oxazole-5-carbaldehyde (Compound 11-c)

The solution of (5E)-2-(4-benzyloxy-2-ethyl-5-methyl-pyrazol-3-yl)-5-(iodomethylene)-4H-oxazole **(11-b,** 2.8 g, 6.62 mmol) in DCE (15 mL) was stirred at 80 °C for 12 hrs under O₂. Then EA (100 mL) was added, the mixture was washed with sat. Na₂SO₃ solution and brine. The organic phase was dried over anhydrous Na₂SO₄ and concentrated. The residue was purified by column chromatography to give **Compound 11-c** (1.0 g), LCMS (M+H)⁺: 312.

### Step 4: preparation of (5E)-2-(4-benzyloxy-2-ethyl-5-methyl-pyrazol-3-yl)oxazole-5-carbaldehyde oxime (Compound 11-d)

To a solution of 2-(4-benzyloxy-2-ethyl-5-methyl-pyrazol-3-yl)oxazole-5-carbaldehyde **(11-c,** 900 mg, 2.89 mmol) in EtOH (2 mL) was added NH₂OH.HCl (301.3 mg, 4.34 mmol) and KOAc (474.1 mg, 5.78 mmol), the mixture was stirred at r.t. for 3 hrs. The mixture was concentrated (< 30 °C), the residue was dissolved in water (30 mL), then extracted with DCM (60 mL×2). The combined organic phase was concentrated to give **Compound 11-d** (800 mg), LCMS (M+H)⁺: 327.

### Step 5: preparation of [2-(4-benzyloxy-2-ethyl-5-methyl-pyrazol-3-yl)oxazol-5-yl]methanamine (Compound 11-e)

To a solution of (5*E*)-2-(4-benzyloxy-2-ethyl-5-methyl-pyrazol-3-yl)oxazole-5-carbaldehyde oxime **(11-d,** 800 mg, 2.45 mmol) in AcOH (20 mL) was added Zn (2.745 g, 49.03 mmol) in portions. The mixture was stirred at r.t. for 16 hrs under N₂. EA (400 mL) was added to the mixture, then sat. Na₂CO₃ solution was added to adjust to pH >7. The mixture was extracted with EA (300 mL×2). The combined organic phase was concentrated to give **Compound 11-e** (600 mg). LCMS (M+H)⁺: 313.

### Step 6: preparation of tert-butyl N-[[2-(4-benzyloxy-2-ethyl-5-methyl-pyrazol-3-yl)oxazol-5-yl]methyl]carbamate (Compound 11-f)

To a solution of [2-(4-benzyloxy-2-ethyl-5-methyl-pyrazol-3-yl)oxazol-5-yl]methanamine **(11-e,** 600 mg, 1.92 mmol) in THF (15 mL) was added Boc₂O (628 mg, 2.88 mmol) and TEA (582 mg, 5.76 mmol), the mixture was stirred at r.t. for 16 hrs. Then the mixture was poured into water (50 mL) and extracted with EA (80 mL×3). The combined organic phase was washed with sat. NaHCO₃ solution and brine, dried over anhydrous Na₂SO₄ and concentrated, the residue was purified by column chromatography to give **Compound 11-f** (660 mg), LCMS (M+H)⁺: 413.

### Step 7: preparation of tert-butyl N-[[2-(4-benzyloxy-2-ethyl-5-methyl-pyrazol-3-yl)-4-[3-[(2,4-dimethoxyphenyl)methylcarbamoyl]-6-methyl-imidazo[1,5-a]pyrazin-1-yl]oxazol-5-yl]methyl]carbamate (Compound 11-g)

The solution of *tert*-butyl N-[[2-(4-benzyloxy-2-ethyl-5-methyl-pyrazol-3-yl)oxazol-5-yl]methyl]carbamate **(11-f,** 560 mg, 1.36 mmol), K₂CO₃ (562.1 mg, 4.07 mmol), Pd(OAc)₂ (91.4 mg, 0.41 mmol), PivOH (83.1 mg, 0.81 mmol), cataCXium A (146.0 mg, 0.41 mmol), 1-bromo-*N*-[(2,4-dimethoxyphenyl)methyl]-6-methyl-imidazo[1,5-a]pyrazine-3-carboxamide **(Intermediate A1,** 550.2 mg, 1.36 mmol) in toluene (25 mL) was stirred at 115 °C for 12 hrs. DCM (100 mL) was added, then the mixture was filtered, the filtrate was concentrated, the residue was purified by column chromatography and then prep-TLC to give **Compound 11-g** (27 mg), LCMS (M+H)⁺: 737.

### Step 8: preparation of 1-[5-(aminomethyl)-2-(2-ethyl-4-hydroxy-5-methyl-pyrazol-3-yl)oxazol-4-yl]-6-methyl-imidazo[1,5-a]pyrazine-3-carboxamide (Example 11)

The solution of *tert*-butyl N-[[2-(4-benzyloxy-2-ethyl-5-methyl-pyrazol-3-yl)-4-[3-[(2,4-dimethoxyphenyl)methylcarbamoyl]-6-methyl-imidazo[1,5-a]pyrazin-1-yl]oxazol-5-yl]methyl]-carbamate **(Compound 11-g,** 27 mg, 0.04 mmol) in TFA (2.09 g, 18.32 mmol) was stirred at 70 °C for 2 hrs. The mixture was purified by pre-HPLC to give Example **11** (4.7 mg), LCMS (M+H)⁺: 397. ¹H NMR (400 MHz, DMSO-*d₆*+D₂O) δ = 9.59 (d, *J=* 1.1 Hz, 1H), 8.99 (s, 1H), 4.72 (s, 2H), 4.48 (q, *J=* 6.8 Hz, 2H), 2.43 (s, 3H), 2.13 (s, 3H), 1.36 (t, *J=* 7.2 Hz, 3H).

### Example 12

### 4-(6-carbamoyl-1-methyl-pyrazolo[4,3-c]pyridin-4-yl)-2-(2-ethyl-4-hydroxy-5-methyl-pyrazol-3-yl)oxazole-5-carboxamide

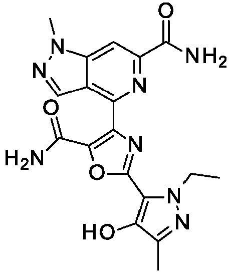

The title compound was prepared according to the following scheme:

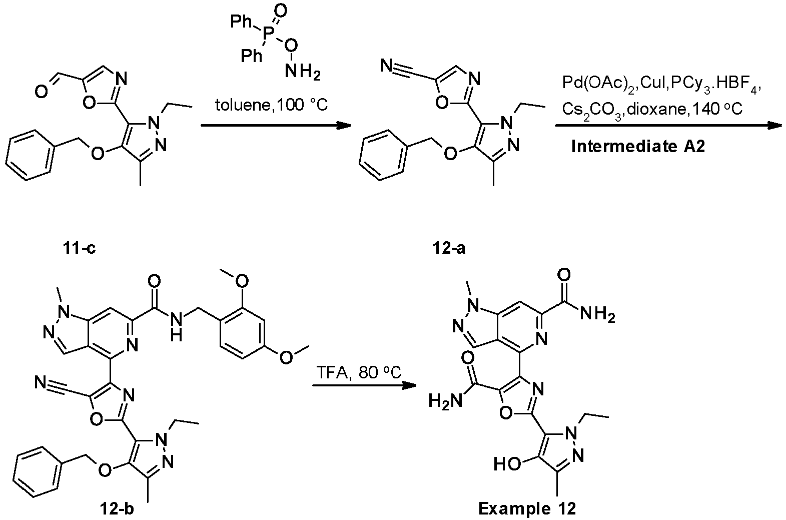

### Step 1: preparation of 2-(4-benzyloxy-2-ethyl-5-methyl-pyrazol-3-yl)oxazole-5-carbonitrile (Compound 12-a)

To a solution of 2-(4-benzyloxy-2-ethyl-5-methyl-pyrazol-3-yl)oxazole-5-carbaldehyde **(11-c,** 3.5 g, 11.24 mmol) in toluene (150 mL) was added *O*-diphenylphosphorylhydroxylamine (3.93 g, 16.86 mmol), the mixture was stirred at 100 °C for 2 hrs. Then the mixture was concentrated. The residue was purified by prep-HPLC to give **Compound 12-a** (1.2 g), LCMS (M+H)⁺: 309.

### Step 2: preparation of 4-[2-(4-benzyloxy-2-ethyl-5-methyl-pyrazol-3-yl)-5-cyano-oxazol-4-yl]-N-[(2,4-dimethoxyphenyl)methyl]-1-methyl-pyrazolo[4,3-c]pyridine-6-carboxamide (Compound 12-b)

The solution of 4-bromo-N-[(2,4-dimethoxyphenyl)methyl]-1-methyl-pyrazolo[4,3-c]pyridine-6-carboxamide **(Intermediate A2,** 262.9 mg, 0.65 mmol), 2-(4-benzyloxy-2-ethyl-5-methyl-pyrazol-3-yl)oxazole-5-carbonitrile **(12-a,** 200 mg, 0.65 mmol), Pd(OAc)₂ (43.7 mg, 0.19 mmol), CuI (247.1 mg, 1.3 mmol), PCy_{3.}HBF₄ (95.5 mg, 0.26 mmol), Cs₂CO₃ (2.108 g, 0.65 mmol) in 1,4-dioxane (4 mL) was stirred at 140 °C for 18 hrs. Then the mixture was concentrated. The residue was purified by prep-HPLC to give **Compound 12-b** (70 mg). LCMS (M+H)⁺: 633.

### Step 3: preparation of 4-(6-carbamoyl-1-methyl-pyrazolo[4,3-c]pyridin-4-yl)-2-(2-ethyl-4-hydroxy-5-methyl-pyrazol-3-yl)oxazole-5-carboxamide (Example 12)

The solution of 4-[2-(4-benzyloxy-2-ethyl-5-methyl-pyrazol-3-yl)-5-cyano-oxazol-4-yl]-N-[(2,4-dimethoxyphenyl)methyl]-1-methyl-pyrazolo[4,3-c]pyridine-6-carboxamide **(12-b,** 10 mg, 0.02 mmol) in TFA (2.0 mL) was stirred at 80 °C for 0.5 h. Then the mixture was concentrated, the residue was purified by prep-HPLC to give **Example 12** (1.6 mg), LCMS (M+H)⁺: 411. ¹H NMR (400 MHz, DMSO-*d₆*) δ = 10.12 (br s, 1H), 8.78 (s, 1H), 8.40 (s, 1H), 8.39 - 8.38 (m, 1H), 8.37 (br s, 1H), 8.14 (br s, 1H), 7.83 (br s, 1H), 4.55 (br d, *J=* 6.8 Hz, 2H), 4.20 (s, 3H), 2.11 (s, 3H), 1.38 (t, *J* = 7.1 Hz, 3H).

### Example 13

### 4-[3-(2-ethyl-5,6-dihydro-4H-cyclopenta[c]pyrazol-3-yl)-1H-1,2,4-triazol-5-yl]-1-methyl-pyrazolo[4,3-c]pyridine-6-carboxamide

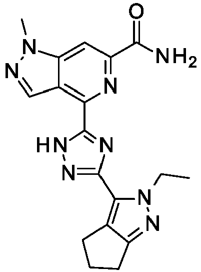

The title compound was prepared according to the following scheme:

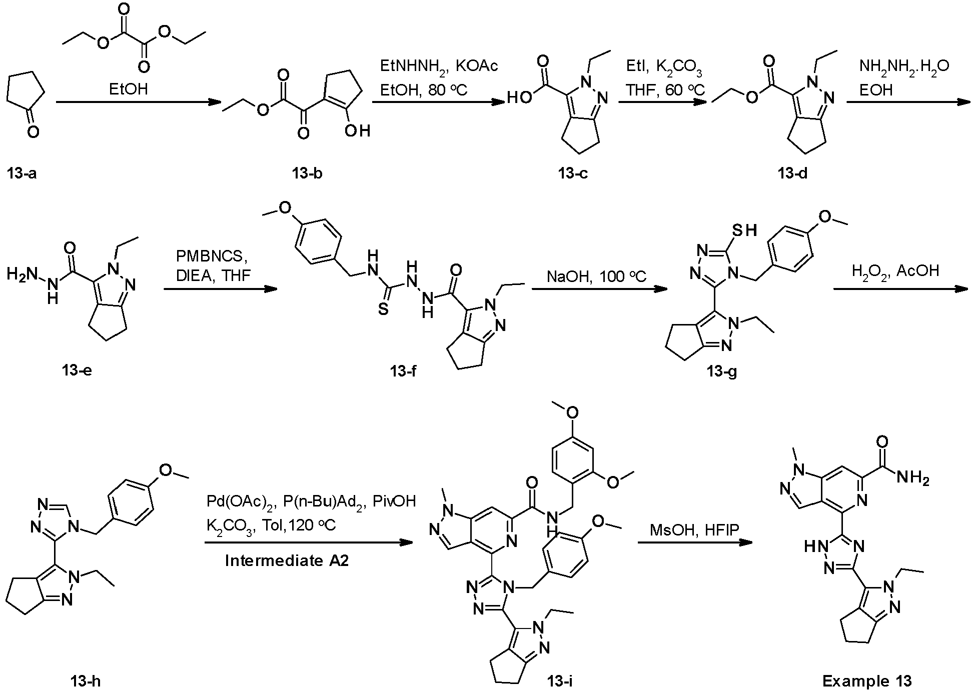

### Step 1: preparation of ethyl 2-(2-hydroxycyclopenten-1-yl)-2-oxo-acetate (Compound 13-b)

To a solution of cyclopentanone **(13-a,** 10.52 mL, 118.88 mmol) in EtOH (60 mL) was added EtONa (42.37 g, 130.77 mmol) dropwise at 0 °C, the mixture was stirred at 0 °C for 0.5 h, then diethyl oxalate (16.15 mL, 118.88 mmol) was added dropwise, then the mixture was stirred at r.t. for 12 hrs. The mixture was concentrated (< 40°C), the residue was poured into water (200 mL), then HCl (30 mL, 1 N) was added to adjust to pH~7, and extracted with EA (500 mL×2). The combined organic phase was washed with brine, dried over anhydrous Na₂SO₄, and concentrated to give **Compound 13-b** (20 g), LCMS (M+H)⁺: 185.

### Step 2: preparation of 2-ethyl-5,6-dihydro-4H-cyclopenta[c]pyrazole-3-carboxylic acid (Compound 13-c)

To a solution of ethyl 2-(2-hydroxycyclopenten-1-yl)-2-oxo-acetate **(13-b,** 15.0 g, 81.44 mmol) in EtOH (150 mL) was added ethylhydrazine dihydrochloride (10.83 g, 81.44 mmol) and KOAc (15.98 g, 162.88 mmol), the mixture was stirred at 80 °C for 12 hrs. Then the mixture was concentrated, the residue was extracted with EA (200 mL×2). The combined organic phase was washed with brine, dried over anhydrous Na₂SO₄, and concentrated to give **Compound 13-c** (8 g), LCMS (M+H)⁺: 181.

### Step 3: preparation of ethyl 2-ethyl-5,6-dihydro-4H-cyclopenta[c]pyrazole-3-carboxylate (Compound 13-d)

To a solution of 2-ethyl-5,6-dihydro-4H-cyclopenta[c]pyrazole-3-carboxylic acid **(13-c,** 8.0 g, 44.4 mmol) in THF (80 mL) was added iodoethane (7.1 mL, 88.79 mmol) and K₂CO₃ (18.38 g, 133.19 mmol), the mixture was stirred at 60 °C for 2 hrs. Water (200 mL) was added, then extracted with EA (300 mL×2). The combined organic phase was washed with brine, dried over anhydrous Na₂SO₄, and concentrated, the residue was purified by column chromatography to give **Compound 13-d** (5.2 g). LCMS (M+H)⁺: 209.

### Step 4~7: preparation of 2-ethyl-3-[4-[(4-methoxyphenyl)methyl]-1,2,4-triazol-3-yl]-5,6-dihydro-4H-cyclopenta[c]pyrazole (Compound 13-h)

**Compound 13-h** was prepared in analogy to the preparation of **Intermediate B1** by using **compound 13-d** instead of isobutoxycarbonyl 4-benzyloxy-2-ethyl-5-methyl-pyrazole-3-carboxylate **(B1-h). Compound 13-h** (400 mg) was obtained. LCMS (M+H)⁺: 324.

### Step 8: preparation of N-[(2,4-dimethoxyphenyl)methyl]-4-[5-(2-ethyl-5,6-dihydro-4H-cyclopenta[c]pyrazol-3-yl)-4-[(4-methoxyphenyl)methyl]-1,2,4-triazol-3-yl]-1-methyl-pyrazolo[4,3-c]pyridine-6-carboxamide (Compound 13-i)

To a solution of 2-ethyl-3-[4-[(4-methoxyphenyl)methyl]-1,2,4-triazol-3-yl]-5,6-dihydro-4*H*-cyclopenta[c]pyrazole **(13-h,** 50.0 mg, 0.15 mmol), 4-bromo-*N*-[(2,4-dimethoxyphenyl)-methyl]-1-methyl-pyrazolo[4,3-c]pyridine-6-carboxamide **(Intermediate A2,** 62.7 mg, 0.15 mmol) in Toluene (5 mL) was added K₂CO₃ (64.0 mg, 0.46 mmol), cataCXium A (27.7 mg, 0.08 mmol), PivOH (7.9 mg, 0.08 mmol) and Pd(OAc)₂ (10.4 mg, 0.05 mmol). The mixture was stirred at 120 °C for 18 hrs under N₂. Then the mixture was filtered, the filtered cake was washed by DCM/MeOH (V/V=20/1), the filtrate was combined and concentrated. The residue was purified by prep-TLC to give **Compound 13-i** (60 mg), LCMS (M+H)⁺: 648.

### Step 9: preparation of 4-[3-(2-ethyl-5,6-dihydro-4H-cyclopenta[c]pyrazol-3-yl)-1H-1,2,4-triazol-5-yl]-1-methyl-pyrazolo[4,3-c]pyridine-6-carboxamide (Example 13)

To a solution of *N*-[(2,4-dimethoxyphenyl)methyl]-4-[5-(2-ethyl-5,6-dihydro-4H-cyclopenta[c]pyrazol-3-yl)-4-[(4-methoxyphenyl)methyl]-1,2,4-triazol-3-yl]-1-methyl-pyrazolo[4,3-c]pyridine-6-carboxamide **(Compound 13-i,** 45 mg, 0.07 mmol) in HFIP (0.5 mL) was added MsOH (66.7 mg, 0.69 mmol), the mixture was stirred at r.t. for 0.5 h. Then the mixture was diluted with ACN (1 mL), then purified by prep-HPLC to give **Example 13** (13.7 mg). LCMS (M+H)⁺: 378. ¹H NMR(400 MHz, DMSO-*d₆*) δ = 15.34 (br s, 1H), 8.81 (br s, 1H), 8.75 (d, *J* = 0.8 Hz, 1H), 8.46 (d, *J=* 0.6 Hz, 1H), 7.89 (br s, 1H), 4.66 (q, *J* = 7.1 Hz, 2H), 4.22 (s, 3H), 2.90 (t, *J=* 7.1 Hz, 2H), 2.71 - 2.63 (m, 2H), 2.47 - 2.40 (m, 2H), 1.42 (t, *J=* 7.1 Hz, 3H).

### Example 14

### 1-[3-[2-(3-hydroxypropyl)-5-methyl-pyrazol-3-yl]-1H-1,2,4-triazol-5-yl]-6-methyl-imidazo[1,5-a]pyrazine-3-carboxamide

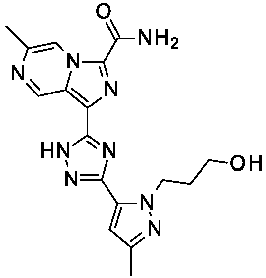

The title compound was prepared according to the following scheme:

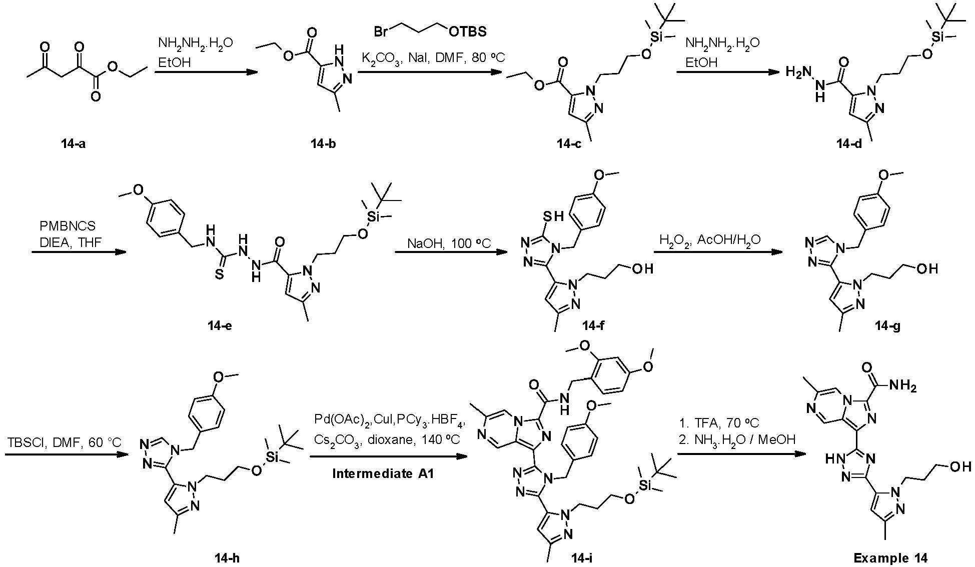

### Step 1: preparation of ethyl 3-methyl-1H-pyrazole-5-carboxylate (compound 14-b)

To a solution of ethyl 2,4-dioxopentanoate **(14-a,** 10 g, 63.23 mmol) in EtOH (40 mL) was added hydrazine hydrate (3.48 g, 69.55 mmol) at 0 °C, the mixture was stirred at 0 °C for 1 h and then concentrated at r.t. to give **compound 14-b** (9.6 g). LCMS (M+H)⁺: 155.

### Step 2: preparation of ethyl 1-(3-((tert-butyldimethylsilyl)oxy)propyl)-3-methyl-1H-pyrazole-5-carboxylate (compound 14-c)

To a solution of ethyl 3-methyl-1*H*-pyrazole-5-carboxylate **(14-b,** 9.6 g, 62.27 mmol) in DMF (50 mL) was added (3-bromopropoxy)-tert-butyldimethylsilane (31.54 g, 124.54 mmol), NaI (18.67 g, 124.54 mmol) and K₂CO₃ (17.21 g, 124.54 mmol), the mixture was stirred at 80 °C for 36 hrs. Then the mixture was diluted with water (800 mL), and extracted with EA (200 mL×3). The combined organic phase was washed with brine, dried over anhydrous Na₂SO₄, and concentrated, the residue was purified by flash column chromatography to give **compound 14-c** (8.0 g), LCMS (M+H)⁺: 327.

### Step 3~6: preparation of 3-[5-[4-[(4-methoxyphenyl)methyl]-1,2,4-triazol-3-yl]-3-methyl-pyrazol-1-yl]propan-1-ol (Compound 14-g)

**Compound 14-g** was prepared in analogy to the preparation of **Intermediate B1** by using **compound 14-c** instead of isobutoxycarbonyl 4-benzyloxy-2-ethyl-5-methyl-pyrazole-3-carboxylate **(B1-h). Compound 14-g** (1.6 g) was obtained. LCMS (M+H)⁺: 328.

### Step 7: preparation of tert-butyl-[3-[5-[4-[(4-methoxyphenyl)methyl]-1,2,4-triazol-3-yl]-3-methyl-pyrazol-1-yl]propoxy]-dimethyl-silane (compound 14-h)

To a solution of 3-[5-[4-[(4-methoxyphenyl)methyl]-1,2,4-triazol-3-yl]-3-methyl-pyrazol-1-yl]propan-1-ol **(14-g,** 800 mg, 2.44 mmol) in DMF (3 mL) was added imidazole (998.2 mg, 14.66 mmol) and TBSCl (1.105 g, 7.33 mmol), the mixture was stirred at 60 °C for 1 h. Then the mixture was concentrated, the residue was purified via flash column chromatography to give **compound 14-h** (600 mg). LCMS (M+H)⁺: 442.

### Step 8: preparation of 1-[5-[2-[3-[tert-butyl(dimethyl)silyl]oxypropyl]-5-methyl-pyrazol-3-yl]-4-[(4-methoxyphenyl)methyl]-1,2,4-triazol-3-yl]-N-[(2,4-dimethoxyphenyl)-methyl]-6-methyl-imidazo[1,5-a]pyrazine-3-carboxamide (compound 14-i)

To a solution of *tert*-butyl-[3-[5-[4-[(4-methoxyphenyl)methyl]-1,2,4-triazol-3-yl]-3-methyl-pyrazol-1-yl]propoxy]-dimethyl-silane **(14-h,** 183.5 mg, 0.45 mmol) in 1,4-dioxane (5 mL) was added 1-bromo-N-[(2,4-dimethoxyphenyl)methyl]-6-methyl-imidazo[1,5-a]pyrazine-3-carboxamide **(Intermediate A1,** 200 mg, 0.45 mmol), Pd(OAc)₂ (30.5 mg, 0.14 mmol), CuI (172.5 mg, 0.91 mmol), PCy_{3.}HBF₄ (66.7 mg, 0.18 mmol) and Cs₂CO₃ (147.2 mg, 0.45 mmol), the mixture was stirred at 140 °C for 16 hrs. Then the mixture was dissolved in DCM/MeOH (30 mL /5 mL), filtered, then the filtrate was concentrated, the residue was purified by prep-TLC to give **compound 14-i** (42.0 mg), LCMS (M+H)⁺: 766.

### Step 9: preparation of 1-[3-[2-(3-hydroxypropyl)-5-methyl-pyrazol-3-yl]-1H-1,2,4-triazol-5-yl]-6-methyl-imidazo[1,5-a]pyrazine-3-carboxamide (Example 14)

The mixture of 1-[5-[2-[3-[*tert*-butyl(dimethyl)silyl]oxypropyl]-5-methyl-pyrazol-3-yl]-4-[(4-methoxyphenyl)methyl]-1,2,4-triazol-3-yl]-*N*-[(2,4-dimethoxyphenyl)-methyl]-6-methyl-imidazo[1,5-a]pyrazine-3-carboxamide **(14-i,** 40 mg, 0.05 mmol) in TFA (2.5 mL) was stirred at 70 °C for 1.5 hrs. Then the mixture was concentrated, the residue was added into MeOH (1 mL) and NH₃.H₂O (0.5 mL), then the suspension was stirred at r.t. for 15 mins. The mixture was concentrated, the residue was purified by prep-HPLC to give **Example 14** (9.2 mg), LCMS (M+H)⁺: 382. ¹H NMR (400 MHz, DMSO-*d₆*) δ = 9.72 (d, *J =* 1.34 Hz, 1H), 9.08 (s, 1H), 8.01 - 7.82 (m, 2H), 6.62 (s, 1H), 4.67 (t, *J* = 7.03 Hz, 2H), 3.45 (t, *J* = 6.11 Hz, 2H), 2.52 (br s, 3H), 2.22 (s, 3H), 1.98 (br t, *J=* 6.72 Hz, 2H).

### Example 15

### 1-[3-(2-ethyl-5-methyl-pyrazol-3-yl)-1H-1,2,4-triazol-5-yl]-6-methyl-imidazo[1,5-a]pyrazine-3-carboxamide

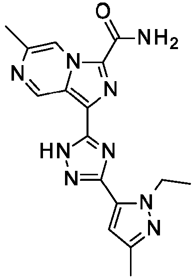

The title compound was prepared according to the following scheme:

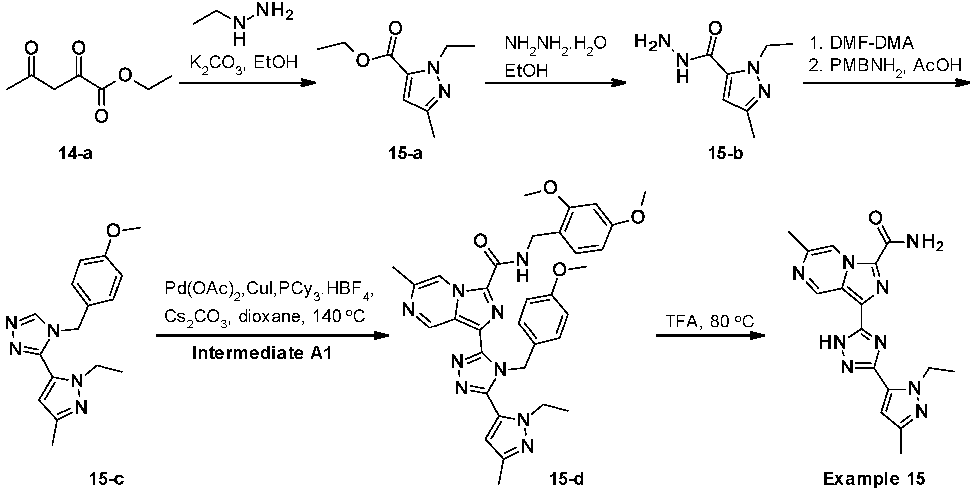

### Step 1: preparation of ethyl 2-ethyl-5-methyl-pyrazole-3-carboxylate (compound 15-a)

To a solution of ethylhydrazine dihydrochloride (1.0 g, 7.52 mmol) in EtOH (12 mL) was added K₂CO₃ (2.28 g, 16.54 mmol) and ethyl 2,4-dioxopentanoate **(14-a,** 1.31 g, 8.27 mmol) dropwise at 0 °C, the mixture was stirred at r.t. for 12 hrs. Then the mixture was concentrated, the residue was extracted with EA (80 mL). The organic layer was washed with water, dried over anhydrous Na₂SO₄, filtered, and concentrated to give **compound 15-a** (400 mg). LCMS (M+H)⁺:183.

### Step 2: preparation of 2-ethyl-5-methyl-pyrazole-3-carbohydrazide (compound 15-b)

To a solution of ethyl 2-ethyl-5-methyl-pyrazole-3-carboxylate **(15-a,** 2.4 g, 13.17 mmol) in EtOH (30 mL) was added hydrazine hydrate (4.76 g, 80.87 mmol), the mixture was stirred at 80 °C for 16 hrs and then concentrated to give compound **15-b** (2 g), LCMS (M+H)⁺: 169.

### Step 3: preparation of 3-(2-ethyl-5-methyl-pyrazol-3-yl)-4-[(4-methoxyphenyl)-methyl]-1,2,4-triazole (compound 15-c)

The suspension of 2-ethyl-5-methyl-pyrazole-3-carbohydrazide (15-b, 2.0 g, 11.89 mmol) in DMF-DMA (20 mL) was stirred at 100 °C for 1 h. Then the mixture was concentrated. The residue was dissolved in toluene (60 mL), then 4-methoxylbenzylamine (2.456 g, 17.92 mmol) and AcOH (10 mL) were added, the mixture was stirred at 100 °C for 16 hrs. Then the mixture was concentrated, the residue was purified by column chromatography to give **compound 15-c** (2.1 g). LCMS (M+H)⁺: 298.

### Step 4: preparation of N-[(2,4-dimethoxyphenyl)methyl]-1-[5-(2-ethyl-5-methyl-pyrazol-3-yl)-4-[(4-methoxyphenyl)methyl]-1,2,4-triazol-3-yl]-6-methyl-imidazo[1,5-a]pyrazine-3-carboxamide (compound 15-d)

To a solution of 3-(2-ethyl-5-methyl-pyrazol-3-yl)-4-[(4-methoxyphenyl)methyl]-1,2,4-triazole (15-c, 200 mg, 0.67 mmol) in 1,4-dioxane (2 mL) was added 1-bromo-N-[(2,4-dimethoxyphenyl)methyl]-6-methyl-imidazo[1,5-a]pyrazine-3-carboxamide **(Intermediate A1,** 272.6 mg, 0.67 mmol), Pd(OAc)₂ (45.3 mg, 0.2 mmol), CuI (256.2 mg, 1.35 mmol), PCy_{3.}HBF₄ (99.0 mg, 0.27 mmol) and Cs₂CO₃ (218.6 mg, 0.67 mmol), the mixture was stirred at 140 °C for 18 hrs. Then the mixture was filtered and concentrated, the residue was purified by prep-TLC to give **compound 15-d** (40 mg). LCMS (M+H)⁺: 622.

### Step 5: preparation of 1-[3-(2-ethyl-5-methyl-pyrazol-3-yl)-1H-1,2,4-triazol-5-yl]-6-methyl-imidazo[1,5-a]pyrazine-3-carboxamide (Example 15)

The solution of *N*-[(2,4-dimethoxyphenyl)methyl]-1-[5-(2-ethyl-5-methyl-pyrazol-3-yl)-4-[(4-methoxyphenyl)methyl]-1,2,4-triazol-3-yl]-6-methyl-imidazo[1,5-a]pyrazine-3-carboxamide **(15-d,** 30 mg, 0.05 mmol) in TFA (1.5 mL) was stirred at 80 °C for 1 h. The mixture was purified by prep-HPLC to give **Example 15** (2.6 mg), LCMS (M+H)⁺: 352. ¹H NMR (400 MHz, DMSO-*d₆*) *δ* = 15.2 - 14.4 (m, 1H), 9.68 (s, 1H), 9.10 (s, 1H), 8.2 - 7.7 (m, 2H), 6.8 - 6.6 (m, 1H), 4.64 (br d, *J=* 6.4 Hz, 2H), 2.5 (s, 3H), 2.23 (s, 3H), 1.41 (t, *J =* 7.2 Hz, 3H).

### Example 16

### 1-[3-(2-ethyl-4-fluoro-5-methyl-pyrazol-3-yl)-1H-1,2,4-triazol-5-yl]-6-methyl-imidazo[1,5-a]pyrazine-3-carboxamide

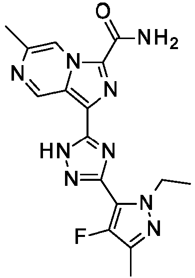

The title compound was prepared according to the following scheme:

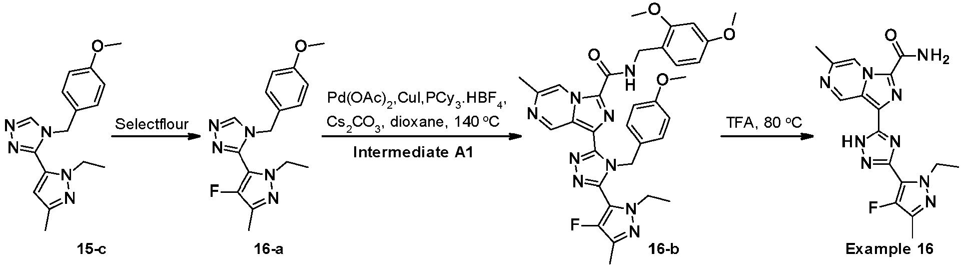

### Step 1: preparation of 3-(2-ethyl-4-fluoro-5-methyl-pyrazol-3-yl)-4-[(4-methoxyphenyl)methyl]-1,2,4-triazole (compound 16-a)

To a solution of 3-(2-ethyl-5-methyl-pyrazol-3-yl)-4-[(4-methoxyphenyl)methyl]-1,2,4-triazole (15-c, 1.6 g, 5.38 mmol) in ACN (50 mL) was added Selectflour (3.8 g, 10.76 mmol), the mixture was stirred at 50 °C for 16 hrs. Then the mixture was concentrated, the residue was purified by column chromatography to give **compound 16-a** (400 mg). LCMS (M+H)⁺: 316.

### Step 2~3: preparation of 1-[3-(2-ethyl-4-fluoro-5-methyl-pyrazol-3-yl)-1H-1,2,4-triazol-5-yl]-6-methyl-imidazo[1,5-a]pyrazine-3-carboxamide (Example 16)

**Example 16** was prepared in analogy to the preparation of **Example 15** by using **compound 16-a** instead of 3-(2-ethyl-5-methyl-pyrazol-3-yl)-4-[(4-methoxyphenyl)methyl]-1,2,4-triazole **(15-c). Example 16** (13.2 mg) was obtained. LCMS (M+H)⁺: 370. ¹H NMR (400 MHz, DMSO-*d₆*) δ = 9.65 (s, 1H), 9.11 (s, 1H), 7.6-8.2 (m, 2H), 4.53 (q, *J =* 7.2 Hz, 2H), 3.31 (br s, 3H), 2.23 (s, 3H), 1.40 (t, *J* = 7.2 Hz, 3H).

### Example 17

### 1-[3-(4-amino-2-ethyl-5-methyl-pyrazol-3-yl)-1H-1,2,4-triazol-5-yl]-6-methyl-imidazo[1,5-a]pyrazine-3-carboxamide

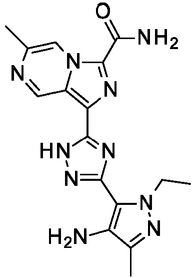

The title compound was prepared according to the following scheme:

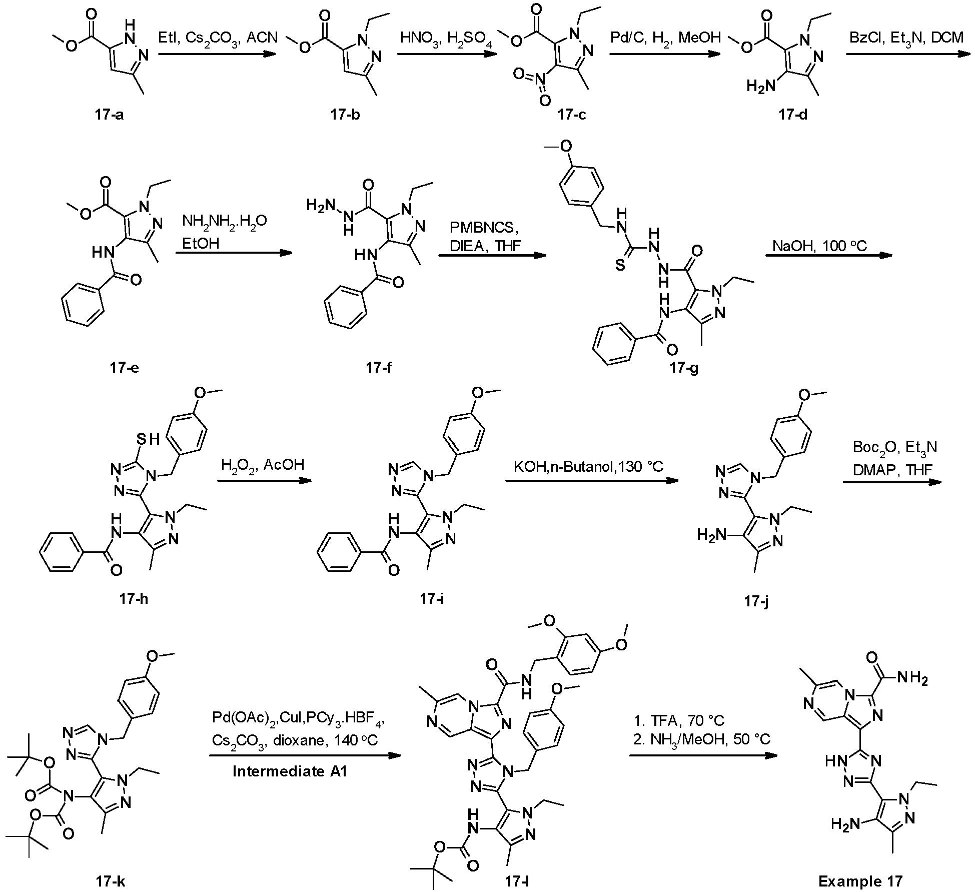

### Step 1: preparation of methyl 2-ethyl-5-methyl-pyrazole-3-carboxylate (Compound 17-b)

To a solution of methyl 3-methyl-1*H*-pyrazole-5-carboxylate **(17-a,** 10.0 g, 71.36 mmol) and Cs₂CO₃ (46.38 g, 142.71 mmol) in ACN (100 mL) was added iodoethane (8.56 mL, 107.04 mmol). The mixture was stirred at 80 °C for 2 hrs. Then the mixture was poured into water (100ml) and extracted with EA (100 mL×2). The combined organic phase was washed with water and brine, dried over anhydrous Na₂SO₄, and concentrated. The residue was purified by flash chromatography to give **Compound 17-b** (5 g). ¹H NMR (400 MHz, DMSO-*d6*) δ = 6.64 (s, 1H), 4.42 (q, *J=* 7.21 Hz, 2H), 3.81 (s, 3H), 2.18 (s, 3H), 1.30 (t, *J=* 7.19 Hz, 3H).

### Step 2: preparation of methyl 2-ethyl-5-methyl-4-nitro-pyrazole-3-carboxylate (Compound 17-c)

To a solution of methyl 2-ethyl-5-methyl-pyrazole-3-carboxylate **(17-b,** 5 g, 29.73 mmol) in H₂SO₄ (23.75 mL, 445.92 mmol) was added HNO₃ (15.45 mL, 311.14 mmol) dropwise at 0 °C. The mixture was stirred at 60 °C for 18 hrs. Then the mixture was poured into ice water (500 mL) and extracted with EA (300 mL×2). The combined organic phase was washed with sat. NaHCO₃ solution and brine, dried over anhydrous Na₂SO₄, and concentrated. The residue was purified by flash column chromatography to give **Compound 17-c** (3.5 g), LCMS (M+H)⁺: 214.

### Step 3: preparation of methyl 4-amino-2-ethyl-5-methyl-pyrazole-3-carboxylate (Compound 17-d)

To a solution of methyl 2-ethyl-5-methyl-4-nitro-pyrazole-3-carboxylate **(17-c,** 2.0 g, 9.38 mmol) in MeOH (40 mL) was added Pd/C (1.0 g, 9.38 mmol), the mixture was stirred at r.t. for 16 hrs under H₂ balloon. Then the mixture was filtered, the filtered cake was washed with MeOH, the combined filtrate was concentrated to give **Compound 17-d** (1.7 g). LCMS (M+H)⁺: 184.

### Step 4: preparation of methyl 4-benzamido-2-ethyl-5-methyl-pyrazole-3-carboxylate (Compound 17-e)

To a solution of methyl 4-amino-2-ethyl-5-methyl-pyrazole-3-carboxylate **(17-d,** 1.4 g, 7.64 mmol) and Et₃N (1.98 mL, 15.28 mmol) in DCM (30 mL) was added BzCl (0.98 mL, 8.41 mmol) at 0 °C, the mixture was stirred at r.t. for 2 hrs. Then the mixture was concentrated. The residue was purified by flash column chromatography to give **Compound 17-d** (2 g), LCMS (M+H)⁺: 288.

### Step 5~8: preparation of N-[1-ethyl-5-[4-[(4-methoxyphenyl)methyl]-1,2,4-triazol-3-yl]-3-methyl-pyrazol-4-yl]benzamide (Compound 17-i)

**Compound 17-i** was prepared in analogy to the preparation of **Intermediate B1** by using **compound 17-e** instead of isobutoxycarbonyl 4-benzyloxy-2-ethyl-5-methyl-pyrazole-3-carboxylate **(B1-h). Compound 17-i** (2 g) was obtained. LCMS (M+H)⁺: 417.

### Step 9: preparation of 1-ethyl-5-[4-[(4-methoxyphenyl)methyl]-1,2,4-triazol-3-yl]-3-methyl-pyrazol-4-amine (Compound 17-j)

To a solution of *N*-[1-ethyl-5-[4-[(4-methoxyphenyl)methyl]-1,2,4-triazol-3-yl]-3-methyl-pyrazol-4-yl]benzamide **(17-i,** 1.0 g, 2.4 mmol) in n-Butanol (20 mL) was added KOH (4.29 g, 76.53 mmol), the mixture was stirred at 130 °C for 16 hrs. Then the mixture was diluted with water (200 mL), and extracted with EA (200 mL×2). The combined organic phase was washed with brine, dried over anhydrous Na₂SO₄, and concentrated to give **Compound 17-j** (400 mg). LCMS (M+H)⁺: 313.

### Step 10: preparation of tert-butyl N-tert-butoxycarbonyl-N-[1-ethyl-5-[4-[(4-methoxyphenyl)methyl]-1,2,4-triazol-3-yl]-3-methyl-pyrazol-4-yl]carbamate (Compound 17-k)

To a solution of 1-ethyl-5-[4-[(4-methoxyphenyl)methyl]-1,2,4-triazol-3-yl]-3-methyl-pyrazol-4-amine **(17-j,** 400 mg, 1.28 mmol), Et₃N (388.0 mg, 3.84 mmol), and DMAP (15.9 mg, 0.13 mmol) in THF (0.3 mL) was added Boc₂O (837.5 mg, 3.84 mmol), the mixture was stirred at r.t. for 16 hrs. Then the mixture was concentrated, the residue was purified by flash column chromatography to give **Compound 17-k** (250 mg). LCMS (M+H)⁺: 513.

### Step 11~12: preparation of 1-[3-(4-amino-2-ethyl-5-methyl-pyrazol-3-yl)-1H-1,2,4-triazol-5-yl]-6-methyl-imidazo[1,5-a]pyrazine-3-carboxamide (Example 17)

**Example 17** was prepared in analogy to the preparation of **Example 14** by using **compound 17-k** instead of *tert*-butyl-[3-[5-[4-[(4-methoxyphenyl)methyl]-1,2,4-triazol-3-yl]-3-methyl-pyrazol-1-yl]propoxy]-dimethyl-silane **(14-h). Example 17** (3.7 mg) was obtained. LCMS (M+H)⁺: 367. ¹H NMR (400 MHz, DMSO-*d₆*+D₂O) δ = 9.63 (br s, 1H), 9.07 (br d, *J=* 3.4 Hz, 1H), 4.51 (br d, *J* = 5.1 Hz, 2H), 2.52 (d, *J=* 1.9 Hz, 3H), 2.15 - 2.03 (m, 3H), 1.31 (br t, *J* = 6.8 Hz, 3H).

### Example 18

### 6-chloro-1-[3-(2-ethyl-4-hydroxy-5-methyl-pyrazol-3-yl)-1H-1,2,4-triazol-5-yl]imidazo[1,5-a]pyrazine-3-carboxamide

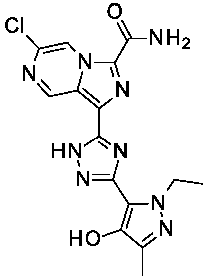

The title compound was prepared according to the following scheme:

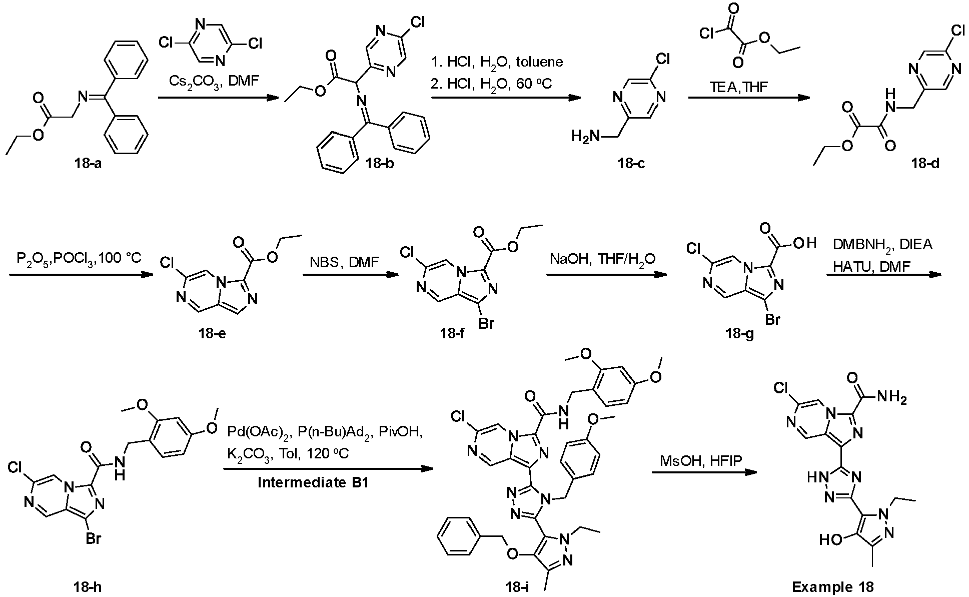

### Step 1: preparation of ethyl 2-(benzhydrylideneamino)-2-(5-chloropyrazin-2-yl)acetate (compound 18-b)

To a solution of 2,5-dichloropyrazine (5.0 g, 33.56 mmol) in DMF (100 mL) was added Cs₂CO₃ (10.94 g, 33.56 mmol) and ethyl 2-(benzhydrylideneamino)acetate **(18-a,** 9.87 g, 36.92 mmol), the mixture was stirred at r.t. for 12 hrs. Then the mixture was diluted with EA (900 mL) and water (1 L), the organic layer was washed with water, then dried over anhydrous Na₂SO₄, and concentrated. The residue was purified by column chromatography to give **compound 18-b** (8.0 g). LCMS (M+H)⁺: 380.

### Step 2: preparation of (5-chloropyrazin-2-yl)methanamine (compound 18-c)

To a solution of ethyl 2-(benzhydrylideneamino)-2-(5-chloropyrazin-2-yl)acetate **(18-b,** 8.0 g, 19.52 mmol) in toluene (36 mL) was added water (12 mL) and HCl (2.0 mL, 24.0 mmol, 12 N), the mixture was stirred at r.t. for 12 hrs. Then the mixture was extracted with toluene (100 mL×3). The aqueous layer was added into water (12 mL) and HCl (1.78 mL, 21.31 mmol, 12 N), the mixture was stirred at 60 °C for 12 hrs and then concentrated to give **compound 18-c** (4.0 g). LCMS (M+H)⁺: 144.

### Step 3~7: preparation of 1-bromo-6-chloro-N-[(2,4-dimethoxyphenyl)methyl]-imidazo[1,5-a]pyrazine-3-carboxamide (Compound 18-h)

**Compound 18-h** was prepared in analogy to the preparation of **Intermediate A1** by using **compound 18-c** instead of 2-(aminomethyl)-5-methylpyrazine **(A1-a). Compound 18-h** (360 mg) was obtained. LCMS (M+H)⁺: 425.

### Step 8: preparation of 1-[5-(4-benzyloxy-2-ethyl-5-methyl-pyrazol-3-yl)-4-[(4-methoxyphenyl)methyl]-1,2,4-triazol-3-yl]-6-chloro-N-[(2,4-dimethoxyphenyl)methyl]-imidazo[1,5-a]pyrazine-3-carboxamide (Compound 18-i)

**Compound 18-i** was prepared in analogy to the preparation of **Compound 3-f** by using **compound 18-h** instead of 8-bromo-N-[(2,4-dimethoxyphenyl)methyl]-3-methyl-pyrrolo[1,2-a]pyrazine-6-carboxamide **(3-e). Compound 18-i** (60 mg) was obtained. LCMS (M+H)⁺: 748.

### Step 9: preparation of 6-chloro-1-[3-(2-ethyl-4-hydroxy-5-methyl-pyrazol-3-yl)-1H-1,2,4-triazol-5-yl]imidazo[1,5-a]pyrazine-3-carboxamide (Example 18)

**Example 18** was prepared in analogy to the preparation of **Example 4** by using **compound 18-i** instead of 8-[5-(4-benzyloxy-2-ethyl-5-methyl-pyrazol-3-yl)-4-[(4-methoxyphenyl)methyl]-1,2,4-triazol-3-yl]-*N*-[(2,4-dimethoxyphenyl)methyl]-7-fluoro-3-methyl-pyrrolo[1,2-a]pyrazine-6-carboxamide **(4-h). Example 18** (2.2 mg) was obtained. LCMS (M+H)⁺: 388. ¹H NMR (400 MHz, DMSO-*d₆*) δ = 9.60 (s, 1H), 9.31 (s, 1H), 4.49 (br d, *J* = 7.4 Hz, 2H), 2.15 (s, 3H), 1.34 (t, *J* = 7.2 Hz, 3H).

### Example 19

### 1-[3-(2-ethyl-4-hydroxy-5-methyl-pyrazol-3-yl)-1H-1,2,4-triazol-5-yl]-6-methyl-imidazo[1,5-a]pyridine-3-carboxamide

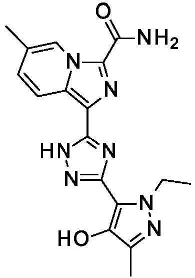

The title compound was prepared according to the following scheme:

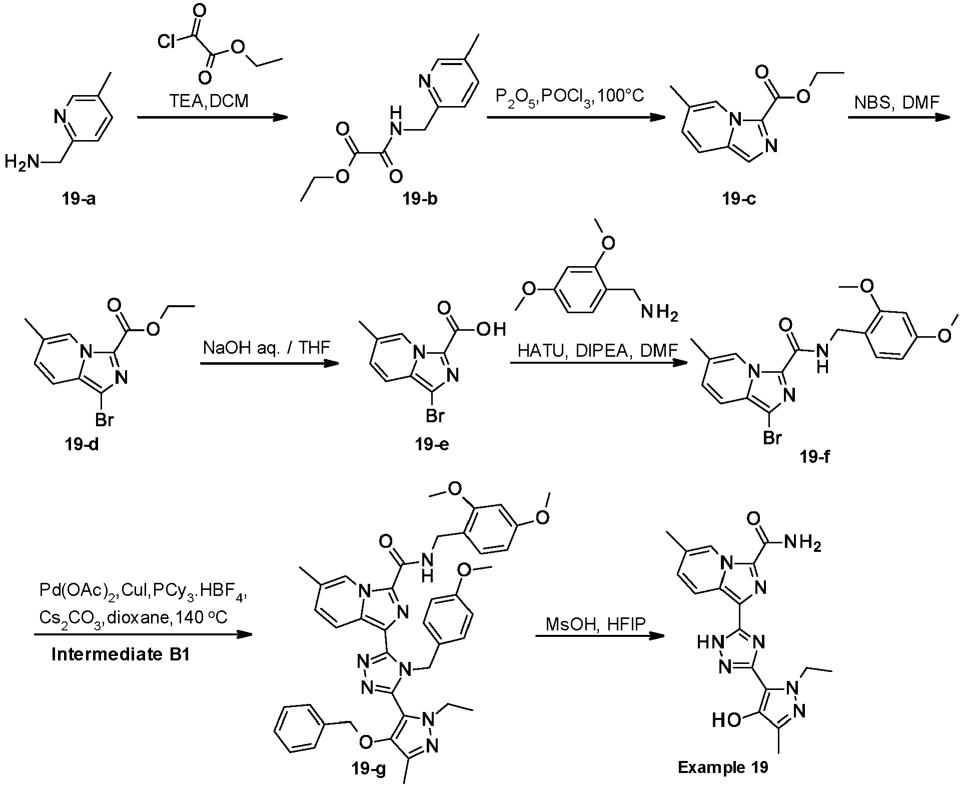

### Step 1~6: preparation of 1-[5-(4-benzyloxy-2-ethyl-5-methyl-pyrazol-3-yl)-4-[(4-methoxyphenyl)methyl]-1,2,4-triazol-3-yl]-N-[(2,4-dimethoxyphenyl)methyl]-6-methyl-imidazo[1,5-a]pyridine-3-carboxamide (Compound 19-g)

**Compound 19-g** was prepared in analogy to the preparation of **Compound 2-a** by using (5-methyl-2-pyridyl)methanamine **(19-a)** and 3-(4-benzyloxy-2-ethyl-5-methyl-pyrazol-3-yl)-4-[(4-methoxyphenyl)methyl]-1,2,4-triazole **(Intermediate B1)** instead of 2-(aminomethyl)-5-methylpyrazine (**A1-a**) and 3-(4-benzyloxy-2-ethyl-5-methyl-pyrazol-3-yl)-1-methyl-1,2,4-triazole **(Intermediate B2). Compound 19-g** (120 mg) was obtained. LCMS (M+H)⁺: 727.

### Step 7: preparation of 1-[3-(2-ethyl-4-hydroxy-5-methyl-pyrazol-3-yl)-1H-1,2,4-triazol-5-yl]-6-methyl-imidazo[1,5-a]pyridine-3-carboxamide (Example 19)

To a solution of 1-[5-(4-benzyloxy-2-ethyl-5-methyl-pyrazol-3-yl)-4-[(4-methoxyphenyl)-methyl]-1,2,4-triazol-3-yl]-*N*-[(2,4-dimethoxyphenyl)methyl]-6-methyl-imidazo[1,5-a]pyridine-3-carboxamide **(19-g,** 120 mg, 0.17 mmol) in HFIP (2.0 mL) was added MsOH (158.5 mg, 1.65 mmol), the mixture was stirred at r.t. for 12 hrs and then concentrated and purified by prep-HPLC to give **Example 19** (12.7 mg), LCMS (M+H)⁺: 367. ¹H NMR (400 MHz, DMSO-*d₆*) δ = 9.31 (s, 1H), 8.28 (d, *J=* 9.3 Hz, 1H), 7.88 - 7.45 (m, 2H), 7.25 (br d, *J* = 8.6 Hz, 1H), 4.46 (q, *J* = 6.8 Hz, 2H), 2.36 (s, 3H), 2.14 (s, 3H), 1.33 (t, *J* = 7.1 Hz, 3H).

### Example 20

### 7-[3-(2-ethyl-4-hydroxy-5-methyl-pyrazol-3-yl)-1H-1,2,4-triazol-5-yl]-3-methyl-pyrrolo[1,2-c]pyrimidine-5-carboxamide

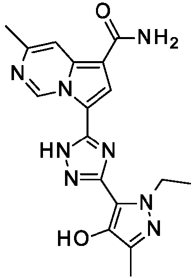

The title compound was prepared according to the following scheme:

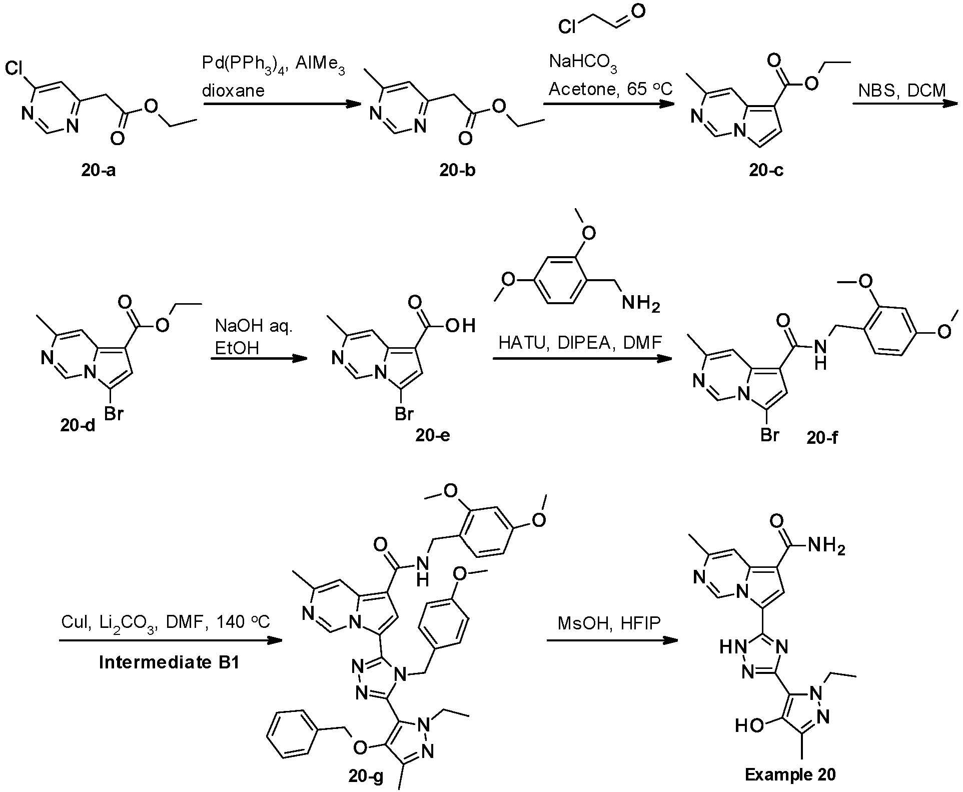

### Step 1: preparation of ethyl 2-(6-methylpyrimidin-4-yl)acetate (compound 20-b)

To a solution of ethyl 2-(6-chloropyrimidin-4-yl)acetate **(20-a,** 4.0 g, 19.94 mmol) in 1,4-dioxane (100 mL) was added Pd(PPh₃)₄ (2.304 g, 1.99 mmol), then trimethylaluminum (29.91 mL, 59.81 mmol) was added slowly. The mixture was stirred at 15 °C for 2 hrs. Then the mixture was poured into HCl (100 mL, 1 N), extracted with EA (50 mL). The aqueous phase was adjusted to pH~9 with sat. K₂CO₃ solution, extracted with EA (100 mL×3). The combined organic phase was dried over anhydrous Na₂SO₄, filtered and concentrated to give **compound 20-b** (3.0 g). LCMS (M+H)⁺: 181.

### Step 2: preparation of ethyl 3-methylpyrrolo[1,2-c]pyrimidine-5-carboxylate (compound 20-c)

To a solution of ethyl 2-(6-methylpyrimidin-4-yl)acetate **(20-b,** 3.0 g, 16.65 mmol) in acetone (100 mL) was added chloroacetaldehyde (1.634 g, 83.24 mmol) and NaHCO₃ (5.594 g, 66.59 mmol), the mixture was stirred at 65 °C for 12 hrs under N₂. Then the mixture was concentrated. The residue was purified by column chromatography to give **compound 20-c** (2.7 g). LCMS (M+H)⁺: 205.

### Step 3: preparation of ethyl 7-bromo-3-methyl-pyrrolo[1,2-c]pyrimidine-5-carboxylate (compound 20-d)

To a solution of ethyl 3-methylpyrrolo[1,2-c]pyrimidine-5-carboxylate **(20-c,** 2.7 g, 13.22 mmol) in DCM (50 mL) was added NBS (2.353 g, 13.22 mmol) at 0 °C, the mixture was stirred at 15 °C for 1 h. Then the mixture was quenched with sat. NaHCO₃ solution (500 mL), extracted with DCM (500 mL×2). The combined organic phase was washed with brine, dried over anhydrous Na₂SO₄, and concentrated. The residue was purified by column chromatography to give **compound 20-d** (1.4 g). LCMS (M+H)⁺: 283.

### Step 4: preparation of 7-bromo-3-methyl-pyrrolo[1,2-c]pyrimidine-5-carboxylic acid (compound 20-e)

To a solution of ethyl 7-bromo-3-methyl-pyrrolo[1,2-c]pyrimidine-5-carboxylate (20-d, 400 mg, 1.41 mmol) in EtOH (6 mL) and water (1 mL) was added NaOH (282.6 mg, 7.06 mmol), the mixture was stirred at 30 °C for 12 hrs. Then water (150 mL) was added, and washed with EA. The aqueous phase was adjusted to pH~5 with HCl (1 N), extracted with EA (100 mL×3), the combined organic phase was dried over anhydrous Na₂SO₄, filtered and concentrated to give **compound 20-e** (150 mg). LCMS (M+H)⁺: 255.

### Step 5: preparation of 7-bromo-N-[(2,4-dimethoxyphenyl)methyl]-3-methyl-pyrrolo[1,2-c]pyrimidine-5-carboxamide (compound 20-f)

To a suspension of 7-bromo-3-methyl-pyrrolo[1,2-c]pyrimidine-5-carboxylic acid **(20-e,** 135 mg, 0.53 mmol) in DMF (1 mL) was added DIEA (0.24 mL, 1.59 mmol), DMBNH₂ (0.1 mL, 0.64 mmol) and HATU (301.9 mg, 0.79 mmol), the mixture was stirred at r.t. for 1 h. Then the mixture was poured into H₂O (100 mL), extracted with EA (150 mL×3). The combined organic phase was washed with brine, dried over anhydrous Na₂SO₄, filtered and concentrated. The residue was purified by column chromatography to give **compound 20-f** (80 mg). LCMS (M+H)⁺: 404.

### Step 6: preparation of 7-[5-(4-benzyloxy-2-ethyl-5-methyl-pyrazol-3-yl)-4-[(4-methoxyphenyl)methyl]-1,2,4-triazol-3-yl]-N-[(2,4-dimethoxyphenyl)methyl]-3-methyl-pyrrolo[1,2-c]pyrimidine-5-carboxamide (compound 20-g)

To a solution of 7-bromo-*N*-[(2,4-dimethoxyphenyl)methyl]-3-methyl-pyrrolo[1,2-c]pyrimidine-5-carboxamide **(compound 20-f,** 150.3 mg, 0.37 mmol) in DMF (10 mL) was added 3-(4-benzyloxy-2-ethyl-5-methyl-pyrazol-3-yl)-4-[(4-methoxyphenyl)methyl]-1,2,4-triazole **(Intermediate B1,** 100.0 mg, 0.25 mmol), Li₂CO₃ (55.0 mg, 0.74 mmol) and CuI (4.7 mg, 0.02 mmol), the mixture was stirred at 140 °C for 12 hrs under N₂. Then the mixture was concentrated. The residue was purified by prep-TLC to give **compound 20-g** (15.0 mg). LCMS (M+H)⁺: 727.

### Step 7: preparation of 7-[3-(2-ethyl-4-hydroxy-5-methyl-pyrazol-3-yl)-1H-1,2,4-triazol-5-yl]-3-methyl-pyrrolo[1,2-c]pyrimidine-5-carboxamide (Example 20)

To a solution of 7-[5-(4-benzyloxy-2-ethyl-5-methyl-pyrazol-3-yl)-4-[(4-methoxyphenyl)-methyl]-1,2,4-triazol-3-yl]-*N*-[(2,4-dimethoxyphenyl)methyl]-3-methyl-pyrrolo[1,2-c]-pyrimidine-5-carboxamide **(compound 20-g,** 10.0 mg, 0.01 mmol) in HFIP (0.5 mL) was added MsOH (105.7 mg, 1.1 mmol), the mixture was stirred at r.t. for 3 hrs. Then the mixture was purified by reversed-phase column chromatography to give **Example 20** (2.5 mg). LCMS (M+H)⁺: 367. ¹H NMR (400 MHz, DMSO-*d₆*) δ = 14.32 - 13.83 (m, 1H), 10.16 (s, 1H), 9.24 - 8.88 (m, 1H), 8.05 (br d, *J* = 3.8 Hz, 2H), 7.86 - 7.67 (m, 1H), 7.18 - 6.98 (m, 1H), 4.48 (q, *J=* 6.8 Hz, 2H), 2.46 (br s, 3H), 2.15 (s, 3H), 1.34 (t, *J =* 7.2 Hz, 3H).

### Example 21

### 1-[2-(2-ethyl-4-hydroxy-5-methyl-pyrazol-3-yl)oxazol-4-yl]-5-methyl-pyrazolo[3,4-c]pyridine-3-carboxamide

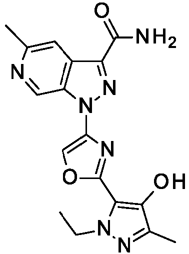

The title compound was prepared according to the following scheme:

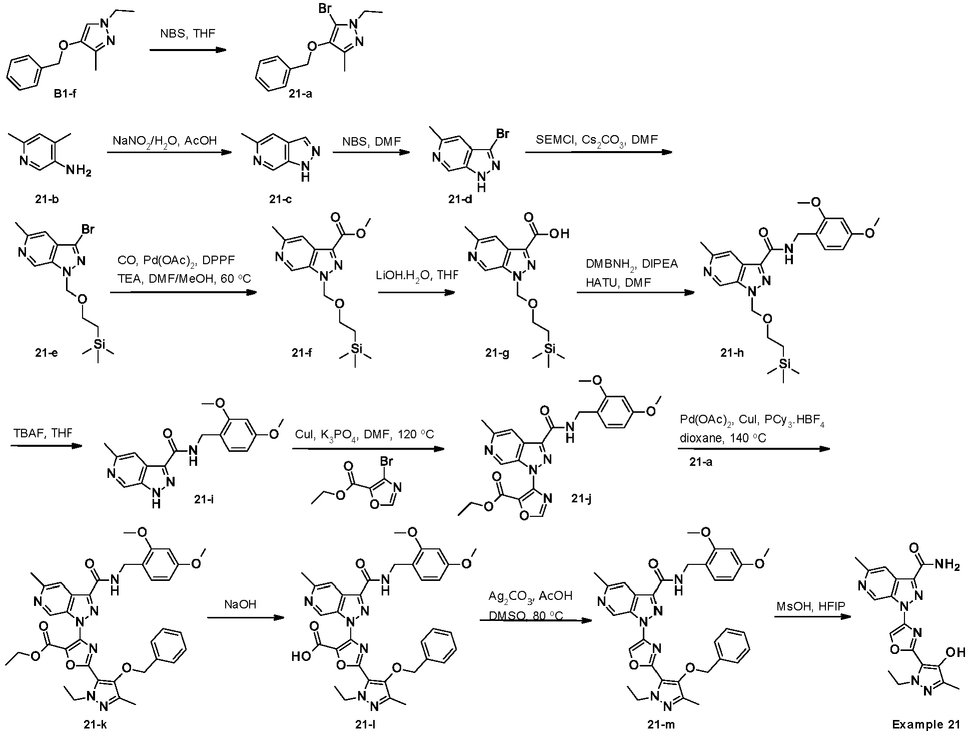

### Step 1: preparation of 4-benzyloxy-5-bromo-1-ethyl-3-methyl-pyrazole (compound 21-a)

To a solution of 4-benzyloxy-1-ethyl-3-methyl-pyrazole **(B1-f,** 1.0 g, 4.62 mmol) in THF (10 mL) was added NBS (0.91 g, 5.09 mmol), the mixture was stirred at r.t. for 2 hrs. Then the mixture was concentrated, the residue was purified by column chromatography to give **compound 21-a** (1.3 g). LCMS (M+H)⁺: 295.

### Step 2: preparation of 5-methyl-1H-pyrazolo[3,4-c]pyridine (compound 21-c)

To a solution of 4,6-dimethylpyridin-3-amine **(21-b,** 10.0 g, 81.85 mmol) in AcOH (200 mL) was added the solution of NaNO₂ (6.21 g, 90.04 mmol) in water (20 mL), the mixture was stirred at 15 °C for 12 hrs. Then the mixture was concentrated, diluted with water (200 mL), and then extracted with EA (200 mL×3). The combined organic phase was dried with anhydrous Na₂SO₄ and concentrated. The residue was purified by column chromatography to give **compound 21-c** (10.0 g). LCMS (M+H)⁺: 134.

### Step 3: preparation of 3-bromo-5-methyl-1H-pyrazolo[3,4-c]pyridine (compound 21-d)

To a solution of 5-methyl-1H-pyrazolo[3,4-c]pyridine **(21-c,** 10.0 g, 75.1 mmol) in DMF (200 mL) was added NBS (14.70 g, 82.61 mmol), the mixture was stirred at 30 °C for 1 h. Then the mixture was concentrated, diluted with water (200 mL), and then extracted with EA (200 mL×3). The combined organic phase was dried with anhydrous Na₂SO₄ and concentrated to give **compound 21-d** (15.9 g). LCMS (M+H)⁺: 212.

### Step 4: preparation of 2-[(3-bromo-5-methyl-pyrazolo[3,4-c]pyridin-1-yl)methoxy]-ethyl-trimethyl-silane (compound 21-e)

To a solution of 3-bromo-5-methyl-1H-pyrazolo[3,4-c]pyridine **(21-d,** 10.0 g, 47.16 mmol) in DMF (20 mL) was added Cs₂CO₃ (30.73 g, 94.32 mmol) and SEMCl (11.79 g, 70.74 mmol), the mixture was stirred at 60 °C for 2 hrs. Then the mixture was poured into H₂O (100 mL) and extracted with EA (200 mL×3). The combined organic phase was washed with brine, dried with anhydrous Na₂SO₄, filtered and concentrated. The residue was purified by column chromatography to give **compound 21-e** (4.0 g). LCMS (M+H)⁺: 342.

### Step 5: preparation of methyl 5-methyl-1-(2-trimethylsilylethoxymethyl)pyrazolo[3,4-c]pyridine-3-carboxylate (compound 21-f)

To a solution of 2-[(3-bromo-5-methyl-pyrazolo[3,4-c]pyridin-1-yl)methoxy]ethyl-trimethyl-silane **(21-e,** 4.0 g, 11.69 mmol) in DMF (30 mL) and MeOH (30 mL) was added DPPF (1.29 g, 2.34 mmol), Pd(OAc)₂ (262.3 mg, 1.17 mmol) and TEA (2.95 g, 29.21 mmol), the mixture was stirred at 60 °C under CO (45 psi) for 16 hrs. Then the mixture was filtered and washed with MeOH. The filtrate was concentrated, the residue was purified by column chromatography to give **compound 21-f** (3.7 g). LCMS (M+H)⁺: 322.

### Step 6: preparation of 5-methyl-1-(2-trimethylsilylethoxymethyl)pyrazolo[3,4-c]pyridine-3-carboxylic acid (compound 21-g)

To a solution of methyl 5-methyl-1-(2-trimethylsilylethoxymethyl)pyrazolo[3,4-c]pyridine-3-carboxylate **(21-f,** 3.4 g, 10.58 mmol) in THF (20 mL) was added the solution of LiOH.H₂O (887.7 mg, 21.15 mmol) in water (20 mL), the mixture was stirred at r.t. for 1 h. Then the mixture was cooled to 0 °C, HCl solution (0.5 N) was added dropwise to adjust to pH~4. The suspension was filtered, the filtered cake was washed with water and dried to give **compound 21-g** (3.2 g). LCMS (M+H)⁺: 308.

### Step 7: preparation of N-[(2,4-dimethoxyphenyl)methyl]-5-methyl-1-(2-trimethylsilylethoxymethyl)pyrazolo[3,4-c]pyridine-3-carboxamide (compound 21-h)

To a solution of 5-methyl-1-(2-trimethylsilylethoxymethyl)pyrazolo[3,4-c]pyridine-3-carboxylic acid **(21-g,** 3.2 g, 10.41 mmol) in DMF (60 mL) was added DIPEA (6.72 g, 52.05 mmol), HATU (5.93 g, 15.61 mmol), and 2,4-dimethoxybenzylamine (2.61 g, 15.61 mmol), the mixture was stirred at r.t. for 1 h. Then the mixture was poured into H₂O (30 mL) and extracted with EA (30 mL×3). The combined organic phase was washed with brine, dried with anhydrous Na₂SO₄, filtered and concentrated. The residue was purified by column chromatography to give **compound 21-h** (4.0 g). LCMS (M+H)⁺: 457.

### Step 8: preparation of N-[(2,4-dimethoxyphenyl)methyl]-5-methyl-1H-pyrazolo[3,4-c]pyridine-3-carboxamide (compound 21-i)

To a solution of *N*-[(2,4-dimethoxyphenyl)methyl]-5-methyl-1-(2-trimethylsilylethoxymethyl)pyrazolo[3,4-c]pyridine-3-carboxamide **(21-h,** 4.0 g, 8.76 mmol) in THF (50 mL) was added TBAF (25.0 mL, 25.0 mmol, 1 N in THF), the mixture was stirred at 60 °C for 16 hrs. Then the mixture was concentrated, the residue was dissolved in DCM (100 mL). Then sat. NaHCO₃ solution (100 mL) was added, the mixture was extracted with DCM (100 mL×3). The combined organic phase was washed with brine, dried with anhydrous Na₂SO₄, filtered and the concentrated. The residue was purified by column chromatography to give **compound 21-i** (2.5 g). LCMS (M+H)⁺: 327.

### Step 9: preparation of ethyl 4-[3-[(2,4-dimethoxyphenyl)methylcarbamoyl]-5-methyl-pyrazolo[3,4-c]pyridin-1-yl]oxazole-5-carboxylate (compound 21-j)

To a solution of N-[(2,4-dimethoxyphenyl)methyl]-5-methyl-1*H*-pyrazolo[3,4-c]pyridine-3-carboxamide **(21-i,** 500.0 mg, 1.53 mmol) and ethyl 4-bromooxazole-5-carboxylate **(10-d,** 674.2 mg, 3.06 mmol) in DMF (6 mL) was added CuI (58.4 mg, 0.31 mmol), (1R,2R)-1,2-diaminocyclohexane (35.0 mg, 0.31 mmol) and K₃PO₄ (650.4 mg, 3.06 mmol), the mixture was stirred at 120 °C for 12 hrs. Then the mixture was filtered and concentrated, the residue was purified by column chromatography to give **compound 21-j** (200.0 mg). LCMS (M+H)⁺: 466.

### Step 10: preparation of ethyl 2-(4-benzyloxy-2-ethyl-5-methyl-pyrazol-3-yl)-4-[3-[(2,4-dimethoxyphenyl)methylcarbamoyl]-5-methyl-pyrazolo[3,4-c]pyridin-1-yl]oxazole-5-carboxylate (compound 21-k)

To a solution of ethyl 4-[3-[(2,4-dimethoxyphenyl)methylcarbamoyl]-5-methyl-pyrazolo[3,4-c]pyridin-1-yl]oxazole-5-carboxylate (21-j, 200.0 mg, 0.43 mmol), 4-benzyloxy-5-bromo-1-ethyl-3-methyl-pyrazole (21-a, 200.0 mg, 0.68 mmol) in 1,4-dioxane (1 mL) was added Pd(OAc)₂ (45.6 mg, 0.2 mmol), PCy₃HBF₄ (99.7 mg, 0.27 mmol), CuI (258.1 mg, 1.36 mmol) and Cs₂CO₃ (220.2 mg, 0.68 mmol), the mixture was stirred at 140 °C for 18 hrs under N₂. Then the mixture was filtered, the filtered cake was washed with DCM/MeOH (V/V=20/1), the filtrate was concentrated, the residue was purified by prep-TLC to give **compound 21-k** (30.0 mg). LCMS (M+H)⁺: 680.

### Step 11: preparation of 2-(4-benzyloxy-2-ethyl-5-methyl-pyrazol-3-yl)-4-[3-[(2,4-dimethoxyphenyl)methylcarbamoyl]-5-methyl-pyrazolo[3,4-c]pyridin-1-yl]oxazole-5-carboxylic acid (compound 21-l)

To a solution of ethyl 2-(4-benzyloxy-2-ethyl-5-methyl-pyrazol-3-yl)-4-[3-[(2,4-dimethoxyphenyl)methylcarbamoyl]-5-methyl-pyrazolo[3,4-c]pyridin-1-yl]oxazole-5-carboxylate **(21-k,** 30.0 mg, 0.04 mmol) in THF/H₂O/MeOH (V/V/V=1/1/1, 1 mL) was added NaOH (17.6 mg, 0.44 mmol), the mixture was stirred at 30 °C for 2 hrs. Then HCl solution (1 N) was added to adjust to pH~4, the mixture was extracted with EA (30 mL×3), the combined organic phase was washed with brine, dried with anhydrous Na₂SO₄, filtered and concentrated to give **compound 21-l** (28.0 mg). LCMS (M+H)⁺: 652.

### Step 12: preparation of 1-[2-(4-benzyloxy-2-ethyl-5-methyl-pyrazol-3-yl)oxazol-4-yl]-N-[(2,4-dimethoxyphenyl)methyl]-5-methyl-pyrazolo[3,4-c]pyridine-3-carboxamide (compound 21-m)

To a solution of 2-(4-benzyloxy-2-ethyl-5-methyl-pyrazol-3-yl)-4-[3-[(2,4-dimethoxyphenyl)methylcarbamoyl]-5-methyl-pyrazolo[3,4-c]pyridin-1-yl]oxazole-5-carboxylic acid **(21-l,** 28.0 mg, 0.04 mmol) in DMSO (1 mL) was added AcOH (0.5 mg, 0.01 mmol) and Ag₂CO₃ (2.4 mg, 0.01 mmol), the mixture was stirred at 80 °C for 12 hrs. Then the mixture was filtered, the filtrate was poured into water (10 mL), extracted with EA (10 mL×2). The combined organic phase was washed with brine, dried with anhydrous Na₂SO₄, filtered and concentrated to give **compound 21-m** (20.0 mg). LCMS (M+H)⁺: 608.

### Step 13: preparation of 1-[2-(2-ethyl-4-hydroxy-5-methyl-pyrazol-3-yl)oxazol-4-yl]-5-methyl-pyrazolo[3,4-c]pyridine-3-carboxamide (Example 21)

To a solution of 1-[2-(4-benzyloxy-2-ethyl-5-methyl-pyrazol-3-yl)oxazol-4-yl]-*N*-[(2,4-dimethoxyphenyl)methyl]-5-methyl-pyrazolo[3,4-c]pyridine-3-carboxamide **(21-m,** 20.0 mg, 0.03 mmol) in HFIP (2.0 mL) was added MsOH (31.6 mg, 0.33 mmol), the mixture was stirred at 50 °C for 12 hrs. Then the mixture was purified by reversed-phase column chromatography to give **Example 21** (3.5 mg). LCMS (M+H)⁺: 368. ¹H NMR (400 MHz, DMSO-*d₆*+D₂O) δ = 9.57 (s, 1H), 8.69 (s, 1H), 8.06 (s, 1H), 4.49 (q, *J=* 7.2 Hz, 2H), 2.66 (s, 3H), 2.15 (s, 3H), 1.39 (t, *J =* 7.2 Hz, 3H).

### Example 22

### 1-[3-(2-ethyl-4-hydroxy-5-methyl-pyrazol-3-yl)-1H-1,2,4-triazol-5-yl]-5-methyl-pyrazolo[3,4-c]pyridine-3-carboxamide

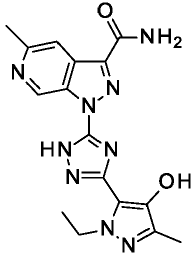

The title compound was prepared according to the following scheme:

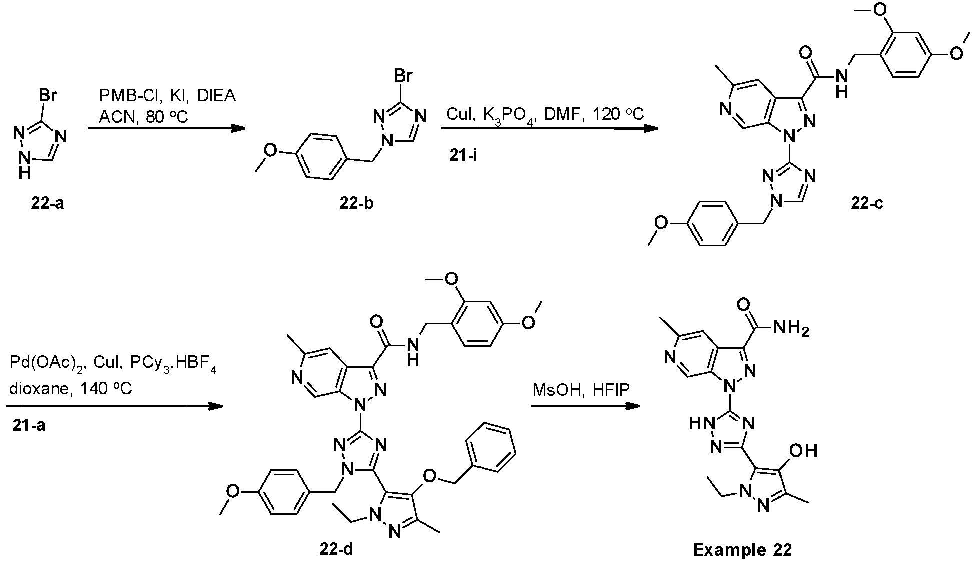

### Step 1: preparation of 3-bromo-1-[(4-methoxyphenyl)methyl]-1,2,4-triazole (compound 22-b)

To a solution of 4-methoxybenzylchloride (6.35 g, 40.55 mmol), DIEA (8.73 g, 67.59 mmol) and KI (2.8 g, 16.9 mmol) in MeCN (50 mL) was added 3-bromo-1H-1,2,4-triazole **(22-a,** 5.0 g, 33.79 mmol), the mixture was stirred at 80 °C for 16 hrs. Then the mixture was concentrated, the residue was purified by column chromatography to give **compound 22-b** (5.0 g). LCMS (M+H)⁺: 268.

### Step 2~3: preparation of 1-[5-(4-benzyloxy-2-ethyl-5-methyl-pyrazol-3-yl)-1-[(4-methoxyphenyl)methyl]-1,2,4-triazol-3-yl]-N-[(2,4-dimethoxyphenyl)methyl]-5-methyl-pyrazolo[3,4-c]pyridine-3-carboxamide (compound 22-d)

**Compound 22-d** was prepared in analogy to the preparation of **compound 21-k** by using **compound 22-b** instead of ethyl 4-bromooxazole-5-carboxylate **(10-d). Compound 22-d** (10 mg) was obtained. LCMS (M+H)⁺: 728.

### Step 4: preparation of 1-[3-(2-ethyl-4-hydroxy-5-methyl-pyrazol-3-yl)-1H-1,2,4-triazol-5-yl]-5-methyl-pyrazolo[3,4-c]pyridine-3-carboxamide (Example 22)

**Example 22** was prepared in analogy to the preparation of **Example 21** by using **compound 22-d** instead of 1-[2-(4-benzyloxy-2-ethyl-5-methyl-pyrazol-3-yl)oxazol-4-yl]-N-[(2,4-dimethoxyphenyl)methyl]-5-methyl-pyrazolo[3,4-c]pyridine-3-carboxamide **(21-m). Example 22** (2.1 mg) was obtained. LCMS (M+H)⁺: 368. ¹H NMR (400 MHz, DMSO-*d₆*+D₂O) δ = 9.64 (s, 1H), 8.12 (s, 1H), 4.48 (q, *J =* 7.2 Hz, 2H), 2.69 (s, 3H), 2.17 (s, 3H), 1.36 (t, *J* = 7.2 Hz, 3H).

### Example 23

### 4-[3-[4-hydroxy-2-(2-imidazol-1-ylethyl)-5-methyl-pyrazol-3-yl]-1H-1,2,4-triazol-5-yl]-1-methyl-pyrazolo[4,3-c]pyridine-6-carboxamide

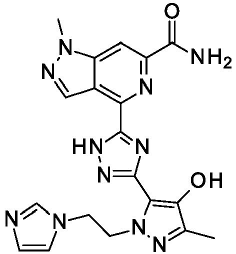

The title compound was prepared according to the following scheme:

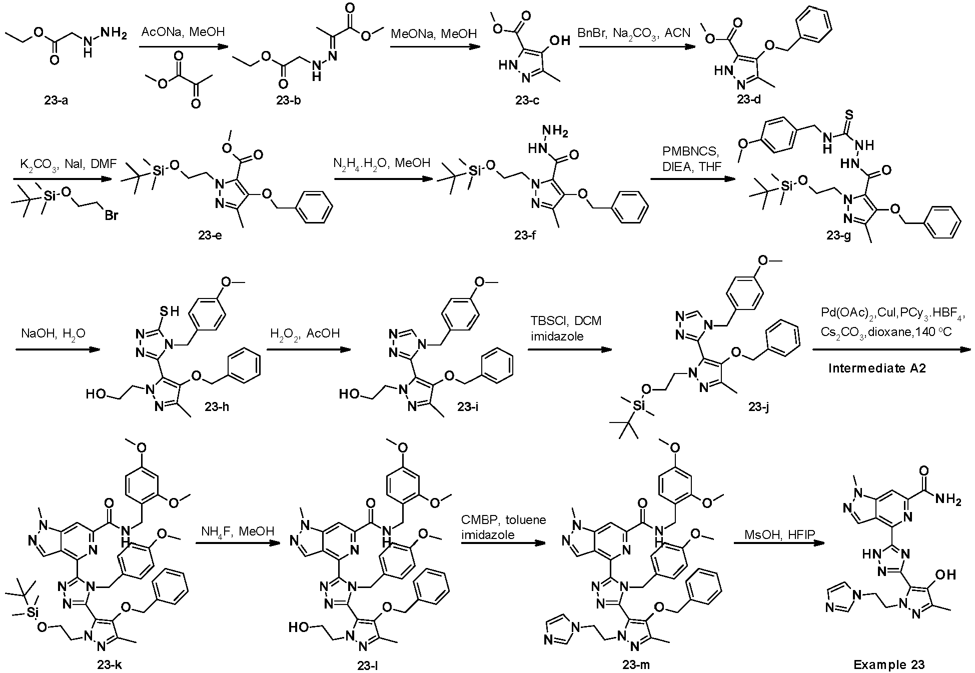

### Step 1: preparation of methyl (2E)-2-[(2-ethoxy-2-oxo-ethyl)hydrazono]propanoate (compound 23-b)

To a solution of methyl 2-oxopropanoate (16.51 g, 161.71 mmol) in MeOH (250 mL) was added AcONa (13.27 g, 161.71 mmol) and ethyl 2-hydrazinoacetate hydrochloride **(23-a,** 25.0 g, 161.71 mmol), the mixture was stirred at r.t. for 18 hrs. Then H₂O (80 mL) was added to the mixture. Then the mixture was concentrated to remove MeOH. The aqueous phase was extracted with EA (400 mL×3). The combined organic phase was washed with brine, dried with anhydrous Na₂SO₄, filtered and concentrated to give **compound 23-b** (28.5 g). LCMS (M+H)⁺: 203.

### Step 2: preparation of methyl 4-hydroxy-3-methyl-1H-pyrazole-5-carboxylate (compound 23-c)

To a solution of methyl (2E)-2-[(2-ethoxy-2-oxo-ethyl)hydrazono]propanoate **(23-b,** 47.0 g, 232.43 mmol) in MeOH (470 mL) was added MeONa (139.46 mL, 697.29 mmol, 5 N in MeOH), the mixture was stirred at 70 °C for 16 hrs. Then HCl (2 N) was added to adjust to pH~7 at 0 °C, then the mixture was extracted with EA (500 mL×3). The combined organic phase was dried with anhydrous Na₂SO₄, filtered and concentrated to give **compound 23-c** (17.0 g). LCMS (M+H)⁺: 157.

### Step 3: preparation of methyl 4-benzyloxy-3-methyl-1H-pyrazole-5-carboxylate (compound 23-d)

**Compound 23-d** was prepared in analogy to the preparation of **compound B1-f** by using **compound 23-c** and Na₂CO₃ instead of 1-ethyl-3-methyl-pyrazol-4-ol **(B1-e)** and Cs₂CO₃. **Compound 23-d** (16.6 g) was obtained. LCMS (M+H)⁺: 247.

### Step 4: preparation of methyl 4-benzyloxy-2-[2-[tert-butyl(dimethyl)silyl]oxyethyl]-5-methyl-pyrazole-3-carboxylate (compound 23-e)

To a solution of methyl 4-benzyloxy-3-methyl-1H-pyrazole-5-carboxylate **(23-d,** 20.0 g, 81.21 mmol) in DMF (200 mL) was added K₂CO₃ (22.45 g, 162.43 mmol), 2-bromoethoxy-*tert-*butyl-dimethyl-silane (23.31 g, 97.46 mmol) and NaI (10.64 g, 71 mmol), the mixture was stirred at 40 °C for 28 hrs. Then additional 2-bromoethoxy-tert-butyl-dimethyl-silane (9.71 g, 40.61 mmol) and K₂CO₃ (11.22 g, 81.21 mmol) were added, the mixture was stirred at 40 °C for another 16 hrs. Then the mixture was poured into water (600 mL), extracted with EA (300 mL×3). The combined organic phase was washed with brine, dried with anhydrous Na₂SO₄, filtered, and concentrated, the residue was purified by column chromatography to give **compound 23-e** (9.0 g). LCMS (M+H)⁺: 405.

### Step 5~8: preparation of 2-[4-benzyloxy-5-[4-[(4-methoxyphenyl)methyl]-1,2,4-triazol-3-yl]-3-methyl-pyrazol-1-yl]ethanol (compound 23-i)

**Compound 23-i** was prepared in analogy to the preparation of **intermediate B1** by using **compound 23-e** instead of isobutoxycarbonyl 4-benzyloxy-2-ethyl-5-methyl-pyrazole-3-carboxylate **(B1-h). Compound 23-i** (3.6 g) was obtained. LCMS (M+H)⁺: 420.

### Step 9: preparation of 2-[4-benzyloxy-S-[4-[(4-methoxyphenyl)methyl]-1,2,4-triazol-3-yl]-3-methyl-pyrazol-1-yl]ethoxy-tert-butyl-dimethyl-silane (compound 23-j)

To a solution of 2-[4-benzyloxy-5-[4-[(4-methoxyphenyl)methyl]-1,2,4-triazol-3-yl]-3-methyl-pyrazol-1-yl]ethanol **(23-i,** 4.0 g, 9.54 mmol) in DCM (100 mL) was added imidazole (3.9 g, 57.21 mmol) and TBSCl (4.31 g, 28.61 mmol), the mixture was stirred at 60 °C for 1 h. Then the mixture was concentrated, the residue was purified by column chromatography to give **compound 23-j** (2.0 g). LCMS (M+H)⁺: 534.

### Step 10: preparation of 4-[5-[4-benzyloxy-2-[2-[tert-butyl(dimethyl)silyl]oxyethyl]-5-methyl-pyrazol-3-yl]-4-[(4-methoxyphenyl)methyl]-1,2,4-triazol-3-yl]-N-[(2,4-dimethoxy-phenyl)methyl]-1-methyl-pyrazolo[4,3-c]pyridine-6-carboxamide(compound 23-k)

**Compound 23-k** was prepared in analogy to the preparation of **compound 1-a** by using **compound 23-j** and **Intermediate A2** instead of ethyl 1-bromo-6-methyl-imidazo[1,5-a]pyrazine-3-carboxylate (**A1-d**) and 3-(4-benzyloxy-2-ethyl-5-methyl-pyrazol-3-yl)-4-[(4-methoxyphenyl)-methyl]-1,2,4-triazole (**Intermediate B1**). **Compound 23-k** (150 mg) was obtained. LCMS (M+H)⁺: 858.

### Step 11: preparation of 4-[5-[4-benzyloxy-2-(2-hydroxyethyl)-5-methyl-pyrazol-3-yl]-4-[(4-methoxyphenyl)methyl]-1,2,4-triazol-3-yl]-N-[(2,4-dimethoxyphenyl)methyl]-1-methyl-pyrazolo[4,3-c]pyridine-6-carboxamide (compound 23-l)

To a solution of 4-[5-[4-benzyloxy-2-[2-[tert-butyl(dimethyl)silyl]oxyethyl]-5-methyl-pyrazol-3-yl]-4-[(4-methoxyphenyl)methyl]-1,2,4-triazol-3-yl]-N-[(2,4-dimethoxyphenyl)-methyl]-1-methyl-pyrazolo[4,3-c]pyridine-6-carboxamide **(23-k,** 150 mg, 0.17 mmol) in MeOH (4 mL) was added NH₄F (232.5 mg, 6.12 mmol), the mixture was stirred at 60 °C for 12 hrs. Then the mixture was concentrated, the residue was purified by column chromatography to give **compound 23-l** (120 mg). LCMS (M+H)⁺: 744.

### Step 12: 4-[5-[4-benzyloxy-2-(2-imidazol-1-ylethyl)-5-methyl-pyrazol-3-yl]-4-[(4-methoxyphenyl)methyl]-1,2,4-triazol-3-yl]-N-[(2,4-dimethoxyphenyl)methyl]-1-methyl-pyrazolo[4,3-c]pyridine-6-carboxamide (compound 23-m)

To a solution of 4-[5-[4-benzyloxy-2-(2-hydroxyethyl)-5-methyl-pyrazol-3-yl]-4-[(4-methoxyphenyl)methyl]-1,2,4-triazol-3-yl]-N-[(2,4-dimethoxyphenyl)methyl]-1-methyl-pyrazolo[4,3-c]pyridine-6-carboxamide (**23-l**, 33 mg, 0.04 mmol), CMBP (0.07 mL, 0.22 mmol) in anhydrous toluene (1.5 mL) was added imidazole (15.1 mg, 0.22 mmol), the mixture was stirred at 60 °C for 12 hrs. Then the mixture was concentrated, the residue was purified by column chromatography to give **compound 23-m** (10 mg). LCMS (M+H)⁺: 794.

### Step 13: preparation of 4-[3-[4-hydroxy-2-(2-imidazol-1-ylethyl)-5-methyl-pyrazol-3-yl]-1H-1,2,4-triazol-5-yl]-1-methyl-pyrazolo[4,3-c]pyridine-6-carboxamide (Example 23)

**Example 23** was prepared in analogy to the preparation of **Example 21** by using **compound 23-m** instead of 1-[2-(4-benzyloxy-2-ethyl-5-methyl-pyrazol-3-yl)oxazol-4-yl]-N-[(2,4-dimethoxyphenyl)methyl]-5-methyl-pyrazolo[3,4-c]pyridine-3-carboxamide **(21-m). Example 23** (2.5 mg) was obtained. LCMS (M+H)⁺: 434. ¹H NMR (400 MHz, DMSO-*d₆*) δ = 15.36 (s, 1H), 8.91 - 8.81 (m, 2H), 8.68 (s, 1H), 8.49 (s, 1H), 8.15 (s, 1H), 7.90 (s, 1H), 7.46 (s, 2H), 4.95 (d, *J=* 4.4 Hz, 2H), 4.69 (d, *J =* 4.4 Hz, 2H), 4.24 (s, 3H), 2.11 (s, 3H).

### Example 24

### 4-[3-[2-(3-cyanopropyl)-4-hydroxy-5-methyl-pyrazol-3-yl]-1H-1,2,4-triazol-5-yl]-1-methyl-pyrazolo[4,3-c]pyridine-6-carboxamide

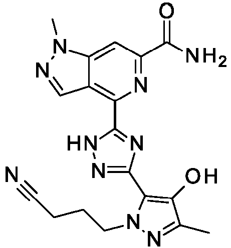

The title compound was prepared according to the following scheme:

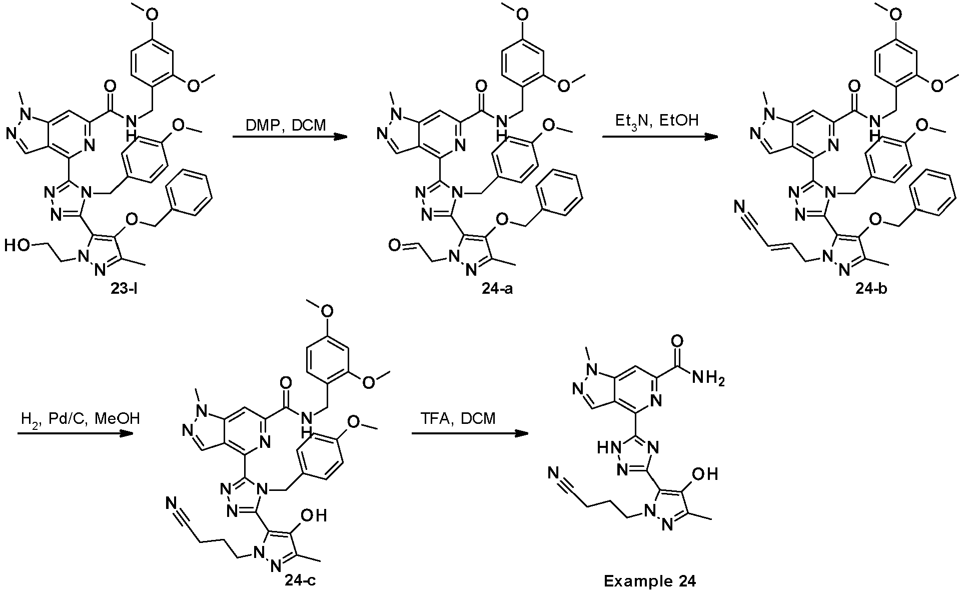

### Step 1: preparation of 4-[5-[4-benzyloxy-5-methyl-2-(2-oxoethyl)pyrazol-3-yl]-4-[(4-methoxyphenyl)methyl]-1,2,4-triazol-3-yl]-N-[(2,4-dimethoxyphenyl)methyl]-1-methyl-pyrazolo[4,3-c]pyridine-6-carboxamide (compound 24-a)

To a solution of 4-[5-[4-benzyloxy-2-(2-hydroxyethyl)-5-methyl-pyrazol-3-yl]-4-[(4-methoxyphenyl)methyl]-1,2,4-triazol-3-yl]-N-[(2,4-dimethoxyphenyl)methyl]-1-methyl-pyrazolo[4,3-c]pyridine-6-carboxamide (23-l, 200 mg, 0.27 mmol) in DCM (5 mL) was added DMP (228.1 mg, 0.54 mmol) at 0 °C, the mixture was stirred at 30 °C for 1 h. Then sat. Na₂SO₃ solution (50 mL) and sat. NaHCO₃ solution (100 mL) were added into the mixture at 0 °C. The mixture was extracted with DCM (100 mL×3). The combined organic phase was washed with brine, dried with anhydrous Na₂SO₄, filtered, and concentrated, the residue was purified by prep-TLC to give **compound 24-a** (100 mg). LCMS (M+H)⁺: 742.

### Step 2: preparation of 4-[5-[4-benzyloxy-2-[(E)-3-cyanoallyl]-5-methyl-pyrazol-3-yl]-4-[(4-methoxyphenyl)methyl]-1,2,4-triazol-3-yl]-N-[(2,4-dimethoxyphenyl)methyl]-1-methyl-pyrazolo[4,3-c]pyridine-6-carboxamide (compound 24-b)

To a solution of 4-[5-[4-benzyloxy-5-methyl-2-(2-oxoethyl)pyrazol-3-yl]-4-[(4-methoxyphenyl)methyl]-1,2,4-triazol-3-yl]-N-[(2,4-dimethoxyphenyl)methyl]-1-methyl-pyrazolo[4,3-c]pyridine-6-carboxamide **(24-a,** 100 mg, 0.13 mmol) in EtOH (3 mL) was added (cyanomethyl)triphenylphosphonium chloride (91.13 mg, 0.27 mmol), then TEA (0.06 mL, 0.4 mmol) in EtOH (3 mL) was added to the mixture dropwise at 0 °C. The mixture was stirred at 30 °C for 3 hrs. Then the mixture was purified by prep-TLC to give **compound 24-b** (80 mg). LCMS (M+H)⁺: 765.

### Step 3: preparation of 4-[5-[2-(3-cyanopropyl)-4-hydroxy-5-methyl-pyrazol-3-yl]-4-[(4-methoxyphenyl)methyl]-1,2,4-triazol-3-yl]-N-[(2,4-dimethoxyphenyl)methyl]-1-methyl-pyrazolo[4,3-c]pyridine-6-carboxamide (compound 24-c)

To a solution of 4-[5-[4-benzyloxy-2-[(E)-3-cyanoallyl]-5-methyl-pyrazol-3-yl]-4-[(4-methoxyphenyl)methyl]-1,2,4-triazol-3-yl]-N-[(2,4-dimethoxyphenyl)methyl]-1-methyl-pyrazolo[4,3-c]pyridine-6-carboxamide **(24-b,** 80 mg, 0.1 mmol) in MeOH (5 mL) was added Pd/C (300 mg) under N₂, the mixture was stirred at r.t. for 2 hrs under H₂ (15 psi). Then the mixture was purified by prep-TLC to give **compound 24-c** (50 mg). LCMS (M+H)⁺: 677.

### Step 4: preparation of 4-[3-[2-(3-cyanopropyl)-4-hydroxy-5-methyl-pyrazol-3-yl]-1H-1,2,4-triazol-5-yl]-1-methyl-pyrazolo[4,3-c]pyridine-6-carboxamide (Example 24)

The solution of 4-[5-[2-(3-cyanopropyl)-4-hydroxy-5-methyl-pyrazol-3-yl]-4-[(4-methoxyphenyl)methyl]-1,2,4-triazol-3-yl]-N-[(2,4-dimethoxyphenyl)methyl]-1-methyl-pyrazolo[4,3-c]pyridine-6-carboxamide **(24-c,** 12 mg, 0.02 mmol) and TFA (8.1 mg, 0.07 mmol) in DCM (1.5 mL) was stirred at 30 °C for 3 hrs. Then the mixture was purified by reversed-phase column chromatography to give **Example 24** (2.2 mg). LCMS (M+H)⁺: 407. ¹H NMR (400 MHz, DMSO-*d₆*) δ = 15.38 (s, 1H), 8.88 (s, 1H), 8.84 (s, 1H), 8.48 (s, 1H), 8.10 (s, 1H), 7.88 (s, 1H), 4.53 - 4.50 (m, 2H), 4.22 (s, 3H), 3.31 (s, 2H), 2.15 (s, 3H), 2.15 - 2.07 (m, 2H).

### Example 25

### 4-[3-[2-(2-cyanoethyl)-4-hydroxy-5-methyl-pyrazol-3-yl]-1H-1,2,4-triazol-5-yl]-1-methyl-pyrazolo[4,3-c]pyridine-6-carboxamide

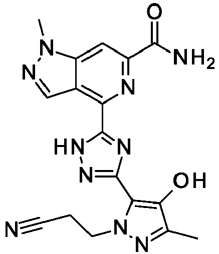

The title compound was prepared according to the following scheme:

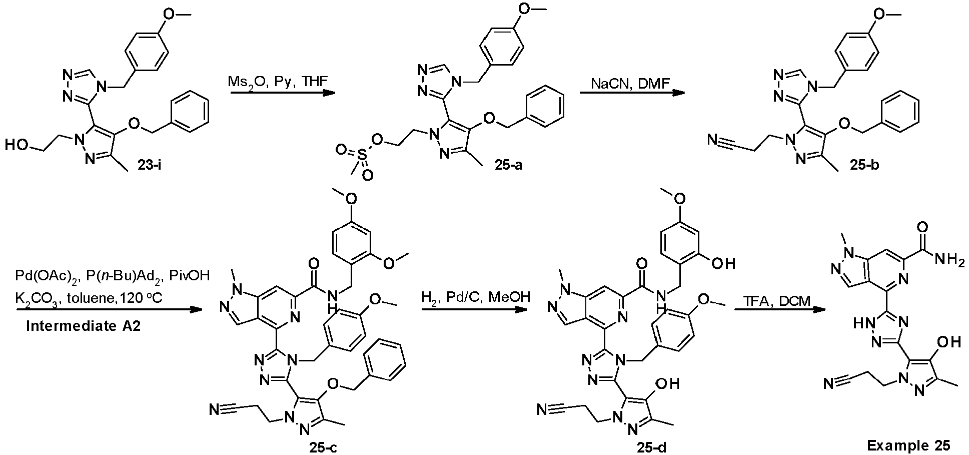

### Step 1: preparation of 2-[4-benzyloxy-5-[4-[(4-methoxyphenyl)methyl]-1,2,4-triazol-3-yl]-3-methyl-pyrazol-1-yl]ethyl methanesulfonate (compound 25-a)

To a solution of 2-[4-benzyloxy-5-[4-[(4-methoxyphenyl)methyl]-1,2,4-triazol-3-yl]-3-methyl-pyrazol-1-yl]ethanol (23-i, 1.0 g, 2.38 mmol) in THF (10 mL) was added Py (565 mg, 7.15 mmol) and Ms₂O (830.5 mg, 4.77 mmol) at 0 °C, the mixture was stirred at r.t. for 1 h. Then H₂O (20 mL) was added into the mixture, the mixture was extracted with DCM (100 mL×2). The combined organic phase was washed with brine, dried with anhydrous Na₂SO₄, filtered, and concentrated to give **compound 25-a** (1.0 g). LCMS (M+H)⁺: 498.

### Step 2: preparation of 3-[4-benzyloxy-5-[4-[(4-methoxyphenyl)methyl]-1,2,4-triazol-3-yl]-3-methyl-pyrazol-1-yl]propanenitrile (compound 25-b)

To a solution of 2-[4-benzyloxy-5-[4-[(4-methoxyphenyl)methyl]-1,2,4-triazol-3-yl]-3-methyl-pyrazol-1-yl]ethyl methanesulfonate **(25-a,** 800 mg, 1.61 mmol) in DMF (8 mL) was added NaCN (760 mg, 15.51 mmol), the mixture was stirred at 60 °C for 18 hrs. Then sat. NaHCO₃ solution (50 mL) was added into the mixture, the mixture was extracted with DCM (50 mL×2). The combined organic phase was washed with brine, dried with anhydrous Na₂SO₄, filtered, and concentrated, the residue was purified by column chromatography to give **compound 25-b** (330.0 mg). LCMS (M+H)⁺: 429.

### Step 3: preparation of 4-[5-[4-benzyloxy-2-(2-cyanoethyl)-5-methyl-pyrazol-3-yl]-4-[(4-methoxyphenyl)methyl]-1,2,4-triazol-3-yl]-N-[(2,4-dimethoxyphenyl)methyl]-1-methyl-pyrazolo[4,3-c]pyridine-6-carboxamide (compound 25-c)

**Compound 25-c** was prepared in analogy to the preparation of **compound 3-f** by using **compound 25-b** and **Intermediate A2** instead of 8-bromo-N-[(2,4-dimethoxyphenyl)-methyl]-3-methyl-pyrrolo[1,2-a]pyrazine-6-carboxamide **(3-e)** and 3-(4-benzyloxy-2-ethyl-5-methyl-pyrazol-3-yl)-4-[(4-methoxyphenyl)methyl]-1,2,4-triazole **(Intermediate B1). Compound 25-c** (120 mg) was obtained. LCMS (M+H)⁺: 753.

### Step 4~5: preparation of 4-[3-[2-(2-cyanoethyl)-4-hydroxy-5-methyl-pyrazol-3-yl]-1H-1,2,4-triazol-5-yl]-1-methyl-pyrazolo[4,3-c]pyridine-6-carboxamide (Example 25)

**Example 25** was prepared in analogy to the preparation of **Example 24** by using **compound 25-c** instead of 4-[5-[4-benzyloxy-2-[(E)-3-cyanoallyl]-5-methyl-pyrazol-3-yl]-4-[(4-methoxyphenyl)methyl]-1,2,4-triazol-3-yl]-N-[(2,4-dimethoxyphenyl)methyl]-1-methyl-pyrazolo[4,3-c]pyridine-6-carboxamide **(24-b). Example 25** (12.4 mg) was obtained. LCMS (M+H)⁺: 393. ¹H NMR (400 MHz, DMSO-*d6*) δ = 15.39 (s, 1H), 8.87 (s, 2H), 8.47 (s, 1H), 8.15 (s, 1H), 7.88 (br d, *J* = 1.2 Hz, 1H), 4.75 (br t, *J =* 6.6 Hz, 2H), 4.23 (s, 3H), 3.09 (t, *J =* 6.6 Hz, 2H), 2.16 (s, 3H).

### Example 26

### 6-(difluoromethyl)-1-[3-(2-ethyl-4-hydroxy-5-methyl-pyrazol-3-yl)-1H-1,2,4-triazol-5-yl]imidazo[1,5-a]pyrazine-3-carboxamide

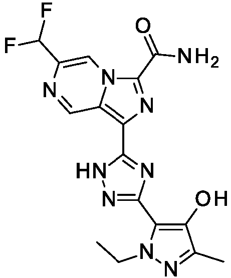

The title compound was prepared according to the following scheme:

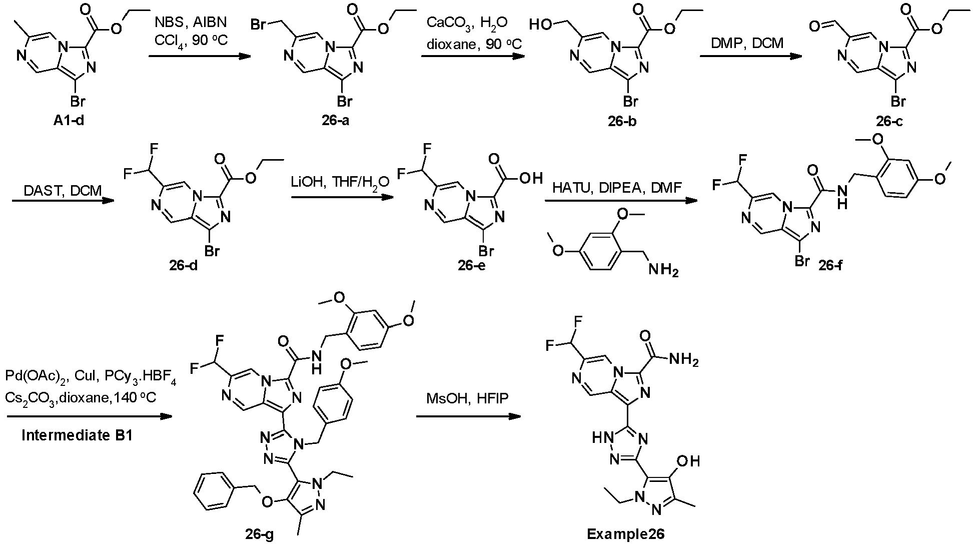

### Step 1: preparation of ethyl 1-bromo-6-(bromomethyl)imidazo[1,5-a]pyrazine-3-carboxylate (compound 26-a)

To a solution of ethyl 1-bromo-6-methyl-imidazo[1,5-a]pyrazine-3-carboxylate (**A1-d**, 1.9 g, 6.69 mmol) in CCl₄ (40 mL) was added NBS (1.31 g, 7.36 mmol) and AIBN (109.8 mg, 0.67 mmol), the mixture was stirred at 90 °C for 16 hrs. Then the mixture was concentrated, the residue was purified by column chromatography to give **compound 26-a** (2.1 g). LCMS (M+H)⁺: 362.

### Step 2: preparation of ethyl 1-bromo-6-(hydroxymethyl)imidazo[1,5-a]pyrazine-3-carboxylate (compound 26-b)

To a solution of ethyl 1-bromo-6-(bromomethyl)imidazo[1,5-a]pyrazine-3-carboxylate **(26-a,** 2.1 g, 5.78 mmol) in 1,4-dioxane (20 mL) was added CaCO₃ (2.89 g, 28.92 mmol) and water (20 mL), the mixture was stirred at 90 °C for 16 hrs. Then water (100 mL) was added into the mixture, the mixture was extracted with EA (50 mL×3). The combined organic phase was washed with brine, dried with anhydrous Na₂SO₄, filtered and concentrated to give **compound 26-b** (1.6 g). LCMS (M+H)⁺: 300.

### Step 3: preparation of ethyl 1-bromo-6-formyl-imidazo[1,5-a]pyrazine-3-carboxylate (compound 26-c)

To a solution of ethyl 1-bromo-6-(hydroxymethyl)imidazo[1,5-a]pyrazine-3-carboxylate **(26-b,** 1.6 g, 5.33 mmol) in DCM (40 mL) was added DMP (3.61 g, 8.53 mmol), the mixture was stirred at r.t. for 1 h. Then the mixture was filtered, the filtered cake was washed with DCM, the filtrate was concentrated, the residue was purified by column chromatography to give **compound 26-c** (1.0 g). LCMS (M+H)⁺: 298.

### Step 4: preparation of ethyl 1-bromo-6-(difluoromethyl)imidazo[1,5-a]pyrazine-3-carboxylate (compound 26-d)

To a solution of ethyl 1-bromo-6-formyl-imidazo[1,5-a]pyrazine-3-carboxylate **(26-c,** 1.0 g, 3.35 mmol) in DCM (20 mL) was added DAST (1.62 g, 10.06 mmol) dropwise at 0 °C, the mixture was stirred at r.t. for 1 h. Then sat. NaHCO₃ solution (100 mL) was added into the mixture, the mixture was extracted with DCM (100 mL×3). The combined organic phase was washed with brine, dried with anhydrous Na₂SO₄, filtered and concentrated, the residue was purified by column chromatography to give **compound 26-d** (650 mg). LCMS (M+H)⁺: 320.

### Step 5~8: preparation of 6-(difluoromethyl)-1-[3-(2-ethyl-4-hydroxy-5-methyl-pyrazol-3-yl)-1H-1,2,4-triazol-5-yl]imidazo[1,5-a]pyrazine-3-carboxamide (Example 26)

**Example 26** was prepared in analogy to the preparation of **Example 5** by using **compound 26-d** instead of methyl 4-acetyl-6-bromo-pyridine-2-carboxylate **(5-f). Example 26** (17.1 mg) was obtained. LCMS (M+H)⁺: 404. ¹H NMR (400 MHz, DMSO-*d₆*+D₂O) δ = 9.78 (s, 1H), 9.53 (s, 1H), 7.15 (t, *J =* 54.4 Hz, 1H), 4.56 - 4.39 (m, 2H), 2.15 (s, 3H), 1.34 (t, *J=* 7.0 Hz, 3H).

### Example 27

### 7-[3-(2-ethyl-4-hydroxy-5-methyl-pyrazol-3-yl)-1,2,4-oxadiazol-5-yl]-3-methyl-pyrrolo[1,2-c]pyrimidine-5-carboxamide

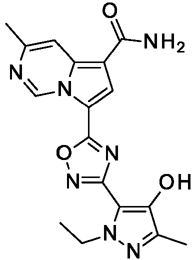

The title compound was prepared according to the following scheme:

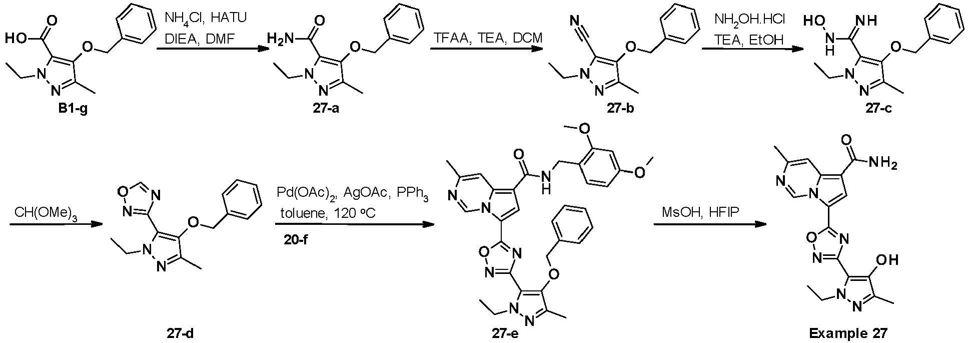

### Step 1: preparation of 4-benzyloxy-2-ethyl-5-methyl-pyrazole-3-carboxamide (compound 27-a)

To a solution of 4-benzyloxy-2-ethyl-5-methyl-pyrazole-3-carboxylic acid **(B1-g,** 1.0 g, 3.84 mmol) in DMF (10 mL) was added DIEA (1.48 g, 11.53 mmol), NH₄Cl (616.5 mg, 11.53 mmol) and HATU (2.19 g, 5.76 mmol), the mixture was stirred at 50 °C for 2 hrs. Then the mixture was concentrated, the residue was purified by reversed-phase column chromatography to give **compound 27-a** (850 mg). LCMS (M+H)⁺: 260.

### Step 2: preparation of 4-benzyloxy-2-ethyl-5-methyl-pyrazole-3-carbonitrile (compound 27-b)

To a solution of 4-benzyloxy-2-ethyl-5-methyl-pyrazole-3-carboxamide **(27-a,** 0.6 g, 2.31 mmol) in DCM (5 mL) was added TEA (708.1 mg, 6.94 mmol), then TFAA (972 mg, 4.63 mmol) in DCM (2 mL) was added dropwise at -5 °C, the mixture was stirred at 0 °C for 1 h. Then NaHCO₃ solution (5%, 100 mL) was added into the mixture, the mixture was extracted with EA (250 mL×2). The combined organic phase was washed with brine, dried with anhydrous Na₂SO₄, filtered and concentrated, the residue was purified by column chromatography to give **compound 27-b** (0.5 g). LCMS (M+H)⁺: 242.

### Step 3: preparation of 4-benzyloxy-2-ethyl-N-hydroxy-5-methyl-pyrazole-3-carboxamidine (compound 27-c)

The mixture of 4-benzyloxy-2-ethyl-5-methyl-pyrazole-3-carbonitrile **(27-b,** 0.4 g, 1.66 mmol), hydroxyamine hydrochloride (0.34 g, 4.97 mmol) and TEA (2.29 mL, 16.58 mmol) in EtOH (10 mL) was stirred at 80 °C for 1 h. Then the mixture was extracted with EA (150 mL×2). The combined organic phase was washed with brine, dried with anhydrous Na₂SO₄, filtered and concentrated, the residue was purified by column chromatography to give **compound 27-c** (450 mg). LCMS (M+H)⁺: 275.

### Step 4: preparation of 3-(4-benzyloxy-2-ethyl-5-methyl-pyrazol-3-yl)-1,2,4-oxadiazole (compound 27-d)

To a solution of 4-benzyloxy-2-ethyl-N-hydroxy-5-methyl-pyrazole-3-carboxamidine **(27-c,** 0.4 g, 1.46 mmol) in trimethyl orthoformate (154.7 mg, 1.46 mmol) was added *p-*toluenesulfonic acid monohydrate (0.04 g, 0.21 mmol), the mixture was stirred at 110 °C for 1 h. Then NaHCO₃ solution (5%, 100 mL) was added into the mixture, the mixture was extracted with EA (150 mL×2). The combined organic phase was washed with brine, dried with anhydrous Na₂SO₄, filtered and concentrated, the residue was purified by column chromatography to give **compound 27-d** (350 mg). LCMS (M+H)⁺: 285.

### Step 5: preparation of 7-[3-(4-benzyloxy-2-ethyl-5-methyl-pyrazol-3-yl)-1,2,4-oxadiazol-5-yl]-N-[(2,4-dimethoxyphenyl)methyl]-3-methyl-pyrrolo[1,2-c]pyrimidine-5-carboxamide (compound 27-e)

To a solution of 3-(4-benzyloxy-2-ethyl-5-methyl-pyrazol-3-yl)-1,2,4-oxadiazole **(27-d,** 200 mg, 0.7 mmol) in toluene (5 mL) was added 7-bromo-N-[(2,4-dimethoxyphenyl)methyl]-3-methyl-pyrrolo[1,2-c]pyrimidine-5-carboxamide **(20-f,** 200 mg, 0.49 mmol), Pd(OAc)₂ (15.8 mg, 0.07 mmol), PPh₃ (36.9 mg, 0.14 mmol) and AgOAc (350.3 mg, 2.11 mmol), the mixture was stirred at 120 °C for 12 hrs. Then the mixture was concentrated, the residue was purified by prep-TLC to give **compound 27-e** (18 mg). LCMS (M+H)⁺: 608.

### Step 6: preparation of 7-[3-(2-ethyl-4-hydroxy-5-methyl-pyrazol-3-yl)-1,2,4-oxadiazol-5-yl]-3-methyl-pyrrolo[1,2-c]pyrimidine-5-carboxamide (Example 27)

**Example 27** was prepared in analogy to the preparation of **Example 21** by using **compound 27-e** instead of 1-[2-(4-benzyloxy-2-ethyl-5-methyl-pyrazol-3-yl)oxazol-4-yl]-N-[(2,4-dimethoxyphenyl)methyl]-5-methyl-pyrazolo[3,4-c]pyridine-3-carboxamide **(21-m). Example 27** (2.1 mg) was obtained. LCMS (M+H)⁺: 368. ¹H NMR (400 MHz, DMSO-*d₆*+D₂O) δ = 10.11 (s, 1H), 8.43 (s, 1H), 8.17 (s, 1H), 4.39 (d, *J =* 6.8 Hz, 2H), 2.54 (s, 3H), 2.15 (s, 3H), 1.34 (t, *J=* 7.2 Hz, 3H).

### Example 28

### 8-[2-(2-ethyl-4-hydroxy-5-methyl-pyrazol-3-yl)oxazol-4-yl]-7-fluoro-3-methyl-pyrrolo[1,2-a]pyrazine-6-carboxamide

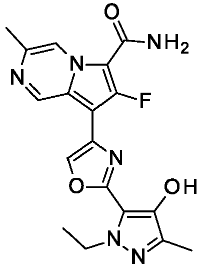

The title compound was prepared according to the following scheme:

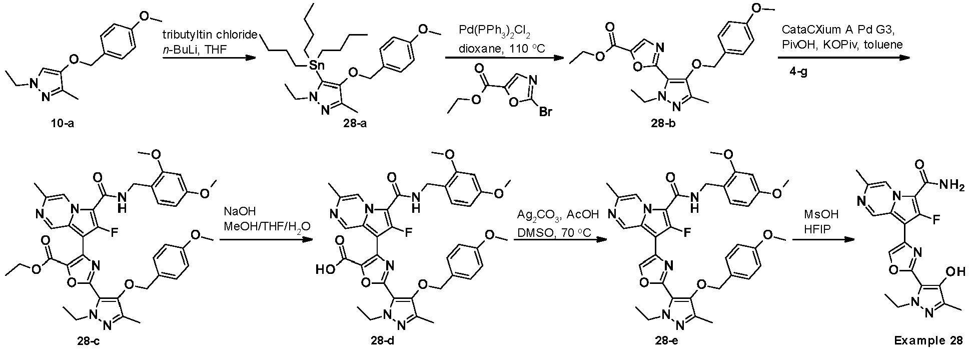

### Step 1: preparation of tributyl-[2-ethyl-4-[(4-methoxyphenyl)methoxy]-5-methyl-pyrazol-3-yl]stannane (compound 28-a)

To a solution of 1-ethyl-4-[(4-methoxyphenyl)methoxy]-3-methyl-pyrazole **(10-a,** 2.0 g, 8.12 mmol) in THF (20 mL) was added n-BuLi (6.5 mL, 16.24 mmol, 2.5 N in hexane) at -78 °C dropwise, the mixture was stirred at -78 °C for 1 h. Then tributyltin chloride (5.7 g, 17.51 mmol) was added into the mixture at -78 °C, the mixture was stirred at r.t. for 3 hrs. Then the mixture was poured into water (100 mL), HCl (1 N) was added to adjust to pH~5. The mixture was extracted with DCM (50 mL×3). The combined organic phase was washed with brine, dried with anhydrous Na₂SO₄, filtered and concentrated, the residue was purified by column chromatography to give **compound 28-a** (3.7 g). LCMS (M+H)⁺: 537.

### Step 2: preparation of ethyl 2-(1-ethyl-4-((4-methoxybenzyl)oxy)-3-methyl-1H-pyrazol-5-yl)oxazole-5-carboxylate (compound 28-b)

To a solution of ethyl 2-bromooxazole-5-carboxylate (1.0 g, 4.55 mmol) in 1,4-Dioxane (40 mL) was added tributyl-[2-ethyl-4-[(4-methoxyphenyl)methoxy]-5-methyl-pyrazol-3-yl]stannane **(28-a,** 3.65 g, 6.82 mmol) and Pd(PPh₃)₂Cl₂ (0.32 g, 0.45 mmol), the mixture was stirred at 110 °C for 18 hrs. Then the mixture was filtered and washed with EA. The filtrate was concentrated, the residue was purified by column chromatography to give **compound 28-b** (720 mg). LCMS (M+H)⁺: 386.

### Step 3: preparation of ethyl 4-[6-[(2,4-dimethoxyphenyl)methylcarbamoyl]-7-fluoro-3-methyl-pyrrolo[1,2-a]pyrazin-8-yl]-2-[2-ethyl-4-[(4-methoxyphenyl)methoxy]-5-methyl-pyrazol-3-yl]oxazole-5-carboxylate (compound 28-c)

To a solution of 8-bromo-*N*-[(2,4-dimethoxyphenyl)methyl]-7-fluoro-3-methyl-pyrrolo[1,2-a]pyrazine-6-carboxamide **(4-g,** 322 mg, 0.76 mmol) and ethyl 2-(1-ethyl-4-((4-methoxybenzyl)oxy)-3-methyl-1H-pyrazol-5-yl)oxazole-5-carboxylate **(28-b,** 294.8 mg, 0.76 mmol) in toluene (1 mL) was added KOPiv (321.8 mg, 2.29 mmol), CataCXium A Pd G3 (167.1 mg, 0.23 mmol) and PivOH (46.8 mg, 0.46 mmol), the mixture was stirred at 120 °C for 12 hrs. Then the mixture was diluted with DCM/MeOH (V/V=10/1, 20 mL), filtered and concentrated, the residue was purified by prep-TLC to give **compound 28-c** (30 mg). LCMS (M+H)⁺: 727.

### Step 4~6: preparation of 8-[2-(2-ethyl-4-hydroxy-5-methyl-pyrazol-3-yl)oxazol-4-yl]-7-fluoro-3-methyl-pyrrolo[1,2-a]pyrazine-6-carboxamide (Example 28)

**Example 28** was prepared in analogy to the preparation of **Example 21** by using **compound 28-c** instead of ethyl 2-(4-benzyloxy-2-ethyl-5-methyl-pyrazol-3-yl)-4-[3-[(2,4-dimethoxyphenyl)methylcarbamoyl]-5-methyl-pyrazolo[3,4-c]pyridin-1-yl]oxazole-5-carboxylate **(21-k). Example 28** (3.7 mg) was obtained. LCMS (M+H)⁺: 385. ¹H NMR (400 MHz, DMSO-*d₆*) δ = 9.55 (s, 1H), 9.15 (s, 1H), 8.77 (br s, 1H), 8.58 (s, 1H), 7.79 (s, 1H), 7.41 (s, 1H), 4.50 (q, *J =* 6.8 Hz, 2H), 2.45 (s, 3H), 2.15 (s, 3H), 1.37 (t, *J* = 7.2 Hz, 3H).

### Example 29

### 1-[2-(2-ethyl-4-hydroxy-5-methyl-pyrazol-3-yl)oxazol-4-yl]-5-methyl-pyrrolo[2,3-c]pyridine-3-carboxamide

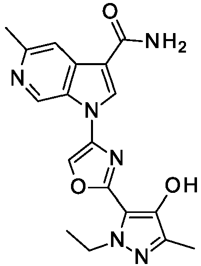

The title compound was prepared according to the following scheme:

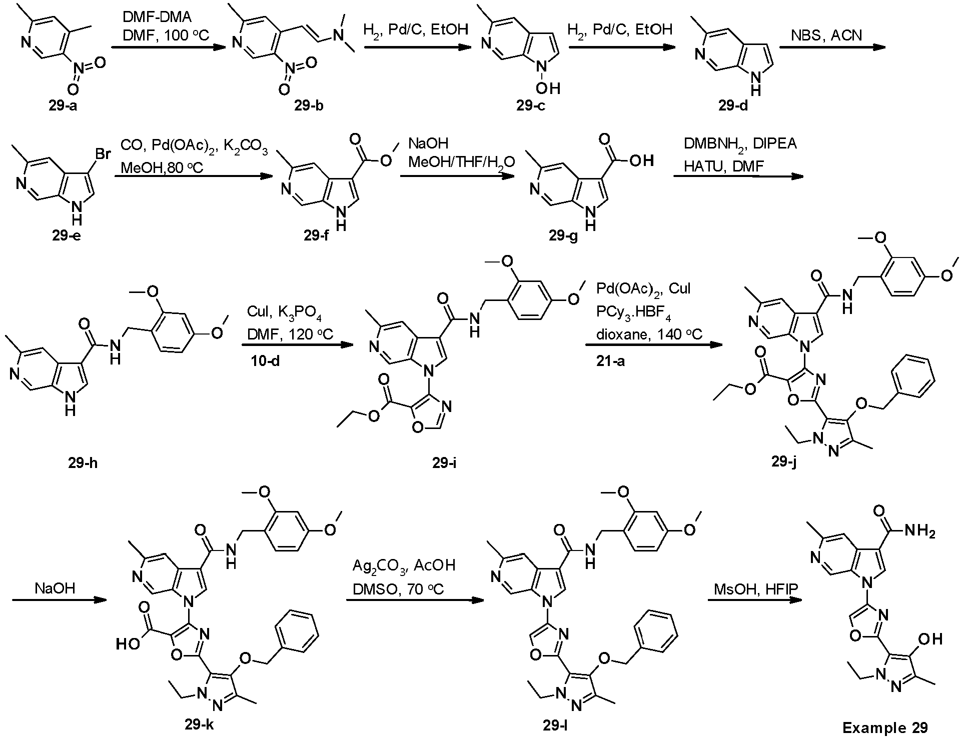

### Step 1: preparation of (E)-N,N-dimethyl-2-(2-methyl-5-nitro-4-pyridyl)ethenamine (compound 29-b)

To a solution of 2,4-dimethyl-5-nitro-pyridine **(29-a,** 20.0 g, 131.45 mmol) in DMF (400 mL) was added DMF-DMA (32.29 g, 271.0 mmol), the mixture was stirred at 100 °C for 2 hrs. Then the mixture was purified by column chromatography to give **compound 29-b** (24.0 g). LCMS (M+H)⁺: 208.

### Step 2: preparation of 1-hydroxy-5-methyl-pyrrolo[2,3-c]pyridine (compound 29-c)

The mixture of (E)-*N,N*-dimethyl-2-(2-methyl-5-nitro-4-pyridyl)ethenamine **(29-b,** 24.0 g, 115.81 mmol) and Pd/C (10.0 g) in EtOH (300 mL) was stirred under H₂ (15 psi) at r.t. for 12 hrs. Then the mixture was filtered through dicalite and concentrated to give **compound 29-c** (20.0 g). LCMS (M+H)⁺: 149.

### Step 3: preparation of 5-methyl-1H-pyrrolo[2,3-c]pyridine (compound 29-d)

The mixture of 1-hydroxy-5-methyl-pyrrolo[2,3-c]pyridine **(29-c,** 20.0 g, 134.99 mmol) and Pd/C (10.0 g) in EtOH (300 mL) was stirred under H₂ (15 psi) at r.t. for 12 hrs. Then the mixture was filtered through dicalite and concentrated, the residue was purified by reversed-phase column chromatography to give **compound 29-d** (10.0 g). LCMS (M+H)⁺: 133.

### Step 4: preparation of 3-bromo-5-methyl-1H-pyrrolo[2,3-c]pyridine (compound 29-e)

To a solution of NBS (4.04 g, 22.7 mmol) in ACN (50 mL) was added 5-methyl-1H-pyrrolo[2,3-c]pyridine **(29-d,** 3.0 g, 22.7 mmol), the mixture was stirred at r.t. for 12 hrs. Then the mixture was filtered and concentrated to give **compound 29-e** (3.0 g). LCMS (M+H)⁺: 211.

### Step 5: preparation of methyl 5-methyl-1H-pyrrolo[2,3-c]pyridine-3-carboxylate (compound 29-f)

To a solution of 3-bromo-5-methyl-1H-pyrrolo[2,3-c]pyridine **(29-e,** 3.0 g, 14.21 mmol) in MeOH (60 mL) was added Pd(OAc)₂ (1.27 g, 5.69 mmol), dicyclohexyl(3-dicyclohexylphosphaniumylpropyl)phosphanium;ditetrafluoroborate (DCPP, 3.48 g, 5.69 mmol) and K₂CO₃ (5.88 g, 42.64 mmol), the mixture was stirred at 80 °C for 12 hrs under CO (50 Psi). Then the mixture was filtrated and concentrated, the residue was purified by reversed-phase column chromatography to give **compound 29-f** (2.0 g). LCMS (M+H)⁺: 191.

### Step 6: preparation of 5-methyl-1H-pyrrolo[2,3-c]pyridine-3-carboxylic acid (compound 29-g)

To a solution of methyl 5-methyl-1H-pyrrolo[2,3-c]pyridine-3-carboxylate (29-f, 2.0 g, 10.52 mmol) in THF/H₂O/MeOH (V/V/V=1/1/1, 30 mL) was added NaOH (4.21 g, 105.15 mmol), the mixture was stirred at 60 °C for 12 hrs. Then the mixture was purified by reversed-phase column chromatography give **compound 29-g** (1.7 g). LCMS (M-H)⁻: 175.

### Step 7: preparation of N-[(2,4-dimethoxyphenyl)methyl]-5-methyl-1H-pyrrolo[2,3-c]pyridine-3-carboxamide (compound 29-h)

**Compound 29-h** was prepared in analogy to the preparation of **compound 21-h** by using **compound 29-g** instead of 5-methyl-1-(2-trimethylsilylethoxymethyl)pyrazolo[3,4-c]pyridine-3-carboxylic acid **(21-g). Compound 29-h** (1.1 g) was obtained. LCMS (M-H)⁻: 324.

### Step 8~12: preparation of 1-[2-(2-ethyl-4-hydroxy-5-methyl-pyrazol-3-yl)oxazol-4-yl]-5-methyl-pyrrolo[2,3-c]pyridine-3-carboxamide (Example 29)

**Example 29** was prepared in analogy to the preparation of **Example 21** by using **compound 29-h** instead of N-[(2,4-dimethoxyphenyl)methyl]-5-methyl-1*H*-pyrazolo[3,4-c]pyridine-3-carboxamide **(21-i). Example 29** (2.9 mg) was obtained. LCMS (M+H)⁺: 367. ¹H NMR (400 MHz, DMSO-*d₆*+D₂O) δ 9.29 (s, 1H), 9.03 (s, 1H), 8.79 (s, 1H), 8.39 (s, 1H), 4.44 (q, *J=* 7.0 Hz, 2H), 2.73 (s, 3H), 2.13 (s, 3H), 1.34 (t, *J =* 7.2 Hz, 3H).

### Example 30

### 8-[3-[2-(2-cyanoethyl)-4-hydroxy-5-methyl-pyrazol-3-yl]-1H-1,2,4-triazol-5-yl]-7-fluoro-3-methyl-pyrrolo[1,2-a]pyrazine-6-carboxamide

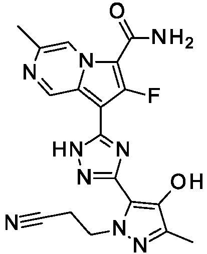

The title compound was prepared according to the following scheme:

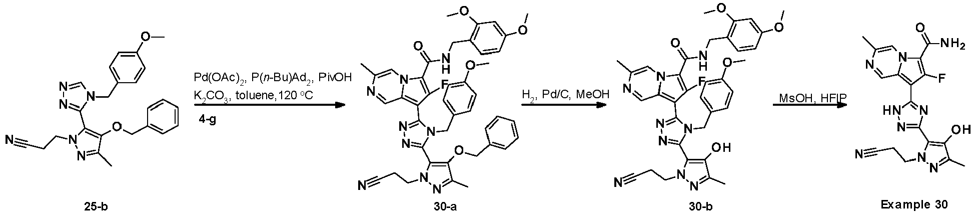

### Step 1~2: preparation of 8-[5-[2-(2-cyanoethyl)-4-hydroxy-5-methyl-pyrazol-3-yl]-4-[(4-methoxyphenyl)methyl]-1,2,4-triazol-3-yl]-N-[(2,4-dimethoxyphenyl)methyl]-7-fluoro-3-methyl-pyrrolo[1,2-a]pyrazine-6-carboxamide (compound 30-b)

**Compound 30-b** was prepared in analogy to the preparation of **compound 25-d** by using **compound 4-g** instead of 4-bromo-N-[(2,4-dimethoxyphenyl)methyl]-1-methyl-pyrazolo[4,3-c]pyridine-6-carboxamide **(Intermediate** A2). **Compound 30-b** (16 mg) was obtained. LCMS (M+H)⁺: 680.

### Step 3: preparation of 8-[3-[2-(2-cyanoethyl)-4-hydroxy-5-methyl-pyrazol-3-yl]-1H-1,2,4-triazol-5-yl]-7-fluoro-3-methyl-pyrrolo[1,2-a]pyrazine-6-carboxamide (Example 30)

**Example 30** was prepared in analogy to the preparation of **Example 21** by using **compound 30-b** instead of 1-[2-(4-benzyloxy-2-ethyl-5-methyl-pyrazol-3-yl)oxazol-4-yl]-N-[(2,4-dimethoxyphenyl)methyl]-5-methyl-pyrazolo[3,4-c]pyridine-3-carboxamide (21-m). **Example 30** (1.5 mg) was obtained. LCMS (M+H)⁺: 410. ¹H NMR (400 MHz, DMSO-*d₆*) δ = 14.20 - 13.95 (m, 1H), 9.61 (s, 1H), 9.38 - 9.26 (m, 1H), 9.22 (s, 1H), 7.89 - 7.77 (m, 1H), 7.57 - 7.39 (m, 1H), 4.77 (br t, *J=* 6.6 Hz, 2H), 3.11 (t, *J=* 6.6 Hz, 2H), 2.48 (s, 3H), 2.18 (s, 3H).

### Example 31

### 1-[3-(4-hydroxy-5-methyl-2-propyl-pyrazol-3-yl)-1H-1,2,4-triazol-5-yl]-5-methyl-pyrazolo[3,4-c]pyridine-3-carboxamide

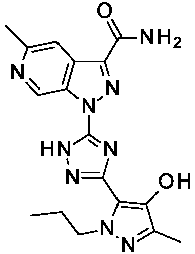

The title compound was prepared according to the following scheme:

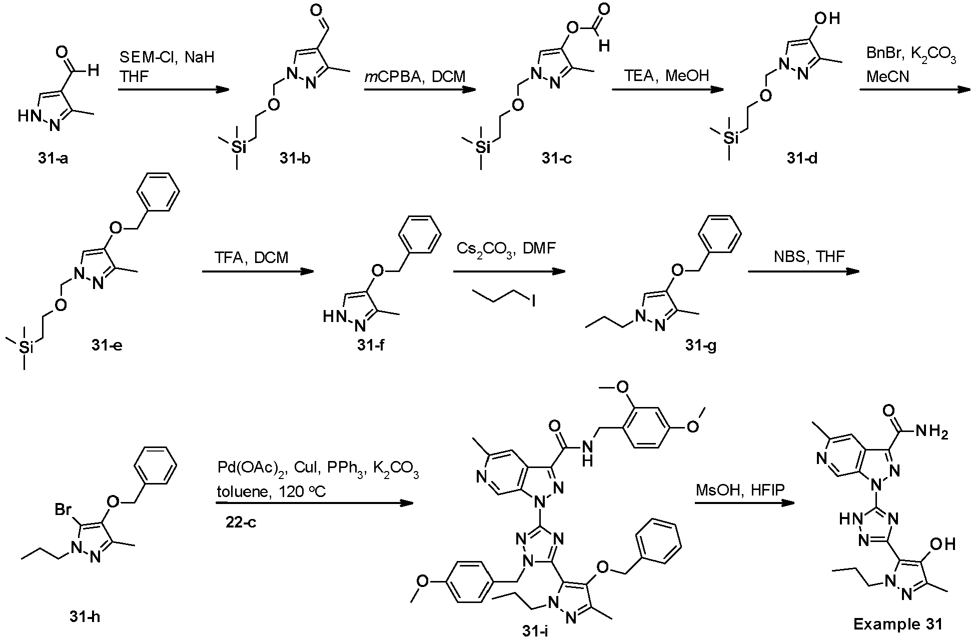

### Step 1: preparation of 3-methyl-1-(2-trimethylsilylethoxymethyl)pyrazole-4-carbaldehyde (compound 31-b)

To a mixture of NaH (871.9 mg, 21.8 mmol) in THF (15 mL) was added the solution of 3-methyl-1H-pyrazole-4-carbaldehyde (32-a, 2.0 g, 18.16 mmol) in THF (15 mL) dropwise at 0 °C, the mixture was stirred at 0 °C for 30 min. Then SEMCl (4.54 g, 27.25 mmol) was added into the mixture dropwise. The mixture was stirred at r.t. for 16 hrs. Then the mixture was poured into water (50 mL), extracted with EA (50 mL×3). The combined organic phase was washed with brine, dried with anhydrous Na₂SO₄, filtered, and concentrated. The residue was purified by column chromatography to give **compound 32-b** (3.8 g). LCMS (M+H)⁺: 241.

### Step 2~4: preparation of 2-[(4-benzyloxy-3-methyl-pyrazol-1-yl)methoxy]ethyl-trimethyl-silane (compound 31-e)

**Compound 31-e** was prepared in analogy to the preparation of **compound B1-f** by using **compound 31-b** instead of 1-ethyl-3-methyl-pyrazole-4-carbaldehyde **(B1-c). Compound 31-e** (3.13 g) was obtained. LCMS (M+H)⁺: 319.

### Step 5: preparation of 4-benzyloxy-3-methyl-1H-pyrazole (compound 31-f)

To a solution of 2-[(4-benzyloxy-3-methyl-pyrazol-1-yl)methoxy]ethyl-trimethyl-silane **(31-e,** 2.0 g, 6.28 mmol) in DCM (30 mL) was added TFA (14.8 g, 129.8 mmol), the mixture was stirred at r.t. for 12 hrs. Then the mixture was concentrated, the residue was dissolved in DCM (10 mL). Then sat. NaHCO₃ solution was added to adjust to pH~8. The mixture was then extracted with DCM (50 mL×3), the combined organic phase was washed with brine, dried with anhydrous Na₂SO₄, filtered, and concentrated. The residue was purified by column chromatography to give **compound 31-f** (1.2 g). LCMS (M+H)⁺: 189.

### Step 6: preparation of 4-benzyloxy-3-methyl-1-propyl-pyrazole (compound 31-g)

To a solution of 4-benzyloxy-3-methyl-1H-pyrazole **(31-f,** 500 mg, 2.66 mmol) in DMF (10 mL) was added 1-iodopropane (903.1 mg, 5.31 mmol) and Cs₂CO₃ (2.59 g, 7.97 mmol), the mixture was stirred at 80 °C for 16 hrs. Then the mixture was diluted with water (10 mL), extracted with EA (30 mL×3). The combined organic phase was washed with brine, dried with anhydrous Na₂SO₄, filtered, and concentrated. The residue was purified by prep-TLC to give **compound 31-g** (450 mg). LCMS (M+H)⁺: 231.

### Step 7: preparation of 4-benzyloxy-5-bromo-3-methyl-1-propyl-pyrazole (compound 31-h)

**Compound 31-h** was prepared in analogy to the preparation of **compound 21-a** by using **compound 31-g** instead of 4-benzyloxy-1-ethyl-3-methyl-pyrazole **(B1-f). Compound 31-h** (200 mg) was obtained. LCMS (M+H)⁺: 309.

### Step 8: preparation of 1-[5-(4-benzyloxy-5-methyl-2-propyl-pyrazol-3-yl)-1-[(4-methoxyphenyl)methyl]-1,2,4-triazol-3-yl]-N-[(2,4-dimethoxyphenyl)methyl]-5-methyl-pyrazolo[3,4-c]pyridine-3-carboxamide (compound 31-i)

The solution of *N*-[(2,4-dimethoxyphenyl)methyl]-1-[1-[(4-methoxyphenyl)methyl]-1,2,4-triazol-3-yl]-5-methyl-pyrazolo[3,4-c]pyridine-3-carboxamide **(22-c,** 120 mg, 0.23 mmol), 4-benzyloxy-5-bromo-3-methyl-1-propyl-pyrazole **(31-h,** 72.2 mg, 0.23 mmol), Pd(OAc)₂ (10.5 mg, 0.05 mmol), CuI (14.3 mg, 0.14 mmol), PPh₃ (33.5 mg, 0.09 mmol) and K₂CO₃ (96.7 mg, 0.7 mmol) in toluene (3 mL) was stirred at 120 °C for 12 hrs under N₂. Then the mixture was diluted with DCM (10 mL) and filtered. The filtrate was concentrated, the residue was purified by prep-TLC to give **compound 31-i** (10 mg). LCMS (M+H)⁺: 742.

### Step 9: preparation of 1-[3-(4-hydroxy-5-methyl-2-propyl-pyrazol-3-yl)-1H-1,2,4-triazol-5-yl]-5-methyl-pyrazolo[3,4-c]pyridine-3-carboxamide (Example 31)

**Example 31** was prepared in analogy to the preparation of **Example 21** by using **compound 31-i** instead of 1-[2-(4-benzyloxy-2-ethyl-5-methyl-pyrazol-3-yl)oxazol-4-yl]-*N-*[(2,4-dimethoxyphenyl)methyl]-5-methyl-pyrazolo[3,4-c]pyridine-3-carboxamide **(21-m). Example 31** (1.7 mg) was obtained. LCMS (M+H)⁺: 382. ¹H NMR (400 MHz, DMSO-*d₆*) δ = 14.20 (br s, 1H), 9.64 (s, 1H), 9.20 (s, 1H), 8.06 (s, 2H), 7.78 (br s, 1H), 4.43 (t, *J =* 6.8 Hz, 2H), 2.67 (s, 3H), 2.18 (s, 3H), 1.86 - 1.75 (m, 2H), 0.87 (t, *J=* 7.2 Hz, 3H).

### Example 32

### 1-[3-[2-(cyclopropylmethyl)-4-hydroxy-5-methyl-pyrazol-3-yl]-1H-1,2,4-triazol-5-yl]-5-methyl-pyrazolo[3,4-c]pyridine-3-carboxamide

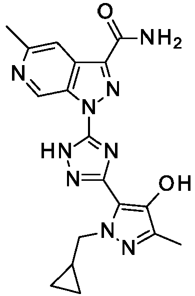

**Example 32** was prepared in analogy to the preparation of **Example 31** by using bromomethylcyclopropane instead of 1-iodopropane. **Example 32** (3.4 mg) was obtained. LCMS (M+H)⁺: 394. ¹H NMR (400 MHz, DMSO-*d₆*) δ = 9.61 (s, 1H), 8.04 - 7.95 (m, 2H), 7.70 (br s, 1H), 4.37 (d, *J =* 6.8 Hz, 2H), 2.65 (s, 3H), 2.15 (s, 3H), 1.37 - 1.28 (m, 1H), 0.47 - 0.41 (m, 2H), 0.40 - 0.35 (m, 2H).

### Example 33

### 1-[3-(2-cyclopropyl-4-hydroxy-5-methyl-pyrazol-3-yl)-1H-1,2,4-triazol-5-yl]-5-methyl-pyrazolo[3,4-c]pyridine-3-carboxamide

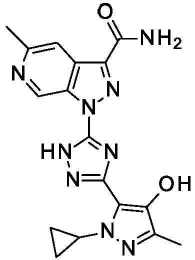

The title compound was prepared according to the following scheme:

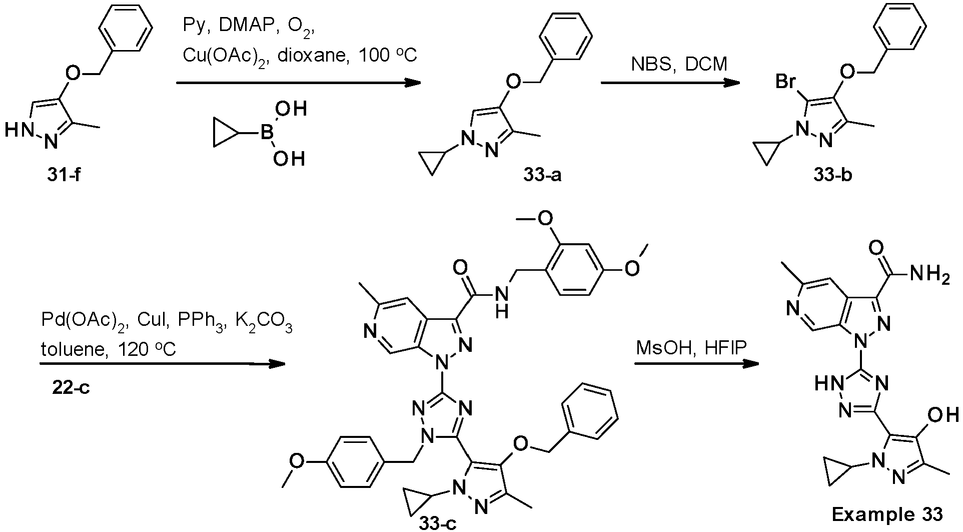

### Step 1: preparation of 4-benzyloxy-1-cyclopropyl-3-methyl-pyrazole (compound 33-a)

To a solution of 4-benzyloxy-3-methyl-1H-pyrazole (31-f, 500 mg, 2.66 mmol) in 1,4-Dioxane (20 mL) was added cyclopropylboronic acid (456.4 mg, 5.31 mmol), Cu(OAc)₂ (482.5 mg, 2.66 mmol), DMAP (1.29 g, 10.63 mmol) and Py (524.6 mg, 6.64 mmol), the mixture was stirred at 100 °C for 16 hrs under O₂. Then the mixture was diluted with water (50 mL), extracted with EA (30 mL×2). The combined organic phase was washed with brine, dried with anhydrous Na₂SO₄, filtered, and concentrated. The residue was purified by column chromatography to give **compound 33-a** (540 mg). LCMS (M+H)⁺: 229.

### Step 2~4: preparation of 1-[3-(2-cyclopropyl-4-hydroxy-5-methyl-pyrazol-3-yl)-1H-1,2,4-triazol-5-yl]-5-methyl-pyrazolo[3,4-c]pyridine-3-carboxamide (Example 33)

**Example 33** was prepared in analogy to the preparation of **Example 31** by using **compound 33-a** instead of 4-benzyloxy-3-methyl-1-propyl-pyrazole **(31-g). Example 33** (1.5 mg) was obtained. LCMS (M+H)⁺: 380. ¹H NMR (400 MHz, DMSO-*d₆*) δ = 14.31 (br s, 1H), 9.67 (d, *J* = 1.2 Hz, 1H), 9.15 (br s, 1H), 8.10-8.00 (m, 2H), 7.77 (br s, 1H), 4.20-4.16 (m, 1H), 2.67 (s, 3H), 2.15 (s, 3H), 1.07-1.05 (m, 2H), 0.99-0.96 (m, 2H).

### Example 34

### 4-[3-[2-(3,3-difluoroallyl)-4-hydroxy-5-methyl-pyrazol-3-yl]-1H-1,2,4-triazol-5-yl]-1-methyl-pyrazolo[4,3-c]pyridine-6-carboxamide

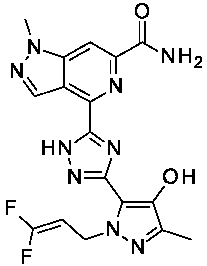

The title compound was prepared according to the following scheme:

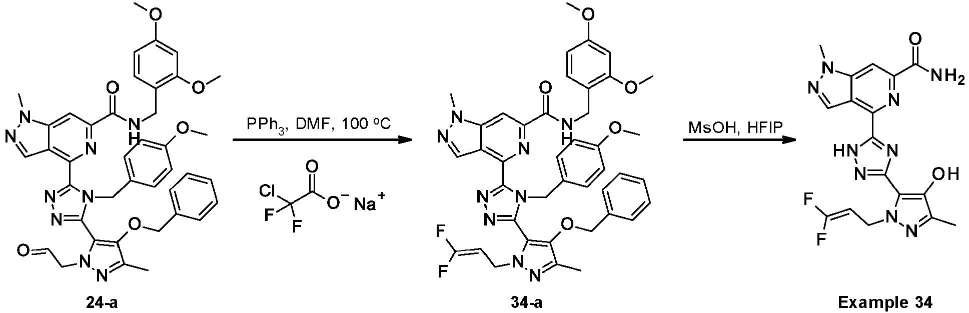

### Step 1: preparation of 4-[5-[4-benzyloxy-2-(3,3-difluoroallyl)-5-methyl-pyrazol-3-yl]-4-[(4-methoxyphenyl)methyl]-1,2,4-triazol-3-yl]-N-[(2,4-dimethoxyphenyl)methyl]-1-methyl-pyrazolo[4,3-c]pyridine-6-carboxamide (compound 34-a)

To a solution of 4-[5-[4-benzyloxy-5-methyl-2-(2-oxoethyl)pyrazol-3-yl]-4-[(4-methoxyphenyl)methyl]-1,2,4-triazol-3-yl]-N-[(2,4-dimethoxyphenyl)methyl]-1-methyl-pyrazolo[4,3-c]pyridine-6-carboxamide **(24-a,** 50 mg, 0.07 mmol) and PPh₃ (22.1 mg, 0.08 mmol) in DMF (1 mL) was added sodium 2-chloro-2,2-difluoro-acetate (16.1 mg, 0.11 mmol), the mixture was stirred at 100 °C for 12 hrs. Then the mixture was added water (50 mL), extracted with EA (50 mL×3). The combined organic phase was washed with water, dried with anhydrous Na₂SO₄, filtered, and concentrated. The residue was purified by prep-TLC to give **compound 34-a** (12 mg). LCMS (M+H)⁺: 776.

### Step 2: preparation of 4-[3-[2-(3,3-difluoroallyl)-4-hydroxy-5-methyl-pyrazol-3-yl]-1H-1,2,4-triazol-5-yl]-1-methyl-pyrazolo[4,3-c]pyridine-6-carboxamide (Example 34)

**Example 34** was prepared in analogy to the preparation of **Example 21** by using **compound 34-a** instead of 1-[2-(4-benzyloxy-2-ethyl-5-methyl-pyrazol-3-yl)oxazol-4-yl]-N-[(2,4-dimethoxyphenyl)methyl]-5-methyl-pyrazolo[3,4-c]pyridine-3-carboxamide **(21-m). Example 34** (1.3 mg) was obtained. LCMS (M+H)⁺: 416. ¹H NMR (400 MHz, METHANOL-*d₄*) δ = 8.86 (s, 1H), 8.43 (s, 1H), 5.26 (br d, *J =* 7.6 Hz, 2H), 4.90 (s, 1H), 4.23 (s, 3H), 2.25 (s, 3H).

### Example 35

### 4-[3-[4-hydroxy-5-methyl-2-[2-(triazol-2-yl)ethyl]pyrazol-3-yl]-1H-1,2,4-triazol-5-yl]-1-methyl-pyrazolo[4,3-c]pyridine-6-carboxamide

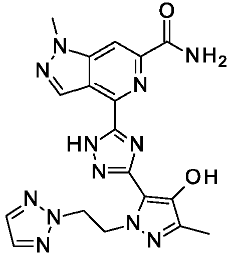

**Example 35** was prepared in analogy to the preparation of **Example 23** by using 1,2,3-triazole instead of imidazole. **Example 35** (7.1 mg) was obtained. LCMS (M+H)⁺: 435. ¹H NMR (400 MHz, DMSO-*d₆*) δ = 15.33 (br s, 1H), 8.90-8.86 (m, 2H), 8.46 (s, 1H), 8.05 (br s, 1H), 7.87 (br s, 1H), 7.66 (s, 2H), 4.98 (t, *J =* 6.2 Hz, 2H), 4.87 (t, *J =* 6.2 Hz, 2H), 4.22 (s, 3H), 2.12 (s, 3H).

### Example 36

### 4-[3-[4-hydroxy-5-methyl-2-[2-(triazol-1-yl)ethyl]pyrazol-3-yl]-1H-1,2,4-triazol-5-yl]-1-methyl-pyrazolo[4,3-c]pyridine-6-carboxamide

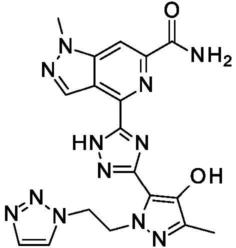

**Example 36** was prepared in analogy to the preparation of **Example 23** by using 1,2,3-triazole instead of imidazole. **Example 36** (5.1 mg) was obtained. LCMS (M+H)⁺: 435. ¹H NMR (400 MHz, DMSO-*d₆*) δ = 15.32 (br s, 1H), 8.86 (s, 2H), 8.46 (s, 1H), 8.08 (s, 1H), 7.8-7.9 (m, 2H), 7.57 (s, 1H), 4.96 (t, *J=* 5.2 Hz, 2H), 4.86 (t, *J =* 5.2 Hz, 2H), 4.23 (s, 3H), 2.12 (s, 3H).

### Example 37

### 4-[3-[2-(3,3-difluoropropyl)-4-hydroxy-5-methyl-pyrazol-3-yl]-1H-1,2,4-triazol-5-yl]-1-methyl-pyrazolo[4,3-c]pyridine-6-carboxamide

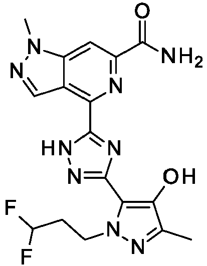

The title compound was prepared according to the following scheme:

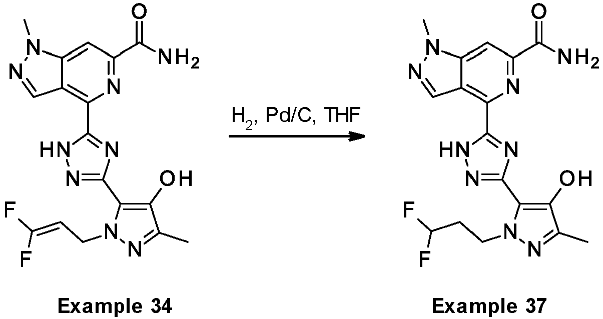

### Step 1: preparation of 4-[3-[2-(3,3-difluoropropyl)-4-hydroxy-5-methyl-pyrazol-3-yl]-1H-1,2,4-triazol-5-yl]-1-methyl-pyrazolo[4,3-c]pyridine-6-carboxamide (Example 37)

To a solution of 4-[3-[2-(3,3-difluoroallyl)-4-hydroxy-5-methyl-pyrazol-3-yl]-1*H*-1,2,4-triazol-5-yl]-1-methyl-pyrazolo[4,3-c]pyridine-6-carboxamide (Example 34, 10 mg, 0.02 mmol) in THF (3 mL) was added Pd/C (10 mg), the mixture was stirred at 30 °C for 12 hrs under H₂ balloon. Then the mixture was purified by reversed-phase column chromatography to give **Example 37** (2.9 mg). LCMS (M+H)⁺: 418. ¹H NMR (400 MHz, DMSO-*d₆*) δ = 15.39 (s, 1H), 8.86 (d, *J =* 1.6 Hz, 1H), 8.83 (s, 1H), 8.47 (s, 1H), 8.07 (s, 1H), 7.87 (d, *J =* 2.6 Hz, 1H), 6.34 - 6.00 (m, 1H), 4.64 (t, *J =* 7.4 Hz, 2H), 4.22 (s, 3H), 2.53 - 2.52 (m, 1H), 2.46 - 2.45 (m, 1H), 2.15 (s, 3H).

### Example 38

### 4-[3-[4-hydroxy-5-methyl-2-[2-(1,2,4-triazol-4-yl)ethyl]pyrazol-3-yl]-1H-1,2,4-triazol-5-yl]-1-methyl-pyrazolo [4,3-c]pyridine-6-carboxamide

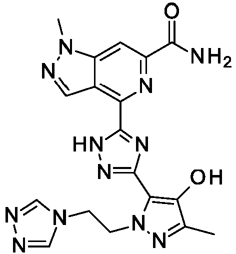

The title compound was prepared according to the following scheme:

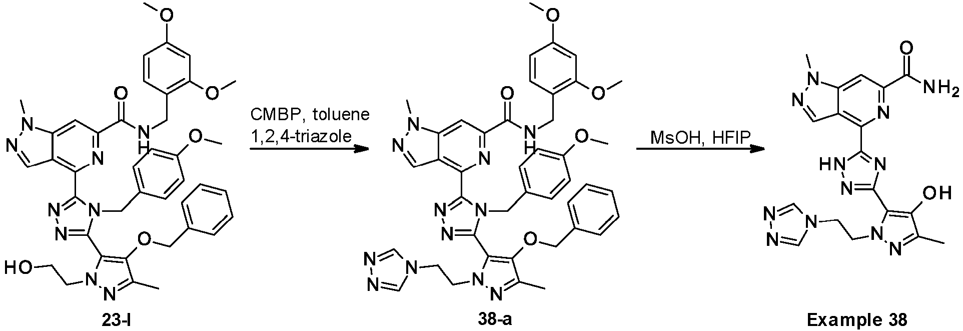

### Step 1: preparation of 4-[5-[4-benzyloxy-5-methyl-2-[2-(1,2,4-triazol-4-ylethyl]pyrazol-3-yl]-4-[(4-methoxyphenyl)methyl]-1,2,4-triazol-3-yl]-N-[(2,4-dimethoxyphenyl)methyl]-1-methyl-pyrazolo[4,3-c]pyridine-6-carboxamide (compound 38-a)

**Compound 38-a** was prepared in analogy to the preparation of **compound 23-m** by using 1,2,4-triazole instead of imidazole. The residue was purified by prep-TLC (DCM/MeOH=30/1), The minor spot (Rf =0.25) was defined as **Compound 38-a** (10 mg) without further structure conformation. LCMS (M+H)⁺: 795.

### Step 2: preparation of 4-[3-[4-hydroxy-5-methyl-2-[2-(1,2,4-triazol-4-yl)ethyl]pyrazol-3-yl]-1H-1,2,4-triazol-5-yl]-1-methyl-pyrazolo[4,3-c]pyridine-6-carboxamide (Example 38)

**Example 38** was prepared in analogy to the preparation of **Example 21** by using **compound 38-a** instead of 1-[2-(4-benzyloxy-2-ethyl-5-methyl-pyrazol-3-yl)oxazol-4-yl]-N-[(2,4-dimethoxyphenyl)methyl]-5-methyl-pyrazolo[3,4-c]pyridine-3-carboxamide **(21-m). Example 38** (2.0 mg) was obtained. LCMS (M+H)⁺: 435. ¹H NMR (400 MHz, DMSO-*d₆*) δ = 15.21 (s, 1H), 8.72 ( s, 1H), 8.24 (s, 1H), 8.09 (s, 1H), 7.93 (s, 1H), 7.86 (s, 1H), 7.61 (s, 1H), 7.34 (s, 1H), 4.87 (t, *J =* 5.8 Hz, 2H), 4.63 (t, *J =* 5.8 Hz, 2H), 2.97 (d, *J =* 4.4 Hz, 3H), 2.09 (s, 3H).

### Example 39

### 4-[3-[4-hydroxy-5-methyl-2-[2-(1,2,4-triazol-1-yl)ethyl]pyrazol-3-yl]-1H-1,2,4-triazol-5-yl]-1-methyl-pyrazolo[4,3-c]pyridine-6-carboxamide

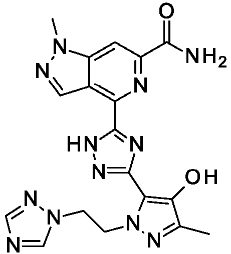

The title compound was prepared according to the following scheme:

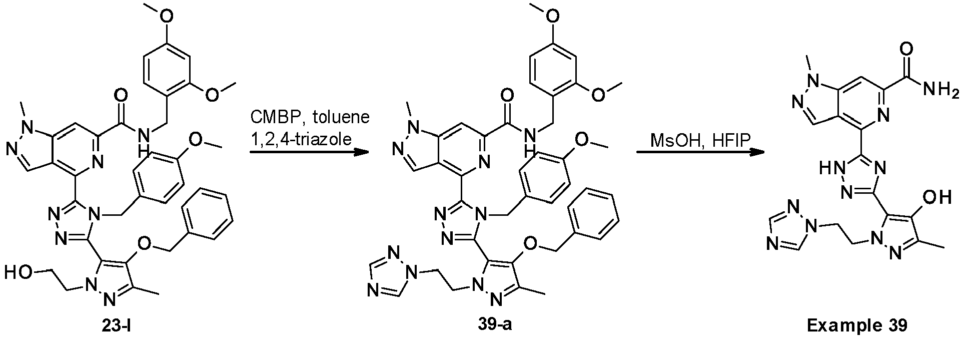

### Step 1: preparation of 4-[5-[4-benzyloxy-5-methyl-2-[2-(1,2,4-triazol-1-yl)ethyl]pyrazol-3-yl]-4-[(4-methoxyphenyl)methyl]-1,2,4-triazol-3-yl]-N-[(2,4-dimethoxyphenyl)methyl]-1-methyl-pyrazolo[4,3-c]pyridine-6-carboxamide (compound 39-a)

**Compound 39-a** was prepared in analogy to the preparation of **compound 23-m** by using 1,2,4-triazole instead of imidazole. The residue was purified by prep-TLC (DCM/MeOH=30/1), The major spot (Rf =0.3) was defined as **Compound 39-a** (20 mg) without further structure conformation. LCMS (M+H)⁺: 795.

### Step 2: preparation of 4-[3-[4-hydroxy-5-methyl-2-[2-(1,2,4-triazol-1-yl)ethyl]pyrazol-3-yl]-1H-1,2,4-triazol-5-yl]-1-methyl-pyrazolo[4,3-c]pyridine-6-carboxamide (Example 39)

**Example 39** was prepared in analogy to the preparation of **Example 21** by using **compound 39-a** instead of 1-[2-(4-benzyloxy-2-ethyl-5-methyl-pyrazol-3-yl)oxazol-4-yl]-*N-*[(2,4-dimethoxyphenyl)methyl]-5-methyl-pyrazolo[3,4-c]pyridine-3-carboxamide **(21-m). Example 39** (6.7 mg) was obtained. LCMS (M+H)⁺: 435. ¹H NMR (400 MHz, DMSO-*d₆*) δ = 15.33 (s, 1H), 8.90 - 8.82 (m, 2H), 8.47 (s, 1H), 8.24 (s, 1H), 8.05 (br s, 1H), 7.89-7.85 (m, 1H), 7.83 (s, 1H), 4.91 (t, *J =* 6.2 Hz, 2H), 4.66 (t, *J =* 6.2 Hz, 2H), 4.23 (s, 3H), 2.13 (s, 3H).

### Example 40

### 1-[3-[4-hydroxy-5-methyl-2-(2-phenylethyl)pyrazol-3-yl]-1H-1,2,4-triazol-5-yl]-5-methyl-pyrazolo[3,4-c]pyridine-3-carboxamide

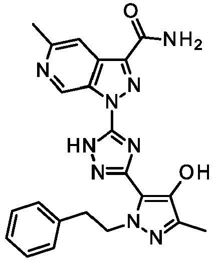

The title compound was prepared according to the following scheme:

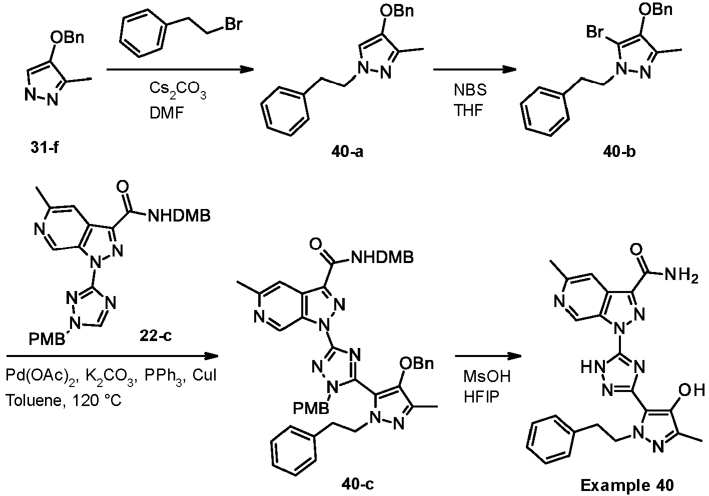

### Step 1: Preparation of 4-benzyloxy-3-methyl-1-(2-phenylethyl)pyrazole (compound 40-a)

To a solution of 4-benzyloxy-3-methyl-1H-pyrazole (31-f, 500 mg, 2.66 mmol) in DMF (4 mL) was added Cs₂CO₃ (2596 mg, 7.97 mmol) and 1-bromo-2-phenylethane (1.09 mL, 7.97 mmol). The mixture was stirred at 100 °C for 12 hrs. The reaction mixture was cooled and diluted with EtOAc (20 mL) and water (20 mL). The organic layer was separated, the aqueous phase was extracted with EtOAc (20 mL x 2). The combined organic layer was dried over Na₂SO₄, and concentrated to give a residue, which was purified via prep-TLC to give **compound 40-a** (500 mg). LCMS (M+H)⁺: 293. ¹H NMR (400 MHz, CHLOROFORM-d) δ = 7.40 - 7.35 (m, 4H), 7.29 - 7.22 (m, 4H), 7.13 - 7.07 (m, 2H), 6.75 (s, 1H), 4.82 (s, 2H), 4.20 - 4.11 (m, 2H), 3.08 (t, *J =* 7.4 Hz, 2H), 2.22 (s, 3H).

### Step 2: Preparation of 4-benzyloxy-5-bromo-3-methyl-1-(2-phenylethyl)pyrazole (compound 40-b)

To a solution of **compound 40-a** (500.0 mg, 1.71 mmol) in THF (5 mL) was added NBS (365 mg, 2.05 mmol). The mixture was stirred at 20 °C for 1 h. The reaction mixture was concentrated and purified by prep-TLC to afford **compound 40-b** (450.0 mg). LCMS (M+H)⁺: 371. ¹H NMR (400 MHz, CHLOROFORM-d) δ = 7.36 (s, 4H), 7.33 - 7.22 (m, 4H), 7.19 - 6.99 (m, 2H), 4.90 (s, 2H), 4.24 (dd, *J* = 7.2, 8.4 Hz, 2H), 3.14 - 3.01 (m, 2H), 2.10 (s, 3H).

### Step 3: Preparation of 1-[5-[4-benzyloxy-5-methyl-2-(2-phenylethyl)pyrazol-3-yl]-1-[(4-methoxyphenyl)methyl]-1,2,4-triazol-3-yl]-N-[(2,4-dimethoxyphenyl)methyl]-5-methyl-pyrazolo[3,4-c]pyridine-3-carboxamide (compound 40-c)

A mixture of **compound 40-b** (200.0 mg, 0.54 mmol), *N*-[(2,4-dimethoxyphenyl)methyl]-1-[1-[(4-methoxyphenyl)methyl]-1,2,4-triazol-3-yl]-5-methyl-pyrazolo[3,4-c]pyridine-3-carboxamide **(22-c,** 276 mg, 0.54 mmol), Pd(OAc)₂ (24 mg, 0.11 mmol), CuI (61 mg, 0.32 mmol), PPh₃ (56 mg, 0.22 mmol), K₂CO₃ (223 mg, 1.62 mmol) in toluene (10 mL) was stirred at 120 °C for 12 hrs under N₂ atmosphere. The reaction mixture was diluted with DCM (10 mL) and filtered. The filtrate was concentrated and purified by prep-TLC to afford **compound 40-c** (10 mg). LCMS (M+H)⁺: 804.

### Step 4: Preparation of 1-[3-[4-hydroxy-5-methyl-2-(2-phenylethyl)pyrazol-3-yl]-1H-1,2,4-triazol-5-yl]-5-methyl-pyrazolo[3,4-c]pyridine-3-carboxamide (Example 40)

A solution of compound 40-c (10 mg, 0.01 mmol) and MsOH (12 mg, 0.12 mmol) in HFIP (0.3 mL) was stirred at 30 °C for 3 hrs. The reaction mixture was purified by HPLC to give **Example 40** (1.8 mg). LCMS (M+H)⁺: 444, ¹H NMR (400 MHz, DMSO-d6) δ = 14.14 (s, 1H), 9.62 (s, 1H), 9.23 (s, 1H), 8.05 (s, 1H), 8.00 - 7.92 (m, 1H), 7.88 - 7.79 (m, 1H), 7.28 - 7.21 (m, 2H), 7.21 - 7.10 (m, 3H), 4.67 (t, *J =* 7.4 Hz, 2H), 3.09 - 3.05 (m, 2H), 2.67 ( s, 3H), 2.18 (s, 3H).

### Example 41

### 1-[3-[2-(3-fluoropropyl)-4-hydroxy-5-methyl-pyrazol-3-yl]-1H-1,2,4-triazol-5-yl]-5-methyl-pyrazolo[3,4-c]pyridine-3-carboxamide

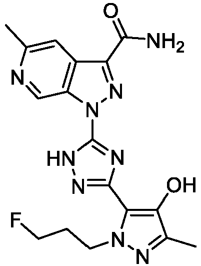

**Example 41** was prepared in analogy to the preparation of **Example 40** by using **compound 41-c** instead of **compound 40-b.**

The **compound 41-c** was prepared according to the following scheme:

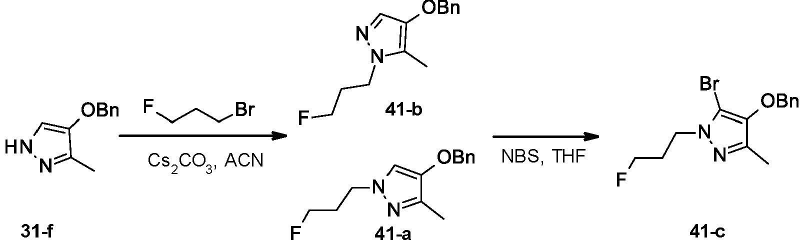

### Step 1: Preparation of 4-benzyloxy-1-(3-fluoropropyl)-3-methyl-pyrazole (compound 41-a) and 4-benzyloxy-1-(3-fluoropropyl)-5-methyl-pyrazole (compound 41-b)

To a solution of 4-benzyloxy-3-methyl-1H-pyrazole (31-f, 500 mg, 2.66 mmol) in acetonitrile (5 mL) was added Cs₂CO₃ (2.6 g, 7.97 mmol, 3.0 eq) and 1-bromo-3-fluoropropane (1.12 g, 7.97 mmol). The mixture was stirred at 80 °C for 12 hrs. The mixture was then filtered and concentrated. The residue was purified by prep-TLC to afford **compound 41-a** (250 mg) and **compound 41-b** (150 mg).

**Compound 41-a,** LCMS (M+H)⁺: 249. ¹H NMR (400 MHz, CHLOROFORM-d) δ = 7.45 - 7.31 (m, 5H), 6.97 (s, 1H), 4.89 (s, 2H), 4.44 (t, *J =* 5.6 Hz, 1H), 4.32 (t, *J =* 5.6 Hz, 1H), 4.08 (t, *J =* 6.7 Hz, 2H), 2.25 - 2.09 (m, 5H).

**Compound 41-b,** LCMS (M+H)⁺: 249. ¹H NMR (400 MHz, CHLOROFORM-d) δ = 7.43 - 7.29 (m, 5H), 7.24 (s, 1H), 4.94 (s, 2H), 4.44 (t, *J =* 5.6 Hz, 1H), 4.32 (t, *J =* 5.6 Hz, 1H), 4.11 (t, J = 6.8 Hz, 2H), 2.29 - 2.06 (m, 5H).

### Step 2: Preparation of 4-benzyloxy-5-bromo-1-(3-fluoropropyl)-3-methyl-pyrazole (compound 41-c)

To a solution of 4-benzyloxy-1-(3-fluoropropyl)-3-methyl-pyrazole (41-a, 220 mg, 0.89 mmol) in THF (2 mL) was added NBS (205 mg, 1.15 mmol). The mixture was stirred at 25 °C for 1 h. The mixture was concentrated, and the residue was purified via flash chromatography on silica gel to afford **compound 41-c** (220 mg). LCMS (M+H)⁺: 327.

**Example 41** (2.4 mg), LCMS (M+H)⁺: 400, ¹H NMR (400 MHz, DMSO-d6) δ = 14.37 - 14.11 (m, 1H), 9.64 (d, *J=* 1.0 Hz, 1H), 9.44 - 9.15 (m, 1H), 8.08 (s, 2H), 7.80 (br s, 1H), 4.60 (t, *J=* 7.0 Hz, 2H), 4.54 (t, *J =* 5.8 Hz, 1H), 4.42 (t, *J =* 5.8 Hz, 1H), 2.68 (s, 3H), 2.23 - 2.13 (m, 5H).

### Example 42

### 1-[3-[4-hydroxy-2-(2-methoxyethyl)-5-methyl-pyrazol-3-yl]-1H-1,2,4-triazol-5-yl]-5-methyl-pyrazolo[3,4-c]pyridine-3-carboxamide

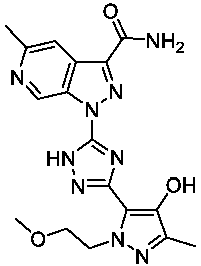

**Example 42** was prepared in analogy to the preparation of **Example 40** by using 2-bromoethyl methyl ether instead of 1-bromo-2-phenylethane in step 1.

**Example 42,** LCMS (M+H)⁺: 398, ¹H NMR (400 MHz, DMSO-*d₆*) δ = 14.23 (br s, 1H), 9.63 (s, 1H), 9.30 - 9.08 (m, 1H), 8.08 (s, 2H), 7.79 (s, 1H), 4.64 (t, *J= 5.6* Hz, 2H), 3.70 (t, *J =* 5.8 Hz, 2H), 3.18 (s, 3H), 2.67 (s, 3H), 2.17 (s, 3H).

### Example 43

### 1-[3-(2-butyl-4-hydroxy-5-methyl-pyrazol-3-yl)-1H-1,2,4-triazol-5-yl]-5-methyl-pyrazolo[3,4-c]pyridine-3-carboxamide

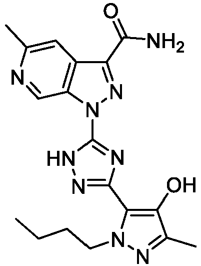

**Example 43** was prepared in analogy to the preparation of **Example 40** by using 1-bromobutane instead of 1-bromo-2-phenylethane in step 1.

**Example 43** (1.5 mg), LCMS (M+H)⁺: 396, ¹H NMR (400 MHz, DMSO-d6) δ = 14.20 (br s, 1H), 9.64 (br s, 1H), 9.19 (br s, 1H), 8.05 (s, 2H), 7.78 (br s, 1H), 4.47 (br t, *J= 7.2* Hz, 2H), 2.67 (s, 3H), 2.18 (s, 3H), 1.82 - 1.72 (m, 2H), 1.32 - 1.26 (m, 2H), 0.87 (br t, *J=* 7.2 Hz, 3H).

### Example 44

### 1-[3-[4-hydroxy-2-(3-methoxypropyl)-5-methyl-pyrazol-3-yl]-1H-1,2,4-triazol-5-yl]-5-methyl-pyrazolo[3,4-c]pyridine-3-carboxamide

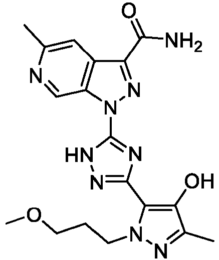

**Example 44** was prepared in analogy to the preparation of **Example 40** by using 1-bromo-3-methoxypropane instead of 1-bromo-2-phenylethane in step 1.

**Example 44** (11.7 mg), LCMS (M+H)⁺: 412, ¹H NMR (400 MHz, DMSO-*d₆*) δ = 9.65 (s, 1H), 8.01 (s, 2H), 7.78 - 7.67 (m, 1H), 7.22 - 6.84 (m, 1H), 4.53 (t, *J=* 7.0 Hz, 2H), 3.29 - 3.29 (m, 2H), 3.18 (s, 3H), 2.65 (s, 3H), 2.15 (s, 3H), 2.05 - 1.96 (m, 2H).

### Example 45

### 4-[3-(4-hydroxy-5-methyl-2-pent-3-ynyl-pyrazol-3-yl)-1H-1,2,4-triazol-5-yl]-1-methyl-pyrazolo[4,3-c]pyridine-6-carboxamide

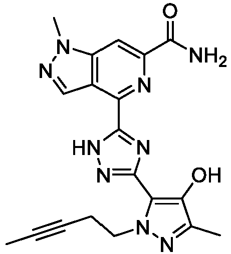

The title compound was prepared according to the following scheme:

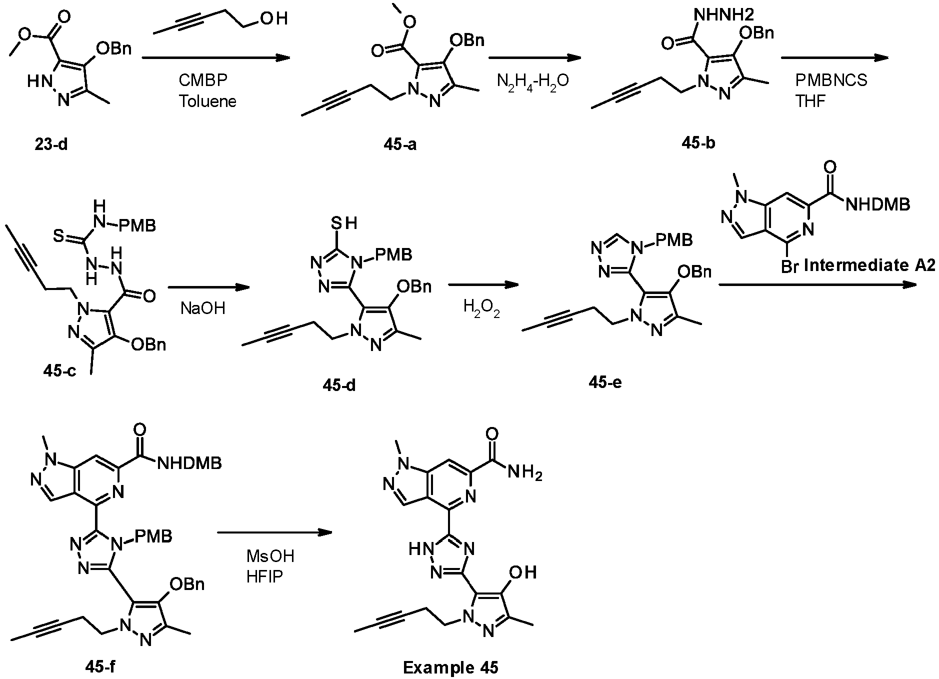

### Step 1: Preparation of methyl 4-benzyloxy-5-methyl-2-pent-3-ynyl-pyrazole-3-carboxylate (compound 45-a)

To a solution of methyl 4-benzyloxy-3-methyl-1H-pyrazole-5-carboxylate (1.5 g, 6.09 mmol) in toluene (15 mL) was added CMBP (7.36 g, 30.46 mmol) and 3-pentyn-1-ol (2.81 mL, 30.46 mmol), the mixture was then stirred at 80 °C for 12 hrs. The reaction was concentrated to give a residue. The residue was purified via column chromatography to afford **compound 45-a** (1.8 g). LCMS (M+H)⁺: 313.

### Step 2: Preparation of 4-benzyloxy-5-methyl-2-pent-3-ynyl-pyrazole-3-carbohydrazide (compound 45-b)

To a solution of **compound 45-a** (1.8 g, 5.76 mmol) in methanol (20 mL) was added hydrazine hydrate (2.94 g, 57.63 mmol) at r.t. Then the mixture was stirred at 80 °C for 4 hrs. The mixture was concentrated to give **compound 45-b** (1.8 g). LCMS (M+H)⁺: 313.

### Step 3: Preparation of 1-[(4-benzyloxy-5-methyl-2-pent-3-ynyl-pyrazole-3-carbonyl)amino]-3-[(4-methoxyphenyl)methyl]thiourea (compound 45-c)

To a yellow solution of **compound 45-b** (1.7 g, 5.44 mmol) and DIPEA (2.69 mL, 16.33 mmol) in THF (80 mL) was added 4-methoxybenzyl isothiocyanate (1.95 g, 10.88 mmol) at r.t. The mixture was stirred for 12 hrs and concentrated to obtained **compound 45-c** (2.3 g). LCMS (M+H)⁺: 492.

### Step 4: Preparation of 5-(4-benzyloxy-5-methyl-2-pent-3-ynyl-pyrazol-3-yl)-4-[(4-methoxyphenyl)methyl]-1,2,4-triazole-3-thiol (compound 45-d)

A yellow solution of **compound 45-c** (2.3 g, 4.68 mmol) in 3N NaOH (25 mL) was stirred at 100 °C for 3 h. The mixture was washed with EA (3 x 900 mL). The combined organic layer was washed with water (3 x 800 mL) and brine (1050 mL), dried over anhydrous sodium sulfate and concentrated, the residue was purified by column chromatography to give **compound 45-d** (2.0 g). LCMS (M+H)⁺: 474.

### Step 5: Preparation of 3-(4-benzyloxy-5-methyl-2-pent-3-ynyl-pyrazol-3-yl)-4-[(4-methoxyphenyl)methyl]-1,2,4-triazole (compound 45-e)

To a solution of **compound 45-d** (2.5 g, 5.28 mmol) in acetic acid (8 mL) was added H₂O₂ (11.27 g, 99.44 mmol) slowly, the mixture was then stirred at r.t. for 1 h. The reaction mixture was poured into saturated aqueous solution of Na₂SO₃ (200 mL) and extracted with ethyl acetate (3 x 250 mL). The combined organic layer was washed with brine (200 mL), dried over Na₂SO₄, filtered and concentrated. The residue was purified via column chromatography to afford **compound 45-e** (1.2 g). LCMS (M+H)⁺: 442.

### Step 6: Preparation of 4-[5-(4-benzyloxy-5-methyl-2-pent-3-ynyl-pyrazol-3-yl)-4-[(4-methoxyphenyl)methyl]-1,2,4-triazol-3-yl]-N-[(2,4-dimethoxyphenyl)methyl]-1-methyl-pyrazolo[4,3-c]pyridine-6-carboxamide (compound 45-f)

To a solution of **compound 45-e** (100 mg, 0.23 mmol) in 1,4-dioxane (2 mL) was added 4-bromo-N-[(2,4-dimethoxyphenyl)methyl]-1-methyl-pyrazolo[4,3-c]pyridine-6-carboxamide **(intermediate A2,** 92 mg, 0.23 mmol), Pd(OAc)₂ (15 mg, 0.07 mmol), CuI (86 mg, 0.45 mmol), PCy₃HBF₄ (33 mg, 0.09 mmol) and Cs₂CO₃ (73 mg, 0.23 mmol) under N₂, the mixture was stirred at 140 °C for 12 hrs. The mixture was added in DCM/MeOH (30 mL/6 mL), then it was filtered. The filtrate was evaporated and purified via prep-TLC to obtain **compound 45-f** (20 mg). LCMS (M+H)⁺: 766.

### Step 7: Preparation of 4-[3-(4-hydroxy-5-methyl-2-pent-3-ynyl-pyrazol-3-yl)-1H-1,2,4-triazol-5-yl]-1-methyl-pyrazolo[4,3-c]pyridine-6-carboxamide (Example 45)

A solution of **compound 45-f** (15.0 mg, 0.02 mmol) and MsOH (19 mg, 0.2 mmol) in HFIP (0.5 mL) was stirred at 30 °C for 3 hrs under N₂ atmosphere. The reaction mixture was purified by reversed phase HPLC to obtain **Example 45** (5.5 mg). LCMS (M+H)⁺: 406, ¹H NMR (400 MHz, DMSO-d6) δ = 15.37 (s, 1H), 8.93 (s, 1H), 8.89 - 8.84 (m, 1H), 8.47 (s, 1H), 8.02 (s, 1H), 7.88 ( s, 1H), 4.57 (t, *J* = 7.4 Hz, 2H), 4.23 (s, 3H), 2.63 (d, *J =* 5.6 Hz, 2H), 2.14 (s, 3H), 1.73 (s, 3H).

### Example 46

### 1-[3-[4-hydroxy-5-methyl-2-[2-(4-pyridyl)ethyl]pyrazol-3-yl]-1H-1,2,4-triazol-5-yl]-5-methyl-pyrazolo[3,4-c]pyridine-3-carboxamide

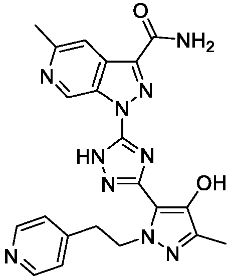

The title compound was prepared according to the following scheme:

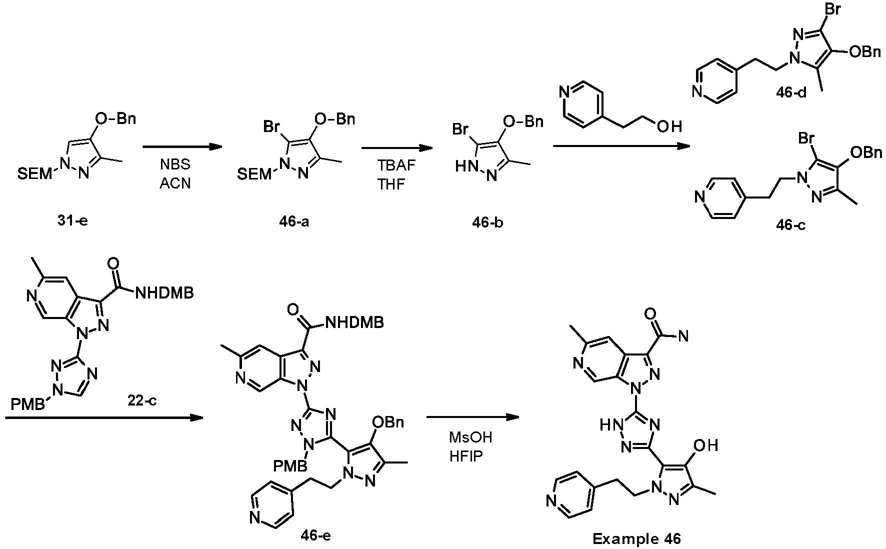

### Step 1: Preparation of 4-(benzyloxy)-5-bromo-3-methyl-1-((2-(trimethylsilyl) ethoxy)methyl)-1H-pyrazole (compound 46-a)

To a stirred mixture of 4-(benzyloxy)-3-methyl-1-((2-(trimethylsilyl)ethoxy)methyl)-1H-pyrazole (31-e, 15.0 g, 47.1 mmol) in ACN (150 mL) was added NBS (16.8 g, 94.2 mmol) in portions. The resulting mixture was stirred for 2 h at 60 °C under nitrogen atmosphere. The mixture was allowed to cool down to 25 °C. The resulting mixture was concentrated and the residue was purified via silica gel column chromatography to give **compound 46-a** (11.0 g). LCMS (M+H)⁺: 397.

### Step 2: Preparation of 4-(benzyloxy)-5-bromo-3-methyl-1H-pyrazole (compound 46-b)

To a stirred mixture of **compound 46-a** (11.0 g, 28 mmol) in THF (30 mL) was added tetrabutylammonium fluoride (150 mL, 150 mmol) (1 M in THF) dropwise. The resulting mixture was stirred for 15 h at 80 °C under nitrogen atmosphere. The mixture was allowed to cool down to 25 °C. The resulting mixture was concentrated and purified via silica gel column chromatography to give **compound 46-b** (4.20 g). LCMS (M+H)⁺: 267.

### Step 3: Preparation of 4-[2-(4-benzyloxy-5-bromo-3-methyl-pyrazol-1-yl)ethyl]pyridin (compound 46-c) and 4-[2-(4-benzyloxy-3-bromo-5-methyl-pyrazol-1-yl)ethyl]pyridine (compound 46-d)

To a solution of **compound 46-b** (500 mg, 1.87 mmol) in Toluene (5 mL) was added CMBP (1353 mg, 5.62 mmol) and 4-pyridineethanol (691 mg, 5.62 mmol). The mixture was stirred at 80 °C for 12 hrs. The mixture was concentrated and purified via prep-NPLC (Welch Ultimate XB-SiOH 250*70*10um, Hexane-EtOH (0.1% FA,10%~50%) to give **compound 46-c** (250 mg) and **compound 46-d** (300 mg).

**Compound 46-c,** LCMS (M+H)⁺: 372, ¹H NMR (400 MHz, CHLOROFORM-d) δ = 8.61 - 8.46 (m, 2H), 7.43 - 7.33 (m, 5H), 7.09 - 7.00 (m, 2H), 4.91 (s, 2H), 4.28 (t, *J=* 7.4 Hz, 2H), 3.12 (t, *J=* 7.4 Hz, 2H), 2.10 (s, 3H).

**Compound 46-d,** LCMS (M+H)⁺: 372, ¹H NMR (400 MHz, CHLOROFORM-d) δ = 8.53 (br d, *J= 5.3* Hz, 2H), 7.37 (s, 5H), 7.08 (d, *J =* 5.9 Hz, 2H), 4.93 (s, 2H), 4.15 (t, *J= 7.0* Hz, 2H), 3.17 (t, *J=* 6.8 Hz, 2H), 1.68 (s, 3H).

### Step 4: Preparation of 1-[5-[4-benzyloxy-5-methyl-2-[2-(4-pyridyl)ethyl]pyrazol-3-yl]-1-[(4-methoxyphenyl)methyl]-1,2,4-triazol-3-yl]-N-[(2,4-dimethoxyphenyl)methyl]-5-methyl-pyrazolo[3,4-c]pyridine-3-carboxamide (compound 46-e)

A mixture of **compound 46-c** (119 mg, 0.32 mmol), N-[(2,4-dimethoxyphenyl)methyl]-1-[1-[(4-methoxyphenyl)methyl]-1,2,4-triazol-3-yl]-5-methyl-pyrazolo[3,4-c]pyridine-3-carboxamide **(22-c,** 150 mg, 0.29 mmol), Pd(OAc)₂ (19 mg, 0.09 mmol), CuI (33 mg, 0.18 mmol), PPh₃ (38 mg, 0.15 mmol), K₂CO₃ (121 mg, 0.88 mmol) in toluene (3 mL) was stirred at 120 °C for 12 hrs under N₂ atmosphere. The reaction mixture was diluted with DCM (10 mL) and filtered. The filtrate was concentrated and purified via prep-TLC to afford **compound 46-e** (35 mg). LCMS (M+H)⁺: 805.

### Step 5: Preparation of 1-[3-[4-hydroxy-5-methyl-2-[2-(4-pyridyl)ethyl]pyrazol-3-yl]-1H-1,2,4-triazol-5-yl]-5-methyl-pyrazolo[3,4-c]pyridine-3-carboxamide (Example 46)

A solution of 1-[5-[4-benzyloxy-5-methyl-2-[2-(4-pyridyl)ethyl]pyrazol-3-yl]-1-[(4-methoxyphenyl)methyl]-1,2,4-triazol-3-yl]-*N*-[(2,4-dimethoxyphenyl)methyl]-5-methyl-pyrazolo[3,4-c]pyridine-3-carboxamide **(46-e,** 30 mg, 0.04 mmol) in HFIP (1.0 mL) was added MsOH (120 mg, 0.88 mmol). The mixture was stirred at 60 °C for 2 hrs and purified via reversed-phase HPLC to give **Example 46** (11.2 mg). LCMS (M+H)⁺: 445, ¹H NMR (400 MHz, DMSO-d6) δ = 14.30 - 14.11 (m, 1H), 9.57 (d, *J =* 1.0 Hz, 1H), 9.45 - 9.23 (m, 1H), 8.65 (d, *J =* 6.4 Hz, 2H), 8.07 (s, 2H), 7.85 (br s, 1H), 7.79 (br d, *J =* 5.8 Hz, 2H), 4.84 (br t, *J =* 7.2 Hz, 2H), 3.38 (br t, *J =* 7.0 Hz, 2H), 2.68 (s, 3H), 2.16 (s, 3H).

### Example 47

### 1-[3-[4-hydroxy-5-methyl-2-[2-(3-pyridyl)ethyl]pyrazol-3-yl]-1H-1,2,4-triazol-5-yl]-5-methyl-pyrazolo[3,4-c]pyridine-3-carboxamide

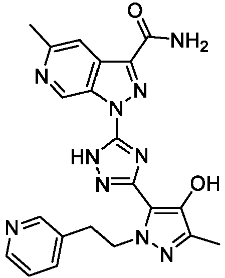

**Example** 47 was prepared in analogy to the preparation of Example 46 by using 3-(2-hydroxyethyl)pyridine instead of 4-pyridineethanol in step 3.

Example 47 (6.9 mg), LCMS (M+H)⁺: 445, ¹H NMR (400 MHz, DMSO-*d₆*) δ = 9.65 (s, 1H), 8.87 (br s, 1H), 8.46 (br s, 1H), 8.37 (br d, *J* = 4.4 Hz, 1H), 8.06 (s, 1H), 7.81 (br s, 1H), 7.69 (br d, *J* = 7.6 Hz, 1H), 7.25 (dd, *J* = 4.6, 7.8 Hz, 1H), 4.70 (br t, *J =* 7.4 Hz, 2H), 3.10 (br t, *J =* 7.4 Hz, 2H), 2.67 (s, 3H), 2.16 (s, 3H).

### Example 48

### 1-[5-[4-hydroxy-5-methyl-2-(2-phenylethyl)pyrazol-3-yl]-2-methyl-1,2,4-triazol-3-yl]-5-methyl-pyrazolo[3,4-c]pyridine-3-carboxamide

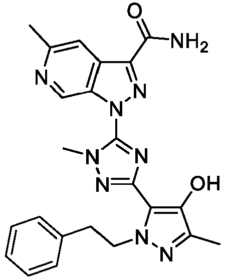

The title compound was prepared according to the following scheme:

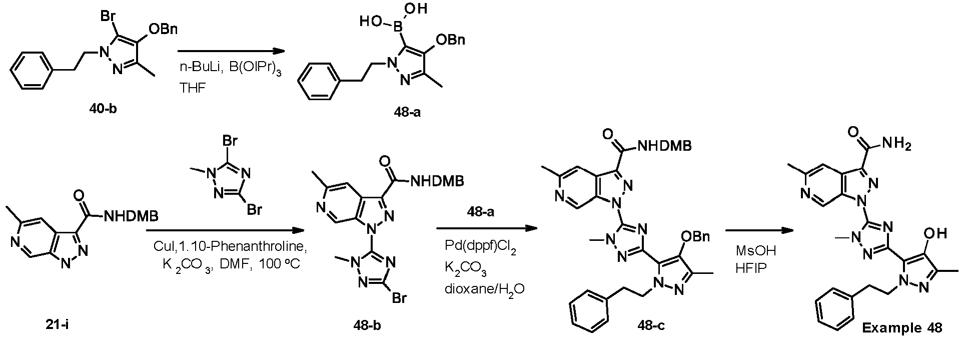

### Step 1: Preparation of [4-benzyloxy-5-methyl-2-(2-phenylethyl)pyrazol-3-yl]boronic acid (compound 48-a)

To a solution of 4-benzyloxy-5-bromo-3-methyl-1-(2-phenylethyl)pyrazole (40-b, 90 mg, 0.24 mmol) in THF (2 mL) was added n-BuLi (2.5M, 0.15 mL, 0.36 mmol) dropwise at -78 °C under N₂ atmosphere. The mixture was stirred at -78 °C for 1 h, and then triisopropyl borate (273 mg, 0.34 mL, 1.45 mmol) was added. The mixture was slowly warmed to r.t. and stirred for 2 hrs. The reaction mixture was poured into aq. NH₄Cl (50 ml) and extracted with EA (3 x 60 mL). The combined organic layer was washed with brine, dried over Na₂SO₄ and concentrated to afford **compound 48-a** (80.0 mg). LCMS (M+H)⁺: 337.

### Step 2: preparation of 1-(5-bromo-2-methyl-1,2,4-triazol-3-yl)-N-[(2,4-dimethoxyphenyl) methyl]-5-methyl-pyrazolo[3,4-c]pyridine-3-carboxamide (compound 48-b)

To a stirred mixture of *N*-(2,4-dimethoxybenzyl)-5-methyl-1H-pyrazolo[3,4-c]pyridine-3-carboxamide (21-i, 800 mg, 2.45 mmol), 3,5-dibromo-1-methyl-1,2,4-triazole (1.76 g, 7.35 mmol) in DMF (15.0 mL) were added copper (I) iodide (233 mg, 1.23 mmol), 1,10-phenanthroline (441 mg, 2.45 mmol) and potassium carbonate (846 mg, 6.13 mmol) in portions. The resulting mixture was stirred for 16 hrs at 100 °C. The mixture was cooled and diluted with ice water (60 mL), and extracted with EtOAc (3 x 80 mL). The combined organic layer was washed with brine (150 mL), dried over sodium sulfate and concentrated. The residue was purified via silica gel column chromatography to give **compound 48-b** (415 mg). LCMS (M+H)⁺: 485.

### Step 3: Preparation of 1-[5-[4-benzyloxy-5-methyl-2-(2-phenylethyl)pyrazol-3-yl]-2-methyl-1,2,4-triazol-3-yl]-N-[(2,4-dimethoxyphenyl)methyl]-5-methyl-pyrazolo[3,4-c]pyridine-3-carboxamide (compound 48-c)

To a solution of 1-(5-bromo-2-methyl-1,2,4-triazol-3-yl)-*N*-[(2,4-dimethoxyphenyl) methyl]-5-methyl-pyrazolo[3,4-c]pyridine-3-carboxamide **(48-b,** 55 mg, 0.11 mmol) and **compound 48-a** (76 mg, 0.23 mmol) in 1,4-dioxane (3 mL) and water (0.3 mL) were added K₂CO₃ (280 mg, 2.04 mmol) and Pd(dppf)Cl₂ (56 mg, 0.07 mmol). The mixture was stirred at 100 °C for 12 hrs under N₂ atmosphere. The mixture was concentrated and purified via prep-TLC to obtain **compound 48-c** (30 mg). LCMS (M+H)⁺: 698.

### Step 4: Preparation of 1-[5-[4-hydroxy-5-methyl-2-(2-phenylethyl)pyrazol-3-yl]-2-methyl-1,2,4-triazol-3-yl]-5-methyl-pyrazolo[3,4-c]pyridine-3-carboxamide (Example 48)

A solution of **compound 48-c** (20 mg, 0.03 mmol) and MsOH (1.0 mL, 0.29 mmol) in HFIP (2.0 mL) was stirred at 30 °C for 3 hrs. The reaction mixture was purified via reversed phase HPLC to obtain **Example 48** (10 mg). LCMS (M+H)⁺: 458, ¹H NMR (400 MHz, DMSO-d6) δ = 9.58 (s, 1H), 8.29 (br s, 1H), 8.07 (s, 1H), 8.05 (s, 1H), 7.91 (s, 1H), 7.25 - 7.11 (m, 5H), 4.58 (t, *J =* 7.6 Hz, 2H), 4.29 (s, 3H), 3.04 (t, *J =* 7.8 Hz, 2H), 2.68 (s, 3H), 2.14 (s, 3H).

### Example 49

### 1-[5-(2-butyl-4-hydroxy-5-methyl-pyrazol-3-yl)-2-methyl-1,2,4-triazol-3-yl]-5-methyl-pyrazolo[3,4-c]pyridine-3-carboxamide

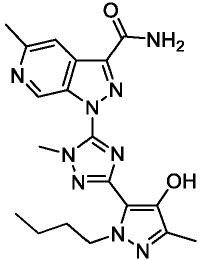

**Example** 49 was prepared in analogy to the preparation of Example 48 by using compound 49-c instead of compound 40-b in step 1.

**Compound** 49-c prepared according to the following scheme:

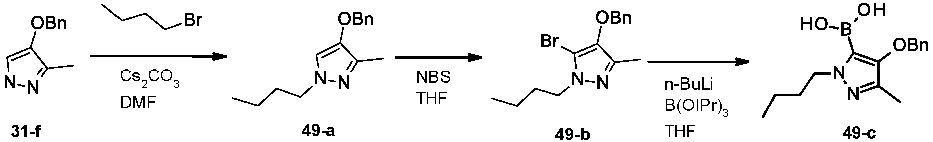

### Step 1: Preparation of 4-benzyloxy-1-butyl-3-methyl-pyrazole (compound 49-a)

To a solution of 4-benzyloxy-3-methyl-1H-pyrazole (31-f, 0.5 g, 2.66 mmol) in DMF (5 mL) was added Cs₂CO₃ (2.6 g, 7.97 mmol) and 1-bromobutane (1.09 g, 7.97 mmol). The mixture was stirred at 100 °C for 12 hrs. The reaction mixture was cooled and diluted with EtOAc (20 mL) and water (20 mL). The two-phase layer was separated, the aqueous phase was extracted with EtOAc (300 mL x 2). All organic layers were combined, dried over Na₂SO₄ and concentrated to give **compound 49-a** (400 mg). LCMS (M+H)⁺: 245.

### Step 2: Preparation of 4-benzyloxy-5-bromo-1-butyl-3-methyl-pyrazole (compound 49-b)

To a solution of **compound 49-a** (900 mg, 3.68 mmol) in THF (10 mL) was added NBS (786 mg, 4.42 mmol). The mixture was stirred at 20 °C for 1 h. The reaction mixture was concentrated and purified via prep-TLC to afford **compound 49-b** (200 mg). LCMS (M+H)⁺: 323, ¹H NMR (400 MHz, CHLOROFORM-d) δ = 7.46 - 7.30 (m, 5H), 4.93 (s, 2H), 4.03 (t, *J* = 7.2 Hz, 2H), 2.10 (s, 3H), 1.83 - 1.72 (m, 2H), 1.37 - 1.26 (m, 2H), 0.94 (t, *J =* 7.4 Hz, 3H).

### Step 3: Preparation of (4-benzyloxy-2-butyl-5-methyl-pyrazol-3-yl) boronic acid (compound 49-c)

To a solution of **compound 49-b** (46 mg, 0.14 mmol) in THF (2 mL) was added n-BuLi (2.5M, 0.09 mL, 0.21 mmol) dropwise at -78 °C under N₂ atmosphere. The mixture was stirred at -78 °C for 0.5 h. Then triisopropyl borate (160 mg, 0.2 mL, 0.85 mmol) was added. The mixture was warmed to 25 °C and stirred for 0.5 h, then it was poured into NH₄Cl aq. (50 ml) and extracted with EA (3 x 50 mL). The combined organic layer was dried over Na₂SO₄ and concentrated to give **compound 49-c** (50.0 mg). LCMS (M+H)⁺: 289.

Example 49, LCMS (M+H)⁺: 410, ¹H NMR (400 MHz, DMSO-d6) δ = 9.59 (d, *J =* 0.8 Hz, 1H), 8.31 (s, 1H), 8.10 (s, 2H), 7.92 (s, 1H), 4.37 (t, *J =* 7.2 Hz, 2H), 4.28 (s, 3H), 2.68 (s, 3H), 2.12 (s, 3H), 1.80 - 1.67 (m, 2H), 1.32 - 1.20 (m, 2H), 0.86 (t, *J =* 7.2 Hz, 3H).

### Example 50

### 1-[3-[4-hydroxy-2-[2-(4-methoxyphenyl)ethyl]-5-methyl-pyrazol-3-yl]-1H-1,2,4-triazol-5-yl]-5-methyl-pyrazolo[3,4-c]pyridine-3-carboxamide

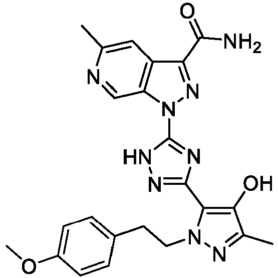

**Example** 50 was prepared in analogy to the preparation of Example 40 by using 4-methoxyphenethyl bromide instead of 1-bromo-2-phenylethane in step 1.

Example 50 (3.2 mg), LCMS (M+H)⁺: 474, ¹H NMR (400 MHz, DMSO-d6) δ = 14.16 (dd, *J =* 2.6, 4.0 Hz, 1H), 9.62 (s, 1H), 9.32 - 9.15 (m, 1H), 8.08 (s, 1H), 8.00 ( s, 1H), 7.88 (br s, 1H), 7.15 (d, *J* = 8.6 Hz, 2H), 6.73 (d, *J =* 8.6 Hz, 2H), 4.66 - 4.58 (m, 2H), 3.67 (s, 3H), 3.00 (d, *J =* 6.8 Hz, 2H), 2.68 (s, 3H), 2.18 (s, 3H).

### Example 51

### 1-[3-(4-hydroxy-5-methyl-2-pent-3-ynyl-pyrazol-3-yl)-1H-1,2,4-triazol-5-yl]-5-methyl-pyrazolo[3,4-c]pyridine-3-carboxamide

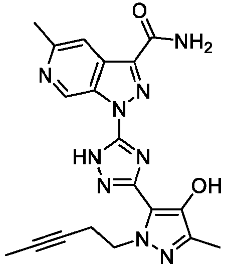

The title compound was prepared according to the following scheme:

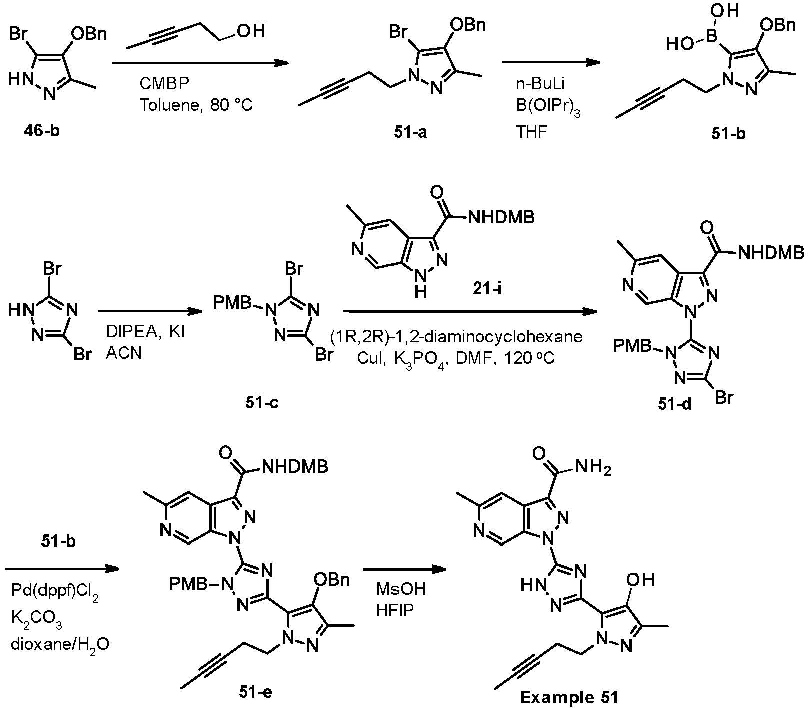

### Step 1: Preparation of 4-benzyloxy-5-bromo-3-methyl-1-pent-3-ynyl-pyrazole (compound 51-a)

To a solution of 4-benzyloxy-5-bromo-3-methyl-1H-pyrazole (46-b, 300 mg, 1.12 mmol), 3-pentyn-1-ol (113 mg, 0.12 mL, 1.35 mmol) in toluene (5 mL) was added CMBP (541 mg, 2.25 mmol). The mixture was stirred at 80 °C for 16 hrs under N₂ balloon. The mixture was concentrated and purified via prep-TLC to give **compound 51-a** (140 mg). LCMS (M+H)⁺: 332.

### Step 2: Preparation of (4-benzyloxy-5-methyl-2-pent-3-ynyl-pyrazol-3-yl) boronic acid (compound 51-b)

To a solution of 4-benzyloxy-5-bromo-3-methyl-1-pent-3-ynyl-pyrazole (51-a, 80 mg, 0.24 mmol) in THF (2 mL) was dropwise added n-BuLi (2.5 M, 0.14 mL, 0.36 mmol) at -70 °C under N₂ atmosphere. The mixture was stirred at -70 °C for 1 h, and then triisopropyl borate (270 mg, 0.33 mL, 1.44 mmol) was added. The mixture was warmed to 20 °C slowly and stirred for 2 hrs. The reaction mixture was poured into saturated aqueous of NH₄Cl (10 ml) at 0 °C and extracted with EtOAc (3 x 10 mL). The combined organic layer was dried over anhydrous Na₂SO₄, filtered and concentrated to afford crude **compound 51-b** (60.0 mg). LCMS (M+H)⁺: 299.

### Step 3: Preparation of 3,5-dibromo-1-[(4-methoxyphenyl)methyl]-1,2,4-triazole (compound 51-c)

A mixture of 3,5-dibromo-1H-1,2,4-triazole (30 g, 132 mmol), 4-methoxybenzylchloride (21.5 mL, 158.69 mmol), KI (10.98 g, 66 mmol), DIEA (43.7 mL, 264 mmol) in MeCN (200 mL) was stirred at 80 °C for 16 hrs under N₂ balloon. The mixture was concentrated and purified via column chromatography to give **compound 51-c** (30 g). LCMS (M+H)⁺: 346, ¹H NMR (400 MHz, DMSO-d6) δ = 7.24 (d, *J =* 8.6Hz, 2H), 6.94 (d, *J =* 8.6 Hz, 2H), 5.32 (s, 2H), 3.74 (s, 3H).

### Step 4: Preparation of 1-[5-bromo-2-[(4-methoxyphenyl)methyl]-1,2,4-triazol-3-yl]-N-[(2,4-dimethylphenyl)methyl]-5-methyl-pyrazolo[3,4-c]pyridine-3-carboxamide (compound 51-d)

To a solution of *N*-[(2,4-dimethoxyphenyl)methyl]-5-methyl-1H-pyrazolo[3,4-c]pyridine-3-carboxamide (21-i, 5.0 g, 15.32 mmol) and **compound 51-c** (7.97 g, 22.98 mmol) in DMF (30 mL) was added CuI (0.59 g, 3.06 mmol), 1-N,2-N-dimethylcyclohexane-1,2-diamine (435 mg, 3.06 mmol) and K₃PO₄ (6.5 g, 30.64 mmol). The mixture was stirred at 120 °C for 12 hrs udner N₂. The reaction mixture was poured into ice water (1000 mL) and extracted with ethyl acetate (3 x 500 mL). The organic layer was washed with water (2 x 500 mL) and brine (2 x 500 mL). The combined organic layer was dried over Na₂SO₄, filtered and concentrated. The residue was triturated with ethyl acetate (30 mL) and filtered to give **compound 51-d** (4.8 g). LCMS (M+H)⁺: 592, ¹H NMR (400 MHz, DMSO-d6) δ = 9.45 (s, 1H), 9.06 (t, *J=* 5.6 Hz, 1H), 8.01 (s, 1H), 7.28 (d, *J =* 8.4 Hz, 2H), 7.16 (d, *J =* 8.4 Hz, 1H), 6.79 (d, *J =* 8.4 Hz, 2H), 6.58 (s, 1H), 6.47 (d, *J =* 8.2 Hz, 1H), 5.89 (s, 2H), 4.49 (d, *J =* 5.6 Hz, 2H), 3.80 (s, 3H), 3.74 (s, 3H), 3.68 (s, 3H), 2.64 (s, 3H).

### Step 5: Preparation of 1-[5-(4-benzyloxy-5-methyl-2-pent-3-ynyl-pyrazol-3-yl)-2-[(4-methoxyphenyl)methyl]-1,2,4-triazol-3-yl]-N-[(3,4-dimethylphenyl)methyl]-5-methyl-pyrazolo[3,4-c]pyridine-3-carboxamide (compound 51-e)

To a solution of **compound 51-d** (80 mg, 0.14 mmol) in 1,4-dioxane (5 mL) and water (0.500 mL) were added **compound 51-b** (60 mg, 0.2 mmol), K₂CO₃ (93 mg, 0.68 mmol) and Pd(dppf)Cl₂.DCM (33 mg, 0.04 mmol). The mixture was stirred at 100 °C for 12 hrs under N₂ atmosphere. The reaction mixture was concentrated, the residue was purified by flash silica gel chromatography to afford **compound 51-e** (50 mg). LCMS (M+H)⁺: 766.

### Step 6: Preparation of 1-[3-(4-hydroxy-5-methyl-2-pent-3-ynyl-pyrazol-3-yl)-1H-1,2,4-triazol-5-yl]-5-methyl-pyrazolo[3,4-c]pyridine-3-carboxamide (Example 51)

To a solution of compound 51-e (90 mg, 0.12 mmol) in HFIP (2 mL) was added MsOH (112 mg, 1.18 mmol) at 25°C. The mixture was stirred at 25 °C for 1 h to give a red solution. The mixture was purified via reversed-phase column to give Example 51 (8 mg). LCMS (M+H)⁺: 406.1, ¹H NMR (400 MHz, DMSO-d6) δ = 14.25 (s, 1H), 9.71 (s, 1H), 9.35 - 9.09 (m, 1H), 8.06 (s, 2H), 7.78 (s, 1H), 4.59 (br t, *J =* 6.8 Hz, 2H), 2.67 (s, 3H), 2.64 - 2.58 (m, 2H), 2.18 (s, 3H), 1.61 (s, 3H).

### Example 52

### 1-[3-[2-[2-(4-fluorophenyl)ethyl]-4-hydroxy-5-methyl-pyrazol-3-yl]-1H-1,2,4-triazol-5-yl]-5-methyl-pyrazolo [3,4-c]pyridine-3-carboxamide

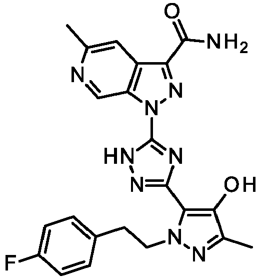

**Example** 52 was prepared in analogy to the preparation of Example 51 by using 1-(2-bromoethyl)-4-fluorobenzene instead of 3-pentyn-1-ol in step 1.

Example 52 (18.12 mg), LCMS (M+H)⁺: 462, ¹H NMR (400 MHz, DMSO-*d₆*) δ = 9.60 (s, 1H), 8.03 (s, 1H), 7.99 (br s, 1H), 7.82 (br s, 1H), 7.27 (dd, *J* = 5.6, 8.4 Hz, 2H), 6.98 (t, *J* = 8.8 Hz, 2H), 4.73 - 4.59 (m, 2H), 3.06 (br t, *J= 7.6* Hz, 2H), 2.67 (s, 3H), 2.17 (s, 3H).

### Example 53

### 1-[3-[4-hydroxy-5-methyl-2-(2-phenylethyl)pyrazol-3-yl]-1H-1,2,4-triazol-5-yl]-6-methyl-imidazo[1,5-a]pyrazine-3-carboxamide

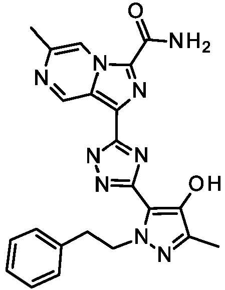

The title compound was prepared according to the following scheme:

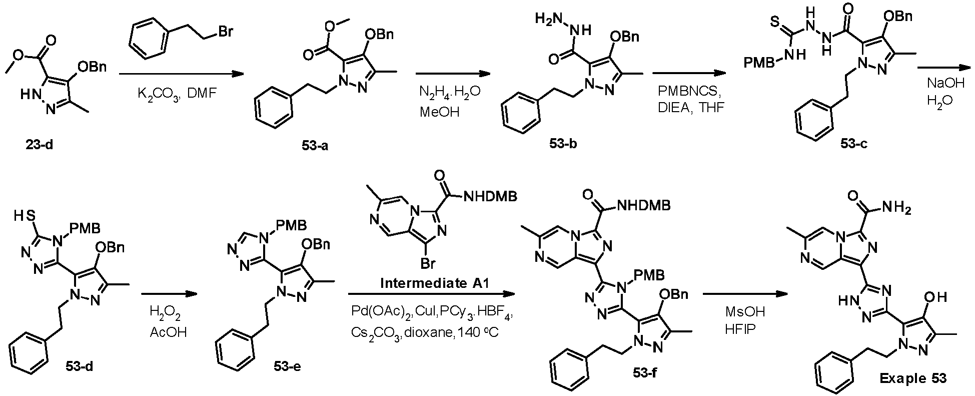

### Step 1: Preparation of methyl 4-benzyloxy-5-methyl-2-(2-phenylethyl)pyrazole-3-carboxylate (compound 53-a)

To a solution of methyl 4-benzyloxy-3-methyl-1H-pyrazole-5-carboxylate (23-d, 2.0 g, 8.12 mmol) in DMF (20 mL) was added 1-bromo-2-phenylethane (2.25 g, 1.66 mL, 12.18 mmol) and K₂CO₃ (3.37 g, 24.36 mmol). The mixture was stirred at 80°C for 12 hrs, and then it was concentrated. The residue was purified via column chromatography to give **compound 53-a** (1.7 g). LCMS (M+H)⁺: 351, ¹H NMR (400 MHz, CHLOROFORM-d) δ = 7.49 - 7.41 (m, 5H), 7.35 (d, *J=* 7.4 Hz, 2H), 7.32 - 7.24 (m, 3H), 4.99 (s, 2H), 4.76 - 4.64 (m, 2H), 3.91 (s, 3H), 3.20 - 3.08 (m, 2H), 2.17 (s, 3H).

### Step 2: Preparation of 4-benzyloxy-5-methyl-2-(2-phenylethyl)pyrazole-3-carbohydrazide (compound 53-b)

To a solution of **compound 53-a** (1.5 g, 4.28 mmol) in methanol (15 mL) was added hydrazine hydrate (2.19 g, 42.81 mmol) at 25°C. Then it was stirred at 80 °C for 4 hrs. The mixture was concentrated to give **compound 53-b** (1.4 g). LCMS (M+H)⁺: 351.

### Step 3: Preparation of 1-[[4-benzyloxy-5-methyl-2-(2-phenylethyl)pyrazole-3-carbonyl]amino]-3-[(4-methoxyphenyl)methyl]thiourea (compound 53-c)

To a yellow solution of **compound 53-b** (1.5 g, 4.28 mmol) and DIPEA (2.12 mL, 12.84 mmol) in THF (20 mL) was added 4-methoxybenzyl isothiocyanate (1.53 g, 8.56 mmol) at 25°C. Then it was stirred at 30 °C for 3 hrs. The mixture was concentrated to obtained **compound 53-c** (2.2 g). LCMS (M+H)⁺: 530.

### Step 4: Preparation of 5-[4-benzyloxy-5-methyl-2-(2-phenylethyl)pyrazol-3-yl]-4-[(4-methoxyphenyl)methyl]-1,2,4-triazole-3-thiol (compound 53-d)

A yellow solution of compound 53-c (2.0 g, 3.78 mmol) in 3N NaOH (20 mL) was stirred at 100 °C for 12 hrs. The mixture was extracted with EA (3 x 100 mL). The organic layer was washed with water (3 x 200 mL) and brine (450 mL), dried over sodium sulfate and concentrated. The residue was purified via column chromatography to give compound 53-d (1.3 g) as a yellow oil. LCMS (M+H)⁺: 512.

### Step 5: Preparation of 3-[4-benzyloxy-5-methyl-2-(2-phenylethyl)pyrazol-3-yl]-4-[(4-methoxyphenyl)methyl]-1,2,4-triazole (compound 53-e)

To a solution of compound 53-d (1.3 g, 2.54 mmol) in acetic acid (4 mL) was added H₂O₂ (2.91 g, 25.68 mmol) slowly, then it was stirred at 25 °C for 1 h. The reaction mixture was poured into saturated solution of Na₂SO₃ (300 mL) and extracted with ethyl acetate (3 x 150 mL). The combined organic layer was washed with brine (300 mL), dried over Na₂SO₄ and concentrated. The residue was purified via column chromatography to afford compound 53-e (1.0 g). LCMS (M+H)⁺: 480.

### Step 6: Preparation of 1-[5-[4-benzyloxy-5-methyl-2-(2-phenylethyl)pyrazol-3-yl]-4-[(4-methoxyphenyl)methyl]-1,2,4-triazol-3-yl]-N-[(2,4-dimethoxyphenyl)methyl]-6-methyl-imidazo[1,5-a]pyrazine-3-carboxamide (compound 53-f)

To a solution of 1-bromo-N-[(2,4-dimethoxyphenyl)methyl]-6-methyl-imidazo[1,5-a]pyrazine-3-carboxamide (Intermediate **A1**, 84 mg, 0.21 mmol) in 1,4-Dioxane (2 mL) was added Pd(OAc)₂ (14 mg, 0.06 mmol), Cs₂CO₃ (67 mg, 0.21 mmol), PCy₃HBF₄ (30 mg, 0.08 mmol), CuI (79 mg, 0.42 mmol) and compound 53-e (0.1 g, 0.21 mmol) in one portion. The mixture was stirred at 140 °C for 12 hr in a sealed tube. (The reaction was set in glove box). The mixture was concentrated and purified via Prep-TLC to give compound 53-f (60 mg). LCMS (M+H)⁺: 804.

### Step 7: 1-[3-[4-hydroxy-5-methyl-2-(2-phenylethyl)pyrazol-3-yl]-1H-1,2,4-triazol-5-yl]-6-methyl-imidazo[1,5-a]pyrazine-3-carboxamide (Example 53)

A solution of compound **53-f** (40 mg, 0.05 mmol) and MsOH (49 mg, 0.5 mmol) in HFIP (2 mL) was stirred at 30 °C for 3 hrs. The reaction mixture was purified by reversed-phase HPLC to obtain Example 53 (18.0 mg). LCMS (M+H)⁺: 444, ¹H NMR (400 MHz, DMSO-d6) δ = 14.17 - 13.83 (m, 1H), 9.68 (d, *J* = 1.4 Hz, 1H), 9.32 - 8.79 (m, 2H), 8.21 - 7.80 (m, 2H), 7.29 - 7.11 (m, 5H), 4.69 (d, *J* = 1.8 Hz, 2H), 3.13 - 2.99 (m, 2H), 2.50 ( s, 3H), 2.16 (s, 3H).

### Example 54

### 1-[3-[4-hydroxy-2-[2-(3-methoxyphenyl)ethyl]-5-methyl-pyrazol-3-yl]-1H-1,2,4-triazol-5-yl]-5-methyl-pyrazolo[3,4-c]pyridine-3-carboxamide

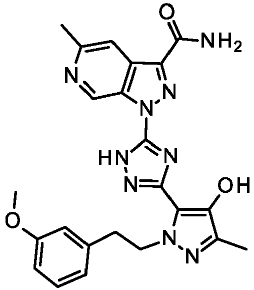

**Example** 54 was prepared in analogy to the preparation of Example 51 by using 1-(2-bromo-ethyl)-3-methoxy-benzene instead of 3-pentyn-1-ol in step 1.

Example 54 (6.9 mg), LCMS (M+H)⁺: 474, ¹H NMR (400 MHz, DMSO-d6) δ = 14.17 (br s, 1H), 9.63 (d, *J* = 1.2 Hz, 1H), 9.34 - 9.15 (m, 1H), 8.08 (s, 1H), 7.98 (br d, *J* = 0.9 Hz, 1H), 7.85 (br s, 1H), 7.09 (t, *J =* 7.8 Hz, 1H), 6.79 (d, *J* = 7.6 Hz, 1H), 6.75 - 6.67 (m, 2H), 4.69 (br t, *J* = 7.6 Hz, 2H), 3.56 (s, 3H), 3.10 - 3.00 (m, 2H), 2.68 (s, 3H), 2.19 (s, 3H).

### Example 55

### 1-(3-(1-(3-fluorophenethyl)-4-hydroxy-3-methyl-1H-pyrazol-5-yl)-1H-1,2,4-triazol-5-yl)-5-methyl-1H-pyrazolo[3,4-c]pyridine-3-carboxamide

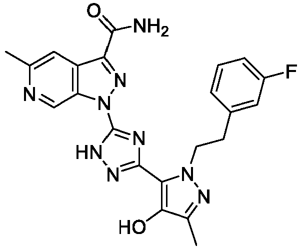

**Example** 55 was prepared in analogy to the preparation of Example 51 by using compound 55-b instead of compound 51-b in step 5.

The compound 55-b was prepared according to the following scheme:

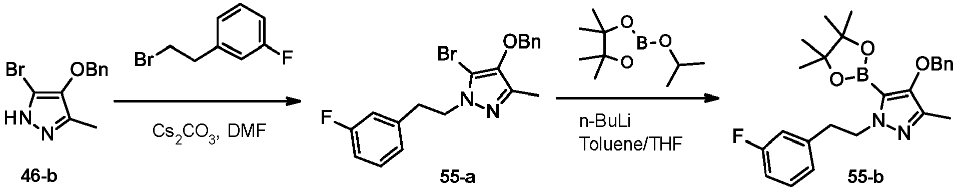

### Step 1: Preparation of 4-(benzyloxy)-5-bromo-1-(3-fluorophenethyl)-3-methyl-1H-pyrazole (compound 55-a)

To a solution of 4-benzyloxy-5-bromo-3-methyl-1H-pyrazole (46-b, 400.0 mg, 1.5 mmol) in DMF (1 mL) was added Cs₂CO₃ (1463.7 mg, 4.49 mmol) and 1-(2-bromoethyl)-3-fluorobenzene (912 mg, 4.49 mmol). The mixture was stirred at 100 °C for 12 hrs. The reaction mixture was diluted with H₂O (50 mL) and extracted with EtOAc (30 mL x 3). The organic layer was washed with brine, dried with Na₂SO₄ and concentrated. The residue was purified via column chromatography and prep-HPLC to afford **compound 55-a** (130 mg). LCMS (M+H)⁺: 389, ¹H NMR (400 MHz, CHLOROFORM-d) δ = 7.43 - 7.33 (m, 5H), 7.26 - 7.21 (m, 1H), 6.98 - 6.87 (m, 2H), 6.82 (br d, *J =* 10.0 Hz, 1H), 4.90 (s, 2H), 4.33 - 4.16 (m, 2H), 3.08 (t, *J =* 7.6 Hz, 2H), 2.09 (s, 3H).

### Step 2: Preparation of 4-(benzyloxy)-1-(3-fluorophenethyl)-3-methyl-5-(4,4,5,5-tetramethyl-1,3,2-dioxaborolan-2-yl)-1H-pyrazole (compound 55-b)

To a solution of 4-(benzyloxy)-5-bromo-1-(3-fluorophenethyl)-3-methyl-1H-pyrazole (55-a, 100 mg, 0.26 mmol) and 2-isopropoxy-4,4,5,5-tetramethyl-1,3,2-dioxaborolane (72 mg, 0.39 mmol) in THF (0.5 mL) and toluene (2.5 mL) was dropwise added n-BuLi (2.5 M, 0.12 mL, 0.31 mmol) at -78 °C under N₂ atmosphere. The mixture was stirred at -78 °C for 1 h, then warmed to 20 °C slowly and stirred for 1 h. The mixture was poured into saturated aqueous solution of NH₄Cl (10 ml) at 0 °C and extracted with EtOAc (3 x 10 mL). The organic layer was washed with brine and dried over anhydrous Na₂SO₄ and concentrated to afford crude **compound 55-b** (100 mg). The crude product was used to next step without purification. LCMS (M+H)⁺: 437.

Example 55 (18 mg), LCMS (M+H)⁺: 462, ¹H NMR (400 MHz, DMSO-*d₆*) δ = 14.16 (br s, 1H), 9.61 (s, 1H), 9.24 (br s, 1H), 8.08 (s, 1H), 7.99 (br s, 1H), 7.83 (br s, 1H), 7.25 - 7.14 (m, 1H), 7.07 - 6.92 (m, 3H), 4.70 (br t, *J =* 7.6 Hz, 2H), 3.10 (br t, *J =* 7.6 Hz, 2H), 2.68 (s, 3H), 2.17 (s, 3H).

### Example 56

### 1-[3-[2-[2-(2-fluorophenyl)ethyl]-4-hydroxy-5-methyl-pyrazol-3-yl]-1H-1,2,4-triazol-5-yl]-5-methyl-pyrazolo [3,4-c]pyridine-3-carboxamide

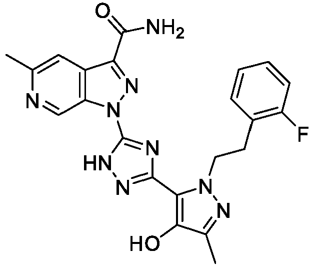

**Example** 56 was prepared in analogy to the preparation of Example 51 by using 2-fluorophenethyl alcohol instead of 3-pentyn-1-ol in step 1 and using 2-isopropoxy-4,4,5,5-tetramethyl-1,3,2-dioxaborolane instead of triisopropyl boratein step 2.

Example 56 (35.3 mg), LCMS (M+H⁺): 462, ¹H NMR (400 MHz, DMSO-d6) δ = 14.35 - 13.88 (m, 1H), 9.63 (s, 1H), 9.03 (s, 1H), 8.11 (s, 1H), 8.02 (s, 1H), 7.81 (s, 1H), 7.29 - 7.15 (m, 2H), 7.04 - 6.92 (m, 2H), 4.72 (t, *J =* 6.8 Hz, 2H), 3.11 (t, *J =* 6.8 Hz, 2H), 2.69 (s, 3H), 2.17 (s, 3H).

### Example 57

### 7-fluoro-8-[3-[4-hydroxy-5-methyl-2-(2-phenylethyl)pyrazol-3-yl]-1H-1,2,4-triazol-5-yl]-3-methyl-pyrrolo[1,2-a]pyrazine-6-carboxamide

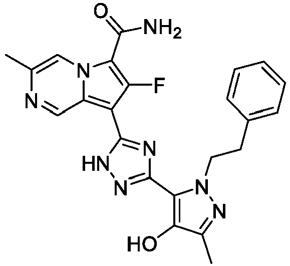

**Example** 57 was prepared in analogy to the preparation of Example 53 by using compound 4-g instead of Intermediate **A1** in step 6.

Example 57 (7.9 mg), LCMS (M+H)⁺: 461, ¹H NMR (400 MHz, DMSO-d6) δ = 14.37 - 13.69 (m, 1H), 9.63 (s, 1H), 9.22 (s, 1H), 9.21 - 8.78 (m, 1H), 7.92 - 7.74 (m, 1H), 7.56 - 7.37 (m, 1H), 7.23 - 7.16 (m, 5H), 4.69 (t, *J =* 7.6 Hz, 2H), 3.08 - 3.02 (m, 2H), 2.49 (s, 3H), 2.16 (s, 3H).

### Example 58

### 1-(2-(4-hydroxy-3-methyl-1-propyl-1H-pyrazol-5-yl)oxazol-4-yl)-5-methyl-1H-pyrazolo[3,4-c]pyridine-3-carboxamide

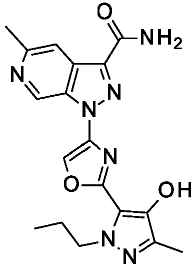

The title compound was prepared according to the following scheme:

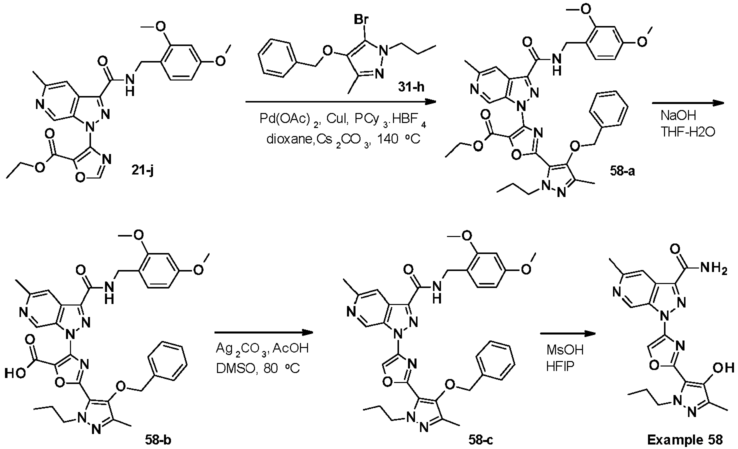

### Step 1: preparation of ethyl 2-(4-(benzyloxy)-3-methyl-1-propyl-1H-pyrazol-5-yl)-4-(3-(2,4-dimethoxybenzylcarbamoyl)-5-methyl-1H-pyrazolo[3,4-c]pyridin-1-yl)oxazole-5-carboxylate (compound 58-a)

To a stirred mixture of ethyl 4-(3-(2,4-dimethoxybenzylcarbamoyl)-5-methyl-1*H-*pyrazolo[3,4-c]pyridin-1-yl)oxazole-5-carboxylate (21-j, 80 mg, 0.17 mmol) and 4-(benzyloxy)-5-bromo-3-methyl-1-propyl-1H-pyrazole (31-h, 106 mg, 0.34 mmol) in 1,4-dioxane (8 mL) was added copper(I) iodide (98 mg, 0.52 mmol), palladium (II) acetate (38 mg, 0.17 mmol), cesium carbonate (111 mg, 0.34 mmol) and tricyclohexylphosphonium tetrafluoroborate (44 mg, 0.12 mmol). The resulting mixture was stirred at 140 °C for 16 hrs under nitrogen atmosphere. The mixture was cooled down and concentrated. The residue was purified via silica gel column chromatography to give **compound 58-a** (40 mg). LCMS (M+H)⁺: 694.

### Step 2: preparation of 2-(4-(benzyloxy)-3-methyl-1-propyl-1H-pyrazol-5-yl)-4-(3-(2,4-dimethoxybenzylcarbamoyl)-5-methyl-1H-pyrazolo[3,4-c]pyridin-1-yl)oxazole-5-carboxylic acid (compound 58-b)

To a stirred mixture of ethyl **compound 58-a** (35 mg, 0.05 mmol) in THF (1 mL) and methanol (1 mL) was added a solution of sodium hydroxide (22 mg, 0.55 mmol) in water (1 mL) dropwise at 0 °C. The resulting mixture was stirred at 25 °C for 16 hrs and then acidified to pH = 5 with HCl (1.0 M aq.) at 0 °C. The suspension was filtered, the wet-cake was dried to give **compound 58-b** (25 mg). LCMS (M+H)⁺: 666.

### Step 3: preparation of 1-(2-(4-(benzyloxy)-3-methyl-1-propyl-1H-pyrazol-5-yl)oxazol-4-yl)-N-(2,4-dimethoxybenzyl)-5-methyl-1H-pyrazolo[3,4-c]pyridine-3-carboxamide (compound 58-c)

To a stirred mixture of **compound 58-b** (20 mg, 0.03 mmol) in DMSO (4 mL) was added acetic acid (0.5 mg, 0.01 mmol) and silver carbonate (2.1 mg, 0.01 mmol). The resulting mixture was stirred at 85 °C for 3 hrs under nitrogen atmosphere. The mixture was poured into ice/water (10 mL) and extracted with EtOAc (3 x 10 mL). The combined organic layer was dried over anhydrous sodium sulfate and concentrated to give **compound 58-c** (18 mg). LCMS (M+H)⁺: 622.

### Step 4: preparation of 1-(2-(4-hydroxy-3-methyl-1-propyl-1H-pyrazol-5-yl)oxazol-4-yl)-5-methyl-1H-pyrazolo[3,4-c]pyridine-3-carboxamide (Example 58)

To a stirred mixture of **compound 58-c** (18 mg, 0.03 mmol,) in 1,1,1,3,3,3-Hexafluoro-2-propanol (0.5 mL) was added methanesulfonic acid (31 mg, 0.32 mmol). The resulting mixture was stirred at 50 °C for 5 hrs under nitrogen atmosphere. The mixture was cooled down to room temperature. The residue was purified via reversed flash chromatography to give **Example 58** (5.7 mg) as a white solid. LCMS (M+H)⁺: 382. ¹H NMR (400 MHz, DMSO-*d₆*) δ = 9.57 (s, 1H), 8.99 (s, 1H), 8.66 (s, 1H), 8.00 (s, 2H), 7.78 (s, 1H), 4.46 - 4.42 (m, 2H), 2.65 (s, 3H), 2.16 (s, 3H), 1.84 - 1.82 (m, 2H), 0.92 - 0.89 (m, 3H).

### Example 59

### 1-(2-(1-butyl-4-hydroxy-3-methyl-1H-pyrazol-5-yl)oxazol-4-yl)-5-methyl-1H-pyrazolo[3,4-c]pyridine-3-carboxamide

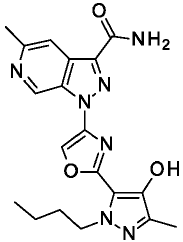

**Example 59** was prepared in analogy to the preparation of Example 58 by using compound 49-b instead of compound 31-h.

**Example** 59 (3.0 mg), LCMS (M+H)⁺: 396, ¹H NMR (400 MHz, DMSO-*d₆*) δ = 9.55 (s, 1H), 9.01 (br s, 1H), 8.66 (s, 1H), 8.00 (s, 2H), 7.78 (s, 1H), 4.49 - 4.46 (m, 2H), 2.65 (s, 3H), 2.16 (s, 3H), 1.81 - 1.77 (m, 2H), 1.33 - 1.31 (m, 2H), 0.91- 0.87 (m, 3H).

### Example 60

### 1-[3-[2-[2-(3-cyanophenyl)ethyl]-4-hydroxy-5-methyl-pyrazol-3-yl]-1H-1,2,4-triazol-5-yl]-5-methyl-pyrazolo[3,4-c]pyridine-3-carboxamide

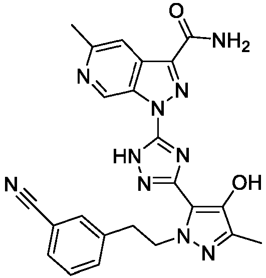

**Example** 60 was prepared in analogy to the preparation of Example 51 by using 3-(2-hydroxyethyl)benzonitrile instead of 3-pentyn-1-ol in step 1 and using 2-isopropoxy-4,4,5,5-tetramethyl-1,3,2-dioxaborolane instead of triisopropyl boratein step 2.

Example 60 (30 mg), LCMS (M+H)⁺: 469, ¹H NMR (400 MHz, DMSO-d6) δ = 14.12 (br s, 1H), 9.60 (s, 1H), 9.25 (br s, 1H), 8.06 (s, 1H), 8.03 (br s, 1H), 7.80 (br s, 1H), 7.61 - 7.55 (m, 2H), 7.53 (br d, *J= 7.8* Hz, 1H), 7.36 (t, *J= 7.8* Hz, 1H), 4.75 (br t, *J=* 7.2 Hz, 2H), 3.16 (br t, *J* = 7.2 Hz, 2H), 2.68 (s, 3H), 2.18 (s, 3H).

### Example 61

### 1-[3-[2-[2-(4-cyanophenyl)ethyl]-4-hydroxy-5-methyl-pyrazol-3-yl]-1H-1,2,4-triazol-5-yl]-5-methyl-pyrazolo[3,4-c]pyridine-3-carboxamide

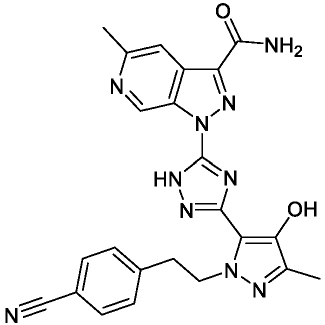

**Example** 61 was prepared in analogy to the preparation of Example 51 by using 4-(2-hydroxyethyl)benzonitrile instead of 3-pentyn-1-ol in step 1 and using 2-isopropoxy-4,4,5,5-tetramethyl-1,3,2-dioxaborolane instead of triisopropyl boratein step 2.

Example 61 (17.6 mg), LCMS (M+H)⁺: 469, ¹H NMR (400 MHz, DMSO-d6) δ = 14.14 ( s, 1H), 9.57 (d, *J=* 1.0 Hz, 1H), 9.42 - 9.15 (m, 1H), 8.06 (s, 2H), 7.86 ( s, 1H), 7.61 (d, *J= 8.2* Hz, 2H), 7.42 (d, *J* = 8.2 Hz, 2H), 4.72 (t, *J =* 7.2 Hz, 2H), 3.17 (t, *J =* 7.2 Hz, 2H), 2.68 (s, 3H), 2.16 (s, 3H).

### Example 62

### Methyl 4-[2-[5-[5-(3-carbamoyl-5-methyl-pyrazolo[3,4-c]pyridin-1-yl)-1H-1,2,4-triazol-3-yl]-4-hydroxy-3-methyl-pyrazol-1-yl]ethyl]benzoate

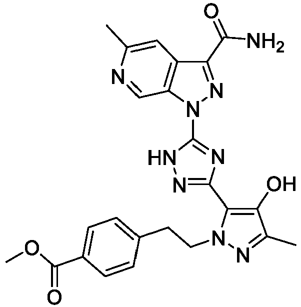

**Example** 62 was prepared in analogy to the preparation of Example 51 by using methyl 4-(2-hydroxyethyl)benzoate instead of 3-pentyn-1-ol in step 1 and using 2-isopropoxy-4,4,5,5-tetramethyl-1,3,2-dioxaborolane instead of triisopropyl boratein step 2.

Example 62 (14 mg), LCMS (M+H)⁺: 502, ¹H NMR (400 MHz, DMSO-*d₆*) δ = 14.13 (s, 1H), 9.57 (d, *J=* 1.0 Hz, 1H), 9.23 (s, 1H), 8.09 - 7.99 (m, 2H), 7.83 (br s, 1H), 7.72 (d, *J =* 8.4 Hz, 2H), 7.32 (d, *J =* 8.0 Hz, 2H), 4.73 (br t, *J =* 7.2 Hz, 2H), 3.79 (s, 3H), 3.17 - 3.12 (m, 2H), 2.67 (s, 3H), 2.17 (s, 3H).

### Example 63

### 1-(3-(1-(3,3-difluoroallyl)-4-hydroxy-3-methyl-1H-pyrazol-5-yl)-1H-1,2,4-triazol-5-yl)-5-methyl-1H-pyrazolo[3,4-c]pyridine-3-carboxamide

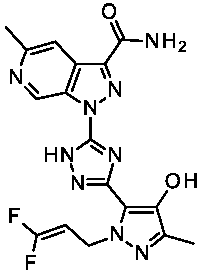

The title compound was prepared according to the following scheme:

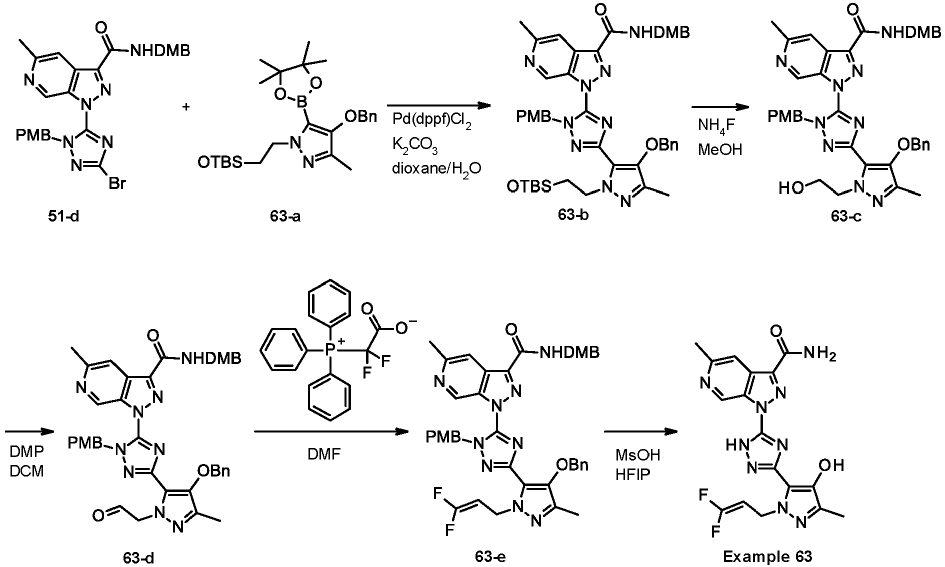

2-[4-benzyloxy-3-methyl-5-(4,4,5,5-tetramethyl-1,3,2-dioxaborolan-2-yl)pyrazol-1-yl]ethoxy-tert-butyl-dimethyl-silane **(compound 63-a)** was prepared in analogy to the preparation of **compound 49-c** by using (2-bromoethoxy)-tert-butyldimethylsilane instead of 1-bromobutane in step 1 and using reagent 2-isopropoxy-4,4,5,5-tetramethyl-1,3,2-dioxaborolane instead of triisopropyl borate in step 3.

### Step 1: 1-[5-[4-benzyloxy-2-[2-[tert-butyl(dimethyl)silyl]oxyethyl]-5-methyl-pyrazol-3-yl]-1-[(4-methoxyphenyl)methyl]-1,2,4-triazol-3-yl]-N-[(2,4-dimethoxyphenyl)methyl]-5-methyl-pyrazolo[3,4-c]pyridine-3-carboxamide (compound 63-b)

To a solution of **compound 63-a** (1.3 g, 2.75 mmol) in 1,4-dioxane (10 mL) and water (1 mL) was added 1-[5-bromo-1-[(4-methoxyphenyl)methyl]-1,2,4-triazol-3-yl]-N-[(2,4-dimethoxyphenyl)methyl]-5-methyl-pyrazolo[3,4-c]pyridine-3-carboxamide (51-d, 1.0 g, 1.69 mmol), K₂CO₃ (1.2 g, 8.44 mmol) and Pd(dppf)Cl₂.DCM (413 mg, 0.51 mmol). The mixture was stirred at 100 °C for 12 hrs under N₂ atmosphere. It was then cooled and poured into water (100 mL). The suspension was extracted with EtOAc (100 mL x 2), the organic phase was washed with brine (20 mL x 2), dried over Na₂SO₄ and concentrated. The residue was purified via column chromatography to give **compound 63-b** (1.1 g). LCMS (M+H)⁺: 858.

### Step 2: 1-[5-[4-benzyloxy-2-(2-hydroxyethyl)-5-methyl-pyrazol-3-yl]-2-[(4-methoxyphenyl)methyl]-1,2,4-triazol-3-yl]-N-[(2,4-dimethoxyphenyl)methyl]-5-methyl-pyrazolo[3,4-c]pyridine-3-carboxamide (compound 63-c)

To a solution of 1-[5-[4-benzyloxy-2-[2-[tert-butyl(dimethyl)silyl]oxyethyl]-5-methyl-pyrazol-3-yl]-2-[(4-methoxyphenyl)methyl]-1,2,4-triazol-3-yl]-N-[(2,4-dimethoxyphenyl)methyl]-5-methyl-pyrazolo[3,4-c]pyridine-3-carboxamide (63-b, 1.1 g, 1.28 mmol) in methanol (10 mL) was added NH₄F (0.95 g, 25.6 mmol). The mixture was stirred at 70 °C for 1 h. The mixture was cooled, filterd and washed with MeOH (40 mL x 2). The filtrate was concentrated and purified with column chromatography to give **compound 63-c** (750 mg). LCMS (M+H)⁺: 744.

### Step 3: 1-[5-[4-benzyloxy-5-methyl-2-(2-oxoethyl)pyrazol-3-yl]-2-[(4-methoxyphenyl)methyl]-1,2,4-triazol-3-yl]-N-[(2,4-dimethoxyphenyl)methyl]-5-methyl-pyrazolo[3,4-c]pyridine-3-carboxamide (compound 63-d)

To a solution of **compound 63-c** (300 mg, 0.40 mmol) in DCM (10 mL) was added Dess-Martin periodinane (684 mg, 1.61 mmol). The mixture was stirred at 25 °C for 2 hrs. It was then poured into a mixture of saturated Na₂SO₃ solution (30 mL) and saturated NaHCO₃ solution (30 mL). The mixture was stirred for 20 mins and extracted with DCM (30 mL). The organic layer was dried over Na₂SO₄, filtered and concentrated to give **compound 63-d** (300 mg). LCMS (M+H)⁺: 742.

### Step 4: 1-[5-[4-benzyloxy-2-(3,3-difluoroallyl)-5-methyl-pyrazol-3-yl]-2-[(4-methoxyphenyl)methyl]-1,2,4-triazol-3-yl]-N-[(2,4-dimethoxyphenyl)methyl]-5-methyl-pyrazolo[3,4-c]pyridine-3-carboxamide (compound 63-e)

To a solution of **compound 63-d** (300 mg, 0.40 mmol) in DMF (1 mL) was added 2-(triphenylphosponio)difluoroacetate (582 mg, 1.62 mmol). The mixture was stirred at 80 °C for 12 hrs. The mixture was poured into water (30 mL), and extracted with EtOAc (30 mL x 2), the organic phase was washed with brine (10 mL x 3), dried over Na₂SO₄, and concentrated. The residue was purified via prep-TLC to give **compound 63-e** (60 mg). LCMS (M+H)⁺: 776.

### Step 5: 1-(3-(1-(3,3-difluoroallyl)-4-hydroxy-3-methyl-1H-pyrazol-5-yl)-1H-1,2,4-triazol-5-yl)-5-methyl-1H-pyrazolo[3,4-c]pyridine-3-carboxamide (Example 63)

To a solution of compound 63-e (55 mg, 0.07 mmol) in HFIP (0.3 mL) was added MsOH (136 mg, 1.42 mmol). The mixture was stirred at 50 °C for 0.5 h. The mixture was then directly purified via prep-HPLC to give Example 63 (30 mg). LCMS (M+H)⁺: 416, ¹H NMR (DMSO-d6, 400 MHz) δ = 14.28 (br s, 1H), 9.62 (s, 1H), 9.34 (br s, 1H), 8.05 (s, 2H), 7.78 (br s, 1H), 5.13 (br d, *J= 7.6* Hz, 2H), 5.02 - 4.90 (m, 1H), 2.66 (s, 3H), 2.18 (s, 3H).

### Example 64

### 1-[3-[4-hydroxy-2-[2-[4-(methoxymethyl)phenyl]ethyl]-5-methyl-pyrazol-3-yl]-1H-1,2,4-triazol-5-yl]-5-methyl-pyrazolo[3,4-c]pyridine-3-carboxamide

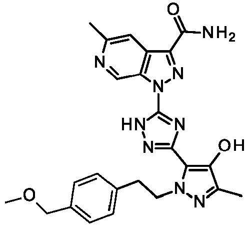

**Example** 64 was prepared in analogy to the preparation of Example 51 by using compound 64-a instead of 3-pentyn-1-ol in step 1 and using 2-isopropoxy-4,4,5,5-tetramethyl-1,3,2-dioxaborolane instead of triisopropyl boratein step 2.

The compound 64-a was prepared according to the following scheme:

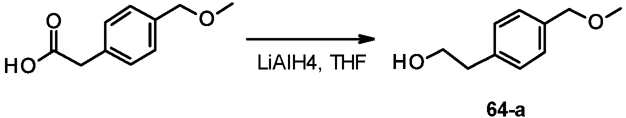

### Step 1: Preparation of 2-[4-(methoxymethyl)phenyl]ethanol (compound 64-a)

To a solution of 2-[4-(methoxymethyl)phenyl]acetic acid (800 mg, 4.44 mmol) in THF (20 mL) was added LiAlH₄ (505 mg, 13.32 mmol) at 0 °C, then the mixture was stirred at 60 °C for 0.5 h. The reaction mixture was added into 1M aqueous solution of HCl (50 mL), extracted with EtOAc (50 mL ×3). The combined organic phase was dried over Na₂SO₄, filtered and concentrated to give **compound 64-a** (650 mg). LCMS (M+H)⁺: 415, ¹H NMR (400 MHz, CHLOROFORM-d) δ = 7.34 - 7.30 (m, 2H), 7.26 - 7.21 (m, 2H), 4.46 (s, 2H), 3.88 (t, *J =* 6.8 Hz, 2H), 3.41 (s, 3H), 2.89 (t, *J =* 6.4 Hz, 2H).

**Example 64** (30 mg), LCMS (M+H)⁺: 488, ¹H NMR (400 MHz, DMSO-d6) δ = 14.16 (br s, 1H), 9.62 (d, *J =* 1.0 Hz, 1H), 9.23 (br s, 1H), 8.08 (s, 1H), 8.00 (br s, 1H), 7.86 (br s, 1H), 7.23 - 7.18 (d, *J=* 8.0 Hz, 2H), 7.11 (d, *J =* 8.0 Hz, 2H), 4.68 (br t, *J =* 7.6 Hz, 2H), 4.32 (s, 2H), 3.23 (s, 3H), 3.10 - 3.03 (br t, *J =* 6.8 Hz, 2H), 2.69 (s, 3H), 2.19 (s, 3H).

### Example 65

### 1-[3-[4-hydroxy-2-[2-[3-(methoxymethyl)phenyl]ethyl]-5-methyl-pyrazol-3-yl]-1H-1,2,4-triazol-5-yl]-5-methyl-pyrazolo[3,4-c]pyridine-3-carboxamide

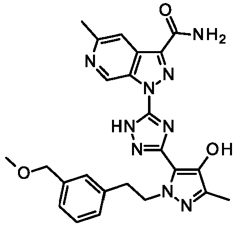

**Example** 65 was prepared in analogy to the preparation of Example 51 by using compound 65-c instead of 3-pentyn-1-ol in step 1 and using 2-isopropoxy-4,4,5,5-tetramethyl-1,3,2-dioxaborolane instead of triisopropyl boratein step 2.

The compound 65-c was prepared according to the following scheme:

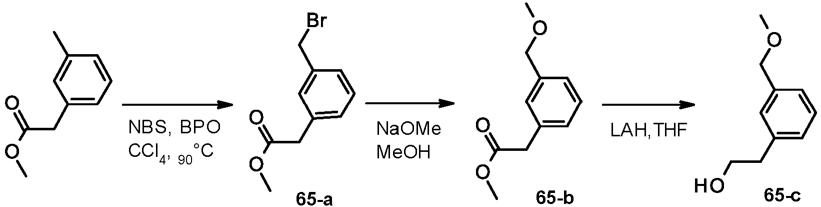

### Step 1: Preparation of methyl 2-[3-(bromomethyl)phenyl]acetate (compound 65-a)

To a solution of methyl 3-methylphenylacetate (9.0 g, 54.81 mmol) in carbon tetrachloride (1 mL) was added BPO (1.33 g, 5.48 mmol), and NBS (9.76 g, 54.81 mmol). The reaction mixture was stirred at 90 °C for 18 hrs under N₂. The reaction mixture was poured into water (100 mL), extracted with DCM (200 mL x 2). The combined organic layer was washed with brine (100 mL), dried over Na₂SO₄, and filtered. The filtrate was concentrated and purified via flash chromatography on silica gel to afford **compound 65-a** (5.0 g). LCMS (M+H)⁺: 243, ¹H NMR (400 MHz, DMSO-d6) δ = 7.43 - 7.29 (m, 3H), 7.27 - 7.18 (m, 1H), 4.70 (s, 2H), 3.70 (s, 2H), 3.62 (s, 3H).

### Step 2: Preparation of methyl 2-[3-(methoxymethyl)phenyl]acetate (compound 65-b)

To a solution of **compound 65-a** (5.0 g, 20.6 mmol) in methanol (50 mL) was added NaOMe (5.0 mL, 25.0 mmol) at 0 °C. The reaction mixture was stirred at 25 °C for 18 hrs and poured into Sat. NH₄Cl (50 mL), it was then extracted with EA (50 mL x 2). The combined organic layer was washed with brine (20 mL), dried over Na₂SO₄ and concentrated. The residue was purified via reversed-phase column to give **compound 65-b** (1.5 g). LCMS (M-CH₃O)⁺: 163.

### Step 3: Preparation of 2-[3-(methoxymethyl)phenyl]ethanol (compound 65-c)

To a solution of **compound 65-b** (1.5g, 7.7 mmol) in THF (15 mL) was added LiAlH₄ (2.5 M, 3.86 mL, 9.64 mmol) at 0 °C. The reaction mixture was stirred at 0 °C for 1 h and poured into 1 N aqueous solution of HCl (20 mL). It was then extracted with EA (30 mL x 2). The combined organic layer was washed with brine (20 mL), dried over Na₂SO₄, and concentrated to give a residue. The residue was purified via column to give **compound 65-c** (600 mg). LCMS (M-CH₃O)⁺: 135.

Example 65 (32.3 mg), LCMS (M+H)⁺: 488, ¹H NMR (400 MHz, DMSO-d6) δ = 14.16 (br d, *J= 2.6* Hz, 1H), 9.65 (d, *J=* 1.0 Hz, 1H), 9.47 - 8.96 (m, 1H), 8.12 (s, 1H), 8.07 (br s, 1H), 7.85 (br s, 1H), 7.20 - 7.04 (m, 4H), 4.75 - 4.65 (m, 2H), 4.17 (s, 2H), 3.09 (s, 3H), 3.07 (br s, 2H), 2.70 (s, 3H), 2.19 (s, 3H).

### Example 66

### 1-(3-(1-(3,3-difluoropropyl)-4-hydroxy-3-methyl-1H-pyrazol-5-yl)-1H-1,2,4-triazol-5-yl)-5-methyl-1H-pyrazolo[3,4-c]pyridine-3-carboxamide

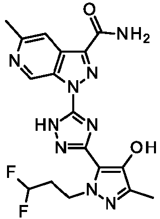

The title compound was prepared according to the following scheme:

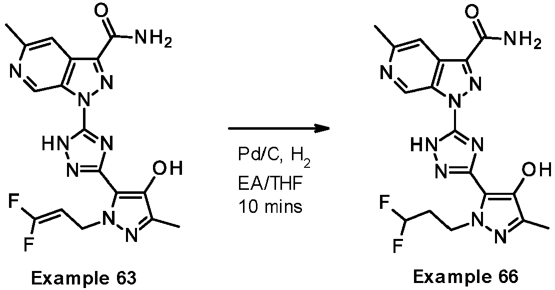

### Step 1: Preparation of 1-(3-(1-(3,3-difluoropropyl)-4-hydroxy-3-methyl-1H-pyrazol-5-yl)-1H-1,2,4-triazol-5-yl) -5-methyl-1H-pyrazolo[3,4-c]pyridine-3-carboxamide (Example 66)

To a solution of 1-(3-(1-(3,3-difluoroallyl)-4-hydroxy-3-methyl-1*H*-pyrazol-5-yl)-1*H-*1,2,4-triazol-5-yl)-5-methyl-1H-pyrazolo[3,4-c]pyridine-3-carboxamide (Example 63, 20 mg, 0.05 mmol) in THF (2 mL) and EtOAc (2 mL) was added Pd/C (20 mg) at 20°C under N₂ atmosphere. The mixture was degassed and purged with H₂ for three times, then it was stirred at 20°C for 10 mins with H₂ balloon. The mixture was purified via prep-HPLC to give Example 66 (5 mg) as a yellow solid. LCMS (M+H)⁺: 418, ¹H NMR (DMSO-d6, 400 MHz) δ = 14.34 - 14.17 (m, 1H), 9.62 (d, *J=* 0.8 Hz, 1H), 9.43 - 9.20 (m, 1H), 8.15 - 7.95 (m, 2H), 7.79 (br s, 1H), 6.34 - 5.98 (m, 1H), 4.66 (t, *J= 7.2* Hz, 2H), 2.67 (s, 3H), 2.43 - 2.35 (m, 2H), 2.18 (s, 3H).

### Example 67

### 1-(2-(1-(3,3-difluoropropyl)-4-hydroxy-3-methyl-1H-pyrazol-5-yl)oxazol-4-yl)-5-methyl-1H-pyrazolo[3,4-c]pyridine-3-carboxamide

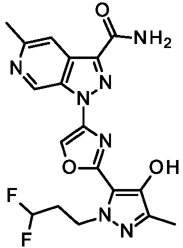

**Example** 67 was prepared in analogy to the preparation of Example 58 by using compound 67-a instead of compound 31-h.

The compound 67-a was prepared according to the following scheme:

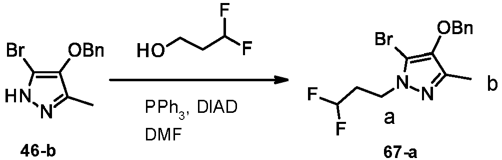

### Step 1: Preparation of 4-(benzyloxy)-5-bromo-1-(3,3-difluoropropyl)-3-methyl-1H-pyrazole (compound 67-a)

To a stirred mixture of 4-(benzyloxy)-5-bromo-3-methyl-1H-pyrazole (46-b, 1.50 g, 5.62 mmol), 3,3-difluoropropan-1-ol (863 mg, 8.98 mmol) and triphenylphosphine (1.91 g, 7.30 mmol) in THF (25 mL) was added diisopropyl azodicarboxylate (1.70 g, 8.42 mmol) dropwise at 0 °C. The resulting mixture was stirred at 40 °C for 4 hrs. It was then cooled and poured into ice/water (50 mL) slowly. The mixture was extracted with EtOAc (3 x 50 mL), the organic layer was dried and concentrated. The residue was purified via silica gel column chromatography to give **compound 67-a** (800 mg). LCMS (M+H)⁺: 345. The structure was confirmed with ¹H NMR and Nuclear Overhauser Effect Spectroscopy (NOESY) (no correlation between Ha and Hb was observed).

**Example** 67 (8.8 mg), LCMS (M+H)⁺: 418. ¹H NMR (400 MHz, DMSO-*d*₆) δ = 9.54 (s, 1H), 9.19 (br s, 1H), 8.67 (s, 1H), 7.99 (s, 2H), 7.77 (s, 1H), 6.31 - 6.01 (m, 1H), 4.69 - 4.66 (m, 2H), 2.64 (s, 3H), 2.46 - 2.29 (m, 2H), 2.16 (s, 3H).

### Example 68

### 1-[3-[4-hydroxy-2-[2-[4-(hydroxymethyl)phenyl]ethyl]-5-methyl-pyrazol-3-yl]-1H-1,2,4-triazol-5-yl]-5-methyl-pyrazolo[3,4-c]pyridine-3-carboxamide

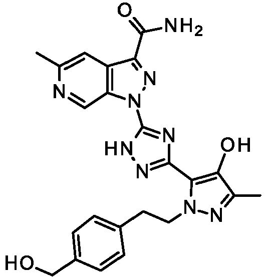

The title compound was prepared according to the following scheme:

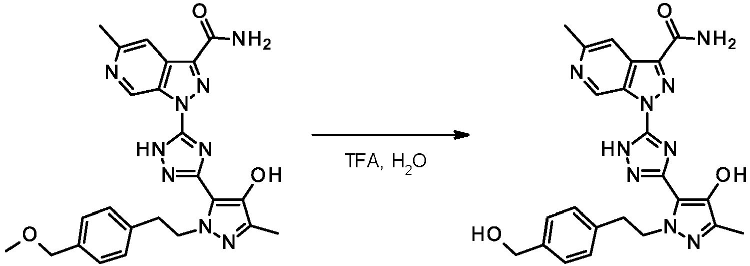

### Example 64 Example 68

A solution of 1-[3-[4-hydroxy-2-[2-[4-(methoxymethyl)phenyl]ethyl]-5-methyl-pyrazol-3-yl]-1H-1,2,4-triazol-5-yl]-5-methyl-pyrazolo[3,4-c]pyridine-3-carboxamide (**Example 64,** 10 mg, 0.02 mmol) in trifluoroacetic acid (2.0 mL, 25.96 mmol) and water (0.2 mL) was stirred at 100 °C for 6 hrs. The mixture was filtered, the filtrate was purified via prep-HPLC to afford **Example 68** (1.4 mg), LCMS (M+H)⁺: 474, ¹H NMR (400 MHz, DMSO-*d*₆) δ = 14.16 (br s, 1H), 9.63 (s, 1H), 9.22 (s, 1H), 8.06 (s, 1H), 7.98 (br s, 1H), 7.84 (br s, 1H), 7.21 - 7.16 (m, 2H), 7.15 - 7.10 (m, 2H), 4.65 (br dd, *J* = 7.6, 8.4 Hz, 2H), 4.41 (s, 2H), 3.08 - 3.02 (m, 2H), 2.68 (s, 3H), 2.18 (s, 3H).

### Example 69

### 7-fluoro-8-[3-[4-hydroxy-2-[2-(4-methoxyphenyl)ethyl]-5-methyl-pyrazol-3-yl]-1H-1,2,4-triazol-5-yl]-3-methyl-pyrrolo[1,2-a]pyrazine-6-carboxamide

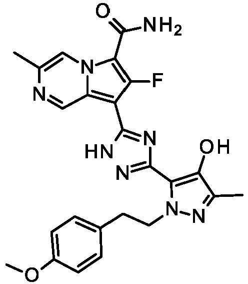

**Example 69** was prepared in analogy to the preparation of **Example 53** by using 4-methoxyphenethyl bromide instead of 1-bromo-2-phenylethane in step 1 and using **compound 4-**g instead of **Intermediate A1** in step 6.

**Example 69** (6.2 mg), LCMS (M+H)⁺: 491, ¹H NMR (400 MHz, DMSO-d6) δ = 14.19 - 13.59 (m, 1H), 9.61 (s, 1H), 9.22 (s, 1H), 9.19 - 8.91 (m, 1H), 7.93 - 7.69 (m, 1H), 7.60 - 7.37 (m, 1H), 7.10 (d, *J* = 8.6 Hz, 2H), 6.76 (d, *J =* 8.6 Hz, 2H), 4.69 - 4.62 (m, 2H), 3.68 (s, 3H), 3.00 - 2.95 (m, 2H), 2.49 - 2.48 (m, 3H), 2.16 (s, 3H).

### Example 70

### 1-[3-[4-hydroxy-2-[2-(4-methoxyphenyl)ethyl]-5-methyl-pyrazol-3-yl]-1H-1,2,4-triazol-5-yl]-6-methyl-imidazo[1,5-a]pyrazine-3-carboxamide

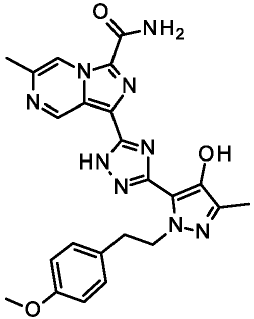

**Example 70** was prepared in analogy to the preparation of **Example 53** by using 4-methoxyphenethyl bromide instead of 1-bromo-2-phenylethane in step 1.

**Example 70** (20 mg), LCMS (M+H)⁺: 474.3, ¹H NMR (400 MHz, DMSO-d6+ D₂O) δ = 9.57 (s, 1H), 9.02 (s, 1H), 7.00 (d, *J* = 8.4 Hz, 2H), 6.66 (d, *J* = 8.4 Hz, 2H), 4.60 (t, *J* = 7.2 Hz, 2H), 3.59 (s, 3H), 2.91 (t, *J =* 7.2 Hz, 2H), 2.45 (s, 3H), 2.12 (s, 3H).

### Example 71

### 1-[3-[4-hydroxy-2-[2-[4-(hydroxymethyl)phenyl]ethyl]-5-methyl-pyrazol-3-yl]-1H-1,2,4-triazol-5-yl]-6-methyl-imidazo[1,5-a]pyrazine-3-carboxamide

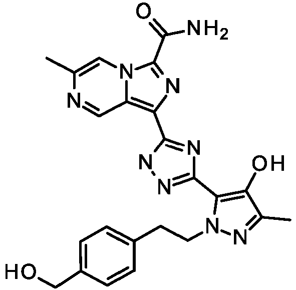

The title compound was prepared according to the following scheme:

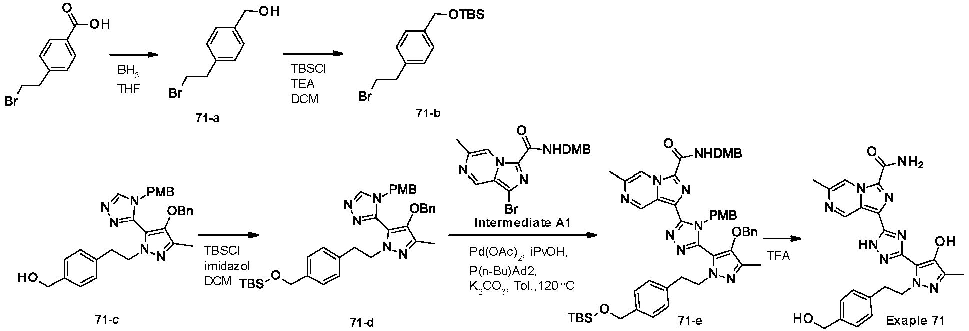

### Step 1: Preparation of [4-(2-bromoethyl)phenyl]methanol (compound 71-a)

To a solution of 4-(2-bromoethyl)benzoic acid (20.0 g, 87.3 mmol) in THF (200 mL) was added BH₃.THF (1.0 M, 174.6 mL, 174.6 mmol) at 0 °C. The reaction mixture warmed to 25 °C and stirred for 2 hrs. It was then poured into 1 N HCl (100 mL) and extracted with EA (200 mL x 2). The combined layer was washed with brine (20 mL), dried over Na₂SO₄ and concentrated to give **compound 71-a** (18.0 g) as light yellow solid. LCMS (M+H-18)⁺: 197.

### Step 2: Preparation of [4-(2-bromoethyl)phenyl]methoxy-tert-butyl-dimethyl-silane (compound 71-b)

To a solution of [4-(2-bromoethyl)phenyl]methanol 71-a (8.0 g, 37.2 mmol), Et₃N (14.3 mL, 111.6 mmol) in DCM (100 mL) was added TBSCl (8.41 g, 55.8 mmol) at 0 °C. The reaction mixture warmed to 25 °C and stirred at for 18 hra. Then it was poured into water (100 mL) and extracted with DCM (100 mL x 2). The combined layer was washed with brine (100 mL), dried over Na₂SO₄, and concentrated to give an oil. The residue was purified via column to give **71-b** (8.0 g). LCMS (M+H-C₆H₁₅SiO)⁺: 197.

### Step 3: Preparation of [4-[2-[4-benzyloxy-5-[4-[(4-methoxyphenyl)methyl]-1,2,4-triazol-3-yl]-3-methyl-pyrazol-1-yl]ethyl]phenyl]methanol (compound 71-c)

**Compound 71-c** was prepared in analogy to the preparation of **Example 53** by using **compound 71-b** instead of 1-bromo-2-phenylethane in step 1.

### Step 4: Preparation of [4-[2-[4-benzyloxy-5-[4-[(4-methoxyphenyl)methyl]-1,2,4-triazol-3-yl]-3-methyl-pyrazol-1-yl]ethyl]phenyl]methoxy-tert-butyl-dimethyl-silane (compound 71-d)

To a yellow solution of **compound 71-c** (1.5 g, 2.9 mmol), imidazole (400 mg, 5.9 mmol) in DCM (20 mL) was added TBSCl (2.96 g, 5.9 mmol) at 25°C. The mixture was stirred for 2 hrs and concentrated. The residue was purified via column chromatography to give **compound 71-d** (1.2 g). LCMS (M+H)⁺: 624.

### Step 5: Preparation of 1-[5-[4-benzyloxy-2-[2-[4-[[tert-butyl(dimethyl)silyl]oxymethyl] phenyl]ethyl]-5-methyl-pyrazol-3-yl]-4-[(4-methoxyphenyl)methyl]-1,2,4-triazol-3-yl]-N-[(2,4-dimethoxyphenyl)methyl]-6-methyl-imidazo[1,5-a]pyrazine-3-carboxamide (compound 71-e)

A suspension of **compound 71-d** (300 mg, 0.48 mmol), 1-bromo-*N*-[(2,4-dimethoxyphenyl)methyl]-6-methyl-imidazo[1,5-a]pyrazine-3-carboxamide (**Intermediate A1,** 194 mg, 0.48 mmol), Pd(OAc)₂ (32.4 mg, 0.14 mmol), PivOH (29 mg, 0.29 mmol), catacxiuma (52 mg, 0.14 mmol), K₂CO₃ (199 mg, 1.44 mmol) in toluene (2 mL) was stirred at 120 °C for 12 hrs under N₂ atmosphere. The mixture was filtered and washed with DCM/MeOH(V/V = 20/1, 20 mL). The filtrate was concentrated and purified by prep-TLC to afford **compound 71-e** (55 mg). LCMS (M+H)⁺: 948.

### Step 6: Preparation of 1-[3-[4-hydroxy-2-[2-[4-(hydroxymethyl)phenyl]ethyl]-5-methyl-pyrazol-3-yl]-1H-1,2,4-triazol-5-yl]-6-methyl-imidazo[1,5-a]pyrazine-3-carboxamide (Example 71)

A solution of **compound 71-e** (50.0 mg, 0.05 mmol) in TFA (3 mL) was stirred at 60 °C for 12 hrs under N₂ atmosphere. The mixture was concentrated and purified via prep-HPLC to give **Example 71** (6.2 mg). LCMS (M+H)⁺: 474, ¹H NMR (400 MHz, DMSO-d₆ + D₂O) δ = 9.68 (s, 1H), 9.10 (s, 1H), 7.36 - 6.83 (m, 4H), 4.66 (br s, 2H), 4.41 (s, 2H), 3.04 (br t, *J* = 7.8 Hz, 2H), 2.50 - 2.50 (m, 3H), 2.16 (s, 3H).

### Example 72

### 1-[3-[4-hydroxy-5-methyl-2-[2-(3-pyridyl)ethyl]pyrazol-3-yl]-1H-1,2,4-triazol-5-yl]-6-methyl-imidazo[1,5-a]pyrazine-3-carboxamide

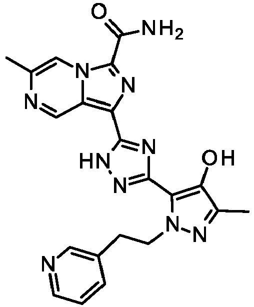

**Example 72** was prepared in analogy to the preparation of **Example 53** by using 2-(3-pyridyl)ethanol instead of 1-bromo-2-phenylethane in step 1 (reaction condition: CMBP, toluene 80 °C instead of K₂CO₃, DMF 80 °C).

**Example 72** (18.7 mg), LCMS (M+H)⁺: 445, ¹H NMR (400 MHz, DMSO+D₂O) δ = 9.65 (s, 1H), 9.12 (s, 1H), 9.05 - 8.81 (m, 1H), 8.35 (br d, *J* = 3.8 Hz, 1H), 7.63 (br d, *J =* 7.4 Hz, 1H), 7.24 (dd, *J =* 4.8, 7.6 Hz, 1H), 4.69 (br t, *J =* 7.2 Hz, 2H), 3.09 (br t, *J =* 7.2 Hz, 2H), 2.49 (br s, 3H), 2.15 (s, 3H).

### Example 73

### 7-fluoro-8-[3-[4-hydroxy-5-methyl-2-[2-(3-pyridyl)ethyl]pyrazol-3-yl]-1H-1,2,4-triazol-5-yl]-3-methyl-pyrrolo[1,2-a]pyrazine-6-carboxamide

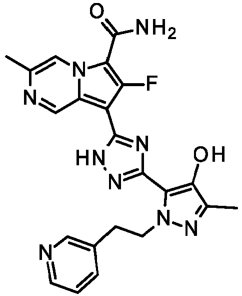

**Example 73** was prepared in analogy to the preparation of **Example 53** by using 2-(3-pyridyl)ethanol instead of 1-bromo-2-phenylethane in step 1 (reaction condition: CMBP, toluene 80 °C instead of K₂CO₃, DMF 80 °C) and using **compound 4-g** instead of **Intermediate A1** in step 6.

**Example 73** (8.91 mg), LCMS (M+H)⁺: 462, ¹H NMR (400 MHz, DMSO-d₆ +D₂O) δ = 9.56 (s, 1H), 9.17 (s, 1H), 8.34 - 8.23 (m, 2H), 7.52 (br d, *J* = 8.4 Hz, 1H), 7.18 (dd, *J* = 4.8, 7.8 Hz, 1H), 4.73 (br t, *J* = 7.2 Hz, 2H), 3.09 (br t, *J* = 7.2 Hz, 2H), 2.46 (s, 3H), 2.13 (s, 3H).

### Example 74

### 1-(2-(4-hydroxy-3-methyl-1-(2-(pyridin-3-yl)ethyl)-1H-pyrazol-5-yl)oxazol-4-yl)-5-methyl-1H-pyrazolo[3,4-c]pyridine-3-carboxamide

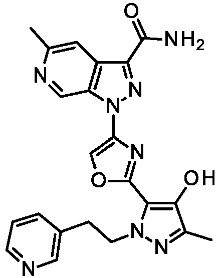

**Example 74** was prepared in analogy to the preparation of **Example 58** by using **compound 74-a** instead of compound **31-h.**

The **compound 74-a** was prepared according to the following scheme:

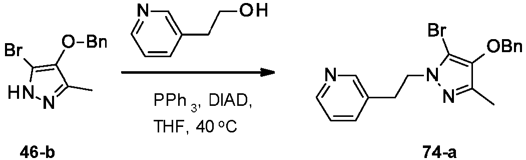

### Step 1. preparation of 3-(2-(4-(benzyloxy)-5-bromo-3-methyl-1H-pyrazol-1-yl)ethyl)pyridine (compound 74-a)

To a stirred mixture of 4-(benzyloxy)-5-bromo-3-methyl-1*H*-pyrazole (**46-b,** 500 mg, 1.90 mmol), 2-(pyridin-3-yl)ethanol (345 mg, 2.80 mmol) and triphenylphosphine (638 mg, 2.40 mmol) in THF (8 mL) was added diisopropyl azodicarboxylate (492 mg, 2.40 mmol) dropwise at 0 °C. The resulting mixture was stirred at 40°C for 4 hrs. The mixture was cooled and concentrated. The residue was purified via silica gel column to give **compound 74-a** (280 mg). LCMS (M+H)⁺: 372.

**Example 74** (15 mg). LCMS (M+H)⁺: 445. ¹H NMR (400 MHz, DMSO-*d*₆) δ = 9.50 (s, 1H), 9.07 (br s, 1H), 8.70 (s, 1H), 8.64 (s, 2H), 8.13 (s, 1H), 8.07 (s, 2H), 7.83 (s, 1H), 7.74 (s, 1H), 4.84 - 4.81 (m, 2H), 3.35 - 3.32 (m, 2H), 2.67 (s, 3H), 2.13 (s, 3H).

### Example 75

### 1-(2-(4-hydroxy-1-(3-methoxyphenethyl)-3-methyl-1H-pyrazol-5-yl)oxazol-4-yl)-5-methyl-1H-pyrazolo[3,4-c]pyridine-3-carboxamide

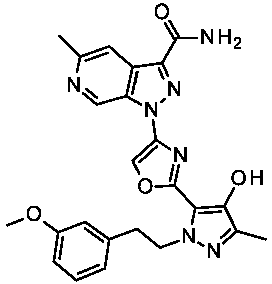

**Example 75** was prepared in analogy to the preparation of **Example 58** by using **compound 75-a** instead of **compound 31-h.**

**Compound 75-a** was prepared in analogy to the preparation of **compound 74-a** by using 2-(3-methoxyphenyl)ethanol instead of 2-(pyridin-3-yl)ethanol.

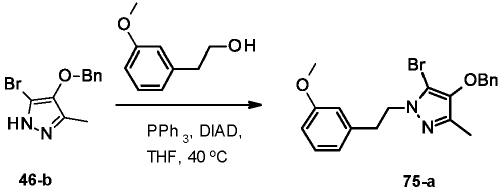

**Example 75** (8.7 mg), LCMS (M+H)⁺: 474. ¹H NMR (400 MHz, DMSO-*d*₆) δ = 9.52 (s, 1H), 8.96 (s, 1H), 8.65 (s, 1H), 8.01 (s, 1H), 7.98 (s, 1H), 7.79 (s, 1H), 7.07 - 7.03 (m, 1H), 6.71 - 6.65 (m, 3H), 4.72 - 4.68 (m, 2H), 3.53 (s, 3H), 3.06 - 3.02 (m, 2H), 2.65 (s, 3H), 2.17 (s, 3H).

### Example 76

### 8-[3-(2-butyl-4-hydroxy-5-methyl-pyrazol-3-yl)-1H-1,2,4-triazol-5-yl]-7-fluoro-3-methyl-pyrrolo[1,2-a]pyrazine-6-carboxamide

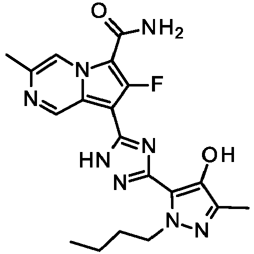

**Example 76** was prepared in analogy to the preparation of **Example 53** by using 1-bromobutane instead of 1-bromo-2-phenylethane in step 1 and using **compound 4-g** instead of **Intermediate A1** in step 6.

**Example 76** (12.3 mg), LCMS (M+H)⁺: 413, ¹H NMR (400 MHz, DMSO-d6) δ = 14.24 - 13.68 (m, 1H), 9.63 (s, 1H), 9.27 - 9.18 (m, 1H), 9.16 - 8.82 (m, 1H), 7.93 - 7.70 (m, 1H), 7.59 - 7.35 (m, 1H), 4.47 (t, *J* = 7.0 Hz, 2H), 2.48 (s, 3H), 2.14 (s, 3H), 1.75 (br t, *J =* 7.4 Hz, 2H), 1.34 - 1.23 (m, 2H), 0.87 (t, *J* = 7.4 Hz, 3H).

### Example 77

### 1-[3-(2-butyl-4-hydroxy-5-methyl-pyrazol-3-yl)-1H-1,2,4-triazol-5-yl]-6-methyl-imidazo[1,5-a]pyrazine-3-carboxamide

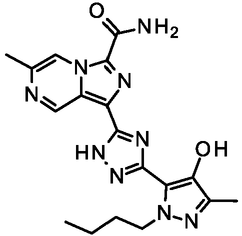

**Example 77** was prepared in analogy to the preparation of **Example 53** by using 1-bromobutane instead of 1-bromo-2-phenylethane in step 1.

**Example 77** (12.1 mg), LCMS (M+H)⁺): 396, ¹H NMR (400 MHz, DMSO-d6) δ = 14.32 - 13.78 (m, 1H), 9.69 (d, *J =* 1.4 Hz, 1H), 9.10 (s, 1H), 9.08 - 8.75 (m, 1H), 8.22 - 7.79 (m, 2H), 4.46 (t, *J =* 6.6 Hz, 2H), 2.49 - 2.48 (m, 3H), 2.15 (s, 3H), 1.75 (br t, *J* = 7.4 Hz, 2H), 1.32 - 1.23 (m, 2H), 0.86 (t, *J =* 7.4 Hz, 3H).

### Example 78

### 1-(2-(4-hydroxy-1-(4-methoxyphenethyl)-3-methyl-1H-pyrazol-5-yl)oxazol-4-yl)-5-methyl-1H-pyrazolo[3,4-c]pyridine-3-carboxamide

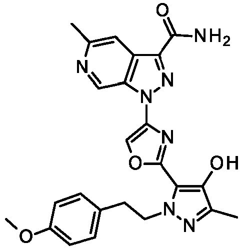

**Example 78** was prepared in analogy to the preparation of **Example 58** by using **compound 78-a** instead of **compound 31-h.**

**Compound 78-a** was prepared in analogy to the preparation of **compound 74-a** by using 2-(4-methoxyphenyl)ethanol instead of 2-(pyridin-3-yl)ethanol.

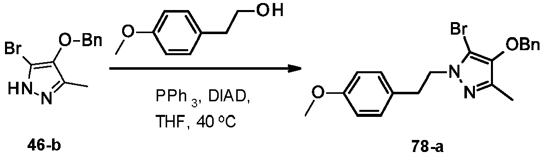

**Example 78** (2.3 mg), LCMS (M+H)⁺: 474. ¹H NMR (400 MHz, DMSO-*d*₆) δ = 9.52 (s, 1H), 8.95 (s, 1H), 8.67 (s, 1H), 8.03 (d, *J =* 5.6 Hz, 1H), 7.99 (s, 1H), 7.80 (s, 1H), 7.06 (d, *J =* 8.4, 2H), 6.70 (d, *J* = 8.4, 2H), 4.67 - 4.63 (m, 2H), 3.65 (s, 3H), 3.02 - 2.98 (m, 2H), 2.66 (s, 3H), 2.17 (s, 3H).

### Example 79

### 7-fluoro-8-[3-(4-hydroxy-5-methyl-2-propyl-pyrazol-3-yl)-1H-1,2,4-triazol-5-yl]-3-methyl-pyrrolo[1,2-a]pyrazine-6-carboxamide

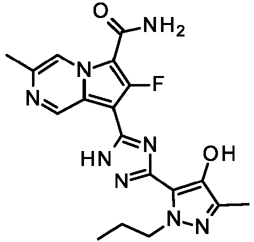

**Example 79** was prepared in analogy to the preparation of **Example 53** by using 1-propanol instead of 1-bromo-2-phenylethane in step 1 (reaction condition: CMBP, toluene 80 °C instead of K₂CO₃, DMF 80 °C) and using **compound 4-g** instead of **Intermediate A1** in step 6.

**Example 79** (18.4 mg), LCMS (M+H)⁺: 399, ¹H NMR (400 MHz, DMSO-d6) δ = 14.43 - 13.53 (m, 1H), 9.70 - 9.58 (m, 1H), 9.23 (s, 1H), 9.14 - 8.81 (m, 1H), 7.87 - 7.74 (m, 1H), 7.52 - 7.39 (m, 1H), 4.43 (br t, *J =* 7.2 Hz, 2H), 2.48 (s, 3H), 2.16 (s, 3H), 1.85 - 1.74 (m, 2H), 0.88 (t, *J* = 7.4 Hz, 3H).

### Example 80

### 1-[3-(4-hydroxy-5-methyl-2-propyl-pyrazol-3-yl)-1H-1,2,4-triazol-5-yl]-6-methyl-imidazo[1,5-a]pyrazine-3-carboxamide

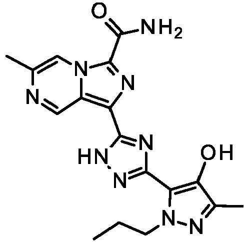

**Example 80** was prepared in analogy to the preparation of **Example 53** by using 1-propanol instead of 1-bromo-2-phenylethane in step 1 (reaction condition: CMBP, toluene 80 °C instead of K₂CO₃, DMF 80 °C).

**Example 80** (4.9 mg) as yellow solid. LCMS (M+H)⁺: 382, ¹H NMR (400 MHz, DMSO-d6+D₂O = 9.67 (s, 1H), 9.08 (s, 1H), 4.45 - 4.32 (m, 2H), 2.47 (br s, 3H), 2.14 (br s, 3H), 1.81 - 1.72 (m, 2H), 0.83 (t, *J =* 7.4 Hz, 3H).

### Example 81

### 1-[3-[4-hydroxy-2-(5-methoxypent-3-ynyl)-5-methyl-pyrazol-3-yl]-1H-1,2,4-triazol-5-yl]-5-methyl-pyrazolo[3,4-c]pyridine-3-carboxamide

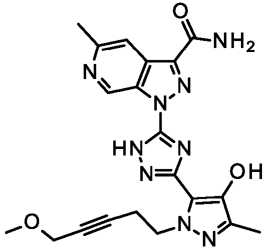

**Example 81** was prepared in analogy to the preparation of **Example 51** by using **compound 81-b** instead of 3-pentyn-1-ol in step 1 and using 2-isopropoxy-4,4,5,5-tetramethyl-1,3,2-dioxaborolane instead of triisopropyl boratein step 2.

The **compound 81-b** was prepared according to the following scheme:

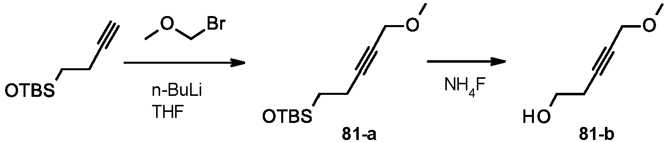

### Step 1: Preparation of tert-butyl-(5-methoxypent-3-ynoxy)-dimethyl-silane (compound 81-a)

To a solution of *tert*-butyl-but-3-ynoxy-dimethyl-silane (5.0 g, 27.1 mmol) in THF (25 mL) was added *n*-BuLi (13.0 mL, 32.6 mmol) drop-wise at -78 °C under N₂ atmosphere. The mixture was stirred at -78 °C for 0.5 h, and then bromomethyl methyl ether (4.41 g, 35.3 mmol) in THF (5 mL) was added at -78°C. The mixture was stirred at -78°C for another 0.5 h. The reaction mixture was poured into water (100 ml) and extracted with ethyl acetate (3 x 250 mL). The combined organic layer was washed with brine and dried over Na₂SO₄. It was then concentrated and purified by column chromatography to give **compound 81-a** (5.6 g). LCMS (M+H)⁺: 229, ¹H NMR (400 MHz, CHLOROFORM-d) δ = 4.08 (d, *J =* 1.8 Hz, 2H), 3.74 (t, *J* = 7.2 Hz, 2H), 3.38 (s, 3H), 2.46 (d, *J =* 1.8 Hz, 2H), 0.91 (s, 9H), 0.08 (s, 6H).

### Step 2: Preparation of 5-methoxypent-3-yn-1-ol (compound 81-b)

To a solution of **compound 81-a** (5.0 g, 21.9 mmol) in methanol (30 mL) was added NH₄F (24.33 g, 656.7 mmol). The mixture was stirred at 60 °C for 1 h. The mixture was filterd and washed with DCM (200 mL x 2). The filtrate was concentrated and purified by column chromatography to give **compound 81-b** (2.4 g). LCMS (M+H)⁺: 115, ¹H NMR (400 MHz, CHLOROFORM-d) δ = 4.11 (d, *J =* 1.8 Hz, 2H), 3.86 - 3.62 (m, 2H), 3.39 (d, *J =* 1.4 Hz, 3H), 2.67 - 2.42 (m, 2H).

**Example 81** (8.6 mg), LCMS (M+H)⁺: 436, ¹H NMR (400 MHz, DMSO-d6) δ = 14.21 (s, 1H), 9.68 (s, 1H), 9.34 - 9.18 (m, 1H), 8.06 (d, *J* = 5.0 Hz, 2H), 7.78 ( d, *J* = 3.2 Hz, 1H), 4.65 ( t, *J* = 6.8 Hz, 2H), 3.96 (s, 2H), 3.10 (s, 3H), 2.76 (t, *J* = 6.8 Hz, 2H), 2.66 (s, 3H), 2.18 (s, 3H).

### Example 82

### 4-(5-(4-hydroxy-3-methyl-1-phenethyl-1H-pyrazol-5-yl)-2H-1,2,4-triazol-3-yl)-1-methyl-1H-pyrazolo[4,3-c]pyridine-6-carboxamide

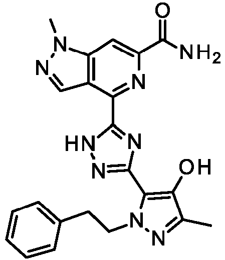

**Example 82** was prepared in analogy to the preparation of **Example 53** by using **Intermediate A2** instead of **Intermediate A1** in step 6.

**Example 82** (8.4 mg), LCMS (M+H)⁺: 444. ¹H NMR (400 MHz, DMSO-*d*₆) δ = 15.33-15.16 (br s, 1H), 8.82 (br s, 1H), 8.79 (s, 2H), 8.46 (s, 1H), 7.88 (s, 1H), 7.26 -7.15 (m, 5H), 4.70 - 4.66 (m, 2H), 4.22 (s, 3H), 3.09 - 3.05 (m, 2H), 2.16 (s, 3H).

### Example 83

### 1-[5-(hydroxymethyl)-2-(4-hydroxy-5-methyl-2-propyl-pyrazol-3-yl)oxazol-4-yl]-5-methyl-pyrazolo[3,4-c]pyridine-3-carboxamide

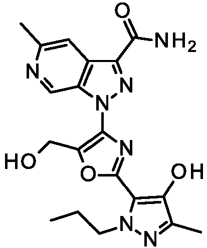

The title compound was prepared according to the following scheme:

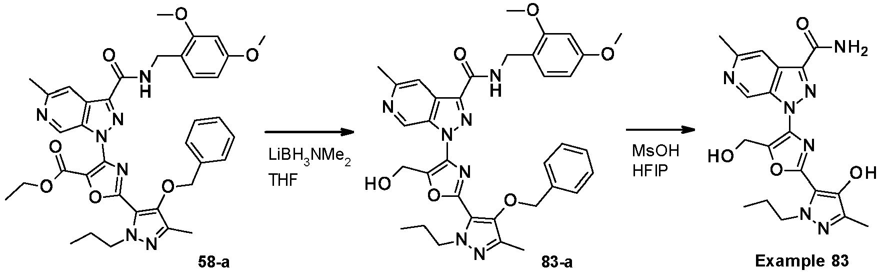

### Step 1: preparation of 1-[2-(4-benzyloxy-5-methyl-2-propyl-pyrazol-3-yl)-5-(hydroxymethyl)oxazol-4-yl]-N-[(2,4-dimethoxyphenyl)methyl]-5-methyl-pyrazolo[3,4-c]pyridine-3-carboxamide (compound 83-a)

To a stirred mixture of ethyl 2-(4-benzyloxy-5-methyl-2-propyl-pyrazol-3-yl)-4-[3-[(2,4-dimethoxyphenyl)methylcarbamoyl]-5-methyl-pyrazolo[3,4-c]pyridin-1-yl]oxazole-5-carboxylate **(compound 58-a,** 200 mg, 0.29 mmol) in THF (15 mL) was added LiBH₃NMe₂ (0.49 mL, 0.49 mmol) (1 M in THF) dropwise at 0 °C. The reaction was stirred at 25 °C for 1 h. The mixture was concentrated and purified via silica gel column chromatography to give **compound 83-a** (100 mg). LCMS (M+H)⁺: 652.

### Step 2: preparation of 1-[5-(hydroxymethyl)-2-(4-hydroxy-5-methyl-2-propyl-pyrazol-3-yl)oxazol-4-yl]-5-methyl-pyrazolo[3,4-c]pyridine-3-carboxamide (Example 83)

A mixture of 1-[2-(4-benzyloxy-5-methyl-2-propyl-pyrazol-3-yl)-5-(hydroxymethyl)oxazol-4-yl]-*N*-[(2,4-dimethoxyphenyl)methyl]-5-methyl-pyrazolo[3,4-c]pyridine-3-carboxamide (**compound 83-a**, 20 mg, 0.03 mmol) and MsOH (23 mg, 0.25 mmol) in HFIP (2 mL) was stirred for 3 hrs at 50 °C under nitrogen atmosphere. The mixture was cooled to room temperature and purified via reversed phase HPLC to give **Example 84** (3.5 mg). LCMS (M+H)⁺: 412, ¹H NMR (400 MHz, DMSO-*d*₆) δ = 9.52 (s, 1H), 8.90 (s, 1H), 8.12 (s, 1H), 8.03 (s, 1H), 7.78 (s, 1H), 5.51 (br s, 1H), 4.92 (s, 2H), 4.43 - 4.40 (m, 2H), 2.65 (s, 3H), 2.16 (s, 3H), 1.83 - 1.78 (m, 2H), 0.90 - 0.86 (m, 3H).

### Example 84

### 1-[5-(4-hydroxy-5-methyl-2-propyl-pyrazol-3-yl)-2-methyl-1,2,4-triazol-3-yl]-5-methyl-pyrazolo[3,4-c]pyridine-3-carboxamide

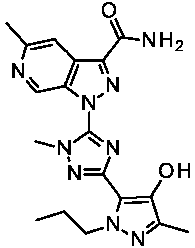

**Example 84** was prepared in analogy to the preparation of **Example 48** by using **compound 31-h** instead of **compound 40-b** and using 2-isopropoxy-4,4,5,5-tetramethyl-1,3,2-dioxaborolane instead of triisopropyl borate in step 1.

**Example 84** (58 mg), LCMS (M+H)⁺: 396, ¹H NMR (400 MHz, DMSO-d6) δ = 9.59 (d, *J* = 1.0 Hz, 1H), 8.30 (s, 1H), 8.09 (s, 2H), 7.92 (s, 1H), 4.33 (t, *J =* 7.2 Hz, 2H), 4.28 (s, 3H), 2.68 (s, 3H), 2.12 (s, 3H), 1.84 - 1.71 (m, 2H), 0.85 (t, *J =* 7.4 Hz, 3H)

### Example 85

### 1-[2-(4-hydroxy-5-methyl-2-propyl-pyrazol-3-yl)oxazol-4-yl]-6-methyl-imidazo[1,5-a]pyrazine-3-carboxamide

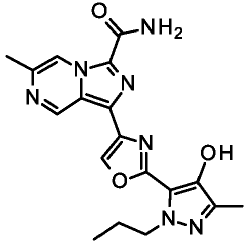

The title compound was prepared according to the following scheme:

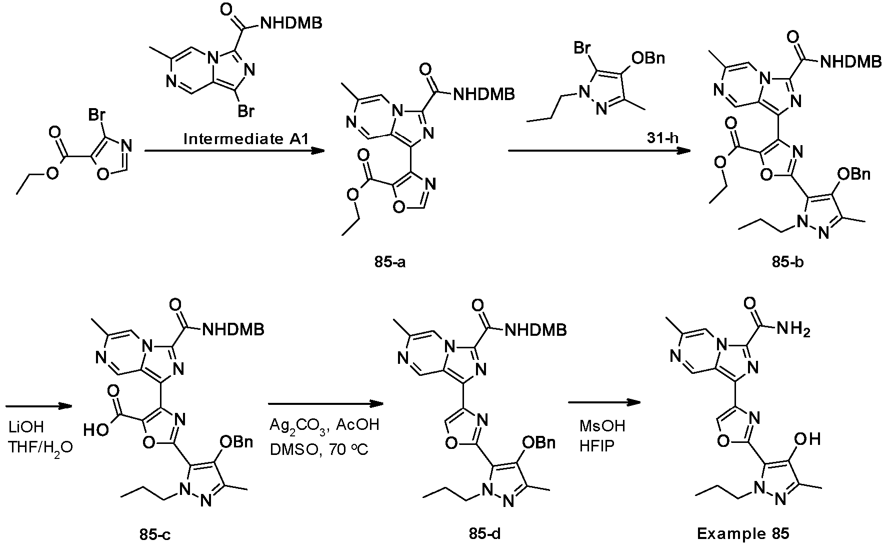

### Step 1: Preparation of ethyl 4-[3-[(2,4-dimethoxyphenyl)methylcarbamoyl]-6-methyl-imidazo[1,5-a]pyrazin-1-yl]oxazole-5-carboxylate (compound 85-a)

To a solution of ethyl 4-bromooxazole-5-carboxylate (896 mg, 4.07 mmol), 1-bromo-N-[(3,4-dimethylphenyl)methyl]-6-methyl-imidazo[1,5-a]pyrazine-3-carboxamide (**Intermediate A1,** 1500 mg, 3.7 mmol) in toluene (1 mL) and water (0.1 mL) was added K₃PO₄ (2357 mg, 11.1 mmol), B₂Pin₂ (1880 mg, 7.4 mmol), Pd(dppf)Cl₂ (541 mg, 0.74 mmol) at 25 °C. The mixture was degassed and purged with N₂ three times. The mixture was heated to 80 °C and stirred for 18 hrs. The mixture was concentrated and purified via flash chromatography on silica gel to afford **compound 85-a** (500 mg). LCMS (M+H)⁺: 466.

### Step 2: Preparation of ethyl 2-(4-benzyloxy-5-methyl-2-propyl-pyrazol-3-yl)-4-[3-[(2,4-dimethoxyphenyl)methylcarbamoyl]-6-methyl-imidazo[1,5-a]pyrazin-1-yl]oxazole-5-carboxylate (compound 85-b)

A mixture of ethyl 4-[3-[(2,4-dimethoxyphenyl)methylcarbamoyl]-6-methyl-imidazo[1,5-a]pyrazin-1-yl]oxazole-5-carboxylate (**85-a**, 200 mg, 0.43 mmol), 4-benzyloxy-5-bromo-3-methyl-1-propyl-pyrazole (**31-h**, 132 mg, 0.43 mmol), Pd(OAc)₂ (29 mg, 0.13 mmol), PivOH (26 mg, 0.26 mmol), catacxiuma (46 mg, 0.13 mmol), K₂CO₃ (178 mg, 1.29 mmol) in toluene (1.5 mL) was stirred at 120 °C for 12 hrs under N₂ atmosphere. The mixture was diluted with DCM (30 mL), filtered and washed with DCM/MeOH=10/1(20 mL). The filtrate was concentrated and purified by prep-TLC to give **compound 85-b** (63 mg). LCMS (M+H)⁺: 694.

### Step 3: Preparation of 2-(4-benzyloxy-5-methyl-2-propyl-pyrazol-3-yl)-4-[3-[(2,4-dimethoxyphenyl)methylcarbamoyl]-6-methyl-imidazo[1,5-a]pyrazin-1-yl]oxazole-5-carboxylic acid (compound 85-c)

To a solution of ethyl 2-(4-benzyloxy-5-methyl-2-propyl-pyrazol-3-yl)-4-[3-[(2,4-dimethoxyphenyl)methylcarbamoyl]-6-methyl-imidazo[1,5-a]pyrazin-1-yl]oxazole-5-carboxylate (**85-b**, 63 mg, 0.09 mmol) in methanol (1 mL) was added 1 N LiOH aq. (1.0 mL). The mixture was stirred at 20 °C for 12 hrs and diluted with water (20 mL). After pH was adjusted to 5 with FA, and the mixture was extracted with DCM (20 mL x 2). The combined organic phase was dried with Na₂SO₄, filtered and concentrated to give **compound 85-c** (60 mg) as crude solid, which was used directly in the next step. LCMS (M+H)⁺: 666.

### Step 4: Preparation of 1-[2-(4-benzyloxy-5-methyl-2-propyl-pyrazol-3-yl)oxazol-4-yl]-N-[(2,4-dimethoxyphenyl)methyl]-6-methyl-imidazo[1,5-a]pyrazine-3-carboxamide (compound 88-d)

To a solution of 2-(4-benzyloxy-5-methyl-2-propyl-pyrazol-3-yl)-4-[3-[(2,4-dimethoxyphenyl)methylcarbamoyl]-6-methyl-imidazo[1,5-a]pyrazin-1-yl]oxazole-5-carboxylic acid (**85-c,** 60 mg, 0.09 mmol) in DMSO (1 mL) was added AcOH (2 mg, 0.04 mmol) and Ag₂CO₃ (10 mg, 0.04 mmol) at 25 °C. The reaction was stirred at 80 °C for 2 hrs. The mixture was cooled and poured into water (30 mL), the aqueous phase was extracted with EtOAc (30 mL x 2). The combined organic phase was washed with brine (20 mL x 3), dried over Na₂SO₄ and concentrated to give **compound 85-d** (50 mg, crude). LCMS (M+H)⁺: 622.

### Step 5: Preparation of 1-[2-(4-hydroxy-5-methyl-2-propyl-pyrazol-3-yl)oxazol-4-yl]-6-methyl-imidazo[1,5-a]pyrazine-3-carboxamide (Example 85)

To a solution of 1-[2-(4-benzyloxy-5-methyl-2-propyl-pyrazol-3-yl)oxazol-4-yl]-N-[(2,4-dimethoxyphenyl)methyl]-6-methyl-imidazo[1,5-a]pyrazine-3-carboxamide (**85-d,** 50 mg, 0.08 mmol) in HFIP (1.0 mL) was added MsOH (0.2 mL). The mixture was stirred at 50 °C for 0.5 h to give a red solution. The mixture was purified directly via prep-HPLC to give **Example 85** (11.8 mg). LCMS (M+H)⁺: 382, ¹H NMR (DMSO-d6, 400 MHz) δ = 9.65 (d, *J* = 1.6 Hz, 1H), 9.05 (s, 1H), 8.81 (br s, 1H), 8.63 (s, 1H), 8.07 (s, 1H), 7.87 (s, 1H), 4.46 (t, *J* = 7.2 Hz, 2H), 2.47 (s, 3H), 2.16 (s, 3H), 1.89 - 1.76 (m, 2H), 0.91 (t, *J =* 7.2 Hz, 3H).

### Example 86

### 1-[2-(2-butyl-4-hydroxy-5-methyl-pyrazol-3-yl)oxazol-4-yl]-6-methyl-imidazo[1,5-a]pyrazine-3-carboxamide

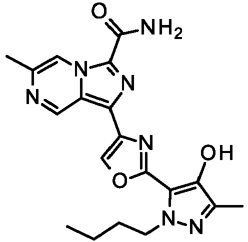

**Example 86** was prepared in analogy to the preparation of **Example 85** by using **compound 49-b** instead of **compound 31-h** in step 2.

**Example 86** (1.1 mg), LCMS (M+H)⁺: 396, ¹H NMR (400 MHz, DMSO-d₆ +D₂O) δ = 9.60 (s, 1H), 8.99 (s, 1H), 8.64 (s, 1H), 4.51 - 4.43 (m, 2H), 2.43 (s, 3H), 2.13 (s, 3H), 1.80 - 1.71 (m, 2H), 1.33 - 1.24 (m, 2H), 0.86 (t, *J =* 7.2 Hz, 3H).

### Example 87

### 1-[3-[4-hydroxy-5-methyl-2-[2-[4-(morpholinomethyl)phenyl]ethyl]pyrazol-3-yl]-1H-1,2,4-triazol-5-yl]-6-methyl-imidazo[1,5-a]pyrazine-3-carboxamide

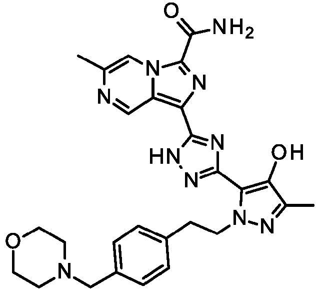

**Example 87** was prepared in analogy to the preparation of **Example 53** by using **compound 87-b** instead of 1-bromo-2-phenylethane in step 1 (reaction condition: CMBP, toluene 80 °C instead of K₂CO₃, DMF 80 °C).

The **compound 87-b** was prepared according to the following scheme:

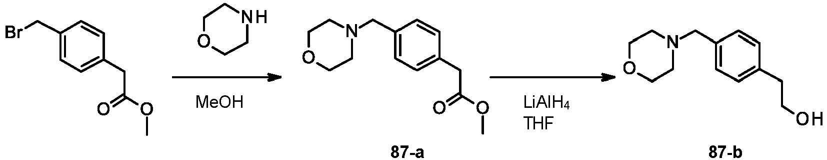

### Step 1: Preparation of methyl 2-(4-(morpholinomethyl)phenyl)acetate (compound 87-a)

To a solution of methyl 2-[4-(bromomethyl)phenyl]acetate (15.0 g, 62 mmol) in methanol (100 mL) was added morpholine (16.0 g, 185 mmol). The mixture was stirred at 25 °C for 3 hrs and concentrated. The residue was treated with water (30 mL) and extracted with ethyl acetate (3 x 25 mL). The combined organic layer was washed with sat. NaCl aq. (50 mL), dried over sodium sulfate, filtered and concentrated to afford **compound 87-a** (16.0 g). LCMS (M+H)⁺: 250.

### Step 2: Preparation of 2-(4-(morpholinomethyl)phenyl)ethan-1-ol (compound 87-b)

To a solution of methyl 2-[4-(morpholinomethyl)phenyl]acetate (**87-a,** 16.0 g, 64 mmol) in THF (200 mL) was added LiAlH₄ (30.8 mL, 77 mmol) at 0 °C. The reaction was stirred at 0 °C for 2 hrs and then quenched via addition of water (150 mL) and 3 N NaOH aq. (120 mL). The aluminium salts were filtered through celite and the filtrate was concentrated to afford **compound 87-b** (14.0 g, crude). LCMS (M+H)⁺: 222.

**Example 87** (23.7 mg), LCMS (M+H)⁺: 543, ¹H NMR (400 MHz, DMSO + D₂O) δ = 9.52 (s, 1H), 8.98 (s, 1H), 7.08 - 6.93 (m, 4H), 4.64 (br t, *J* = 6.8 Hz, 2H), 3.45 (br s, 4H), 3.27 (s, 2H), 2.95 (br t, *J =* 7.1 Hz, 2H), 2.43 (s, 3H), 2.19 (br s, 4H), 2.11 (s, 3H).

### Example 88

### 1-[2-[4-hydroxy-5-methyl-2-[2-[4-(morpholinomethyl)phenyl]ethyl]pyrazol-3-yl]oxazol-4-yl]-5-methyl-pyrazolo[3,4-c]pyridine-3-carboxamide

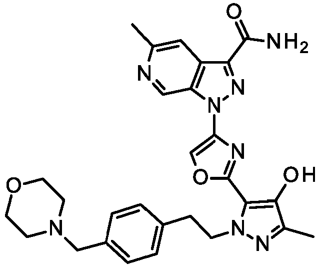

**Example 88** was prepared in analogy to the preparation of **Example 58** by using **compound 88-a** instead of **compound 31-h.**

The **compound 88-a** was prepared in analogy to the preparation of **compound 46-c** by using **compound 87-b** instead of 4-pyridineethanol in step 3.

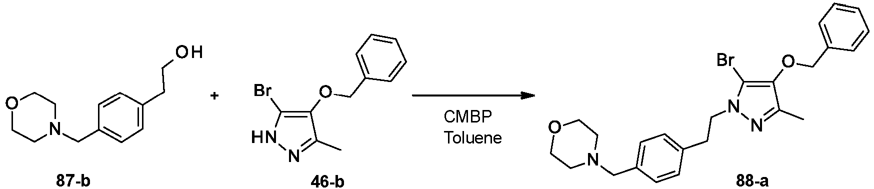

**Example 88** (8.5 mg), LCMS (M+H)⁺: 543, ¹H NMR (400 MHz, DMSO-*d*₆) δ = 9.53 (d, *J* = 1.0 Hz, 1H), 9.16 - 8.84 (m, 1H), 8.66 (s, 1H), 8.05 (s, 1H), 8.00 (s, 1H), 7.81 (s, 1H), 7.32 - 7.21 (m, 4H), 4.73 (br t, *J =* 7.4 Hz, 2H), 4.25 (s, 2H), 4.00 - 3.86 (m, 4H), 3.23 - 3.12 (m, 4H), 3.10 - 2.98 (m, 2H), 2.67 (s, 3H), 2.16 (s, 3H).

### Example 89

### 1-[2-[4-hydroxy-2-[2-[4-(methoxymethyl)phenyl]ethyl]-5-methyl-pyrazol-3-yl]oxazol-4-yl]-5-methyl-pyrazolo [3,4-c]pyridine-3-carboxamide

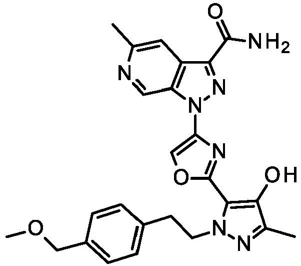

**Example 89** was prepared in analogy to the preparation of **Example 58** by using **compound 89-a** instead of compound **31-h.**

The **compound 89-a** was prepared in analogy to the preparation of **compound 46-c** by using **compound 64-a** instead of 4-pyridineethanol in step 3.

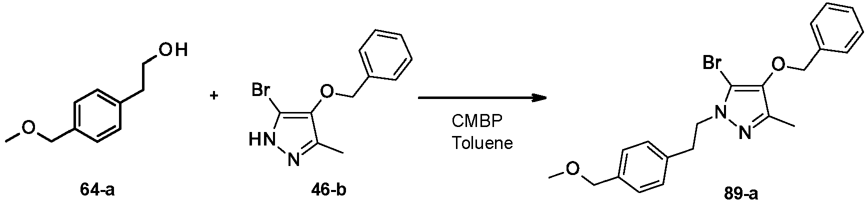

**Example 89** (4.2 mg), LCMS (M+H)⁺: 488, ¹H NMR (400 MHz, DMSO-*d*₆) δ = 9.53 (d, *J* = 1.0 Hz, 1H), 9.04 - 8.87 (m, 1H), 8.66 (s, 1H), 8.04 (s, 1H), 8.00 (s, 1H), 7.80 (s, 1H), 7.14 - 7.04 (m, 4H), 4.69 (t, *J* = 7.4 Hz, 2H), 4.28 (s, 2H), 3.20 (s, 3H), 3.06 (t, *J* = 7.4 Hz, 2H), 2.67 (s, 3H), 2.17 (s, 3H).

### Example 90

### 1-[3-[4-hydroxy-5-methyl-2-(2-thiazol-2-ylethyl)pyrazol-3-yl]-1H-1,2,4-triazol-5-yl]-6-methyl-imidazo[1,5-a]pyrazine-3-carboxamide

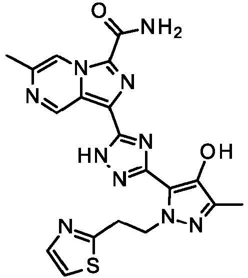

**Example 90** was prepared in analogy to the preparation of **Example 53** by using **compound 90-a** instead of 1-bromo-2-phenylethane in step 1 (reaction condition: CMBP, toluene 80 °C instead of K₂CO₃, DMF 80 °C).

The compound 90-a was prepared according to the following scheme:

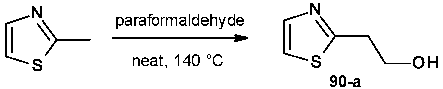

### Step 1: Preparation of 2-thiazol-2-ylethanol (compound 90-a)

A solution of 2-methylthiazole (5 g, 50.43 mmol) in paraformaldehyde (5 g, 50.43 mmol) was slowly heated to 140 °C and stirred for 3 hrs. The reaction mixture was concentrated and purified via column chromatography to affored **compound 90-a** (3 g) as colorless oil. ¹H NMR (400 MHz, CHLOROFORM-d) δ = 7.71 (d, *J* = 3.4 Hz, 1H), 7.24 (d, *J* = 3.4 Hz, 1H), 4.05 (t, *J* = 5.6 Hz, 2H), 3.25 (t, *J =* 5.6 Hz, 2H).

**Example 90** (5.6 mg), LCMS (M+H)⁺: 451, ¹H NMR (400 MHz, DMSO-d6 + D₂O) δ = 9.66 (s, 1H), 9.03 (s, 1H), 7.60 (d, *J* = 3.4 Hz, 1H), 7.44 (d, *J* = 3.4 Hz, 1H), 4.83 (br t, *J =* 6.8 Hz, 2H), 3.49 (br t, *J* = 7.0 Hz, 2H), 2.45 (s, 3H), 2.12 (s, 3H).

### Example 91

### 8-[3-(4-hydroxy-5-methyl-2-propyl-pyrazol-3-yl)-1H-1,2,4-triazol-5-yl]-3-methyl-pyrrolo[1,2-a]pyrazine-6-carboxamide

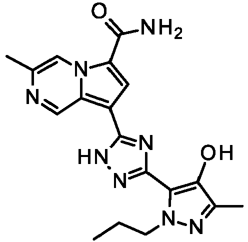

The title compound was prepared according to the following scheme:

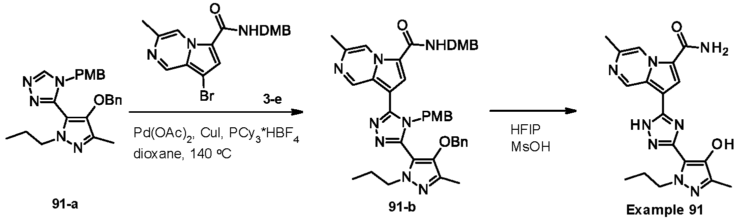

### Step 1: Preparation of 3-(4-benzyloxy-5-methyl-2-propyl-pyrazol-3-yl)-4-[(4-methoxyphenyl)methyl]-1,2,4-triazole (compound 91-a)

The **compound 91-a** was prepared in analogy to the preparation of **compound 53-e** by using 1-iodopropane instead of 1-bromo-2-phenylethane in step 1.

### Step 2: Preparation of 8-[5-(4-benzyloxy-5-methyl-2-propyl-pyrazol-3-yl)-4-[(4-methoxyphenyl)methyl]-1,2,4-triazol-3-yl]-N-[(2,4-dimethoxyphenyl)methyl]-3-methyl-pyrrolo[1,2-a]pyrazine-6-carboxamide (compound 91-b)

A mixture of 3-(4-benzyloxy-5-methyl-2-propyl-pyrazol-3-yl)-4-[(4-methoxyphenyl)methyl]-1,2,4-triazole (**91-a,** 155 mg, 0.37 mmol), 8-bromo-N-[(2,4-dimethoxyphenyl)methyl]-3-methyl-pyrrolo[1,2-a]pyrazine-6-carboxamide (**3-e,** 150 mg, 0.37 mmol), Pd(OAc)₂ (25 mg, 0.11 mmol), CuI (141 mg, 0.74 mmol), PCy₃HBF₄ (54 mg, 0.15 mmol), Cs₂CO₃ (120 mg, 0.37 mmol) in 1,4-dioxane (3 mL) was stirred at 140 °C for 18 hrs under N₂ atmosphere. The mixture was cooled, filtered and washed with DCM/MeOH (20 mL, v/v = 10/1). The filtrate was concentrated and purified via prep-TLC to give **compound 91-b** (100 mg). LCMS (M+H)⁺: 741.

### Step 3: Preparation of 8-[3-(4-hydroxy-5-methyl-2-propyl-pyrazol-3-yl)-1H- 1,2,4-triazol-5-yl]-3-methyl-pyrrolo[1,2-a]pyrazine-6-carboxamide (Example 91)

To a solution of 8-[5-(4-benzyloxy-5-methyl-2-propyl-pyrazol-3-yl)-4-[(4-methoxyphenyl)methyl]-1,2,4-triazol-3-yl]-N-[(2,4-dimethoxyphenyl)methyl]-3-methylpyrrolo[1,2-a]pyrazine-6-carboxamide (**91-a**, 100 mg, 0.13 mmol) in HFIP (2 mL) was added MsOH (259 mg, 2.7 mmol). The mixture was stirred at 50 °C for 0.5 h to give a red solution. The mixture was purified via prep-HPLC to give **Example 91** (46.8 mg). LCMS (M+H)⁺: 381, ¹H NMR (DMSO-d6, 400 MHz) δ = 13.86 (s, 1H), 9.65 (s, 1H), 9.36 (s, 1H), 9.22 - 8.77 (m, 1H), 8.16 (s, 2H), 7.55 (s, 1H), 4.43 (br t, *J* = 6.8 Hz, 2H), 2.47 (s, 3H), 2.15 (s, 3H), 1.84 - 1.73 (m, 2H), 0.86 (t, *J =* 7.4 Hz, 3H).

### Example 92

### 1-[5-(4-hydroxy-5-methyl-2-propyl-pyrazol-3-yl)-2-methyl-1,2,4-triazol-3-yl]-6-methyl-imidazo[1,5-a]pyrazine-3-carboxamide

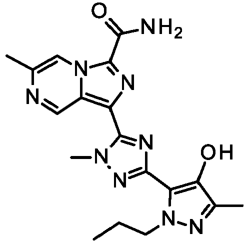

The title compound was prepared according to the following scheme:

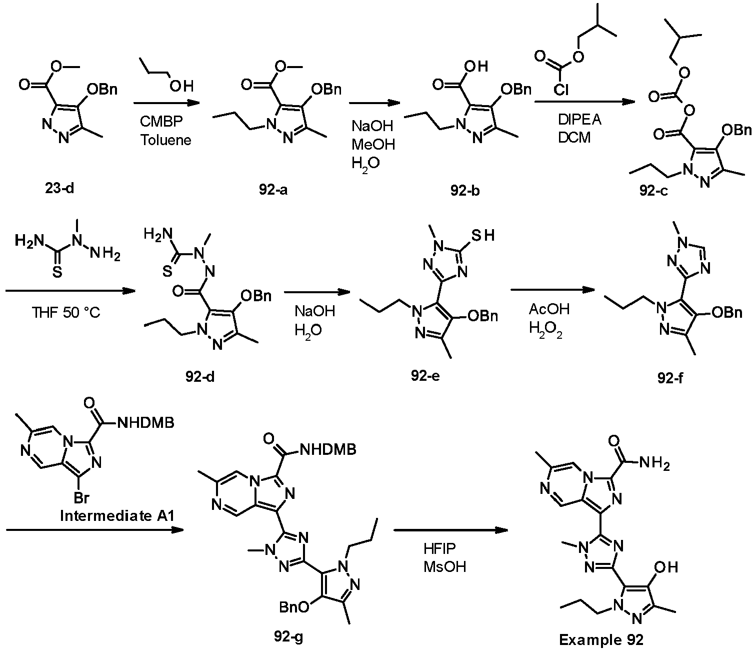

### Step 1: Preparation of methyl 4-benzyloxy-5-methyl-2-propyl-pyrazole-3-carboxylate (compound 92-a)

To a solution of methyl 4-benzyloxy-3-methyl-1*H*-pyrazole-5-carboxylate (**23-d,** 2.00 g, 8.1 mmol) in toluene (10 mL) was added 1-propanol (585 mg, 9.7 mmol) and cyanomethylenetributylphosphorane (2550 mg, 10.5 mmol). The reaction was stirred at 80 °C for 12 hrs. The mixture was purified via column chromatography to afford **compound 92-a** (2.0 g). LCMS (M+H)⁺: 289.

### Step 2: Preparation of 4-benzyloxy-5-methyl-2-propyl-pyrazole-3-carboxylic acid (compound 92-b)

To a solution of methyl 4-benzyloxy-5-methyl-2-propyl-pyrazole-3-carboxylate (**92-a,** 870 mg, 3.0 mmol) in methanol (10 mL) was added LiOH.H₂O (9.0 mg, 9.0 mmol) in H₂O (5 mL). The reaction was stirred at 25 °C for 2 hrs. The mixture was cooled to 0 °C and adjusted pH = 3 with 1 N HCl aq, then EtOAc (100 mL) and water 100 mL) were added. After separation, the aqueous phase was extracted with EtOAc (100 mL x 3). The combined organic layer was washed with brine (400 mL), dried over Na₂SO₄, filtered, and concentrated to give **compound 92-b** (1.6 g, crude). LCMS (M+H)⁺: 275.

### Step 3: Preparation of isobutoxycarbonyl 4-benzyloxy-5-methyl-2-propyl-pyrazole-3-carboxylate (compound 92-c)

To a solution of 4-benzyloxy-5-methyl-2-propyl-pyrazole-3-carboxylic acid (**92-b,** 1.5 g, 5.47 mmol) in DCM (30 mL) was added DIPEA (2.1 g, 16.4 mmol) and isobutyl chloroformate (1.5 g, 10.9 mmol). The reaction was stirred at 20 °C for 2 hrs and concentrated to give **compound 92-c** (2.0 g). LCMS (M+H)⁺: 375.

### Step 4: Preparation of 1-[(4-benzyloxy-5-methyl-2-propyl-pyrazole-3-carbonyl)amino]-1-methyl-thiourea (compound 92-d)

To a solution of 1-amino-1-methyl-thiourea (860 mg, 8.15 mmol) in THF (40 mL), was added isobutoxycarbonyl 4-benzyloxy-5-methyl-2-propyl-pyrazole-3-carboxylate (**92-c,** 1.5 g, 4.07 mmol), then the reaction was stirred at 50 °C for 12 hrs. The mixture was poured into water (200 mL) and extracted with EtOAc (3 x 200 mL). The combined organic layers was washed with sat. NaHCO₃ aq. (300 mL) and brine (300 mL), then dried over anhydrous Na₂SO₄. The organic layer was concentrated to afford crude **compound 92-d** (2.0 g). LCMS (M+H)⁺: 362.

### Step 5: Preparation of 5-(4-benzyloxy-5-methyl-2-propyl-pyrazol-3-yl)-2-methyl-1,2,4-triazole-3-thiol (compound 92-e)

To a solution of 1-[(4-benzyloxy-5-methyl-2-propyl-pyrazole-3-carbonyl)amino]-1-methyl-thiourea (**92-d,** 2.00 g, 5.5 mmol) in water (12 mL) was added NaOH (720 mg, 18.0 mmol). The reaction was heated to 100 °C and stirred at for 6 hrs. The mixture was poured into water (300 ml) and extracted with EtOAc (3 x 300 mL). The combined organic layer was washed with sat. NaHCO₃ aq. (300 mL) and brine (200 mL), then dried over anhydrous Na₂SO₄. The filtered organic layer was concentrated to afford **compound 92-e** (807 mg). LCMS (M+H)⁺: 344.

### Step 6: Preparation of 3-(4-benzyloxy-5-methyl-2-propyl-pyrazol-3-yl)-1-methyl-1,2,4-triazole (compound 92-f)

To a solution of 5-(4-benzyloxy-5-methyl-2-propyl-pyrazol-3-yl)-2-methyl-1,2,4-triazole-3-thiol (**92-e,** 800 mg, 2.3 mmol) in acetic acid (6 mL) was added H₂O₂ (132 mg, 11.6 mmol) slowly at 0 °C. The reaction was warmed to 20 °C and stirred for 1 h. The mixture was poured into sat. Na₂SO₃ aq. (80 mL) and stirred at 0 °C for 30 mins. Then it was adjusted pH to 7-8 with NaHCO₃. The mixture was extracted with ethyl acetate (3 x 50 mL). The combined organic layer was washed with brine (100 mL), dried over Na₂SO₄, filtered and concentrated. The residue was purified via column chromatography to afford **compound 92-f** (480 mg). LCMS (M+H)⁺: 312.

### Step 7: Preparation of 1-[5-(4-benzyloxy-5-methyl-2-propyl-pyrazol-3-yl)-2-methyl-1,2,4-triazol-3-yl]-N-[(2,4-dimethoxyphenyl)methyl]-6-methyl-imidazo[1,5-a]pyrazine-3-carboxamide (compound 92-g)

To a solution of 3-(4-benzyloxy-5-methyl-2-propyl-pyrazol-3-yl)-1-methyl-1,2,4-triazole (**92-f,** 100 mg, 0.32 mmol) in Toluene (0.5 mL) was added 1-bromo-N-[(2,4-dimethoxyphenyl)methyl]-6-methyl-imidazo[1,5-a]pyrazine-3-carboxamide (**Intermediate A1,** 130 mg, 0.32 mmol), catacxiuma (34 mg, 0.1 mmol), Pd(OAc)₂ (22 mg, 0.1 mmol), PivOH (20 mg, 0.19 mmol) and K₂CO₃ (133 mg, 0.96 mmol). The mixture was stirred at 120 °C for 12 hrs and then it was filtered and washed with DCM/MeOH (20/1, 100ml). The filtrate was concentrated and purified via Pre-TLC to afford **compound 92-g** (80 mg). LCMS (M+H)⁺: 636.

### Step 8: Preparation of 1-[5-(4-hydroxy-5-methyl-2-propyl-pyrazol-3-yl)-2-methyl-1,2,4-triazol-3-yl]-6-methyl-imidazo[1,5-a]pyrazine-3-carboxamide (Example 92)

To a solution of 1-[5-(4-benzyloxy-5-methyl-2-propyl-pyrazol-3-yl)-2-methyl-1,2,4-triazol-3-yl]-N-[(2,4-dimethoxyphenyl)methyl]-6-methyl-imidazo[1,5-a]pyrazine-3-carboxamide (**92-g,** 75 mg, 0.12 mmol) in HFIP (1 mL) was added MsOH (226 mg, 2.36 mmol). The mixture was stirred at 50 °C for 1 h. The reaction mixture was filtered and the filtrate was purified via Prep-HPLC to afford **Example 92** (22.7 mg) as a yellow solid. LCMS (M+H)⁺: 396, ¹H NMR (400 MHz, DMSO-d6) δ = 9.71 (s, 1H), 9.15 (s, 1H), 8.37 (s, 1H), 7.97 (s, 1H), 7.91 (d, *J* = 1.4 Hz, 1H), 4.45 (s, 3H), 4.36 (t, *J =* 7.2 Hz, 2H), 2.51 - 2.50 (m, 3H), 2.12 (s, 3H), 1.91 - 1.70 (m, 2H), 0.85 (t, *J* = 7.4 Hz, 3H).

### Example 93

### 7-fluoro-8-[2-(4-hydroxy-5-methyl-2-propyl-pyrazol-3-yl)oxazol-4-yl]-3-methyl-pyrrolo[1,2-a]pyrazine-6-carboxamide

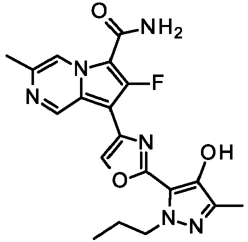

The title compound was prepared according to the following scheme:

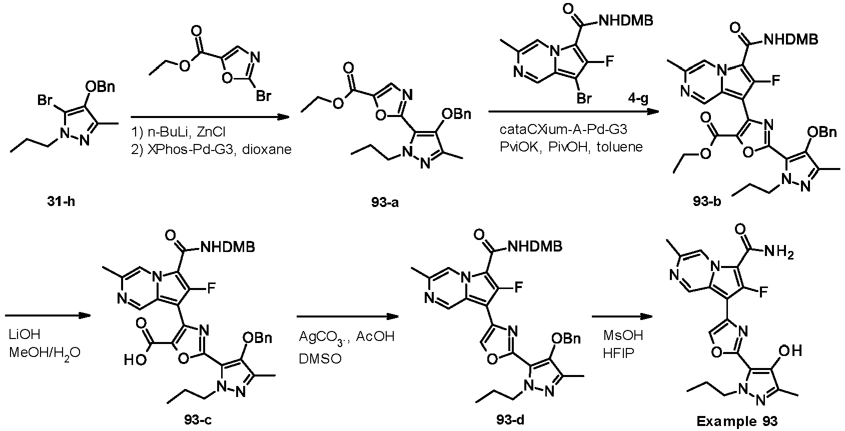

### Step 1: Preparation of ethyl 2-(4-benzyloxy-5-methyl-2-propyl-pyrazol-3-yl)oxazole-5-carboxylate (compound 93-a)

To a solution of 4-benzyloxy-5-bromo-3-methyl-1-propyl-pyrazole (**31-h,** 500 mg, 1.6 mmol) in THF (3 mL) was added n-BuLi (650 µL, 1.6 mmol) dropwise at -63 °C. The reaction was stirred at the temperature for 10 mins and ZnCl₂ (3.2 mL, 1.6 mmol) was added. Keep stirring at the temperature for 30 min, the reaction was warmed to r.t. and stirred for 1 h. The zincate solution (c=0.23 M) was used in the next step. A mixture of ethyl 2-bromooxazole-5-carboxylate (320 mg, 1.5 mmol) and XPhos-Pd-G₃ (137 mg, 0.16 mmol) in 1,4-dioxane (6 mL) was degassed for 5 mins. The above zincate solution was added at 20 °C and the reaction was heated to 80 °C for 90 mins. The reaction was quenched by poured into ice water containing 2.5 mL 1 N HCl. The mixture was extracted with EtOAc (20 mL x 3). The combined organic phase was concentrated and purified via flash silica gel chromatography to afford **compound 93-a** (230 mg). LCMS (M+H)⁺: 370.

### Step 2: Preparation of ethyl 2-(4-benzyloxy-5-methyl-2-propyl-pyrazol-3-yl)-4-[6-[(2,4-dimethoxyphenyl)methylcarbamoyl]-7-fluoro-3-methyl-pyrrolo[1,2-a]pyrazin-8-yl]oxazole-5-carboxylate (compound 93-b)

To a solution of ethyl 2-(4-benzyloxy-5-methyl-2-propyl-pyrazol-3-yl)oxazole-5-carboxylate (**93-a,** 230 mg, 0.62 mmol) in Toluene (3 mL) was added 8-bromo-*N*-[(2,4-dimethoxyphenyl)methyl]-7-fluoro-3-methyl-pyrrolo[1,2-a]pyrazine-6-carboxamide (**4-g,** 263 mg, 0.62 mmol), potassium pivalate (131 mg, 0.93 mmol), PivOH (31 mg, 0.31 mmol) and cataCXium-A-Pd-G₃ (181 mg, 0.25 mmol). The reaction was stirred at 120 °C for 18 hrs under N₂ atmosphere. The mixture was filtered, the wet-cake was washed by DCM/MeOH (V/V = 20:1, 30mL). The filtrate was concentrated and purified via Prep-TLC to give a crude product. Then it was purified again via reversed-phase to afford **compound 93-b** (40 mg). LCMS (M+H)⁺: 711.

### Step 3: Preparation of 2-(4-benzyloxy-5-methyl-2-propyl-pyrazol-3-yl)-4-[6-[(2,4-dimethoxyphenyl)methylcarbamoyl]-7-fluoro-3-methyl-pyrrolo[1,2-a]pyrazin-8-yl]oxazole-5-carboxylic acid (compound 93-c)

To a solution of ethyl 2-(4-benzyloxy-5-methyl-2-propyl-pyrazol-3-yl)-4-[6-[(2,4-dimethoxyphenyl)methylcarbamoyl]-7-fluoro-3-methyl-pyrrolo[1,2-a]pyrazin-8-yl]oxazole-5-carboxylate (**93-b,** 40 mg, 0.06 mmol) in Methanol (4 mL) and water (2 mL) was added LiOH.H₂O (12 mg, 0.28 mmol). The reaction was stirred at 20 °C for 12 hrs. The mixture was acidified with HCl (6 N) to pH = 3 ~ 4 and extracted with DCM (10 mL x 3). The organic layer was washed with brine, dried over Na₂SO₄ and concentrated to afford crude **compound 93-c** (33 mg). LCMS (M+H)⁺: 683.

### Step 4: Preparation of 8-[2-(4-benzyloxy-5-methyl-2-propyl-pyrazol-3-yl)oxazol-4-yl]-N-[(2,4-dimethoxyphenyl)methyl]-7-fluoro-3-methyl-pyrrolo[1,2-a]pyrazine-6-carboxamide (compound 93-d)

To a solution of 2-(4-benzyloxy-5-methyl-2-propyl-pyrazol-3-yl)-4-[6-[(2,4-dimethoxyphenyl)methylcarbamoyl]-7-fluoro-3-methyl-pyrrolo[1,2-a]pyrazin-8-yl]oxazole-5-carboxylic acid (**93-c,** 33 mg, 0.05 mmol) in DMSO (1 mL) was added AcOH (1.2 mg, 0.02 mmol) and Ag₂CO₃ (5.3 mg, 0.02 mmol) at 20 °C. The reaction was stirred at 80 °C for 2 hrs. The mixture was poured into water (10 mL), and extracted with EtOAc (10 mL x 2). The combined organic phase was washed with brine (20 mL x 3), dried over Na₂SO₄, filtered and concentrated to afford crude **compound 93-d** (25 mg). LCMS (M+H)⁺: 639.

### Step 5: Preparation of 7-fluoro-8-[2-(4-hydroxy-5-methyl-2-propyl-pyrazol-3-yl)oxazol-4-yl]-3-methyl-pyrrolo[1,2-a]pyrazine-6-carboxamide (Example 93)

To a solution of 8-[2-(4-benzyloxy-5-methyl-2-propyl-pyrazol-3-yl)oxazol-4-yl]-*N*-[(2,4-dimethoxyphenyl)methyl]-7-fluoro-3-methyl-pyrrolo[1,2-a]pyrazine-6-carboxamide (**93-d,** 25 mg, 0.04 mmol) in HFIP (1 mL) was added MsOH (113 mg, 1.17 mmol). The reaction was stirred at 50 °C for 2 hrs to give a red solution. The mixture was filtered and the filtrate was purified via Prep-HPLC to afford **Example 93** (13.2 mg). LCMS (M+H)⁺: 399. ¹H NMR (400 MHz, DMSO-*d*₆) δ = 9.56 (d, *J* = 1.2 Hz, 1H), 9.14 (s, 1H), 8.83 (br s, 1H), 8.57 (s, 1H), 7.91 - 7.69 (m, 1H), 7.52 - 7.30 (m, 1H), 4.44 (t, *J =* 7.2 Hz, 2H), 2.45 (s, 3H), 2.14 (s, 3H), 1.87 - 1.74 (m, 2H), 0.90 (t, *J =* 7.2 Hz, 3H).

### Example 94

### 8-[2-(4-hydroxy-5-methyl-2-propyl-pyrazol-3-yl)oxazol-4-yl]-3-methyl-pyrrolo[1,2-a]pyrazine-6-carboxamide

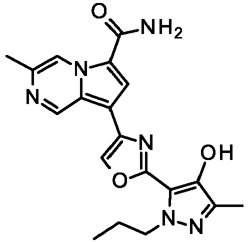

**Example 94** was prepared in analogy to the preparation of **Example 93** by using **compound 3-e** instead of **compound 4-g** in step 2.

**Example 94** (43.8 mg), LCMS (M+H)⁺: 381. ¹H NMR (DMSO-d6, 400 MHz) δ = 9.51 (d, *J =* 1.2 Hz, 1H), 9.31 (s, 1H), 8.75 - 8.62 (s, 2H), 8.22 - 8.05 (m, 1H), 7.99 (s, 1H), 7.54 - 7.51 (m, 1H), 4.44 (t, *J =* 7.2 Hz, 2H), 2.46 (s, 3H), 2.14 (s, 3H), 1.84 - 1.74 (m, 2H), 0.88 (t, *J =* 7.2 Hz, 3H).

### BIOLOGICAL EXAMPLES

### 1. THP1-Dual reporter gene assay (Agonist_THP1_EC₅₀)

This is the cellular reporter assay to evaluate compounds' agonism to interferon regulatory factor (IRF) pathway in THP1-Dual^{™} cells (InvivoGen, cat number thpd-nfis). THP1-Dual^{™} cells were derived from the human THP-1 monocyte cell line by stable integration lucia luciferase gene, a new secreted luciferase reporter gene, under the control of an ISG54 (interferon-stimulated gene) minimal promoter in conjunction with five interferon (IFN)-stimulated response elements. As a result, THP1-Dual^{™} cells allow the study of the IRF pathway, by assessing the activity of Lucia luciferase. Lucia luciferase protein is readily measurable in the cell culture supernatant when using QUANTI-Luc^{™} (InvivoGen, cat: rep-qlcg-500).

After 20-24 hrs compound treatment in THP1-Dual cells, the compounds' agonism to IRF pathway and cell toxicity were tested by measuring luminescence and OD 450 on an Envision plate reader.

On the day of experiment, 30 µL of test medium (RPMI 1640, 2 mM L-glutamine, 25 mM HEPES, 10% heat-inactivated fetal bovine serum) was dispensed in a white 384-well plate (Grenier, cat: 781098) containing the serially diluted compounds (final 1% DMSO) transferred by the Echo550 machine. Then 30 µL of cell suspension (~33,000 cells, 1.1 x10⁶ cells/mL) per well was added immediately by thermo multidrop combi dispenser for incubation 20-24 h at 37 °C, 5% CO₂. At the end of the incubation, 10 µL of cellular supernatant was transferred to proxiplate 384-plus plate for IRF detection, and then 10 µL of QUANTI-Luc^{™} Gold solution was added to the plate that proceeded with the measurement immediately. To detect cell viability, 30 µL of the CCK-8 working solution (Dojindo Molecular Technologies, cat: CK04-20) was added to the cell plate, which was Incubated for 2 hrs in the incubator to measure the absorbance at 450 nm using Envision.

**Table 1: The activity of the compounds of present invention in THP1-Dual^{™} cells assays**

| **Example No.** | **Agonist_THP1_EC₅₀ (µM)** | **Example No.** | **Agonist_THP1_EC₅₀ (µM)** |
|---|---|---|---|
| **1** | 0.202 | **55** | 0.013 |
| **3** | 0.606 | **56** | 0.013 |
| **4** | 0.162 | **57** | 0.062 |
| **9** | 0.829 | **58** | 0.006 |
| **10** | 0.419 | **59** | 0.010 |
| **18** | 0.548 | **60** | 0.023 |
| **19** | 0.408 | **61** | 0.047 |
| **20** | 0.283 | **62** | 0.044 |
| **21** | 0.141 | **63** | 0.026 |
| **22** | 0.082 | **64** | 0.013 |
| **23** | 0.275 | **65** | 0.017 |
| **24** | 0.127 | **66** | 0.027 |
| **25** | 0.050 | **67** | 0.018 |
| **26** | 0.340 | **68** | 0.027 |
| **27** | 0.292 | **69** | 0.093 |
| **28** | 0.134 | **70** | 0.006 |
| **29** | 0.618 | **71** | 0.015 |
| **30** | 0.321 | **72** | 0.015 |
| **31** | 0.019 | **73** | 0.039 |
| **32** | 0.036 | **74** | 0.170 |
| **33** | 0.135 | **75** | 0.049 |
| **34** | 0.012 | **76** | 0.015 |
| **35** | 0.079 | **77** | 0.017 |
| **36** | 0.240 | **78** | 0.090 |
| **37** | 0.014 | **79** | 0.005 |
| **39** | 0.225 | **80** | 0.020 |
| **40** | 0.011 | **81** | 0.065 |
| **41** | 0.048 | **82** | 0.008 |
| **42** | 0.045 | **83** | 0.022 |
| **43** | 0.015 | **84** | 0.034 |
| **44** | 0.046 | **85** | 0.049 |
| **45** | 0.009 | **86** | 0.016 |
| **46** | 0.097 | **87** | 0.053 |
| **47** | 0.033 | **88** | 0.179 |
| **48** | 0.064 | **89** | 0.055 |
| **49** | 0.058 | **90** | 0.030 |
| **50** | 0.025 | **91** | 0.016 |
| **51** | 0.034 | **92** | 0.222 |
| **52** | 0.013 | **93** | 0.006 |
| **53** | 0.012 | **94** | 0.015 |
| **54** | 0.031 | | |

### 2. hPBMC cytokine release assay (Agonist_hPBMC_IFN-β_EC₅₀)

This is the cytokines concentration detection with Luminex assay for human PBMC supernatant. Human peripheral blood mononuclear cells (PBMC) are treated by compounds with appropriate time. Multiple cytokines are released in the supernatant in response to the compound stimulation. Luminex assay can measure the cytokines concentration in supernatant and the compounds activity on human PBMC can be evaluated.

Frozen PBMC was thawed and cultured in PRMI1640 (GIBCO 11875-093) whole medium with 10% Heat inactivated-FBS (HI-FBS), 1% NEAA, 1% sodium pyruvate and 1% Penicillin-Streptomycin in CO₂ incubator overnight. Cells were centrifuged at 400×g for 5 minutes at 4 °C and the pellet was re-suspended with complete RPMI1640 medium to concentration of 1x10⁶ cells/ml in cell culture flask T75 (Thermo Fisher Scientific). Compounds were serially diluted with DMSO and distributed to 96-well flat bottom cell plate (Corning 3599) with Echo555. 90 µL/well PBMC cells were seeded into the plates with Multidrop (Thermo Fisher Scientific). After 24 hours of incubation in CO₂ incubator, cells were centrifuged at 400×g for 5 minutes at room temperature, 80 µL supernatant each well was collected for cytokines release test with luminex assay of Human custom ProcartaPlex 5-plex kit (Thermo Fisher Scientific PPX-05). 10 µL supernatant or standards were added to a mixture of color-coded beads, 5 µL Biotinylated detection antibodies specific to the analytes of interest were added and formed an antibodyantigen sandwich. 10 µL Phycoerythrin (PE)-conjugated streptavidin is added to the plate and then read signal of each well on the Luminex 200^{™}. The raw data was analyzed by Millipore Analyst Software and cytokine concentrations were generated accordingly. The EC₅₀ of corresponding cytokine such as interferon-β (IFN-β) was calculated by the 4-parameter sigmoidal concentration response model fitting.

**Table 2: The activity of the compounds of present invention in hPBMC assays**

| **Example No.** | **Agonist_hPBMC_ IFN-β_EC₅₀ (µM)** | **Example No.** | **Agonist_hPBMC_ IFN-β_EC₅₀ (µM)** |
|---|---|---|---|
| **31** | 0.032 | **76** | 0.014 |
| **34** | 0.011 | **77** | 0.020 |
| **40** | 0.046 | **79** | 0.022 |
| **45** | 0.009 | **80** | 0.042 |
| **52** | 0.018 | **82** | 0.029 |
| **53** | 0.026 | **83** | 0.023 |
| **55** | 0.032 | **84** | 0.018 |
| **58** | 0.017 | **86** | 0.030 |
| **59** | 0.007 | **91** | 0.038 |
| **64** | 0.035 | **93** | 0.009 |
| **67** | 0.024 | **94** | 0.006 |
| **70** | 0.010 | | |

### 3. Single dose pharmacokinetics (SDPK) of selected examples

This is the single dose pharmacokinetics (PK) study in female C57/BL6 mice. The purpose of this study was to determine the pharmacokinetics of selected compounds following a single intravenous bolus injection in female C57BL/6J mice. Briefly, 3 female C57BL/6J mice (available from Shanghai Lingchang Biotechnology Co., Ltd) were treated with a single dose of compound intravenously at 1 mg/kg (IV). Blood samples were collected at 5 min, 15 min, 30 min, 1 h, 2 h, 4 h, 7 h and 24 h post-dose. Blood samples were placed on wet ice until centrifugation to obtain plasma samples. The compound concentration in plasma samples was determined using LC-MS/MS method. The pharmacokinetic parameters were calculated by noncompartmental analysis.

**Table 3: Results of SDPK**

| **Example No.** | **1 mg/kg, iv** | | |
|---|---|---|---|
| | **CL (mL/min/kg)** | **C₀ (ng/mL)** | **AUC₀₋ₗₐₛₜ (h×ng/mL)** |
| **4** | 137 | 1720 | 122 |
| **31** | 30 | 5504 | 597 |
| **40** | 114 | 1599 | 151 |
| **79** | 111 | 2196 | 150 |

### 4. Prior art compounds profile

Reference compouds AC01 and AE01 were prepared according to WO 2022/195462 and tested in the assays described above. The results are shown in Table 4 below.

**Table 4: The activities in hPBMC assays and pharmacological properties of the prior art compounds**

| **Example No. (as disclosed in** WO 2022/195462 | **Structure** | **Agonist_ hPBMC_ IFN-β_EC₅₀ (µM)** | **1 mg/kg, iv** | | |
|---|---|---|---|---|---|
| | | | **CL (mL/min/kg)** | **C₀ (ng/mL)** | **AUC₀₋ₗₐₛₜ (h×ng/mL)** |
| **AC01** | 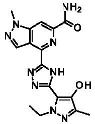 | 0.028 | 170 | 997 | 102 |
| **AE01** | 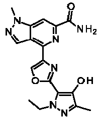 | 0.040 | | | |

## Claims

1. A compound of formula (I), 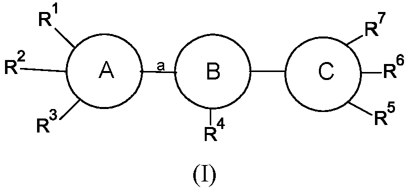 wherein
substituted ring A is selected from 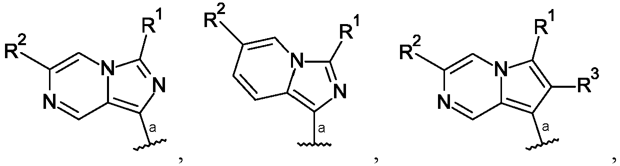 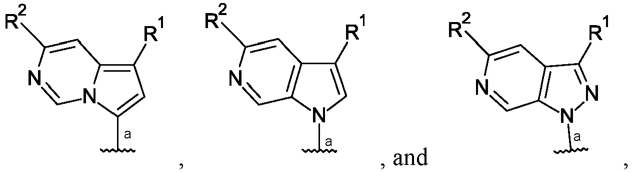 each of R¹, R² and R³ is independently absent or selected from C₁₋₆alkyl, haloC₁₋₆alkyl, hydroxylC₁₋₆alkyl, aminoC₁₋₆alkyl, -C(O)C₁₋₆alkyl, C₁₋₆alkylamino, (C₁₋₆alkyl)₂amino, C₁₋₆alkyl-S-, C₁₋₆alkylSO₂, C₁₋₆alkoxy, haloC₁₋₆alkoxy, C₁₋₆alkoxycarbonyl, carboxyl, hydroxyl, cyano, amino, halogen, acyl, carbamoyl, and a 5 to 8 membered monocyclic heterocyclyl containing one to three heteroatoms selected from N, O, and S, wherein said heterocyclyl can be further substituted by halogen, C₁₋₆alkyl, C₁₋₆alkoxy or amino;
Ring B is a 5 to 8 membered monocyclic or bicyclic heteroaryl ring or heterocyclyl ring containing one to four heteroatoms selected from N, O, and S,
R⁴ is absent or selected from C₁₋₆alkyl, haloC₁₋₆alkyl, hydroxylC₁₋₆alkyl, aminoC₁₋₆alkyl, - C(O)C₁₋₆alkyl, C₁₋₆alkylamino, (C₁₋₆alkyl)₂amino, C₁₋₆alkyl-S-, C₁₋₆alkylSO₂, C₁₋₆alkoxy, haloC₁₋₆alkoxy, C₁₋₆alkoxycarbonyl, carboxyl, hydroxyl, cyano, amino, halogen, and carbamoyl;
Bond a is a bond connecting ring A and ring B;
Ring C is a 5 to 8 membered monocyclic or bicyclic heteroaryl ring or heterocyclyl ring containing one to four heteroatoms selected from N, O, and S,
each of R⁵, R⁶ and R⁷ is independently absent or selected from C₁₋₆alkyl, haloC₁₋₆alkyl, hydroxylC₁₋₆alkyl, aminoC₁₋₆alkyl, -C(O)C₁₋₆alkyl, C₁₋₆alkylamino, (C₁₋₆alkyl)₂amino, C₁₋₆alkyl-S-, C₁₋₆alkylSO₂, C₁₋₆alkoxy, C₁₋₆alkoxyC₁₋₆alkyl, haloC₁₋₆alkoxy, C₁₋₆alkoxycarbonyl, haloC₂₋₆alkenyl, C₃₋₇cycloalkyl, C₃₋₇cycloalkylC₁₋₆alkyl, C₂₋₆alkynyl, C₁₋₆alkoxyC₂₋₆alkynyl, carboxyl, hydroxyl, cyano, amino, halogen, carbamoyl, cyanoC₁₋₆alkyl, imidazolylC₁₋₆alkyl, triazolylC₁₋₆alkyl, pyridinylC₁₋₆alkyl, thiazolylC₁₋₆alkyl, and phenylC₁₋₆alkyl, wherein the phenylC₁₋₆alkyl is optionally substituted by halogen, cyano, hydroxylC₁₋₆alkyl, morpholinoC₁₋₆alkyl, C₁₋₆alkoxy, C₁₋₆alkoxyC₁₋₆alkyl, and C₁₋₆alkoxycarbonyl, or
two of R⁵, R⁶, and R⁷ together with the atoms to which they are bonded form a 5 to 6 membered ring having 0 to 2 heteroatoms selected from N, O, and S;
or a pharmaceutically acceptable salt thereof.

2. The compound according to claim 1, wherein
substituted ring A is selected from 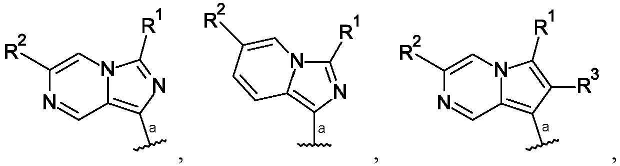 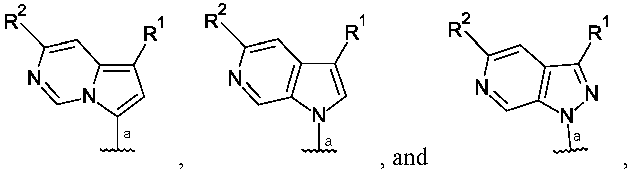
R¹ is carbamoyl,
R² is selected from C₁₋₆alkyl, haloC₁₋₆alkyl, halogen, acyl, and a 5 to 8 membered monocyclic heterocyclyl containing one to three heteroatoms selected from N, O, and S,
R³ is absent or halogen;
Ring B is selected from 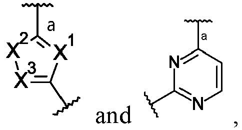
each of X¹, X² and X³ is independently selected from C, N, and O,
R⁴ is absent or selected from C₁₋₆alkyl, haloC₁₋₆alkyl, aminoC₁₋₆alkyl, hydroxyC₁₋₆alkyl, and carbamoyl;
Ring C is pyrazolyl,
R⁵ is selected from C₁₋₆alkyl, hydroxylC₁₋₆alkyl, haloC₁₋₆alkyl, cyanoC₁₋₆alkyl, haloC₂₋₆alkenyl, C₃₋₇cycloalkyl, C₃₋₇cycloalkylC₁₋₆alkyl, C₁₋₆alkoxyC₁₋₆alkyl, C₂₋₆alkynyl, C₁₋₆alkoxyC₂₋₆alkynyl, imidazolylC₁₋₆alkyl, triazolylC₁₋₆alkyl, pyridinylC₁₋₆alkyl, thiazolylC₁₋₆alkyl, and phenylC₁₋₆alkyl, wherein the phenylC₁₋₆alkyl is optionally substituted by halogen, cyano, hydroxylC₁₋₆alkyl, morpholinoC₁₋₆alkyl, C₁₋₆alkoxy, C₁₋₆alkoxyC₁₋₆alkyl, and C₁₋₆alkoxycarbonyl,
R⁶ is absent or C₁₋₆alkyl, R⁷ is absent or selected from hydroxyl, halogen, and amino, or R⁶ and R⁷ together with the atoms to which they are bonded form a 5 to 6 membered ring containing zero to two heteroatoms selected from N, O, and S.

3. A compound according to any one of claims 1 or 2, wherein
substituted ring A is selected from 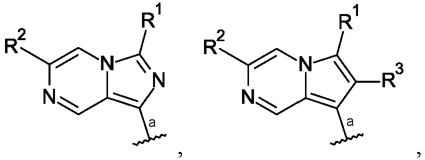 and 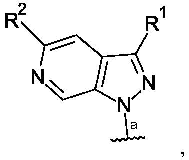
R¹ is carbamoyl,
R² is C₁₋₆alkyl,
R³ is absent or halogen.

4. A compound according to any one of claims 1 or 2, wherein
R¹ is carbamoyl;
R² is selected from methyl, difluoromethyl, acetyl, chloro, and imidazolyl;
R³ is absent or fluoro.

5. A compound according to any one of claims 1 - 4, wherein
R¹ is carbamoyl;
R² is methyl;
R³ is absent or fluoro.

6. A compound according to any one of claims 1 - 5, wherein
ring B is selected from triazolyl, oxazolyl, oxadiazolyl, and pyrimidinyl.

7. A compound according to any one of claims 1 - 6, wherein
substituted ring B is selected from

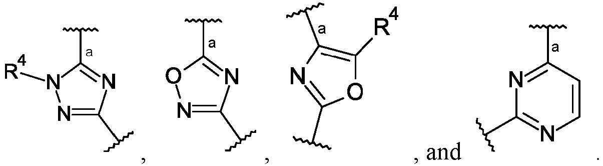

8. A compound according to any one of claims 1 - 7, wherein
substituted ring B is selected from 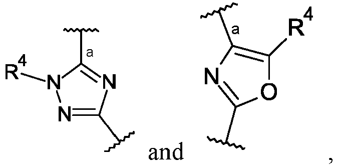
R⁴ is absent or selected from C₁₋₆alkyl and hydroxyC₁₋₆alkyl.

9. A compound according to any one of claims 1 - 7, wherein
R⁴ is absent or selected from methyl, difluoromethyl, aminomethyl, hydroxymethyl, and carbamoyl.

10. A compound according to any one of claims 1 - 9, wherein
R⁵ is selected from ethyl, propyl, butyl, cyclopropyl, cyclopropylmethyl, 3,3-difluoropropyl, 3,3-difluoroallyl, 2-cyanoethyl, 3-cyanopropyl, 3-hydroxypropyl, 2-imidazol-1-ylethyl, 2-(triazol-2-yl)ethyl, 2-(triazol-1-yl)ethyl, 2-(1,2,4-triazol-4-yl)ethyl, 2-(1,2,4-triazol-1-yl)ethyl, 3-fluoro-propyl, 2-methoxyethyl, 3-methoxypropyl, 1-pent-3-ynyl, 5-methoxypent-3-ynyl, 2-phenylethyl, 2-(4-methoxyphenyl)ethyl, 2-(3-methoxyphenyl)ethyl, 2-[4-(methoxymethyl)phenyl]ethyl, 2-[3-(methoxymethyl)phenyl]ethyl, 2-(4-fluorophenyl)ethyl, 2-(3-fluorophenyl)ethyl, 2-(3-cyanophenyl)ethyl, 2-(4-cyanophenyl)ethyl, 2-(4-methoxycarbonylphenyl)-ethyl, 2-[4-(hydroxymethyl)phenyl]ethyl, 2-pyridin-4-ylethyl, 2-(3-pyridyl)ethyl, 2-[4-(morpholinomethyl)phenyl]ethyl, and 2-thiazol-2-ylethyl,
R⁶ is absent or methyl, R⁷ is absent, fluoro, amino, or hydroxyl, or R⁶ and R⁷ together with the atoms to which they are bonded form a 5 membered ring.

11. A compound according to claim 1, wherein
substituted ring A is selected from 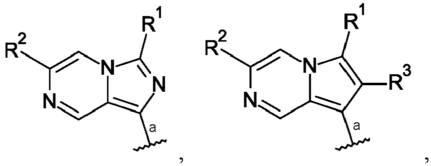 and 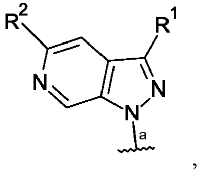
R¹ is carbamoyl,
R² is C₁₋₆alkyl,
R³ is absent or halogen;
substituted ring B is selected from 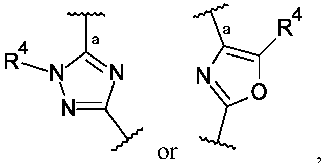 R⁴ is absent or selected from C₁₋₆alkyl and hydroxyC₁₋₆alkyl;
substituted ring C is 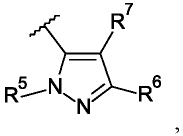
R⁵ is selected from C₁₋₆alkyl, haloC₁₋₆alkyl, haloC₂₋₆alkenyl, C₂₋₆alkynyl, C₃₋₇cycloalkylC₁₋₆alkyl, pyridinylC₁₋₆alkyl, phenylC₁₋₆alkyl, wherein the phenylC₁₋₆alkyl is optionally substituted by halogen, C₁₋₆alkoxy, C₁₋₆alkoxyC₁₋₆alkyl, hydroxylC₁₋₆alkyl, or morpholinoC₁₋₆alkyl,
R⁶ is C₁₋₆alkyl,
R⁷ is hydroxyl.

12. A compound according to claim 1, selected from:
1-[3-(2-ethyl-4-hydroxy-5-methyl-pyrazol-3-yl)-1*H*-1,2,4-triazol-5-yl]-6-methyl-imidazo[1,5-a]pyrazine-3-carboxamide;
1-[5-(2-ethyl-4-hydroxy-5-methyl-pyrazol-3-yl)-2-methyl-1,2,4-triazol-3-yl]-6-methyl-imidazo[1,5-a]pyrazine-3-carboxamide;
8-[3-(2-ethyl-4-hydroxy-5-methyl-pyrazol-3-yl)-1*H*-1,2,4-triazol-5-yl]-3-methyl-pyrrolo[1,2-a]pyrazine-6-carboxamide;
8-[3-(2-ethyl-4-hydroxy-5-methyl-pyrazol-3-yl)-1*H*-1,2,4-triazol-5-yl]-7-fluoro-3-methyl-pyrrolo[1,2-a]pyrazine-6-carboxamide;
4-acetyl-6-[3-(2-ethyl-4-hydroxy-5-methyl-pyrazol-3-yl)-1*H*-1,2,4-triazol-5-yl]pyridine-2-carboxamide;
6-[3-(2-ethyl-4-hydroxy-5-methyl-pyrazol-3-yl)-1*H*-1,2,4-triazol-5-yl]-4-imidazol-1-yl-pyridine-2-carboxamide;
4-[2-(2-ethyl-4-hydroxy-5-methyl-pyrazol -3-yl)pyrimidin-4-yl]-1-methyl-pyrazolo[4,3-c]pyridine-6-carboxamide;
1-[2-(2-ethyl-4-hydroxy-5-methyl-pyrazol-3-yl)pyrimidin-4-yl]-6-methyl-imidazo[1,5-a]pyrazine-3-carboxamide;
1-[2-(2-ethyl-4-hydroxy-5-methyl-pyrazol-3-yl)oxazol-4-yl]-6-methyl-imidazo[1,5-a]pyrazine-3-carboxamide;
4-[5-(difluoromethyl)-2-(2-ethyl-4-hydroxy-5-methyl-pyrazol-3-yl)oxazol-4-yl]-1-methyl-pyrazolo[4,3-c]pyridine-6-carboxamide;
1-[5-(aminomethyl)-2-(2-ethyl-4-hydroxy-5-methyl-pyrazol-3-yl)oxazol-4-yl]-6-methyl-imidazo[1,5-a]pyrazine-3-carboxamide;
4-(6-carbamoyl-1-methyl-pyrazolo[4,3-c]pyridin-4-yl)-2-(2-ethyl-4-hydroxy-5-methyl-pyrazol-3-yl)oxazole-5-carboxamide;
4-[3-(2-ethyl-5,6-dihydro-4*H*-cyclopenta[c]pyrazol-3-yl)-1*H*-1,2,4-triazol-5-yl]-1-methyl-pyrazolo[4,3-c]pyridine-6-carboxamide;
1-[3-[2-(3-hydroxypropyl)-5-methyl-pyrazol-3-yl]-1*H*-1,2,4-triazol-5-yl]-6-methyl-imidazo[1,5-a]pyrazine-3-carboxamide;
1-[3-(2-ethyl-5-methyl-pyrazol-3-yl)-1*H*-1,2,4-triazol-5-yl]-6-methyl-imidazo[1,5-a]pyrazine-3-carboxamide;
1-[3-(2-ethyl-4-fluoro-5-methyl-pyrazol-3 -yl)-1*H*-1,2,4-triazol-5-yl]-6-methyl-imidazo[1,5-a]pyrazine-3-carboxamide;
1-[3-(4-amino-2-ethyl-5-methyl-pyrazol-3-yl)-1*H*-1,2,4-triazol-5-yl]-6-methyl-imidazo[1,5-a]pyrazine-3-carboxamide;
6-chloro-1-[3-(2-ethyl-4-hydroxy-5-methyl-pyrazol-3-yl)-1*H*-1,2,4-triazol-5-yl]imidazo[1,5-a]pyrazine-3-carboxamide;
1-[3-(2-ethyl-4-hydroxy-5-methyl-pyrazol-3-yl)-1*H*-1,2,4-triazol-5-yl]-6-methyl-imidazo[1,5-a]pyridine-3 -carboxamide;
7-[3-(2-ethyl-4-hydroxy-5-methyl-pyrazol-3-yl)-1*H*-1,2,4-triazol-5-yl]-3-methyl-pyrrolo[1,2-c]pyrimidine-5-carboxamide;
1-[2-(2-ethyl-4-hydroxy-5-methyl-pyrazol-3-yl)oxazol-4-yl]-5-methyl-pyrazolo[3,4-c]pyridine-3 -carboxamide;
1-[3-(2-ethyl-4-hydroxy-5-methyl-pyrazol-3-yl)-1*H*-1,2,4-triazol-5-yl]-5-methyl-pyrazolo[3,4-c]pyridine-3-carboxamide;
4-[3-[4-hydroxy-2-(2-imidazol-1-ylethyl)-5-methyl-pyrazol-3-yl]-1*H*-1,2,4-triazol-5-yl]-1-methyl-pyrazolo[4,3-c]pyridine-6-carboxamide;
4-[3-[2-(3-cyanopropyl)-4-hydroxy-5-methyl-pyrazol-3-yl]-1*H*-1,2,4-triazol-5-yl]-1-methyl-pyrazolo[4,3-c]pyridine-6-carboxamide;
4-[3-[2-(2-cyanoethyl)-4-hydroxy-5-methyl-pyrazol-3-yl]-1*H*-1,2,4-triazol-5-yl]-1-methyl-pyrazolo[4,3-c]pyridine-6-carboxamide;
6-(difluoromethyl)-1-[3-(2-ethyl-4-hydroxy-5-methyl-pyrazol-3-yl)-1*H*-1,2,4-triazol-5-yl]imidazo[1,5-a]pyrazine-3-carboxamide;
7-[3-(2-ethyl-4-hydroxy-5-methyl-pyrazol-3-yl)-1,2,4-oxadiazol-5-yl]-3-methyl-pyrrolo[1,2-c]pyrimidine-5-carboxamide;
8-[2-(2-ethyl-4-hydroxy-5-methyl-pyrazol-3-yl)oxazol-4-yl]-7-fluoro-3-methyl-pyrrolo[1,2-a]pyrazine-6-carboxamide;
1-[2-(2-ethyl-4-hydroxy-5-methyl-pyrazol-3-yl)oxazol-4-yl]-5-methyl-pyrrolo[2,3-c]pyridine-3 -carboxamide;
8-[3-[2-(2-cyanoethyl)-4-hydroxy-5-methyl-pyrazol-3-yl]-1*H*-1,2,4-triazol-5-yl]-7-fluoro-3-methyl-pyrrolo[1,2-a]pyrazine-6-carboxamide;
1-[3-(4-hydroxy-5-methyl-2-propyl-pyrazol-3-yl)-1*H*-1,2,4-triazol-5-yl]-5-methyl-pyrazolo[3,4-c]pyridine-3-carboxamide;
1-[3-[2-(cyclopropylmethyl)-4-hydroxy-5-methyl-pyrazol-3-yl]-1*H*-1,2,4-triazol-5-yl]-5-methyl-pyrazolo[3,4-c]pyridine-3-carboxamide;
1-[3-(2-cyclopropyl-4-hydroxy-5-methyl-pyrazol-3-yl)-1*H*-1,2,4-triazol-5-yl]-5-methyl-pyrazolo[3,4-c]pyridine-3-carboxamide;
4-[3-[2-(3,3-difluoroallyl)-4-hydroxy-5-methyl-pyrazol-3-yl]-1*H*-1,2,4-triazol-5-yl]-1-methyl-pyrazolo[4,3-c]pyridine-6-carboxamide;
4-[3-[4-hydroxy-5-methyl-2-[2-(triazol-2-yl)ethyl]pyrazol-3-yl]-1*H*-1,2,4-triazol-5-yl]-1-methyl-pyrazolo[4,3-c]pyridine-6-carboxamide;
4-[3-[4-hydroxy-5-methyl-2-[2-(triazol-1-yl)ethyl]pyrazol-3-yl]-1*H*-1,2,4-triazol-5-yl]-1-methyl-pyrazolo[4,3-c]pyridine-6-carboxamide;
4-[3-[2-(3,3-difluoropropyl)-4-hydroxy-5-methyl-pyrazol-3-yl]-1*H*-1,2,4-triazol-5-yl]-1-methyl-pyrazolo[4,3-c]pyridine-6-carboxamide;
4-[3-[4-hydroxy-5-methyl-2-[2-(1,2,4-triazol-4-yl)ethyl]pyrazol-3-yl]-1*H*-1,2,4-triazol-5-yl]-1-methyl-pyrazolo[4,3-c]pyridine-6-carboxamide;
4-[3-[4-hydroxy-5-methyl-2-[2-(1,2,4-triazol-1-yl)ethyl]pyrazol-3-yl]-1*H*-1,2,4-triazol-5-yl]-1-methyl-pyrazolo[4,3-c]pyridine-6-carboxamide;
1-[3-[4-hydroxy-5-methyl-2-(2-phenylethyl)pyrazol-3-yl]-1*H*-1,2,4-triazol-5-yl]-5-methyl-pyrazolo[3,4-c]pyridine-3-carboxamide;
1-[3-[2-(3-fluoropropyl)-4-hydroxy-5-methyl-pyrazol-3-yl]-1*H*-1,2,4-triazol-5-yl]-5-methyl-pyrazolo[3,4-c]pyridine-3-carboxamide;
1-[3-[4-hydroxy-2-(2-methoxyethyl)-5-methyl-pyrazol-3-yl]-1*H*-1,2,4-triazol-5-yl]-5-methyl-pyrazolo[3,4-c]pyridine-3-carboxamide ;
1-[3-(2-butyl-4-hydroxy-5-methyl-pyrazol-3-yl)-1*H*-1,2,4-triazol-5-yl]-5-methyl-pyrazolo[3,4-c]pyridine-3-carboxamide;
1-[3-[4-hydroxy-2-(3-methoxypropyl)-5-methyl-pyrazol-3-yl]-1*H*-1,2,4-triazol-5-yl]-5-methyl-pyrazolo[3,4-c]pyridine-3-carboxamide;
4-[3-(4-hydroxy-5-methyl-2-pent-3-ynyl-pyrazol-3-yl)-1*H*-1,2,4-triazol-5-yl]-1-methyl-pyrazolo[4,3-c]pyridine-6-carboxamide;
1-[3-[4-hydroxy-5-methyl-2-[2-(4-pyridyl)ethyl]pyrazol-3-yl]-1*H*-1,2,4-triazol-5-yl]-5-methyl-pyrazolo[3,4-c]pyridine-3-carboxamide;
1-[3-[4-hydroxy-5-methyl-2-[2-(3-pyridyl)ethyl]pyrazol-3-yl]-1*H*-1,2,4-triazol-5-yl]-5-methyl-pyrazolo[3,4-c]pyridine-3-carboxamide;
1-[5-[4-hydroxy-5-methyl-2-(2-phenylethyl)pyrazol-3-yl]-2-methyl-1,2,4-triazol-3-yl]-5-methyl-pyrazolo[3,4-c]pyridine-3-carboxamide;
1-[5-(2-butyl-4-hydroxy-5-methyl-pyrazol-3-yl)-2-methyl-1,2,4-triazol-3-yl]-5-methyl-pyrazolo[3,4-c]pyridine-3-carboxamide;
1-[3-[4-hydroxy-2-[2-(4-methoxyphenyl)ethyl]-5-methyl-pyrazol-3-yl]-1*H*-1,2,4-triazol-5-yl]-5-methyl-pyrazolo[3,4-c]pyridine-3-carboxamide;
1-[3-(4-hydroxy-5-methyl-2-pent-3-ynyl-pyrazol-3-yl)-1*H*-1,2,4-triazol-5-yl]-5-methyl-pyrazolo[3,4-c]pyridine-3-carboxamide;
1-[3-[2-[2-(4-fluorophenyl)ethyl]-4-hydroxy-5-methyl-pyrazol-3-yl]-1*H*-1,2,4-triazol-5-yl]-5-methyl-pyrazolo[3,4-c]pyridine-3-carboxamide;
1-[3-[4-hydroxy-5-methyl-2-(2-phenylethyl)pyrazol-3-yl]-1*H*-1,2,4-triazol-5-yl]-6-methyl-imidazo[1,5-a]pyrazine-3-carboxamide;
1-[3-[4-hydroxy-2-[2-(3-methoxyphenyl)ethyl]-5-methyl-pyrazol-3-yl]-1*H*-1,2,4-triazol-5-yl]-5-methyl-pyrazolo[3,4-c]pyridine-3-carboxamide;
1-(3-(1-(3-fluorophenethyl)-4-hydroxy-3-methyl-1*H*-pyrazol-5-yl)-1*H*-1,2,4-triazol-5-yl)-5-methyl-1*H*-pyrazolo[3,4-c]pyridine-3-carboxamide;
1-[3-[2-[2-(2-fluorophenyl)ethyl]-4-hydroxy-5-methyl-pyrazol-3-yl]-1*H*-1,2,4-triazol-5-yl]-5-methyl-pyrazolo[3,4-c]pyridine-3-carboxamide;
7-fluoro-8-[3-[4-hydroxy-5-methyl-2-(2-phenylethyl)pyrazol-3-yl]-1*H*-1,2,4-triazol-5-yl]-3-methyl-pyrrolo[1,2-a]pyrazine-6-carboxamide;
1-(2-(4-hydroxy-3-methyl-1-propyl-1H-pyrazol-5-yl)oxazol-4-yl)-5-methyl-1*H-*pyrazolo[3,4-c]pyridine-3-carboxamide;
1-(2-(1-butyl-4-hydroxy-3-methyl-1*H*-pyrazol-5-yl)oxazol-4-yl)-5-methyl-1*H-*pyrazolo[3,4-c]pyridine-3-carboxamide;
1-[3-[2-[2-(3-cyanophenyl)ethyl]-4-hydroxy-5-methyl-pyrazol-3-yl]-1*H*-1,2,4-triazol-5-yl]-5-methyl-pyrazolo[3,4-c]pyridine-3-carboxamide;
1-[3-[2-[2-(4-cyanophenyl)ethyl]-4-hydroxy-5-methyl-pyrazol-3-yl]-1*H*-1,2,4-triazol-5-yl]-5-methyl-pyrazolo[3,4-c]pyridine-3-carboxamide;
Methyl 4-[2-[5-[5-(3-carbamoyl-5-methyl-pyrazolo[3,4-c]pyridin-1-yl)-1*H*-1,2,4-triazol-3-yl]-4-hydroxy-3-methyl-pyrazol-1-yl]ethyl]benzoate;
1-(3-(1-(3,3-difluoroallyl)-4-hydroxy-3-methyl-1*H*-pyrazol-5-yl)-1*H*-1,2,4-triazol-5-yl)-5-methyl-1*H*-pyrazolo[3,4-c]pyridine-3-carboxamide;
1-[3-[4-hydroxy-2-[2-[4-(methoxymethyl)phenyl]ethyl]-5-methyl-pyrazol-3-yl]-1*H*-1,2,4-triazol-5-yl]-5-methyl-pyrazolo[3,4-c]pyridine-3-carboxamide;
1-[3-[4-hydroxy-2-[2-[3-(methoxymethyl)phenyl]ethyl]-5-methyl-pyrazol-3-yl]-1*H*-1,2,4-triazol-5-yl]-5-methyl-pyrazolo[3,4-c]pyridine-3-carboxamide;
1-(3-(1-(3,3-difluoropropyl)-4-hydroxy-3-methyl-1*H*-pyrazol-5-yl)-1*H*-1,2,4-triazol-5-yl)-5-methyl-1*H*-pyrazolo[3,4-c]pyridine-3-carboxamide;
1-(2-(1-(3,3-difluoropropyl)-4-hydroxy-3-methyl-1*H*-pyrazol-5-yl)oxazol-4-yl)-5-methyl-1*H*-pyrazolo[3,4-c]pyridine-3-carboxamid;
1-[3-[4-hydroxy-2-[2-[4-(hydroxymethyl)phenyl]ethyl]-5-methyl-pyrazol-3-yl]-1*H*-1,2,4-triazol-5-yl]-5-methyl-pyrazolo[3,4-c]pyridine-3-carboxamide;
7-fluoro-8-[3-[4-hydroxy-2-[2-(4-methoxyphenyl)ethyl]-5-methyl-pyrazol-3-yl]-1*H*-1,2,4-triazol-5-yl]-3-methyl-pyrrolo[1,2-a]pyrazine-6-carboxamide;
1-[3-[4-hydroxy-2-[2-(4-methoxyphenyl)ethyl]-5-methyl-pyrazol-3-yl]-1*H*-1,2,4-triazol-5-yl]-6-methyl-imidazo[1,5-a]pyrazine-3-carboxamide;
1-[3-[4-hydroxy-2-[2-[4-(hydroxymethyl)phenyl]ethyl]-5-methyl-pyrazol-3-yl]-1*H*-1,2,4-triazol-5-yl]-6-methyl-imidazo[1,5-a]pyrazine-3-carboxamide;
1-[3-[4-hydroxy-5-methyl-2-[2-(3-pyridyl)ethyl]pyrazol-3-yl]-1*H*-1,2,4-triazol-5-yl]-6-methyl-imidazo[1,5-a]pyrazine-3-carboxamide;
7-fluoro-8-[3-[4-hydroxy-5-methyl-2-[2-(3-pyridyl)ethyl]pyrazol-3-yl]-1*H*-1,2,4-triazol-5-yl]-3-methyl-pyrrolo[1,2-a]pyrazine-6-carboxamide;
1-(2-(4-hydroxy-3-methyl-1-(2-(pyridin-3-yl)ethyl)-1*H*-pyrazol-5-yl)oxazol-4-yl)-5-methyl-1*H*-pyrazolo[3,4-c]pyridine-3-carboxamide;
1-(2-(4-hydroxy-1-(3-methoxyphenethyl)-3-methyl-1*H*-pyrazol-5-yl)oxazol-4-yl)-5-methyl-1*H*-pyrazolo[3,4-c]pyridine-3-carboxamide;
8-[3-(2-butyl-4-hydroxy-5-methyl-pyrazol-3-yl)-1*H*-1,2,4-triazol-5-yl]-7-fluoro-3-methyl-pyrrolo[1,2-a]pyrazine-6-carboxamide;
1-[3-(2-butyl-4-hydroxy-5-methyl-pyrazol-3-yl)-1*H*-1,2,4-triazol-5-yl]-6-methyl-imidazo[1,5-a]pyrazine-3-carboxamide;
1-(2-(4-hydroxy-1-(4-methoxyphenethyl)-3-methyl-1*H*-pyrazol-5-yl)oxazol-4-yl)-5-methyl-1*H*-pyrazolo[3,4-c]pyridine-3-carboxamide;
7-fluoro-8-[3-(4-hydroxy-5-methyl-2-propyl-pyrazol-3-yl)-1*H*-1,2,4-triazol-5-yl]-3-methyl-pyrrolo[1,2-a]pyrazine-6-carboxamide;
1-[3-(4-hydroxy-5-methyl-2-propyl-pyrazol-3-yl)-1*H*-1,2,4-triazol-5-yl]-6-methyl-imidazo[1,5-a]pyrazine-3-carboxamide;
1-[3-[4-hydroxy-2-(5-methoxypent-3-ynyl)-5-methyl-pyrazol-3-yl]-1*H*-1,2,4-triazol-5-yl]-5-methyl-pyrazolo[3,4-c]pyridine-3-carboxamide;
4-(5-(4-hydroxy-3-methyl-1-phenethyl-1*H*-pyrazol-5-yl)-2*H*-1,2,4-triazol-3-yl)-1-methyl-1*H*-pyrazolo[4,3-c]pyridine-6-carboxamide;
1-[5-(hydroxymethyl)-2-(4-hydroxy-5-methyl-2-propyl-pyrazol-3-yl)oxazol-4-yl]-5-methyl-pyrazolo[3,4-c]pyridine-3-carboxamide;
1-[5-(4-hydroxy-5-methyl-2-propyl-pyrazol-3-yl)-2-methyl-1,2,4-triazol-3-yl]-5-methyl-pyrazolo[3,4-c]pyridine-3-carboxamide;
1-[2-(4-hydroxy-5-methyl-2-propyl-pyrazol-3-yl)oxazol-4-yl]-6-methyl-imidazo[1,5-a]pyrazine-3-carboxamide;
1-[2-(2-butyl-4-hydroxy-5-methyl-pyrazol-3-yl)oxazol-4-yl]-6-methyl-imidazo[1,5-a]pyrazine-3-carboxamide;
1-[3-[4-hydroxy-5-methyl-2-[2-[4-(morpholinomethyl)phenyl]ethyl]pyrazol-3-yl]-1*H*-1,2,4-triazol-5-yl]-6-methyl-imidazo[1,5-a]pyrazine-3-carboxamide;
1-[2-[4-hydroxy-5-methyl-2-[2-[4-(morpholinomethyl)phenyl]ethyl]pyrazol-3-yl]oxazol-4-yl]-5-methyl-pyrazolo[3,4-c]pyridine-3-carboxamide;
1-[2-[4-hydroxy-2-[2-[4-(methoxymethyl)phenyl]ethyl]-5-methyl-pyrazol-3-yl]oxazol-4-yl]-5-methyl-pyrazolo[3,4-c]pyridine-3-carboxamide;
1-[3-[4-hydroxy-5-methyl-2-(2-thiazol-2-ylethyl)pyrazol-3-yl]-1*H*-1,2,4-triazol-5-yl]-6-methyl-imidazo[1,5-a]pyrazine-3-carboxamide;
8-[3-(4-hydroxy-5-methyl-2-propyl-pyrazol-3-yl)-1*H*-1,2,4-triazol-5-yl]-3-methyl-pyrrolo[1,2-a]pyrazine-6-carboxamide;
1-[5-(4-hydroxy-5-methyl-2-propyl-pyrazol-3-yl)-2-methyl-1,2,4-triazol-3-yl]-6-methyl-imidazo[1,5-a]pyrazine-3-carboxamide;
7-fluoro-8-[2-(4-hydroxy-5-methyl-2-propyl-pyrazol-3-yl)oxazol-4-yl]-3-methyl-pyrrolo[1,2-a]pyrazine-6-carboxamide;
8-[2-(4-hydroxy-5-methyl-2-propyl-pyrazol-3-yl)oxazol-4-yl]-3-methyl-pyrrolo[1,2-a]pyrazine-6-carboxamide;
or a pharmaceutically acceptable salt thereof.

13. A pharmaceutical composition comprising a compound or a pharmaceutically acceptable salt thereof according to any one of claims 1 to 12, and a pharmaceutically acceptable excipient.

14. A compound or a pharmaceutically acceptable salt thereof according to any one of claims 1 to 12 for use as therapeutically active substance.

15. A compound or a pharmaceutically acceptable salt thereof according to any one of claims 1 to 12 for use in the treatment of cancer.

## Patentansprüche

1. Verbindung der Formel (I), 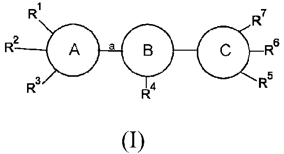 wobei
der substituierte Ring A ausgewählt ist aus 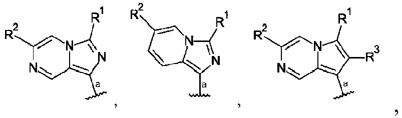 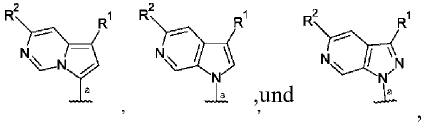
R¹, R² und R³ jeweils unabhängig voneinander abwesend sind oder ausgewählt sind aus C₁₋₆-Alkyl, Halogen-C₁₋₆-alkyl, Hydroxyl-C₁₋₆-alkyl, Amino-C₁₋₆-alkyl, -C(O)-C₁₋₆-Alkyl, C₁₋₆-Alkylamino, (C₁₋₆-Alkyl)₂-amino, C₁₋₆-Alkyl-S-, C₁₋₆-Alkyl-SO₂, C₁₋₆-Alkoxy, Halogen-C₁₋₆-alkoxy, C₁₋₆-Alkoxycarbonyl, Carboxyl, Hydroxyl, Cyano, Amino, Halogen, Acyl, Carbamoyl und einem 5- bis 8-gliedrigen monocyclischen Heterocyclyl, das ein bis drei Heteroatome, ausgewählt aus N, O und S, enthält, wobei das Heterocyclyl ferner durch Halogen, C₁₋₆-Alkyl, C₁₋₆-Alkoxy oder Amino substituiert sein kann;
Ring B ein 5- bis 8-gliedriger monocyclischer oder bicyclischer Heteroarylring oder ein Heterocyclylring ist, der ein bis vier Heteroatome enthält, ausgewählt aus N, O und S,
R⁴ abwesend ist oder ausgewählt ist aus C₁₋₆-Alkyl, Halogen-C₁₋₆-alkyl, Hydroxyl-C₁₋₆-alkyl, Amino-C₁₋₆-alkyl, -C(O)-C₁₋₆-Alkyl, C₁₋₆-Alkylamino, (C₁₋₆-Alkyl)₂-amino, C₁₋₆-Alkyl-S-, C₁₋₆-Alkyl-SO₂, C₁₋₆-Alkoxy, Halogen-C₁₋₆-alkoxy, C₁₋₆-Alkoxycarbonyl, Carboxyl, Hydroxyl, Cyano, Amino, Halogen und Carbamoyl;
Bindung a eine Bindung ist, die Ring A und Ring B verbindet;
Ring C ein 5- bis 8-gliedriger monocyclischer oder bicyclischer Heteroarylring oder
Heterocyclylring ist, der ein bis vier Heteroatome enthält, ausgewählt aus N, O und S, R⁵, R⁶ und R⁷ jeweils unabhängig abwesend sind oder ausgewählt sind aus C₁₋₆-Alkyl, Halogen-C₁₋₆-alkyl, Hydroxyl-C₁₋₆-alkyl, Amino-C₁₋₆-alkyl, -C(O)-C₁₋₆-Alkyl, C₁₋₆-Alkylamino, (C₁₋₆-Alkyl)₂-amino, C₁₋₆-Alkyl-S-, C₁₋₆-Alkyl-SO₂, C₁₋₆-Alkoxy, C₁₋₆-Alkoxy-C₁₋₆-alkyl, Halogen-C₁₋₆-alkoxy, C₁₋₆-Alkoxycarbonyl, Halogen-C₂₋₆-alkenyl, C₃₋₇-Cycloalkyl, C₃₋₇-Cycloalkyl-C₁₋₆-alkyl, C₂₋₆-Alkinyl, C₁₋₆-Alkoxy-C₂₋₆-alkinyl, Carboxyl, Hydroxyl, Cyano, Amino, Halogen, Carbamoyl, Cyano-C₁₋₆-alkyl, Imidazolyl-C₁₋₆-alkyl, Triazolyl-C₁₋₆-alkyl, Pyridinyl-C₁₋₆-alkyl, Thiazolyl-C₁₋₆-alkyl und Phenyl-C₁₋₆-alkyl, wobei das Phenyl-C₁₋₆-alkyl gegebenenfalls durch Halogen, Cyano, Hydroxyl-C₁₋₆-alkyl, Morpholino-C₁₋₆-alkyl, C₁₋₆-Alkoxy, C₁₋₆-Alkoxy-C₁₋₆-alkyl und C₁₋₆-Alkoxycarbonyl substituiert ist, oder
zwei von R⁵, R⁶ und R⁷ zusammen mit den Atomen, an die sie gebunden sind, einen 5- bis 6- gliedrigen Ring mit 0 bis 2 Heteroatomen bilden, ausgewählt aus N, O und S;
oder ein pharmazeutisch verträgliches Salz davon.

2. Verbindung nach Anspruch 1, wobei
der substituierte Ring A ausgewählt ist aus 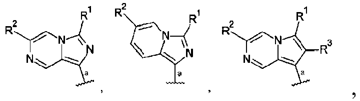 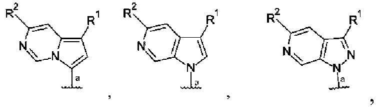
R¹ Carbamoyl ist,
R² ausgewählt ist aus C₁₋₆-Alkyl, Halogen-C₁₋₆-alkyl, Halogen, Acyl und einem 5- bis 8- gliedrigen monocyclischen Heterocyclyl, das ein bis drei Heteroatome enthält, ausgewählt aus N, O und S,
R³ abwesend oder Halogen ist;
Ring B ausgewählt ist aus 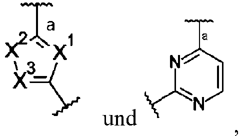
jedes von X¹, X² und X³ unabhängig ausgewählt ist aus C, N und O,
R⁴ abwesend ist oder ausgewählt ist aus C₁₋₆-Alkyl, Halogen-C₁₋₆-alkyl, Amino-C₁₋₆-alkyl,
Hydroxy-C₁₋₆-alkyl und Carbamoyl;
Ring C Pyrazolyl ist,
R⁵ ausgewählt ist aus C₁₋₆-Alkyl, Hydroxyl-C₁₋₆-alkyl, Halogen-C₁₋₆-alkyl, Cyano-C₁₋₆-alkyl, Halogen-C₂₋₆-alkenyl, C₃₋₇-Cycloalkyl, C₃₋₇-Cycloalkyl-C₁₋₆-alkyl, C₁₋₆-Alkoxy-C₁₋₆-alkyl, C₂₋₆-Alkinyl, C₁₋₆-Alkoxy-C₂₋₆-alkinyl, Imidazolyl-C₁₋₆-alkyl, Triazolyl-C₁₋₆-alkyl, Pyridinyl-C₁₋₆-alkyl, Thiazolyl-C₁₋₆-alkyl und Phenyl-C₁₋₆-alkyl, wobei das Phenyl-C₁₋₆-alkyl gegebenenfalls substituiert ist mit Halogen, Cyano, Hydroxyl-C₁₋₆-alkyl, Morpholino-C₁₋₆-alkyl, C₁₋₆-Alkoxy, C₁₋₆-Alkoxy-C₁₋₆-alkyl und C₁₋₆-Alkoxycarbonyl,
R⁶ abwesend oder C₁₋₆-Alkyl ist, R⁷ abwesend oder ausgewählt ist aus Hydroxyl, Halogen und Amino oder R⁶ und R⁷ zusammen mit den Atomen, an die sie gebunden sind, einen 5- bis 6-gliedrigen Ring bilden, der null bis zwei Heteroatome enthält, ausgewählt aus N, O und S.

3. Verbindung nach einem der Ansprüche 1 oder 2, wobei
der substituierte Ring A ausgewählt ist aus 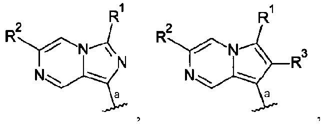 und 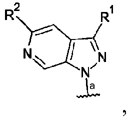
R¹ Carbamoyl ist,
R² C₁₋₆-Alkyl ist,
R³ abwesend oder Halogen ist.

4. Verbindung nach einem der Ansprüche 1 oder 2, wobei
R¹ Carbamoyl ist;
R² ausgewählt ist aus Methyl, Difluormethyl, Acetyl, Chlor und Imidazolyl;
R³ abwesend oder Fluor ist.

5. Verbindung nach einem der Ansprüche 1-4, wobei
R¹ Carbamoyl ist;
R² Methyl ist;
R³ abwesend oder Fluor ist.

6. Verbindung nach einem der Ansprüche 1-5, wobei
Ring B ausgewählt ist aus Triazolyl, Oxazolyl, Oxadiazolyl und Pyrimidinyl.

7. Verbindung nach einem der Ansprüche 1-6, wobei
der ubstituierte Ring B ausgewählt ist aus

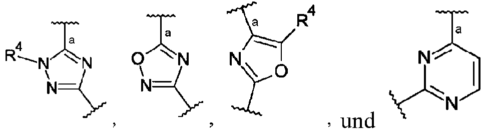

8. Verbindung nach einem der Ansprüche 1-7, wobei
der substituierte Ring B ausgewählt ist aus 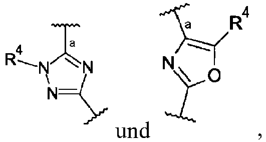
R⁴ abwesend ist oder ausgewählt ist aus C₁₋₆-Alkyl und Hydroxy-C₁₋₆-alkyl.

9. Verbindung nach einem der Ansprüche 1-7, wobei
R⁴ abwesend ist oder ausgewählt ist aus Methyl, Difluormethyl, Aminomethyl, Hydroxymethyl und Carbamoyl.

10. Verbindung nach einem der Ansprüche 1-9, wobei
R⁵ ausgewählt ist aus Ethyl, Propyl, Butyl, Cyclopropyl, Cyclopropylmethyl, 3,3-Difluorpropyl, 3,3-Difluorallyl, 2-Cyanoethyl, 3-Cyanopropyl, 3-Hydroxypropyl, 2-Imidazol-1-ylethyl, 2-(Triazol-2-yl)ethyl, 2-(Triazol-1-yl)ethyl, 2-(1,2,4-Triazol-4-yl)ethyl, 2-(1,2,4-Triazol-1-yl)ethyl, 3-Fluorpropyl, 2-Methoxyethyl, 3-Methoxypropyl, 1-Pent-3-inyl, 5-Methoxypent-3-inyl, 2-Phenylethyl, 2-(4-Methoxyphenyl)ethyl, 2-(3-Methoxyphenyl)ethyl, 2-[4-(Methoxymethyl)phenyl]ethyl, 2-[3-(Methoxymethyl)phenyl]ethyl, 2-(4-Fluorphenyl)ethyl, 2-(3-Fluorphenyl)ethyl, 2-(3-Cyanophenyl)ethyl, 2-(4-Cyanophenyl)ethyl, 2-(4-Methoxycarbonylphenyl)ethyl, 2-[4-(Hydroxymethyl)phenyl]ethyl, 2-Pyridin-4-ylethyl, 2-(3-Pyridyl)ethyl, 2-[4-(Morpholinomethyl)phenyl]ethyl und 2-Thiazol-2-ylethyl,
R⁶ abwesend oder Methyl ist, R⁷ abwesend, Fluor, Amino oder Hydroxyl ist oder R⁶ und R⁷ zusammen mit den Atomen, an die sie gebunden sind, einen 5-gliedrigen Ring bilden.

11. Verbindung nach Anspruch 1, wobei
der substituierte Ring A ausgewählt ist aus 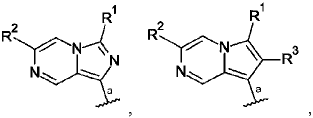 und 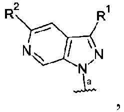
R¹ Carbamoyl ist,
R² C₁₋₆-Alkyl ist,
R³ abwesend oder Halogen ist;
der substituierte Ring B ausgewählt ist aus 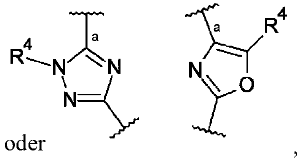
R⁴ abwesend ist oder ausgewählt ist aus C₁₋₆-Alkyl und Hydroxy-C₁₋₆-alkyl;
der substituierte Ring C 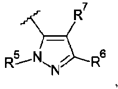 ist,
R⁵ ausgewählt ist aus C₁₋₆-Alkyl, Halogen-C₁₋₆-alkyl, Halogen-C₂₋₆-alkenyl, C₂₋₆-Alkinyl, C₃₋₇-Cycloalkyl-C₁₋₆-alkyl, Pyridinyl-C₁₋₆-alkyl, Phenyl-C₁₋₆-alkyl, wobei das Phenyl-C₁₋₆-alkyl gegebenenfalls substituiert ist mit Halogen, C₁₋₆-Alkoxy, C₁₋₆-Alkoxy-C₁₋₆-alkyl, Hydroxyl-C₁₋₆-alkyl oder Morpholino-C₁₋₆-alkyl,
R⁶ C₁₋₆-Alkyl ist,
R⁷ Hydroxyl ist.

12. Verbindung nach Anspruch 1, ausgewählt aus:
1-[3-(2-Ethyl-4-hydroxy-5-methyl-pyrazol-3-yl)-1*H*-1,2,4-triazol-5-yl]-6-methyl-imidazo[1,5-a]pyrazin-3-carboxamid;
1-[5-(2-Ethyl-4-hydroxy-5-methyl-pyrazol-3-yl)-2-methyl-1,2,4-triazol-3-yl]-6-methyl-imidazo[1,5-a]pyrazin-3-carboxamid;
8-[3-(2-Ethyl-4-hydroxy-5-methyl-pyrazol-3-yl)-1*H*-1,2,4-triazol-5-yl]-3-methyl-pyrrolo[1,2-a]pyrazin-6-carboxamid;
8-[3-(2-Ethyl-4-hydroxy-5-methyl-pyrazol-3-yl)-1*H*-1,2,4-triazol-5-yl]-7-fluor-3-methyl-pyrrolo[1,2-a]pyrazin-6-carboxamid;
4-Acetyl-6-[3-(2-ethyl-4-hydroxy-5-methyl-pyrazol-3-yl)-1*H*-1,2,4-triazol-5-yl]pyridin-2-carboxamid;
6-[3-(2-Ethyl-4-hydroxy-5-methyl-pyrazol-3-yl)-1*H*-1,2,4-triazol-5-yl]-4-imidazol-1-yl-pyridin-2-carboxamid;
4-[2-(2-Ethyl-4-hydroxy-5-methyl-pyrazol-3-yl)pyrimidin-4-yl]-1-methyl-pyrazolo-[4,3-c]pyridin-6-carboxamid;
1-[2-(2-Ethyl-4-hydroxy-5-methyl-pyrazol-3-yl)pyrimidin-4-yl]-6-methyl-imidazo-[1,5-a]-pyrazin-3-carboxamid;
1-[2-(2-Ethyl-4-hydroxy-5-methyl-pyrazol-3-yl)oxazol-4-yl]-6-methyl-imidazo[1,5-a]-pyrazin-3-carboxamid;
4-[5-(Difluormethyl)-2-(2-ethyl-4-hydroxy-5-methyl-pyrazol-3-yl)oxazol-4-yl]-1-methyl-pyrazolo[4,3-c]pyridin-6-carboxamid;
1-[5-(Aminomethyl)-2-(2-ethyl-4-hydroxy-5-methyl-pyrazol-3-yl)oxazol-4-yl]-6-methyl-imidazo[1,5-a]pyrazin-3-carboxamid;
4-(6-Carbamoyl-1-methyl-pyrazolo[4,3-c]pyridin-4-yl)-2-(2-ethyl-4-hydroxy-5-methyl-pyrazol-3-yl)oxazol-5-carboxamid;
4-[3-(2-Ethyl-5,6-dihydro-4*H*-cyclopenta[c]pyrazol-3-yl)-1*H*-1,2,4-triazol-5-yl]-1-methyl-pyrazolo[4,3-c]pyridin-6-carboxamid;
1-[3-[2-(3-Hydroxypropyl)-5-methyl-pyrazol-3-yl]-1*H*-1,2,4-triazol-5-yl]-6-methyl-imidazo[1,5-a]pyrazin-3-carboxamid;
1-[3-(2-Ethyl-5-methyl-pyrazol-3-yl)-1*H*-1,2,4-triazol-5-yl]-6-methyl-imidazo[1,5-a]-pyrazin-3-carboxamid;
1-[3-(2-Ethyl-4-fluor-5-methyl-pyrazol-3-yl)-1*H*-1,2,4-triazol-5-yl]-6-methyl-imidazo[1,5-a]pyrazin-3-carboxamid;
1-[3-(4-Amino-2-ethyl-5-methyl-pyrazol-3-yl)-1*H*-1,2,4-triazol-5-yl]-6-methyl-imidazo[1,5-a]pyrazin-3-carboxamid;
6-Chlor-1-[3-(2-ethyl-4-hydroxy-5-methyl-pyrazol-3-yl)-1*H*-1,2,4-triazol-5-yl]-imidazo[1,5-a]pyrazin-3-carboxamid;
1-[3-(2-Ethyl-4-hydroxy-5-methyl-pyrazol-3-yl)-1*H*-1,2,4-triazol-5-yl]-6-methyl-imidazo[1,5-a]pyridin-3-carboxamid;
7-[3-(2-Ethyl-4-hydroxy-5-methyl-pyrazol-3-yl)-1*H*-1,2,4-triazol-5-yl]-3-methyl-pyrrolo[1,2-c]pyrimidin-5-carboxamid;
1-[2-(2-Ethyl-4-hydroxy-5-methyl-pyrazol-3-yl)oxazol-4-yl]-5-methyl-pyrazolo-[3,4-c]pyridin-3-carboxamid;
1-[3-(2-Ethyl-4-hydroxy-5-methyl-pyrazol-3-yl)-1*H*-1,2,4-triazol-5-yl]-5-methyl-pyrazolo[3,4-c]pyridin-3-carboxamid;
4-[3-[4-Hydroxy-2-(2-imidazol-1-ylethyl)-5-methyl-pyrazol-3-yl]-1*H*-1,2,4-triazol-5-yl]-1-methyl-pyrazolo[4,3-c]pyridin-6-carboxamid;
4-[3-[2-(3-Cyanopropyl)-4-hydroxy-5-methyl-pyrazol-3-yl]-1*H*-1,2,4-triazol-5-yl]-1-methyl-pyrazolo[4,3-c]pyridin-6-carboxamid;
4-[3-[2-(2-Cyanoethyl)-4-hydroxy-5-methyl-pyrazol-3-yl]-1*H*-1,2,4-triazol-5-yl]-1-methyl-pyrazolo[4,3-c]pyridin-6-carboxamid;
6-(Difluormethyl)-1-[3-(2-ethyl-4-hydroxy-5-methyl-pyrazol-3-yl)-1*H*-1,2,4-triazol-5-yl]imidazo[1,5-a]pyrazin-3-carboxamid;
7-[3-(2-Ethyl-4-hydroxy-5-methyl-pyrazol-3-yl)-1,2,4-oxadiazol-5-yl]-3-methyl-pyrrolo[1,2-c]pyrimidin-5-carboxamid;
8-[2-(2-Ethyl-4-hydroxy-5-methyl-pyrazol-3-yl)oxazol-4-yl]-7-fluor-3-methyl-pyrrolo[1,2-a]pyrazin-6-carboxamid;
1-[2-(2-Ethyl-4-hydroxy-5-methyl-pyrazol-3-yl)oxazol-4-yl]-5-methyl-pyrrolo-[2,3-c]pyridin-3-carboxamid;
8-[3-[2-(2-Cyanoethyl)-4-hydroxy-5-methyl-pyrazol-3-yl]-1*H*-1,2,4-triazol-5-yl]-7-fluor-3-methyl-pyrrolo[1,2-a]pyrazin-6-carboxamid;
1-[3-(4-Hydroxy-5-methyl-2-propyl-pyrazol-3-yl)-1*H*-1,2,4-triazol-5-yl]-5-methyl-pyrazolo[3,4-c]pyridin-3-carboxamid;
1-[3-[2-(Cyclopropylmethyl)-4-hydroxy-5-methyl-pyrazol-3-yl]-1*H*-1,2,4-triazol-5-yl]-5-methyl-pyrazolo[3,4-c]pyridin-3-carboxamid;
1-[3-(2-Cyclopropyl-4-hydroxy-5-methyl-pyrazol-3-yl)-1*H*-1,2,4-triazol-5-yl]-5-methyl-pyrazolo[3,4-c]pyridin-3-carboxamid;
4-[3-[2-(3,3-Difluorallyl)-4-hydroxy-5-methyl-pyrazol-3-yl]-1*H*-1,2,4-triazol-5-yl]-1-methyl-pyrazolo[4,3-c]pyridin-6-carboxamid;
4-[3-[4-Hydroxy-5-methyl-2-[2-(triazol-2-yl)ethyl]pyrazol-3-yl]-1*H*-1,2,4-triazol-5-yl]-1-methyl-pyrazolo[4,3-c]pyridin-6-carboxamid;
4-[3-[4-Hydroxy-5-methyl-2-[2-(triazol-1-yl)ethyl]pyrazol-3-yl]-1*H*-1,2,4-triazol-5-yl]-1-methyl-pyrazolo[4,3-c]pyridin-6-carboxamid;
4-[3-[2-(3,3-Difluorpropyl)-4-hydroxy-5-methyl-pyrazol-3-yl]-1*H*-1,2,4-triazol-5-yl]-1-methyl-pyrazolo[4,3-c]pyridin-6-carboxamid;
4-[3-[4-Hydroxy-5-methyl-2-[2-(1,2,4-triazol-4-yl)ethyl]pyrazol-3-yl]-1*H*-1,2,4-triazol-5-yl]-1-methyl-pyrazolo[4,3-c]pyridin-6-carboxamid;
4-[3-[4-Hydroxy-5-methyl-2-[2-(1,2,4-triazol-1-yl)ethyl]pyrazol-3-yl]-1*H*-1,2,4-triazol-5-yl]-1-methyl-pyrazolo[4,3-c]pyridin-6-carboxamid;
1-[3-[4-Hydroxy-5-methyl-2-(2-phenylethyl)pyrazol-3-yl]-1*H*-1,2,4-triazol-5-yl]-5-methyl-pyrazolo[3,4-c]pyridin-3-carboxamid;
1-[3-[2-(3-Fluorpropyl)-4-hydroxy-5-methyl-pyrazol-3-yl]-1*H*-1,2,4-triazol-5-yl]-5-methyl-pyrazolo[3,4-c]pyridin-3-carboxamid;
1-[3-[4-Hydroxy-2-(2-methoxyethyl)-5-methyl-pyrazol-3-yl]-1*H*-1,2,4-triazol-5-yl]-5-methyl-pyrazolo[3,4-c]pyridin-3-carboxamid;
1-[3-(2-Butyl-4-hydroxy-5-methyl-pyrazol-3-yl)-1*H*-1,2,4-triazol-5-yl]-5-methyl-pyrazolo[3,4-c]pyridin-3-carboxamid;
1-[3-[4-Hydroxy-2-(3-methoxypropyl)-5-methyl-pyrazol-3-yl]-1*H*-1,2,4-triazol-5-yl]-5-methyl-pyrazolo[3,4-c]pyridin-3-carboxamid;
4-[3-(4-Hydroxy-5-methyl-2-pent-3-inyl-pyrazol-3-yl)-1*H*-1,2,4-triazol-5-yl]-1-methyl-pyrazolo[4,3-c]pyridin-6-carboxamid;
1-[3-[4-Hydroxy-5-methyl-2-[2-(4-pyridyl)ethyl]pyrazol-3-yl]-1*H*-1,2,4-triazol-5-yl]-5-methyl-pyrazolo[3,4-c]pyridin-3-carboxamid;
1-[3-[4-Hydroxy-5-methyl-2-[2-(3-pyridyl)ethyl]pyrazol-3-yl]-1*H*-1,2,4-triazol-5-yl]-5-methyl-pyrazolo[3,4-c]pyridin-3-carboxamid;
1-[5-[4-Hydroxy-5-methyl-2-(2-phenylethyl)pyrazol-3-yl]-2-methyl-1,2,4-triazol-3-yl]-5-methyl-pyrazolo[3,4-c]pyridin-3-carboxamid;
1-[5-(2-Butyl-4-hydroxy-5-methyl-pyrazol-3-yl)-2-methyl-1,2,4-triazol-3-yl]-5-methyl-pyrazolo[3,4-c]pyridin-3-carboxamid;
1-[3-[4-Hydroxy-2-[2-(4-methoxyphenyl)ethyl]-5-methyl-pyrazol-3-yl]-1*H*-1,2,4-triazol-5-yl]-5-methyl-pyrazolo[3,4-c]pyridin-3-carboxamid;
1-[3-(4-Hydroxy-5-methyl-2-pent-3-inyl-pyrazol-3-yl)-1*H*-1,2,4-triazol-5-yl]-5-methyl-pyrazolo[3,4-c]pyridin-3-carboxamid;
1-[3-[2-[2-(4-Fluorphenyl)ethyl]-4-hydroxy-5-methyl-pyrazol-3-yl]-1*H*-1,2,4-triazol-5-yl]-5-methyl-pyrazolo[3,4-c]pyridin-3-carboxamid;
1-[3-[4-Hydroxy-5-methyl-2-(2-phenylethyl)pyrazol-3-yl]-1*H*-1,2,4-triazol-5-yl]-6-methyl-imidazo[1,5-a]pyrazin-3-carboxamid;
1-[3-[4-Hydroxy-2-[2-(3-methoxyphenyl)ethyl]-5-methyl-pyrazol-3-yl]-1*H*-1,2,4-triazol-5-yl]-5-methyl-pyrazolo[3,4-c]pyridin-3-carboxamid;
1-(3-(1-(3-Fluorphenethyl)-4-hydroxy-3-methyl-1*H*-pyrazol-5-yl)-1*H*-1,2,4-triazol-5-yl)-5-methyl-1*H*-pyrazolo[3,4-c]pyridin-3-carboxamid;
1-[3-[2-[2-(2-Fluorphenyl)ethyl]-4-hydroxy-5-methyl-pyrazol-3-yl]-1*H*-1,2,4-triazol-5-yl]-5-methyl-pyrazolo[3,4-c]pyridin-3-carboxamid;
7-Fluor-8-[3-[4-hydroxy-5-methyl-2-(2-phenylethyl)pyrazol-3-yl]-1*H*-1,2,4-triazol-5-yl]-3-methyl-pyrrolo[1,2-a]pyrazin-6-carboxamid;
1-(2-(4-Hydroxy-3-methyl-1-propyl-1*H*-pyrazol-5-yl)oxazol-4-yl)-5-methyl-1*H-*pyrazolo[3,4-c]pyridin-3-carboxamid;
1-(2-(1-Butyl-4-hydroxy-3-methyl-1*H*-pyrazol-5-yl)oxazol-4-yl)-5-methyl-1*H-*pyrazolo[3,4-c]pyridin-3-carboxamid;
1-[3-[2-[2-(3-Cyanophenyl)ethyl]-4-hydroxy-5-methyl-pyrazol-3-yl]-1*H*-1,2,4-triazol-5-yl]-5-methyl-pyrazolo[3,4-c]pyridin-3-carboxamid;
1-[3-[2-[2-(4-Cyanophenyl)ethyl]-4-hydroxy-5-methyl-pyrazol-3-yl]-1*H*-1,2,4-triazol-5-yl]-5-methyl-pyrazolo[3,4-c]pyridin-3-carboxamid;
Methyl-4-[2-[5-[5-(3-carbamoyl-5-methyl-pyrazolo[3,4-c]pyridin-1-yl)-1*H*-1,2,4-triazol-3-yl]-4-hydroxy-3-methyl-pyrazol-1-yl]ethyl]benzoat;
1-(3-(1-(3,3-Difluorallyl)-4-hydroxy-3-methyl-1*H*-pyrazol-5-yl)-1*H*-1,2,4-triazol-5-yl)-5-methyl-1*H*-pyrazolo[3,4-c]pyridin-3-carboxamid;
1-[3-[4-Hydroxy-2-[2-[4-(methoxymethyl)phenyl]ethyl]-5-methyl-pyrazol-3-yl]-1*H-*1,2,4-triazol-5-yl]-5-methyl-pyrazolo[3,4-c]pyridin-3-carboxamid;
1-[3-[4-Hydroxy-2-[2-[3-(methoxymethyl)phenyl]ethyl]-5-methyl-pyrazol-3-yl]-1*H-*1,2,4-triazol-5-yl]-5-methyl-pyrazolo[3,4-c]pyridin-3-carboxamid;
1-(3-(1-(3,3-Difluorpropyl)-4-hydroxy-3-methyl-1*H*-pyrazol-5-yl)-1*H*-1,2,4-triazol-5-yl)-5-methyl-1*H*-pyrazolo[3,4-c]pyridin-3-carboxamid;
1-(2-(1-(3,3-Difluorpropyl)-4-hydroxy-3-methyl-1*H*-pyrazol-5-yl)oxazol-4-yl)-5-methyl-1*H*-pyrazolo[3,4-c]pyridin-3-carboxamid;
1-[3-[4-Hydroxy-2-[2-[4-(hydroxymethyl)phenyl]ethyl]-5-methyl-pyrazol-3-yl]-1*H-*1,2,4-triazol-5-yl]-5-methyl-pyrazolo[3,4-c]pyridin-3-carboxamid;
7-Fluor-8-[3-[4-hydroxy-2-[2-(4-methoxyphenyl)ethyl]-5-methyl-pyrazol-3-yl]-1*H-*1,2,4-triazol-5-yl]-3-methyl-pyrrolo[1,2-a]pyrazin-6-carboxamid;
1-[3-[4-Hydroxy-2-[2-(4-methoxyphenyl)ethyl]-5-methyl-pyrazol-3-yl]-1*H*-1,2,4-triazol-5-yl]-6-methyl-imidazo[1,5-a]pyrazin-3-carboxamid;
1-[3-[4-Hydroxy-2-[2-[4-(hydroxymethyl)phenyl]ethyl]-5-methyl-pyrazol-3-yl]-1*H-*1,2,4-triazol-5-yl]-6-methyl-imidazo[1,5-a]pyrazin-3-carboxamid;
1-[3-[4-Hydroxy-5-methyl-2-[2-(3-pyridyl)ethyl]pyrazol-3-yl]-1*H*-1,2,4-triazol-5-yl]-6-methyl-imidazo[1,5-a]pyrazin-3-carboxamid;
7-Fluor-8-[3-[4-hydroxy-5-methyl-2-[2-(3-pyridyl)ethyl]pyrazol-3-yl]-1*H*-1,2,4-triazol-5-yl]-3-methyl-pyrrolo[1,2-a]pyrazin-6-carboxamid;
1-(2-(4-Hydroxy-3-methyl-1-(2-(pyridin-3-yl)ethyl)-1*H*-pyrazol-5-yl)oxazol-4-yl)-5-methyl-1*H*-pyrazolo[3,4-c]pyridin-3-carboxamid;
1-(2-(4-Hydroxy-1-(3-methoxyphenethyl)-3-methyl-1*H*-pyrazol-5-yl)oxazol-4-yl)-5-methyl-1*H*-pyrazolo[3,4-c]pyridin-3-carboxamid;
8-[3-(2-Butyl-4-hydroxy-5-methyl-pyrazol-3-yl)-1*H*-1,2,4-triazol-5-yl]-7-fluor-3-methyl-pyrrolo[1,2-a]pyrazin-6-carboxamid;
1-[3-(2-Butyl-4-hydroxy-5-methyl-pyrazol-3-yl)-1*H*-1,2,4-triazol-5-yl]-6-methyl-imidazo[1,5-a]pyrazin-3-carboxamid;
1-(2-(4-Hydroxy-1-(4-methoxyphenethyl)-3-methyl-1*H*-pyrazol-5-yl)oxazol-4-yl)-5-methyl-1*H*-pyrazolo[3,4-c]pyridin-3-carboxamid;
7-Fluor-8-[3-(4-hydroxy-5-methyl-2-propyl-pyrazol-3-yl)-1*H*-1,2,4-triazol-5-yl]-3-methyl-pyrrolo[1,2-a]pyrazin-6-carboxamid;
1-[3-(4-Hydroxy-5-methyl-2-propyl-pyrazol-3-yl)-1*H*-1,2,4-triazol-5-yl]-6-methyl-imidazo[1,5-a]pyrazin-3-carboxamid;
1-[3-[4-Hydroxy-2-(5-methoxypent-3-inyl)-5-methyl-pyrazol-3-yl]-1*H*-1,2,4-triazol-5-yl]-5-methyl-pyrazolo[3,4-c]pyridin-3-carboxamid;
4-(5-(4-Hydroxy-3-methyl-1-phenethyl-1*H*-pyrazol-5-yl)-1*H*-1,2,4-triazol-3-yl)-1-methyl-1*H*-pyrazolo[4,3-c]pyridin-6-carboxamid;
1-[5-(Hydroxymethyl)-2-(4-hydroxy-5-methyl-2-propyl-pyrazol-3-yl)oxazol-4-yl]-5-methyl-pyrazolo[3,4-c]pyridin-3-carboxamid;
1-[5-(4-Hydroxy-5-methyl-2-propyl-pyrazol-3-yl)-2-methyl-1,2,4-triazol-3-yl]-5-methyl-pyrazolo[3,4-c]pyridin-3-carboxamid;
1-[2-(4-Hydroxy-5-methyl-2-propyl-pyrazol-3-yl)oxazol-4-yl]-6-methyl-imidazo-[1,5-a]pyrazin-3-carboxamid;
1-[2-(2-Butyl-4-hydroxy-5-methyl-pyrazol-3-yl)oxazol-4-yl]-6-methyl-imidazo[1,5-a]-pyrazin-3-carboxamid;
1-[3-[4-Hydroxy-5-methyl-2-[2-[4-(morpholinomethyl)phenyl]ethyl]pyrazol-3-yl]-1*H-*1,2,4-triazol-5-yl]-6-methyl-imidazo[1,5-a]pyrazin-3-carboxamid;
1-[2-[4-Hydroxy-5-methyl-2-[2-[4-(morpholinomethyl)phenyl]ethyl]pyrazol-3-yl]oxazol-4-yl]-5-methyl-pyrazolo[3,4-c]pyridin-3-carboxamid;
1-[2-[4-Hydroxy-2-[2-[4-(methoxymethyl)phenyl]ethyl]-5-methyl-pyrazol-3-yl]oxazol-4-yl]-5-methyl-pyrazolo[3,4-c]pyridin-3-carboxamid;
1-[3-[4-Hydroxy-5-methyl-2-(2-thiazol-2-ylethyl)pyrazol-3-yl]-1*H*-1,2,4-triazol-5-yl]-6-methyl-imidazo[1,5-a]pyrazin-3-carboxamid;
8-[3-(4-Hydroxy-5-methyl-2-propyl-pyrazol-3-yl)-1*H*-1,2,4-triazol-5-yl]-3-methyl-pyrrolo[1,2-a]pyrazin-6-carboxamid;
1-[5-(4-Hydroxy-5-methyl-2-propyl-pyrazol-3-yl)-2-methyl-1,2,4-triazol-3-yl]-6-methyl-imidazo[1,5-a]pyrazin-3-carboxamid;
7-Fluor-8-[2-(4-hydroxy-5-methyl-2-propyl-pyrazol-3-yl)oxazol-4-yl]-3-methyl-pyrrolo[1,2-a]pyrazin-6-carboxamid;
8-[2-(4-Hydroxy-5-methyl-2-propyl-pyrazol-3-yl)oxazol-4-yl]-3-methyl-pyrrolo[1,2-a]-pyrazin-6-carboxamid;
oder ein pharmazeutisch verträgliches Salz davon.

13. Pharmazeutische Zusammensetzung, umfassend eine Verbindung oder ein pharmazeutisch verträgliches Salz davon nach einem der Ansprüche 1 bis 12 und einen pharmazeutisch verträglichen Exzipienten.

14. Verbindung oder ein pharmazeutisch verträgliches Salz davon nach einem der Ansprüche 1 bis 12 zur Verwendung als therapeutisch wirksame Substanz.

15. Verbindung oder ein pharmazeutisch verträgliches Salz davon nach einem der Ansprüche 1 bis 12 zur Verwendung bei der Behandlung von Krebs.

## Revendications

1. Composé de formule (I), 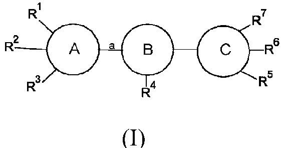 dans lequel
le cycle A substitué est choisi parmi 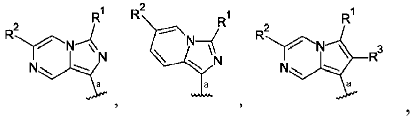 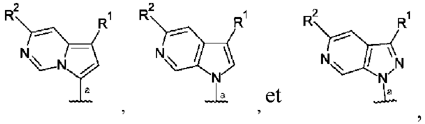
chacun parmi R¹, R² et R³ est indépendamment absent ou choisi parmi un alkyle à C₁₋₆, un halogénoalkyle en C₁₋₆, un hydroxylalkyle en C₁₋₆, un aminoalkyle en C₁₋₆, un -C(O)alkyle en C₁₋₆, un alkyle en C₁₋₆-amino, un (alkyle en C₁₋₆)₂amino, un alkyle en C₁₋₆-S-, un alkyle en C₁₋₆-SO₂, un alcoxy en C₁₋₆, un halogénoalcoxy en C₁₋₆, un alcoxy en C₁₋₆-carbonyle, un carboxyle, un hydroxyle, un cyano, un amino, un halogène, un acyle, un carbamoyle et un hétérocyclyle monocyclique à 5 à 8 chaînons contenant un à trois hétéroatomes choisis parmi N, O et S, dans lequel ledit hétérocyclyle peut être en outre substitué par un halogène, un alkyle en C₁₋₆, un alcoxy en C₁₋₆ ou un amino ;
le cycle B représente un cycle hétéroaryle ou un cycle hétérocyclyle monocyclique ou bicyclique à 5 à 8 chaînons contenant un à quatre hétéroatomes choisis parmi N, O et S,
R⁴ est absent ou choisi parmi un alkyle en C₁₋₆, un halogénoalkyle en C₁₋₆, un hydroxylalkyle en C₁₋₆, un aminoalkyle en C₁₋₆, un -C(O)alkyle en C₁₋₆, un alkyle en C₁₋₆-amino, un (alkyle en C₁₋₆)zamino, un alkyle en C₁₋₆-S-, un alkyle en C₁₋₆-SO₂, un alcoxy en C₁₋₆, un halogénoalcoxy en C₁₋₆, un alcoxy en C₁₋₆-carbonyle, un carboxyle, un hydroxyle, un cyano, un amino, un halogène et un carbamoyle ;
la liaison a est une liaison reliant le cycle A et le cycle B ;
le cycle C représente un cycle hétéroaryle ou un cycle hétérocyclyle monocyclique ou bicyclique à 5 à 8 chaînons contenant un à quatre hétéroatomes choisis parmi N, O et S, chacun parmi R⁵, R⁶ et R⁷ est indépendamment absent ou choisi parmi un alkyle en C₁₋₆, un halogénoalkyle en C₁₋₆, un hydroxylalkyle en C₁₋₆, un aminoalkyle en C₁₋₆, un -C(O)alkyle en C₁₋₆, un alkyle en C₁₋₆-amino, un (alkyle en C₁₋₆)₂amino, un alkyle en C₁₋₆-S-, un alkyle en C₁₋₆-SO₂, un alcoxy en C₁₋₆, un alcoxy en C₁₋₆-alkyle en C₁₋₆, un halogénoalcoxy en C₁₋₆, un alcoxy en C₁₋₆-carbonyle, un halogénoalcényle en C₂₋₆, un cycloalkyle en C₃₋₇, un cycloalkyle en C₃₋₇-alkyle en C₁₋₆, un alcynyle en C₂₋₆, un alcoxy en C₁₋₆-alcynyle en C₂₋₆, un carboxyle, un hydroxyle, un cyano, un amino, un halogène, un carbamoyle, un cyanoalkyle en C₁₋₆, un imidazolylalkyle en C₁₋₆, un triazolylalkyle en C₁₋₆, un pyridinylalkyle en C₁₋₆, un thiazolylalkyle en C₁₋₆ et un phénylalkyle en C₁₋₆, dans lequel le phénylalkyle en C₁₋₆ est éventuellement substitué par un halogène, un cyano, un hydroxylalkyle en C₁₋₆, un morpholinoalkyle en C₁₋₆, un alcoxy en C₁₋₆, un alcoxy en C₁₋₆-alkyle en C₁₋₆ et un alcoxy en C₁₋₆-carbonyle, ou
deux parmi R⁵, R⁶ et R⁷ conjointement avec les atomes auxquels ils sont liés forment un cycle à 5 à 6 chaînons ayant 0 à 2 hétéroatomes choisis parmi N, O et S ;
ou un sel pharmaceutiquement acceptable de celui-ci.

2. Composé selon la revendication 1, dans lequel
le cycle A substitué est choisi parmi 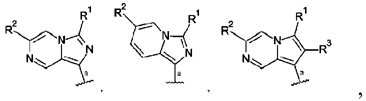 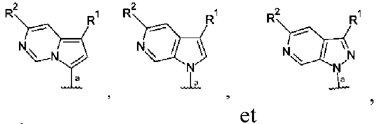
R¹ représente un carbamoyle,
R² est choisi parmi un alkyle en C₁₋₆, un halogénoalkyle en C₁₋₆, un halogène, un acyle et un hétérocyclyle monocyclique à 5 à 8 chaînons contenant un à trois hétéroatomes choisis parmi N, O et S,
R³ est absent ou représente un halogène ;
le cycle B est choisi parmi 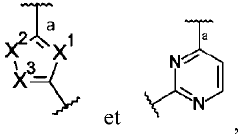
chacun parmi X¹, X² et X³ est indépendamment choisi parmi C, N et O,
R⁴ est absent ou choisi parmi un alkyle en C₁₋₆, un halogénoalkyle en C₁₋₆, un aminoalkyle en C₁₋₆, un hydroxyalkyle en C₁₋₆ et un carbamoyle ;
le cycle C représente un pyrazolyle,
R⁵ est choisi parmi un alkyle en C₁₋₆, un hydroxylalkyle en C₁₋₆, un halogénoalkyle en C₁₋₆, un cyanoalkyle en C₁₋₆, un halogénoalcényle en C₂₋₆, un cycloalkyle en C₃₋₇, un cycloalkyle en C₃₋₇-alkyle en C₁₋₆, un alcoxy en C₁₋₆-alkyle en C₁₋₆, un alcynyle en C₂₋₆, un alcoxy en C₁₋₆-alcynyle en C₂₋₆, un imidazolylalkyle en C₁₋₆, un triazolylalkyle en C₁₋₆, un pyridinylalkyle en C₁₋₆, un thiazolylalkyle en C₁₋₆ et un phénylalkyle en C₁₋₆, dans lequel le phénylalkyle en C₁₋₆ est éventuellement substitué par un halogène, un cyano, un hydroxylalkyle en C₁₋₆, un morpholinoalkyle en C₁₋₆, un alcoxy en C₁₋₆, un alcoxy en C₁₋₆-alkyle en C₁₋₆ et un alcoxy en C₁₋₆-carbonyle,
R⁶ est absent ou un alkyle en C₁₋₆, R⁷ est absent ou choisi parmi un hydroxyle, un halogène et un amino, ou R⁶ et R⁷ conjointement avec les atomes auxquels ils sont liés forment un cycle à 5 à 6 chaînons contenant zéro à deux hétéroatomes choisis parmi N, O et S.

3. Composé selon l'une quelconque des revendications 1 ou 2, dans lequel
le cycle A substitué est choisi parmi 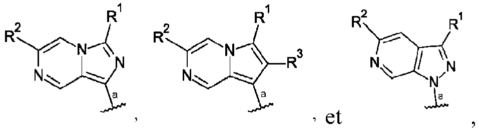
R¹ représente un carbamoyle,
R² représente un alkyle en C₁₋₆,
R³ est absent ou représente un halogène.

4. Composé selon l'une quelconque des revendications 1 ou 2, dans lequel
R¹ représente un carbamoyle ;
R² est choisi parmi un méthyle, un difluorométhyle, un acétyle, un chlore et un imidazolyle ;
R³ est absent ou représente un fluor.

5. Composé selon l'une quelconque des revendications 1 à 4, dans lequel
R¹ représente un carbamoyle ;
R² représente un méthyle ;
R³ est absent ou représente un fluor.

6. Composé selon l'une quelconque des revendications 1 à 5, dans lequel
le cycle B est choisi parmi un triazolyle, un oxazolyle, un oxadiazolyle et un pyrimidinyle.

7. Composé selon l'une quelconque des revendications 1 à 6, dans lequel
le cycle B substitué est choisi parmi

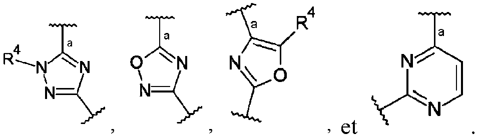

8. Composé selon l'une quelconque des revendications 1 à 7, dans lequel
le cycle B substitué est choisi parmi 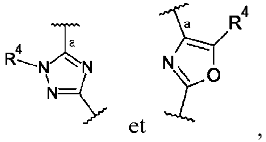
R⁴ est absent ou choisi parmi un alkyle en C₁₋₆ et un hydroxyalkyle en C₁₋₆.

9. Composé selon l'une quelconque des revendications 1 à 7, dans lequel
R⁴ est absent ou choisi parmi un méthyle, un difluorométhyle, un aminométhyle, un hydroxyméthyle et un carbamoyle.

10. Composé selon l'une quelconque des revendications 1 à 9, dans lequel
R⁵ est choisi parmi un éthyle, un propyle, un butyle, un cyclopropyle, un cyclopropylméthyle, un 3,3-difluoropropyle, un 3,3-difluoroallyle, un 2-cyanoéthyle, un 3-cyanopropyle, un 3-hydroxypropyle, un 2-imidazol-1-yléthyle, un 2-(triazol-2-yl)éthyle, un 2-(triazol-1-yl)éthyle, un 2-(1,2,4-triazol-4-yl)éthyle, un 2-(1,2,4-triazol-1-yl)éthyle, un 3-fluoro-propyle, un 2-méthoxyéthyle, un 3-méthoxypropyle, un 1-pent-3-ynyle, un 5-méthoxypent-3-ynyle, un 2-phényléthyle, un 2-(4-méthoxyphényl)éthyle, un 2-(3-méthoxyphényl)éthyle, un 2-[4-(méthoxyméthyl)phényl]éthyle, un 2-[3-(méthoxyméthyl)phényl]éthyle, un 2-(4-fluorophényl)éthyle, un 2-(3-fluorophényl)éthyle, un 2-(3-cyanophényl)éthyle, un 2-(4-cyanophényl)éthyle, un 2-(4-méthoxycarbonylphényl)-éthyle, un 2-[4-(hydroxyméthyl)phényl]éthyle, un 2-pyridin-4-yléthyle, un 2-(3-pyridyl)éthyle, un 2-[4-(morpholinométhyl)phényl]éthyle et un 2-thiazol-2-yléthyle,
R⁶ est absent ou représente un méthyle, R⁷ est absent ou représente un fluor, un amino ou un hydroxyle, ou R⁶ et R⁷ conjointement avec les atomes auxquels ils sont liés forment un cycle à 5 chaînons.

11. Composé selon la revendication 1, dans lequel
le cycle A substitué est choisi parmi 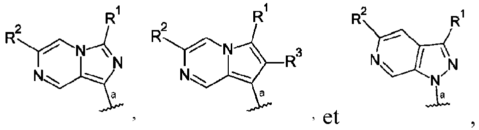
R¹ représente un carbamoyle,
R² représente un alkyle en C₁₋₆,
R³ est absent ou représente un halogène ;
le cycle B substitué est choisi parmi 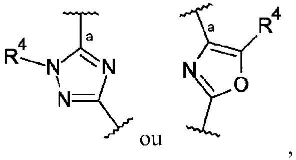
R⁴ est absent ou choisi parmi un alkyle en C₁₋₆ et un hydroxyalkyle en C₁₋₆ ;
le cycle C substitué représente 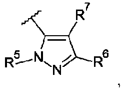
R⁵ est choisi parmi un alkyle en C₁₋₆, un halogénoalkyle en C₁₋₆, un halogénoalcényle en C₂₋₆, un alcényle en C₂₋₆, un cycloalkyle en C₃₋₇-alkyle en C₁₋₆, un pyridinylalkyle en C₁₋₆, un phénylalkyle en C₁₋₆, dans lequel le phénylalkyle en C₁₋₆ est éventuellement substitué par un halogène, un alcoxy en C₁₋₆, un alcoxy en C₁₋₆-alkyle en C₁₋₆, un hydroxylalkyle en C₁₋₆ ou un morpholinoalkyle en C₁₋₆,
R⁶ représente un alkyle en C₁₋₆,
R⁷ représente un hydroxyle.

12. Composé selon la revendication 1, choisi parmi :
le 1-[3-(2-éthyl-4-hydroxy-5-méthyl-pyrazol-3-yl)-1H-1,2,4-triazol-5-yl]-6-méthyl-imidazo[1,5-a]pyrazine-3-carboxamide ;
le 1-[5-(2-éthyl-4-hydroxy-5-méthyl-pyrazol-3-yl)-2-méthyl-1,2,4-triazol-3-yl]-6-méthyl-imidazo[1,5-a]pyrazine-3-carboxamide ;
le 8-[3-(2-éthyl-4-hydroxy-5-méthyl-pyrazol-3-yl)-1H-1,2,4-triazol-5-yl]-3-méthyl-pyrrolo[1,2-a]pyrazine-6-carboxamide ;
le 8-[3-(2-éthyl-4-hydroxy-5-méthyl-pyrazol-3-yl)-1*H*-1,2,4-triazol-5-yl]-7-fluoro-3-méthyl-pyrrolo[1,2-a]pyrazine-6-carboxamide ;
le 4-acétyl-6-[3-(2-éthyl-4-hydroxy-5-méthyl-pyrazol-3-yl)-1*H*-1,2,4-triazol-5-yl]pyridine-2-carboxamide ;
le 6-[3-(2-éthyl-4-hydroxy-5-méthyl-pyrazol-3-yl)-1*H*-1,2,4-triazol-5-yl]-4-imidazol-1-yl-pyridine-2-carboxamide ;
le 4-[2-(2-éthyl-4-hydroxy-5-méthyl-pyrazol-3-yl)pyrimidin-4-yl]-1-méthyl-pyrazolo[4,3-c]pyridine-6-carboxamide ;
le 1-[2-(2-éthyl-4-hydroxy-5-méthyl-pyrazol-3-yl)pyrimidin-4-yl]-6-méthyl-imidazo[1,5-a]pyrazine-3-carboxamide ;
le 1-[2-(2-éthyl-4-hydroxy-5-méthyl-pyrazol-3-yl)oxazol-4-yl]-6-méthyl-imidazo[1,5-a]pyrazine-3-carboxamide ;
le 4-[5-(difluorométhyl)-2-(2-éthyl-4-hydroxy-5-méthyl-pyrazol-3-yl)oxazol-4-yl]-1-méthyl-pyrazolo[4,3-c]pyridine-6-carboxamide ;
le 1-[5-(aminométhyl)-2-(2-éthyl-4-hydroxy-5-méthyl-pyrazol-3-yl)oxazol-4-yl]-6-méthyl-imidazo[1,5-a]pyrazine-3-carboxamide ;
le 4-(6-carbamoyl-1-méthyl-pyrazolo[4,3-c]pyridin-4-yl)-2-(2-éthyl-4-hydroxy-5-méthyl-pyrazol-3-yl)oxazole-5-carboxamide ;
le 4-[3-(2-éthyl-5,6-dihydro-4*H*-cyclopenta[c]pyrazol-3-yl)-1*H*-1,2,4-triazol-5-yl]-1-méthyl-pyrazolo[4,3-c]pyridine-6-carboxamide ;
le 1-[3-[2-(3-hydroxypropyl)-5-méthyl-pyrazol-3-yl]-1*H*-1,2,4-triazol-5-yl]-6-méthyl-imidazo[1,5-a]pyrazine-3-carboxamide ;
le 1-[3-(2-éthyl-5-méthyl-pyrazol-3-yl)-1*H*-1,2,4-triazol-5-yl]-6-méthyl-imidazo[1,5-a]pyrazine-3-carboxamide ;
le 1-[3-(2-éthyl-4-fluoro-5-méthyl-pyrazol-3-yl)-1*H*-1,2,4-triazol-5-yl]-6-méthyl-imidazo[1,5-a]pyrazine-3-carboxamide ;
le 1-[3-(4-amino-2-éthyl-5-méthyl-pyrazol-3-yl)-1*H*-1,2,4-triazol-5-yl]-6-méthyl-imidazo[1,5-a]pyrazine-3-carboxamide ;
le 6-chloro-1-[3-(2-éthyl-4-hydroxy-5-méthyl-pyrazol-3-yl)-1*H*-1,2,4-triazol-5-yl]imidazo[1,5-a]pyrazine-3-carboxamide ;
le 1-[3-(2-éthyl-4-hydroxy-5-méthyl-pyrazol-3-yl)-1*H*-1,2,4-triazol-5-yl]-6-méthyl-imidazo[1,5-a]pyridine-3-carboxamide ;
le 7-[3-(2-éthyl-4-hydroxy-5-méthyl-pyrazol-3-yl)-1*H*-1,2,4-triazol-5-yl]-3-méthyl-pyrrolo[1,2-c]pyrimidine-5-carboxamide ;
le 1-[2-(2-éthyl-4-hydroxy-5-méthyl-pyrazol-3-yl)oxazol-4-yl]-5-méthyl-pyrazolo[3,4-c]pyridine-3-carboxamide ;
le 1-[3-(2-éthyl-4-hydroxy-5-méthyl-pyrazol-3-yl)-1*H*-1,2,4-triazol-5-yl]-5-méthyl-pyrazolo[3,4-c]pyridine-3-carboxamide ;
le 4-[3-[4-hydroxy-2-(2-imidazol-1-yléthyl)-5-méthyl-pyrazol-3-yl]-1*H*-1,2,4-triazol-5-yl]-1-méthyl-pyrazolo[4,3-c]pyridine-6-carboxamide ;
le 4-[3-[2-(3-cyanopropyl)-4-hydroxy-5-méthyl-pyrazol-3-yl]-1*H*-1,2,4-triazol-5-yl]-1-méthyl-pyrazolo[4,3-c]pyridine-6-carboxamide ;
le 4-[3-[2-(2-cyanoéthyl)-4-hydroxy-5-méthyl-pyrazol-3-yl]-1*H*-1,2,4-triazol-5-yl]-1-méthyl-pyrazolo[4,3-c]pyridine-6-carboxamide ;
le 6-(difluorométhyl)-1-[3-(2-éthyl-4-hydroxy-5-méthyl-pyrazol-3-yl)-1*H*-1,2,4-triazol-5-yl]imidazo[1,5-a]pyrazine-3-carboxamide ;
le 7-[3-(2-éthyl-4-hydroxy-5-méthyl-pyrazol-3-yl)-1,2,4-oxadiazol-5-yl]-3-méthyl-pyrrolo[1,2-c]pyrimidine-5-carboxamide ;
le 8-[2-(2-éthyl-4-hydroxy-5-méthyl-pyrazol-3-yl)oxazol-4-yl]-7-fluoro-3-méthyl-pyrrolo[1,2-a]pyrazine-6-carboxamide ;
le 1-[2-(2-éthyl-4-hydroxy-5-méthyl-pyrazol-3-yl)oxazol-4-yl]-5-méthyl-pyrrolo[2,3-c]pyridine-3-carboxamide ;
le 8-[3-[2-(2-cyanoéthyl)-4-hydroxy-5-méthyl-pyrazol-3-yl]-1*H*-1,2,4-triazol-5-yl]-7-fluoro-3-méthyl-pyrrolo[1,2-a]pyrazine-6-carboxamide ;
le 1-[3-(4-hydroxy-5-méthyl-2-propyl-pyrazol-3-yl)-1*H*-1,2,4-triazol-5-yl]-5-méthyl-pyrazolo[3,4-c]pyridine-3-carboxamide ;
le 1-[3-[2-(cyclopropylméthyl)-4-hydroxy-5-méthyl-pyrazol-3-yl]-1*H*-1,2,4-triazol-5-yl]-5-méthyl-pyrazolo[3,4-c]pyridine-3-carboxamide ;
le 1-[3-(2-cyclopropyl-4-hydroxy-5-méthyl-pyrazol-3-yl)-1*H*-1,2,4-triazol-5-yl]-5-méthyl-pyrazolo[3,4-c]pyridine-3-carboxamide ;
le 4-[3-[2-(3,3-difluoroallyl)-4-hydroxy-5-méthyl-pyrazol-3-yl]-1*H*-1,2,4-triazol-5-yl]-1-méthyl-pyrazolo[4,3-c]pyridine-6-carboxamide ;
le 4-[3-[4-hydroxy-5-méthyl-2-[2-(triazol-2-yl)éthyl]pyrazol-3-yl]-1*H*-1,2,4-triazol-5-yl]-1-méthyl-pyrazolo[4,3-c]pyridine-6-carboxamide ;
le 4-[3-[4-hydroxy-5-méthyl-2-[2-(triazol-1-yl)éthyl]pyrazol-3-yl]-1*H*-1,2,4-triazol-5-yl]-1-méthyl-pyrazolo[4,3-c]pyridine-6-carboxamide ;
le 4-[3-[2-(3,3-difluoropropyl)-4-hydroxy-5-méthyl-pyrazol-3-yl]-1*H*-1,2,4-triazol-5-yl]-1-méthyl-pyrazolo[4,3-c]pyridine-6-carboxamide ;
le 4-[3-[4-hydroxy-5-méthyl-2-[2-(1,2,4-triazol-4-yl)éthyl]pyrazol-3-yl]-1*H*-1,2,4-triazol-5-yl]-1-méthyl-pyrazolo[4,3-c]pyridine-6-carboxamide ;
le 4-[3-[4-hydroxy-5-méthyl-2-[2-(1,2,4-triazol-1-yl)éthyl]pyrazol-3-yl]-1*H*-1,2,4-triazol-5-yl]-1-méthyl-pyrazolo[4,3-c]pyridine-6-carboxamide ;
le 1-[3-[4-hydroxy-5-méthyl-2-(2-phényléthyl)pyrazol-3-yl]-1*H*-1,2,4-triazol-5-yl]-5-méthyl-pyrazolo[3,4-c]pyridine-3-carboxamide ;
le 1-[3-[2-(3-fluoropropyl)-4-hydroxy-5-méthyl-pyrazol-3-yl]-1*H*-1,2,4-triazol-5-yl]-5-méthyl-pyrazolo[3,4-c]pyridine-3-carboxamide ;
le 1-[3-[4-hydroxy-2-(2-méthoxyéthyl)-5-méthyl-pyrazol-3-yl]-1*H*-1,2,4-triazol-5-yl]-5-méthyl-pyrazolo[3,4-c]pyridine-3-carboxamide ;
le 1-[3-(2-butyl-4-hydroxy-5-méthyl-pyrazol-3-yl)-1*H*-1,2,4-triazol-5-yl]-5-méthyl-pyrazolo[3,4-c]pyridine-3-carboxamide ;
le 1-[3-[4-hydroxy-2-(3-méthoxypropyl)-5-méthyl-pyrazol-3-yl]-1*H*-1,2,4-triazol-5-yl]-5-méthyl-pyrazolo[3,4-c]pyridine-3-carboxamide ;
le 4-[3-(4-hydroxy-5-méthyl-2-pent-3-ynyl-pyrazol-3-yl)-1*H*-1,2,4-triazol-5-yl]-1-méthyl-pyrazolo[4,3-c]pyridine-6-carboxamide ;
le 1-[3-[4-hydroxy-5-méthyl-2-[2-(4-pyridyl)éthyl]pyrazol-3-yl]-1*H*-1,2,4-triazol-5-yl]-5-méthyl-pyrazolo[3,4-c]pyridine-3-carboxamide ;
le 1-[3-[4-hydroxy-5-méthyl-2-[2-(3-pyridyl)éthyl]pyrazol-3-yl]-1*H*-1,2,4-triazol-5-yl]-5-méthyl-pyrazolo[3,4-c]pyridine-3-carboxamide ;
le 1-[5-[4-hydroxy-5-méthyl-2-(2-phényléthyl)pyrazol-3-yl]-2-méthyl-1,2,4-triazol-3-yl]-5-méthyl-pyrazolo[3,4-c]pyridine-3-carboxamide ;
le 1-[5-(2-butyl-4-hydroxy-5-méthyl-pyrazol-3-yl)-2-méthyl-1,2,4-triazol-3-yl]-5-méthyl-pyrazolo[3,4-c]pyridine-3-carboxamide ;
le 1-[3-[4-hydroxy-2-[2-(4-méthoxyphényl)éthyl]-5-méthyl-pyrazol-3-yl]-1*H*-1,2,4-triazol-5-yl]-5-méthyl-pyrazolo[3,4-c]pyridine-3-carboxamide ;
le 1-[3-(4-hydroxy-5-méthyl-2-pent-3-ynyl-pyrazol-3-yl)-1*H*-1,2,4-triazol-5-yl]-5-méthyl-pyrazolo[3,4-c]pyridine-3-carboxamide ;
le 1-[3-[2-[2-(4-fluorophényl)éthyl]-4-hydroxy-5-méthyl-pyrazol-3-yl]-1*H*-1,2,4-triazol-5-yl]-5-méthyl-pyrazolo[3,4-c]pyridine-3-carboxamide ;
le 1-[3-[4-hydroxy-5-méthyl-2-(2-phényléthyl)pyrazol-3-yl]-1*H*-1,2,4-triazol-5-yl]-6-méthyl-imidazo[1,5-a]pyrazine-3-carboxamide ;
le 1-[3-[4-hydroxy-2-[2-(3-méthoxyphényl)éthyl]-5-méthyl-pyrazol-3-yl]-1*H*-1,2,4-triazol-5-yl]-5-méthyl-pyrazolo[3,4-c]pyridine-3-carboxamide ;
le 1-(3-(1-(3-fluorophénéthyl)-4-hydroxy-3-méthyl-1*H*-pyrazol-5-yl)-1*H*-1,2,4-triazol-5-yl)-5-méthyl-1*H*-pyrazolo[3,4-c]pyridine-3-carboxamide ;
le 1-[3-[2-[2-(2-fluorophényl)éthyl]-4-hydroxy-5-méthyl-pyrazol-3-yl]-1*H*-1,2,4-triazol-5-yl]-5-méthyl-pyrazolo[3,4-c]pyridine-3-carboxamide ;
le 7-fluoro-8-[3-[4-hydroxy-5-méthyl-2-(2-phényléthyl)pyrazol-3-yl]-1*H*-1,2,4-triazol-5-yl]-3-méthyl-pyrrolo[1,2-a]pyrazine-6-carboxamide ;
le 1-(2-(4-hydroxy-3-méthyl-1-propyl-1*H*-pyrazol-5-yl)oxazol-4-yl)-5-méthyl-1*H-*pyrazolo[3,4-c]pyridine-3-carboxamide ;
le 1-(2-(1-butyl-4-hydroxy-3-méthyl-1*H*-pyrazol-5-yl)oxazol-4-yl)-5-méthyl-1*H-*pyrazolo[3,4-c]pyridine-3-carboxamide ;
le 1-[3-[2-[2-(3-cyanophényl)éthyl]-4-hydroxy-5-méthyl-pyrazol-3-yl]-1*H*-1,2,4-triazol-5-yl]-5-méthyl-pyrazolo[3,4-c]pyridine-3-carboxamide ;
le 1-[3-[2-[2-(4-cyanophényl)éthyl]-4-hydroxy-5-méthyl-pyrazol-3-yl]-1*H*-1,2,4-triazol-5-yl]-5-méthyl-pyrazolo[3,4-c]pyridine-3-carboxamide ;
le 4-[2-[5-[5-(3-carbamoyl-5-méthyl-pyrazolo[3,4-c]pyridin-1-yl)-1*H*-1,2,4-triazol-3-yl]-4-hydroxy-3-méthyl-pyrazol-1-yl]éthyl]benzoate de méthyle ;
le 1-(3-(1-(3,3-difluoroallyl)-4-hydroxy-3-méthyl-1*H*-pyrazol-5-yl)-1*H*-1,2,4-triazol-5-yl)-5-méthyl-1*H*-pyrazolo[3,4-c]pyridine-3-carboxamide ;
le 1-[3-[4-hydroxy-2-[2-[4-(méthoxyméthyl)phényl]éthyl]-5-méthyl-pyrazol-3-yl]-1*H-*1,2,4-triazol-5-yl]-5-méthyl-pyrazolo[3,4-c]pyridine-3-carboxamide ;
le 1-[3-[4-hydroxy-2-[2-[3-(méthoxyméthyl)phényl]éthyl]-5-méthyl-pyrazol-3-yl]-1*H-*1,2,4-triazol-5-yl]-5-méthyl-pyrazolo[3,4-c]pyridine-3-carboxamide ;
le 1-(3-(1-(3,3-difluoropropyl)-4-hydroxy-3-méthyl-1*H*-pyrazol-5-yl)-1*H*-1,2,4-triazol-5-yl)-5-méthyl-1*H*-pyrazolo[3,4-c]pyridine-3-carboxamide ;
le 1-(2-(1-(3,3-difluoropropyl)-4-hydroxy-3-méthyl-1*H*-pyrazol-5-yl)oxazol-4-yl)-5-méthyl-1*H*-pyrazolo[3,4-c]pyridine-3-carboxamide ;
le 1-[3-[4-hydroxy-2-[2-[4-(hydroxyméthyl)phényl]éthyl]-5-méthyl-pyrazol-3-yl]-1*H-*1,2,4-triazol-5-yl]-5-méthyl-pyrazolo[3,4-c]pyridine-3-carboxamide ;
le 7-fluoro-8-[3-[4-hydroxy-2-[2-(4-méthoxyphényl)éthyl]-5-méthyl-pyrazol-3-yl]-1*H-*1,2,4-triazol-5-yl]-3-méthyl-pyrrolo[1,2-a]pyrazine-6-carboxamide ;
le 1-[3-[4-hydroxy-2-[2-(4-méthoxyphényl)éthyl]-5-méthyl-pyrazol-3-yl]-1*H*-1,2,4-triazol-5-yl]-6-méthyl-imidazo[1,5-a]pyrazine-3-carboxamide ;
le 1-[3-[4-hydroxy-2-[2-[4-(hydroxyméthyl)phényl]éthyl]-5-méthyl-pyrazol-3-yl]-1*H-*1,2,4-triazol-5-yl]-6-méthyl-imidazo[1,5-a]pyrazine-3-carboxamide ;
le 1-[3-[4-hydroxy-5-méthyl-2-[2-(3-pyridyl)éthyl]pyrazol-3-yl]-1*H*-1,2,4-triazol-5-yl]-6-méthyl-imidazo[1,5-a]pyrazine-3-carboxamide ;
le 7-fluoro-8-[3-[4-hydroxy-5-méthyl-2-[2-(3-pyridyl)éthyl]pyrazol-3-yl]-1*H*-1,2,4-triazol-5-yl]-3-méthyl-pyrrolo[1,2-a]pyrazine-6-carboxamide ;
le 1-(2-(4-hydroxy-3-méthyl-1-(2-(pyridin-3-yl)éthyl)-1*H*-pyrazol-5-yl)oxazol-4-yl)-5-méthyl-1*H*-pyrazolo[3,4-c]pyridine-3-carboxamide ;
le 1-(2-(4-hydroxy-1-(3-méthoxyphénéthyl)-3-méthyl-1*H*-pyrazol-5-yl)oxazol-4-yl)-5-méthyl-1*H*-pyrazolo[3,4-c]pyridine-3-carboxamide ;
le 8-[3-(2-butyl-4-hydroxy-5-méthyl-pyrazol-3-yl)-1*H*-1,2,4-triazol-5-yl]-7-fluoro-3-méthyl-pyrrolo[1,2-a]pyrazine-6-carboxamide ;
le 1-[3-(2-butyl-4-hydroxy-5-méthyl-pyrazol-3-yl)-1*H*-1,2,4-triazol-5-yl]-6-méthyl-imidazo[1,5-a]pyrazine-3-carboxamide ;
le 1-(2-(4-hydroxy-1-(4-méthoxyphénéthyl)-3-méthyl-1*H*-pyrazol-5-yl)oxazol-4-yl)-5-méthyl-1*H*-pyrazolo[3,4-c]pyridine-3-carboxamide ;
le 7-fluoro-8-[3-(4-hydroxy-5-méthyl-2-propyl-pyrazol-3-yl)-1*H*-1,2,4-triazol-5-yl]-3-méthyl-pyrrolo[1,2-a]pyrazine-6-carboxamide ;
le 1-[3-(4-hydroxy-5-méthyl-2-propyl-pyrazol-3-yl)-1*H*-1,2,4-triazol-5-yl]-6-méthyl-imidazo[1,5-a]pyrazine-3-carboxamide ;
le 1-[3-[4-hydroxy-2-(5-méthoxypent-3-ynyl)-5-méthyl-pyrazol-3-yl]-1*H*-1,2,4-triazol-5-yl]-5-méthyl-pyrazolo[3,4-c]pyridine-3-carboxamide ;
le 4-(5-(4-hydroxy-3-méthyl-1-phénéthyl-1*H*-pyrazol-5-yl)-1*H*-1,2,4-triazol-3-yl)-1-méthyl-1*H*-pyrazolo[4,3-c]pyridine-6-carboxamide ;
le 1-[5-(hydroxyméthyl)-2-(4-hydroxy-5-méthyl-2-propyl-pyrazol-3-yl)oxazol-4-yl]-5-méthyl-pyrazolo[3,4-c]pyridine-3-carboxamide ;
le 1-[5-(4-hydroxy-5-méthyl-2-propyl-pyrazol-3-yl)-2-méthyl-1,2,4-triazol-3-yl]-5-méthyl-pyrazolo[3,4-c]pyridine-3-carboxamide ;
le 1-[2-(4-hydroxy-5-méthyl-2-propyl-pyrazol-3-yl)oxazol-4-yl]-6-méthyl-imidazo[1,5-a]pyrazine-3-carboxamide ;
le 1-[2-(2-butyl-4-hydroxy-5-méthyl-pyrazol-3-yl)oxazol-4-yl]-6-méthyl-imidazo[1,5-a]pyrazine-3-carboxamide ;
le 1-[3-[4-hydroxy-5-méthyl-2-[2-[4-(morpholinométhyl)phényl]éthyl]pyrazol-3-yl]-1*H*-1,2,4-triazol-5-yl]-6-méthyl-imidazo[1,5-a]pyrazine-3-carboxamide ;
le 1-[2-[4-hydroxy-5-méthyl-2-[2-[4-(morpholinométhyl)phényl]éthyl]pyrazol-3-yl]oxazol-4-yl]-5-méthyl-pyrazolo[3,4-c]pyridine-3-carboxamide ;
le 1-[2-[4-hydroxy-2-[2-[4-(méthoxyméthyl)phényl]éthyl]-5-méthyl-pyrazol-3-yl]oxazol-4-yl]-5-méthyl-pyrazolo[3,4-c]pyridine-3-carboxamide ;
le 1-[3-[4-hydroxy-5-méthyl-2-(2-thiazol-2-yléthyl)pyrazol-3-yl]-1*H*-1,2,4-triazol-5-yl]-6-méthyl-imidazo[1,5-a]pyrazine-3-carboxamide ;
le 8-[3-(4-hydroxy-5-méthyl-2-propyl-pyrazol-3-yl)-1*H*-1,2,4-triazol-5-yl]-3-méthyl-pyrrolo[1,2-a]pyrazine-6-carboxamide ;
le 1-[5-(4-hydroxy-5-méthyl-2-propyl-pyrazol-3-yl)-2-méthyl-1,2,4-triazol-3-yl]-6-méthyl-imidazo[1,5-a]pyrazine-3-carboxamide ;
le 7-fluoro-8-[2-(4-hydroxy-5-méthyl-2-propyl-pyrazol-3-yl)oxazol-4-yl]-3-méthyl-pyrrolo[1,2-a]pyrazine-6-carboxamide ;
le 8-[2-(4-hydroxy-5-méthyl-2-propyl-pyrazol-3-yl)oxazol-4-yl]-3-méthyl-pyrrolo[1,2-a]pyrazine-6-carboxamide ;
ou un sel pharmaceutiquement acceptable de ceux-ci.

13. Composition pharmaceutique comprenant un composé ou un sel pharmaceutiquement acceptable de celui-ci selon l'une quelconque des revendications 1 à 12 et un excipient pharmaceutiquement acceptable.

14. Composé ou sel pharmaceutiquement acceptable de celui-ci selon l'une quelconque des revendications 1 à 12 pour une utilisation comme substance thérapeutiquement active.

15. Composé ou sel pharmaceutiquement acceptable de celui-ci selon l'une quelconque des revendications 1 à 12 pour une utilisation dans le traitement du cancer.
